# EUROPEAN PATENT SPECIFICATION

(11) **EP 3 752 644 B1**
(45) Date of publication and mention of the grant of the patent: **16.07.2025**
(21) Application number: 19705173.3
(22) Date of filing: 13.02.2019
(51) Int. Cl.: C12Q 1/6886

(54) **TUMOR MINIMAL RESIDUAL DISEASE STRATIFICATION**
STRATIFIZIERUNG VON MINIMALER RESTERKRANKUNG BEI TUMOR
STRATIFICATION DES PATHOLOGIES TUMORALES RÉSIDUELLES MINIMALES

(30) Priority: 13.02.2018 GB 201802312
(43) Date of publication of application: 23.12.2020
(73) Proprietor: VIB VZW, 9052 Gent (BE); Katholieke Universiteit Leuven, K.U.Leuven R&D, 3000 Leuven (BE)
(72) Inventor: MARINE, Jean-Christophe, 3210 Linden (BE); RAMBOW, Florian, 3000 Leuven (BE)
(86) International application number: PCT/EP2019/053549
(87) International publication number: WO 2019/158586

(56) References cited:
- US-A- 5 543 296
- FLORENCIA PAULA MADORSKY ROWDO ET AL: "In vitro long-term treatment with MAPK inhibitors induces melanoma cells with resistance plasticity to inhibitors while retaining sensitivity to CD8 T cells", ONCOLOGY REPORTS, vol. 37, no. 3, 1 March 2017 (2017-03-01), pages 1367 - 1378, XP055586435, ISSN: 1021-335X, DOI: 10.3892/or.2017.5363
- MARIA CRISTINA RAPANOTTI ET AL: "Minimal residual disease in melanoma: circulating melanoma cells and predictive role of MCAM/MUC18/MelCAM/CD146", CELL DEATH DISCOVERY, vol. 3, 6 March 2017 (2017-03-06), pages 17005, XP055584844, DOI: 10.1038/cddiscovery.2017.5
- A ROGIERS ET AL: "Portraying drug-tolerant state dynamics and heterogeneity by single-cell RNAseq", PIGMENT CELL MELANOMA RESEARCH: SMR CONGRESS 2017 ABSTRACTS, vol. 31, no. 1, 22 December 2017 (2017-12-22), https://onlinelibrary.wiley.com/doi/full/10.1111/pcmr.12656, pages 199, XP055586514
- FLORIAN RAMBOW ET AL: "Toward Minimal Residual Disease-Directed Therapy in Melanoma", CELL, vol. 174, no. 4, 1 August 2018 (2018-08-01), AMSTERDAM, NL, pages 843 - 855.e19, XP055575360, ISSN: 0092-8674, DOI: 10.1016/j.cell.2018.06.025
- JUDITH VANHORNE ET AL: "Cloning and characterization of the human GFRA2 locus and investigation of the gene in Hirschsprung disease", HUMAN GENETICS, vol. 108, no. 5, 22 May 2001 (2001-05-22), BERLIN, DE, pages 409 - 415, XP055603418, ISSN: 0340-6717, DOI: 10.1007/s004390100506

## Description

### FIELD OF THE INVENTION

The invention relates to the field of tumor disease stratification, in particular melanoma disease stratification. In particular it relates to the methods for tumor analysis, such as for determining tumor cell heterogeneity during treatment. These methods are helpful in selecting or optimizing tumor therapy, or in predicting responses to tumor therapy. The invention further relates to methods for screening for cytotoxic or cytostatic compounds targeting one or more of the heterogeneous tumor cell populations occurring such as during therapy, such as during the minimal residual disease phase.

### BACKGROUND

A major obstacle to successful targeted therapy is the acquisition by cancer cells of a plethora of resistance-conferring genetic alterations that greatly attenuate or suppress drug response. Different mutational events can be selected in distinct drug-resistant clones from the same patient (Kemper et al. 2015, EMBO Mol Med 7:1104-1118) and even co-occur within the same lesion, thus creating genetic intra-tumor heterogeneity (Burrell et al. 2013, Nature 501:338-345). These findings have highlighted the need to improve effectiveness of treatment before mutational acquired resistance prevails. Recent *cell culture* findings indicate that acquired mutational resistance to cancer drugs may follow a transient and reversible "drug-tolerant" phase in which a small subpopulation of drug-tolerant cells remain viable whereas the vast majority of the cell population is rapidly killed (Sharma et al. 2010, Cell 141:69-80). Importantly, the emergence of these "drug-tolerant persisters" (DTPs) or "induced drug-tolerant" cells (IDTCs; (Menon et al. 2015, Oncogene 34:4545)) is observed at a frequency much higher than would be expected due to mutational mechanisms. Drug tolerance is therefore thought to be caused by the selection of a small subpopulation of cancer cells, that are intrinsically refractory to the effects of anticancer drugs possibly via enhanced drug efflux (Roesch et al. 2013, Cancer Cell 23:811-825; Trumpp & Wiestler 2008, Oncology 5:337-347). Yet another, non-mutually exclusive scenario proposes that the drug-tolerant phenotype is transiently acquired by a small proportion of cancer cells, through non-mutational mechanisms such as epigenetic and/or transcriptome reprogramming (Menon et al. 2015, Oncogene 34:4545; Shaffer et al. 2017, Nature 546:431-435; Sharma et al. 2010, Cell 141:69-80). The latter model is consistent with accumulating clinical evidence that cancer patients treated with a variety of anticancer drugs can be successfully re-treated with the same drug after a "drug holiday", i.e., treated with the same drug but with intermittent drug-free periods.

Identification and characterization of the drug-tolerant subpopulation may allow their selective ablation before more permanent/stable resistance mechanisms are established (Sharma et al. 2010, Cell 141:69-80). In keeping with this possibility, upregulation of the melanoma survival oncogene MITF was shown to drive an early non-mutational and reversible drug tolerance state in cultured melanoma cell lines exposed to a BRAF-inhibitor and pharmacological suppression of MITF expression by nelfinavir sensitized melanoma cells to MAPK-pathway inhibition (Smith et al. 2016, Cancer Cell 29:270-284). This observation is consistent with previous reports indicating that MITF can indeed provide resistance to MAPK-inhibition through various mechanisms and that enhanced MITF expression is linked to innate/intrinsic resistance (Gopal et al. 2014, Cancer Res 74:7037-7047; Haq et al. 2013, Cancer Cell 23:302-315; Haq et al. 2013, Proc Natl Acad Sci USA 110:4321-4326; Ji et al. 2015, J Invest Dermatol 135:1863-1872; Johannessen et al. 2013, Nature 504:138-142; Muller et al. 2014, Nature Comm 5:5712; Smith et al. 2013, J Natl Cancer Inst 105:33-46; Van Allen et al. 2014, Cancer Discovery 4:94-109; Wellbrock and Arozarena 2015, Pigment Cell Melanoma Res 28:390-406). Importantly, it has been proposed that the initial response phase to MAPK inhibitor treatment is uniform, while the BRAF-driven signaling network readjusts and melanoma cells quickly adapt to the new input (Lito et al. 2012, Cancer Cell 22:668-682; Smith et al. 2016, Cancer Cell 29:270-284; von Kriegsheim et al. 2009, Nature Cell Biol 11:1458-1464).

In apparent contrast with these findings, melanoma cells with an invasive gene expression signature, characterized by low levels of expression of both MITF and SOX10 and high levels of AXL and EGFR, exhibit increased intrinsic resistance to MAPK-inhibition (Kemper et al. 2014, EMBO Mol Med 7:1104-1118; Shaffer et al. 2017, Nature 546:431-435; Titz et al. 2016, Cell Discov 2:16028; Verfaillie et al. 2015, Nature Commun 6:6683). It has therefore been suggested that drug-induced phenotype switching from a proliferative to an invasive cell state may be an alternative route towards drug tolerance and/or resistance. Whether these distinct MITFhigh and/or MITFlow transcriptional cell states contribute to drug tolerance *in vivo,* and if so whether they occur within different tumors or within the same lesion is unknown. This is a critical issue, one that has important clinical implications. Indeed, if the response to MAPK-inhibition is uniform, as proposed based on bulk sequencing analyses, then targeting the driver of this newly established drug-tolerant state (i.e. MITF high) should significantly prolong response and delay or even prevent the occurrence of genetically acquired resistance. In contrast, if different subpopulations of drug-tolerant cells can emerge within the same lesion, probing the magnitude of cellular heterogeneity and understanding the molecular mechanisms underlying the selection of drug-tolerant subpopulations will be essential for developing rational therapies that prevent the occurrence of acquired resistance.

Using single-cell RNA-sequencing, Tirosh et al. 2016 (Science 352:189-196) revealed the co-existence of MITFhigh and AXL-high populations within the same melanoma lesion/tumor, with a shift to AXL-high and MET-high upon treatment with MAPK-pathway inhibitors. The authors catalogued AXL and NGFR in the same transcriptional program that is negatively correlated with the MITF-high program.

A metabolic gene expression signature (*CAV1, CD36, MLXIPL, CPT1C, CYP2E1*) associated with the epithelial-mesenchymal program across multiple cancers was established (Nath and Chan 2016, Sci Rep 6:18669). Inhibition of one of the gene products, CD36, was demonstrated to target metastasis of oral squamous cell carcinoma (Pascual et al. 2017, Nature 541:41-45; WO 2017/055411).

A neural stem cell-like population of melanoma cells was reported by Rogiers et al. 2018 (Pigment cell melanoma research 31(1), SMR Congress 2017 abstracts page 199; published online 22 December 2017).

### SUMMARY OF THE INVENTION

In particular, the invention relates to methods of analysis of a human tumor, such methods comprising detecting in a biological sample from the human tumor or in a biological sample comprising human tumor nucleic acids an increased expression level:
- of 4 to 8 genes selected from gene signature A1 consisting of the genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; and wherein detection of an increased expression level of the selected genes is indicating the emergence or presence of neural drug tolerant tumor cells (NDTCs); and
- of 4 to 8 genes selected from gene signature B1 consisting of the genes SLC7A8, DLX5, TRIM67, CD36, PAX3, IP6K3, UBXN10, KIAA1161, LSMEM1; and wherein detection of an increased expression level of the selected genes is indicating the emergence or presence of hypometabolic tumor cells (HMTCs); and
- of 4 to 8 genes selected from gene signature C1 consisting of the genes SLC24A5, PMEL, FABP7, SLC45A2, KIT, EDNRB, TRPM1, APOE, MLANA, MLPH, TYRP1, GPR143, TYR, RAB27A, SNAI2, MITF, DCT; and wherein detection of an increased expression level of the selected genes is indicating the emergence or presence of pigmented tumor cells; and
- of 4 to 8 genes selected from gene signature D1 consisting of VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4; and wherein detection of an increased expression level of the selected genes is indicating the emergence or presence of invasive tumor cells; and
wherein increased expression level of a selected gene is determined compared to a reference expression level of that selected gene.

Such methods in particular are for use in selecting therapy or in optimizing therapy for a patient having a tumor, or for use in predicting the response of a tumor to therapy. In one embodiment, such methods are further comprising the step of selecting therapy for a patient having a tumor wherein the selected therapy is normalizing the expression levels of a selected gene detected to be increased in the biological sample compared to the reference expression level of the selected gene.

Such therapy is in one embodiment chosen from:
- a compound that is cytotoxic or cytostatic for the population of cells in the tumor with, compared to reference expression levels, increased expression levels of the 4 to 8 genes selected from gene signature A1; and/or
- a compound that is cytotoxic or cytostatic for the population of cells in the tumor with, compared to reference expression levels, increased expression levels of the 4 to 8 genes selected from gene signature B1; and/or
- a compound that is cytotoxic or cytostatic for the population of cells in the tumor with, compared to reference expression levels, increased expression levels of the 4 to 8 genes selected from gene signature C1; and/or
- a compound that is cytotoxic or cytostatic for the cells in the tumor with, compared to reference expression levels, increased expression levels of the 4 to 8 genes selected from gene signature D1.

Such compound can be selected from:
- a compound that is cytotoxic or cytostatic for the population of cells in the tumor with, compared to reference expression levels, increased expression levels of the 4 to 8 genes selected from gene signature A1, is an antagonist of retinoid X receptor gamma (RXRG), an antagonist of RXRG combined with a FAK-inhibitor, a CD36 antagonist, or a CD36 antagonist combined with an antifolate drug, a melanocyte-directed enzyme prodrug, an antibody drug conjugate wherein the antibody is targeting GPNMB, or nelfinavir;
- a compound that is cytotoxic or cytostatic for the population of cells in the tumor with, compared to reference expression levels, increased expression levels of the 4 to 8 genes selected from gene signature B1, is a CD36 antagonist, an inhibitor of PAX3, or a CD36 antagonist combined with an antifolate drug, a melanocyte-directed enzyme prodrug, an antibody drug conjugate wherein the antibody is targeting GPNMB, or nelfinavir;
- a compound that is cytotoxic or cytostatic for the population of cells in the tumor with, compared to reference expression levels, increased expression levels of the 4 to 8 genes selected from gene signature C1, is an antifolate drug, a melanocyte-directed enzyme prodrug, an antibody drug conjugate wherein the antibody is targeting GPNMB, or nelfinavir;
- a compound that is cytotoxic or cytostatic for the cells in the tumor with, compared to reference expression levels, increased expression levels of the 4 to 8 genes selected from gene signature D1, is an inhibitor of AXL.

In one embodiment to any one of these methods, the tumor is melanoma.

In a further embodiment to any of these methods, the expression level is an mRNA expression level or a protein expression level. In particular, the mRNA expression level is determined by RNA-sequencing, PCR, RT-PCR, gene expression profiling, serial analysis of gene expression, microarray analysis, whole genome sequencing, or is determined based on at least one of an amplification reaction, a sequencing reaction, a melting reaction, a hybridization reaction or a reverse hybridization reaction.

In a further embodiment to any of the above methods, the expression level of at most 250 genes is determined.

The invention further relates to methods for screening for cytotoxic or cytostatic compounds, such methods comprising:
- culturing tumor cells;
- applying a therapy to the cultured tumor cells, wherein the therapy induces the occurrence of one or more populations of tumor cells that are reversibly resistant to the therapy, and wherein the populations are one or more of:
   - a population with increased expression of 4 to 8 genes selected from gene signature A1 consisting of the genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; and/or
   - a population with increased expression of 4 to 8 genes selected from gene signature B1 consisting of the genes SLC7A8, DLX5, TRIM67, CD36, PAX3, IP6K3, UBXN10, KIAA1161, LSMEM1; and/or
   - a population with increased expression of 4 to 8 genes selected from gene signature C1 consisting of the genes SLC24A5, PMEL, FABP7, SLC45A2, KIT, EDNRB, TRPM1, APOE, MLANA, MLPH, TYRP1, GPR143, TYR, RAB27A, SNAI2, MITF, DCT; and/or
   - a population with increased expression of 4 to 8 genes selected from gene signature D1 consisting of VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4;
      wherein the increased expression of a selected gene is determined compared to a reference expression level of the selected gene;
- contacting the one or more populations of tumor cells that are reversibly resistant to the therapy with a compound that is a candidate compound cytotoxic or cytostatic to one or more of the populations of tumor cells that are reversibly resistant to the therapy;
- identifying a compound cytotoxic or cytostatic to one or more of the populations of tumor cells that are reversibly resistant to the therapy.

The invention further relates to methods for screening for cytotoxic or cytostatic compounds, the method comprising:
- culturing tumor cells;
- applying a therapy to the cultured tumor cells, wherein the therapy induces the occurrence of one or more populations of tumor cells that are reversibly resistant to the therapy, and wherein the populations are one or more of:
   - a population with increased expression of 4 to 8 genes selected from gene signature A1 consisting of the genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; and/or
   - a population with increased expression of 4 to 8 genes selected from gene signature B1 consisting of the genes SLC7A8, DLX5, TRIM67, CD36, PAX3, IP6K3, UBXN10, KIAA1161, LSMEM1; and/or
   - a population with increased expression of 4 to 8 genes selected from gene signature C1 consisting of the genes SLC24A5, PMEL, FABP7, SLC45A2, KIT, EDNRB, TRPM1, APOE, MLANA, MLPH, TYRP1, GPR143, TYR, RAB27A, SNAI2, MITF, DCT; and/or
   - a population with increased expression of 4 to 8 genes selected from gene signature D1 consisting of VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4;
      wherein the increased expression of a selected gene is determined compared to a reference expression level of the selected gene;
- contacting the one or more populations of tumor cells that are reversibly resistant to the therapy with a compound that is a candidate compound for modifying expression or function of a gene selected from any of gene signatures A1, B1, C1, or D1;
- identifying as cytotoxic or cytostatic compound a compound that is modifying expression or function a gene selected from any of gene signatures A1, B1, C1, or D1.

### DESCRIPTION TO THE FIGURES

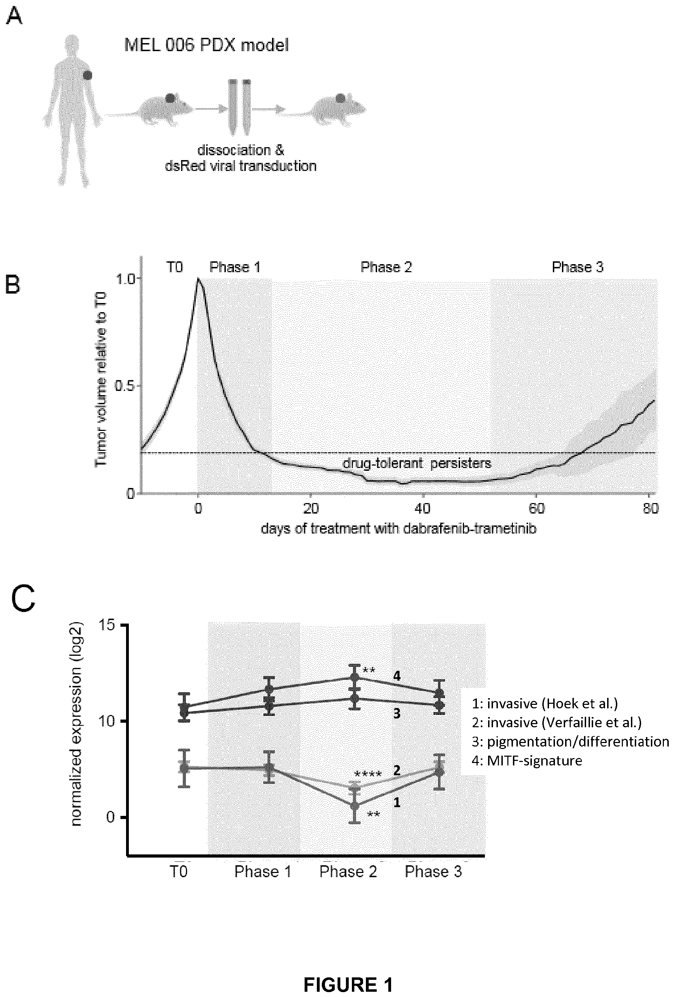

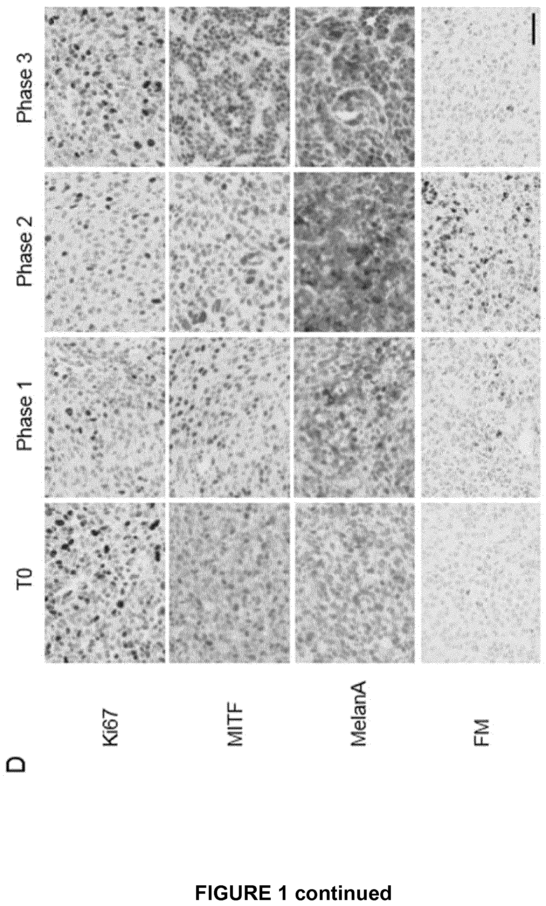

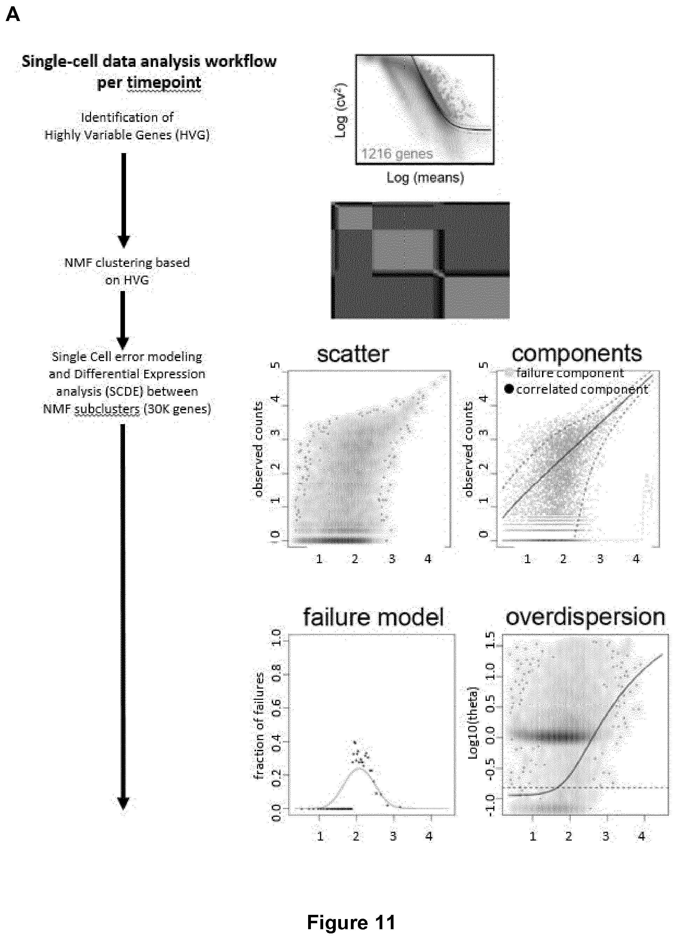

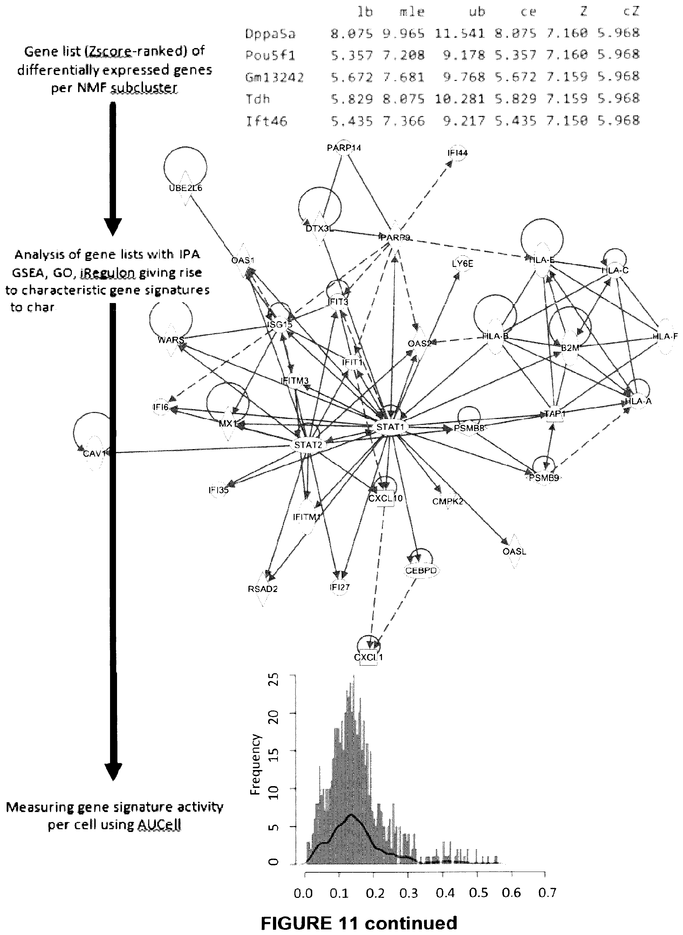

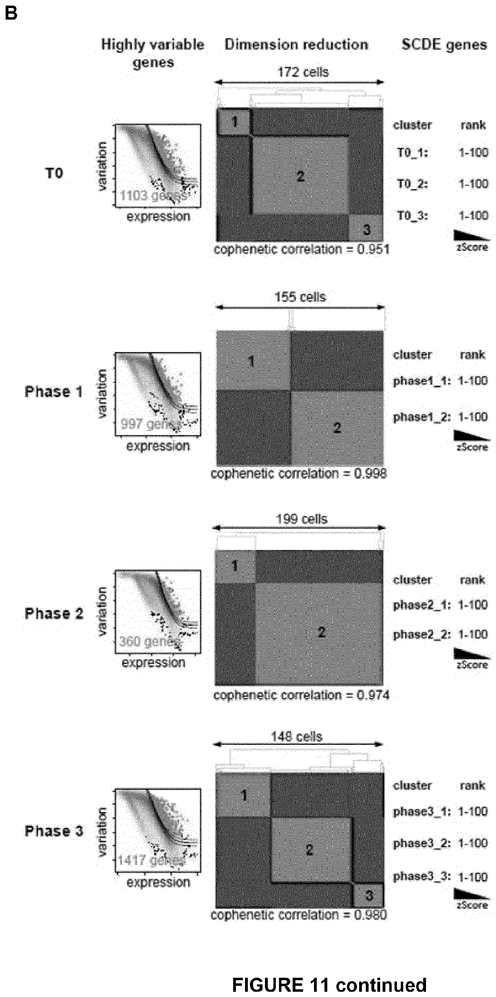

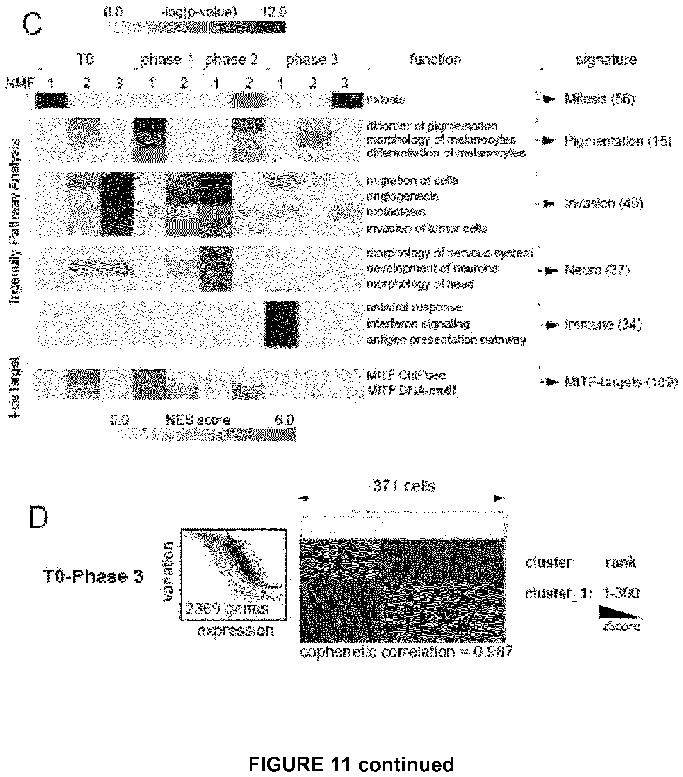

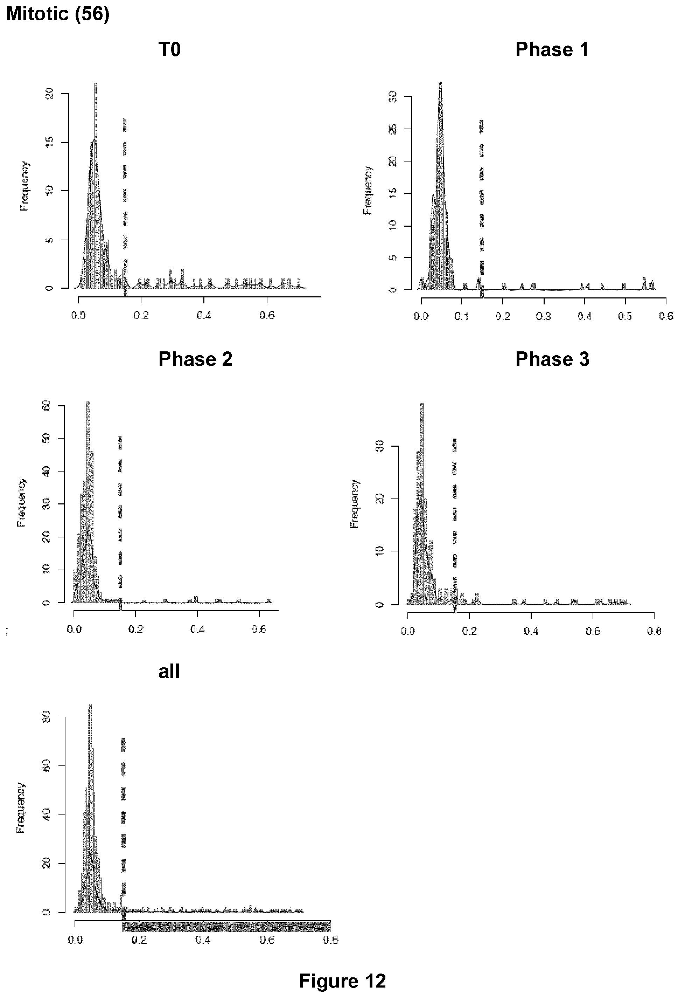

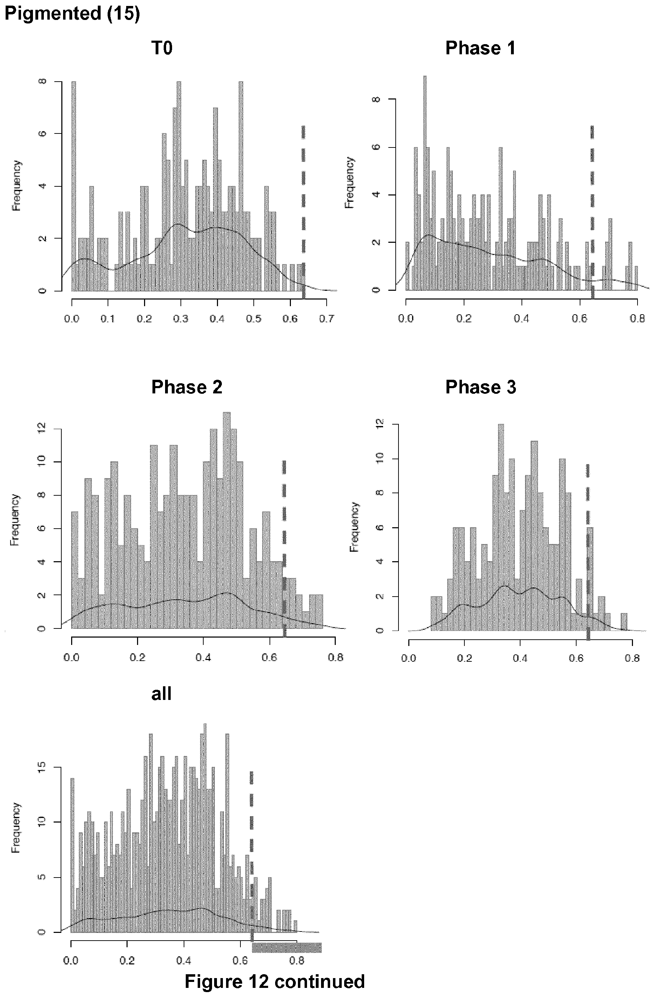

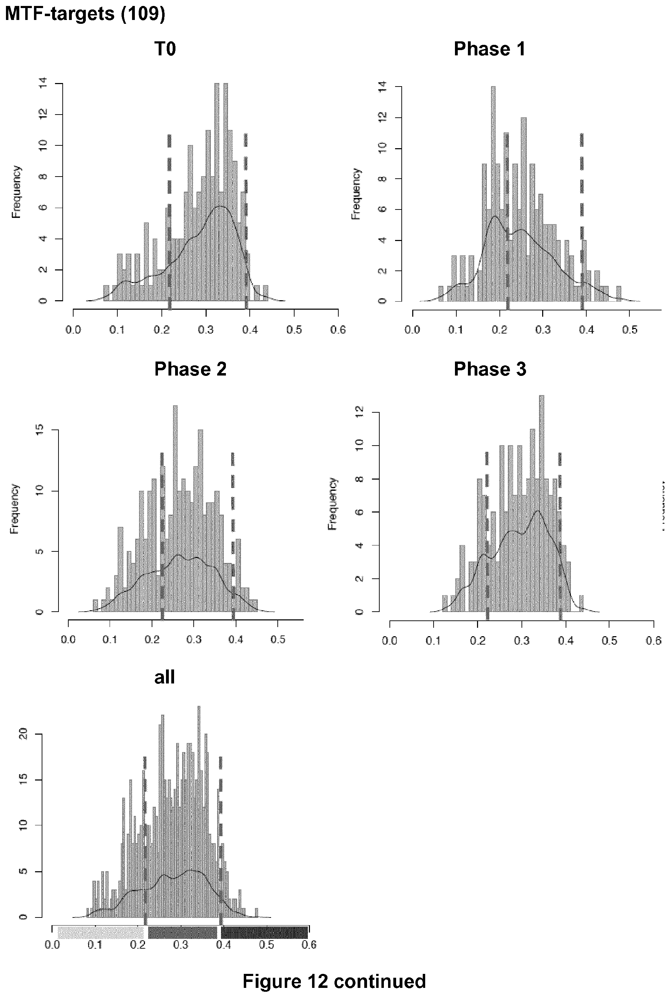

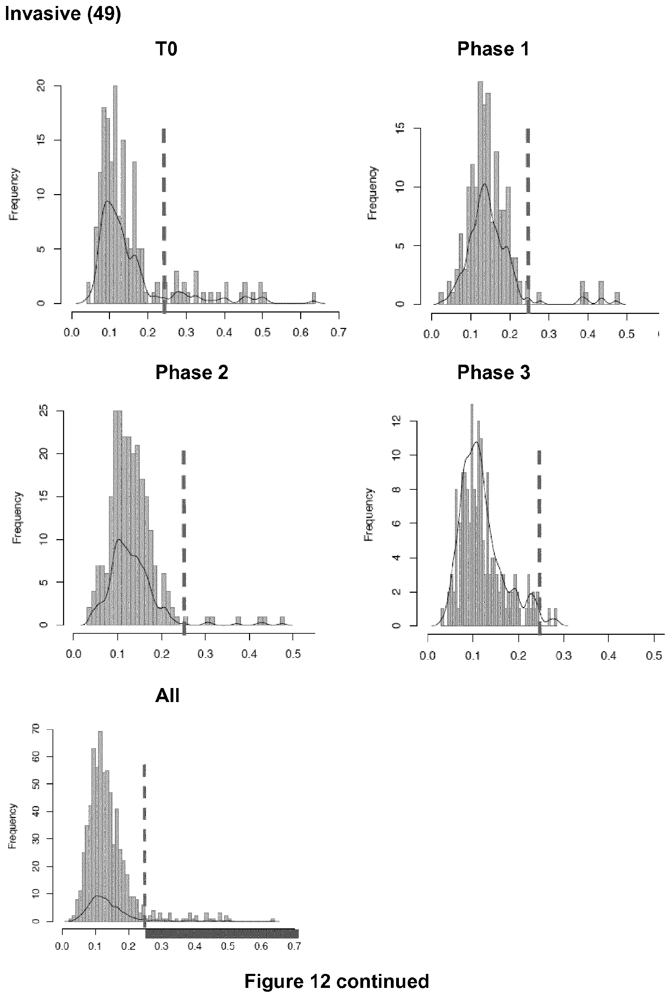

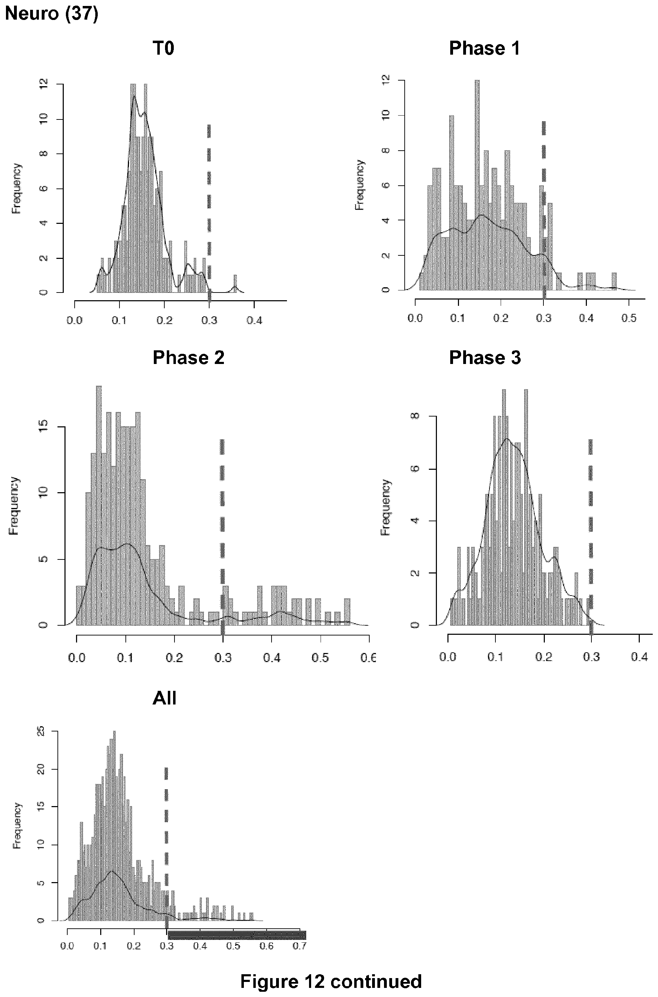

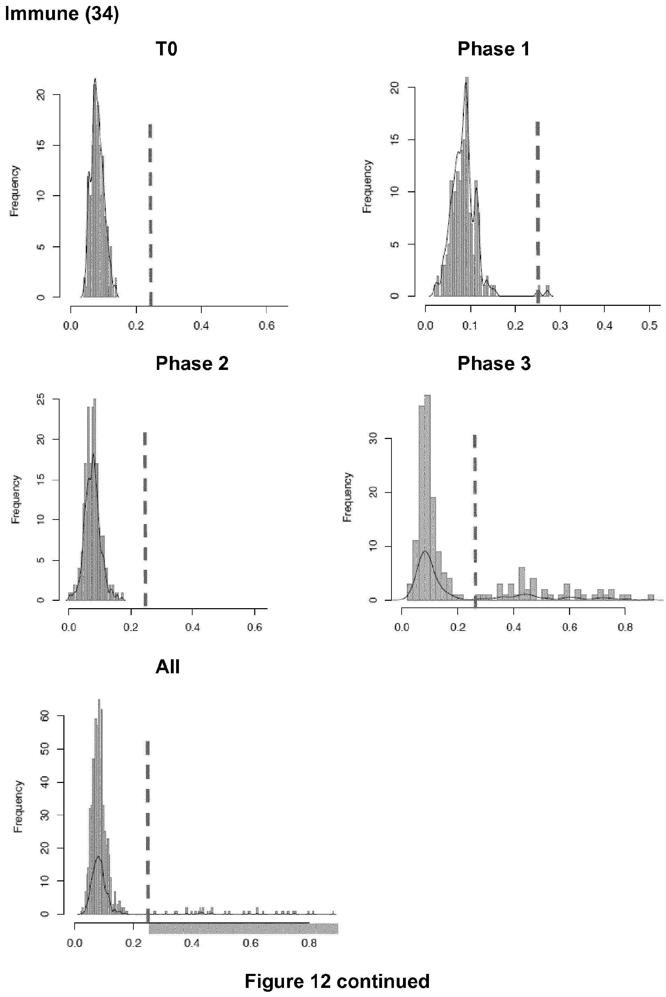

**FIGURE 1****. MAPK-inhibition induces MITF high and low drug-tolerant cells**
   A) Schematic diagram demonstrating the establishment of dsRed-expressing melanoma patient-derived xenograft model MEL006.
   B) Tumor volumes relative to baseline (T0) upon treatment with BRAF-MEK combination dabrafenib (30 mg/kg) and trametinib (0.3 mg/kg) via daily oral gavage; n = 29, standard error bands.
   C) Dynamics of known gene expression signatures during BRAF&MEK inhibition based on bulk RNA sequencing.
   D) Immunohistochemistry staining of MEL006 sections showing decreased Ki67 levels during phases 1 and 2, increased MITF expression heterogeneity and increased MelanA expression and pigmentation in a selected subset of melanoma cells at phase 2. Scale bar 50 µm. FM: Fontana-Masson silver method.

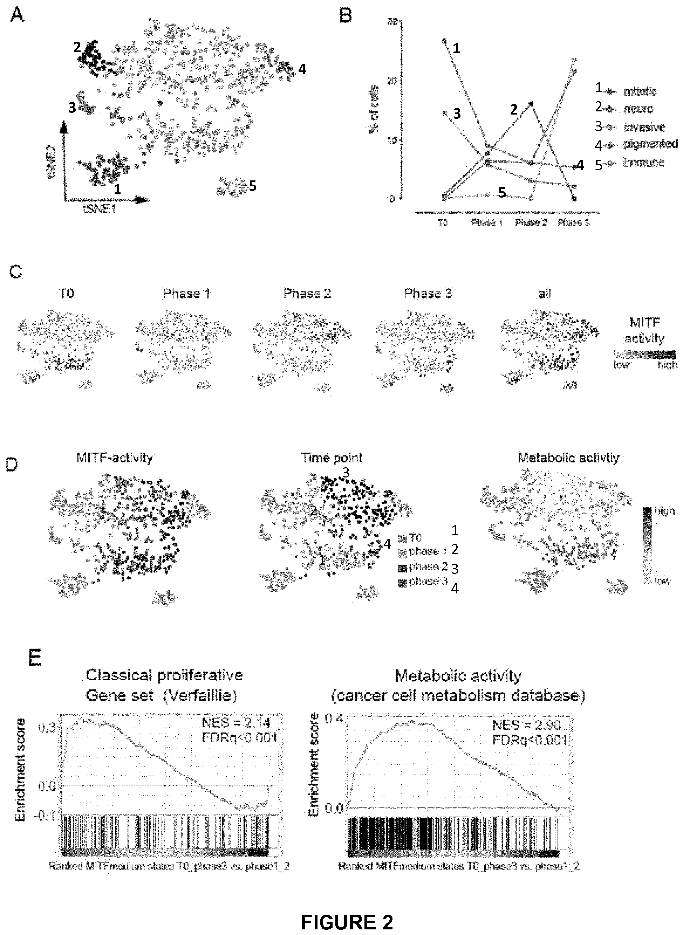

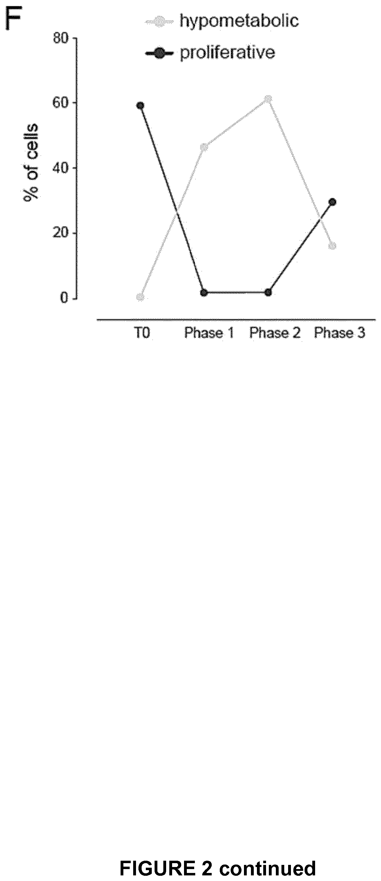

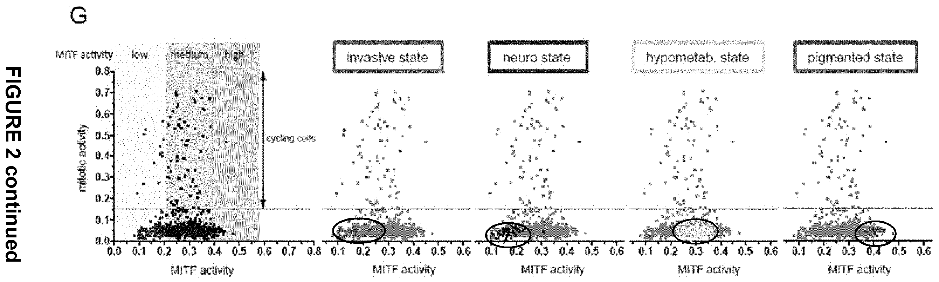

**FIGURE 2****. Single cell RNA-seq identifies multiple drug-tolerant transcriptional cell states**
   A) Single-cell transcriptomics allow the identification of different cell states. Shown is the projection of 674 cells in a two-dimensional space by tSNE. The cell state identity was inferred by enrichment analysis (Figure 11). Cells in a higher state were colored using the AUCell measure (Figure 12).
   B) Dynamics of the different cellular states show coexisting drug tolerant states at Phase 2.
   C) Shown are different tSNE plots of the different treatment phases color coded based on the expressional activity of either the MITF state.
   D) Two distinct MITF states of medium expressional activity are distinguishable. They differ by time and metabolic activity. MITF-medium cells of T0 and phase 3 are metabolically more active compared to phase 1 and 2 cells.
   E) MITF medium cells of T0 and phase 3 show significant enrichment for the classical proliferative and metabolically active signature compared to phase1&2 MITF medium cells, as shown by gene set enrichment analysis.
   F) Quantification of classical proliferative and hypometabolic cells based on their single cell expression profiles shows enrichment of hypometabolic MITF medium cells during tolerance (phase 2).
   G) Enriched drug-tolerant cells of different states were projected into a mitotic and MITF-activity space. Cells of the neuro and hypometabolic state do not cross the mitotic threshold inferred by AUCell, suggesting that these states are composed of dormant cells.

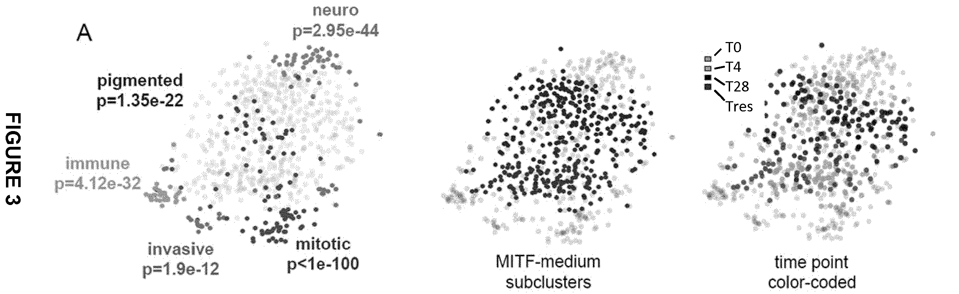

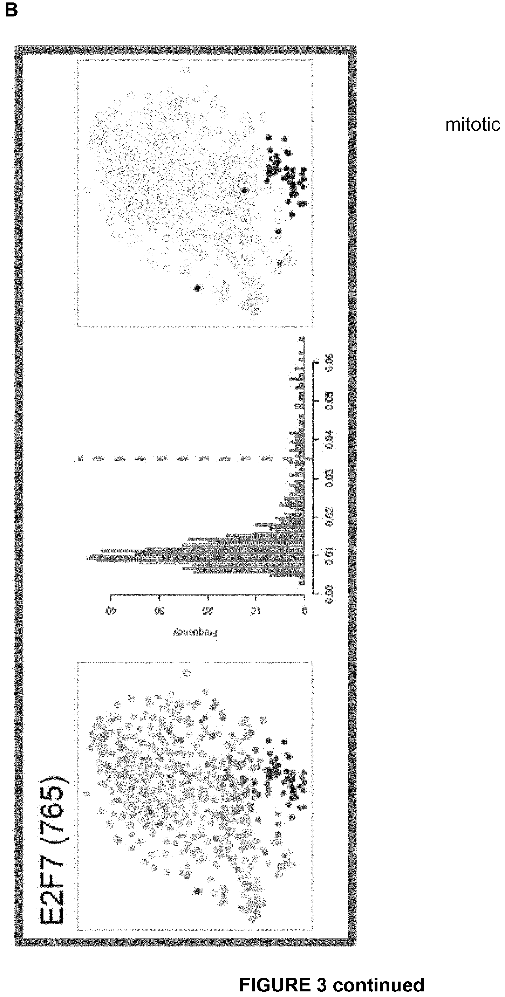

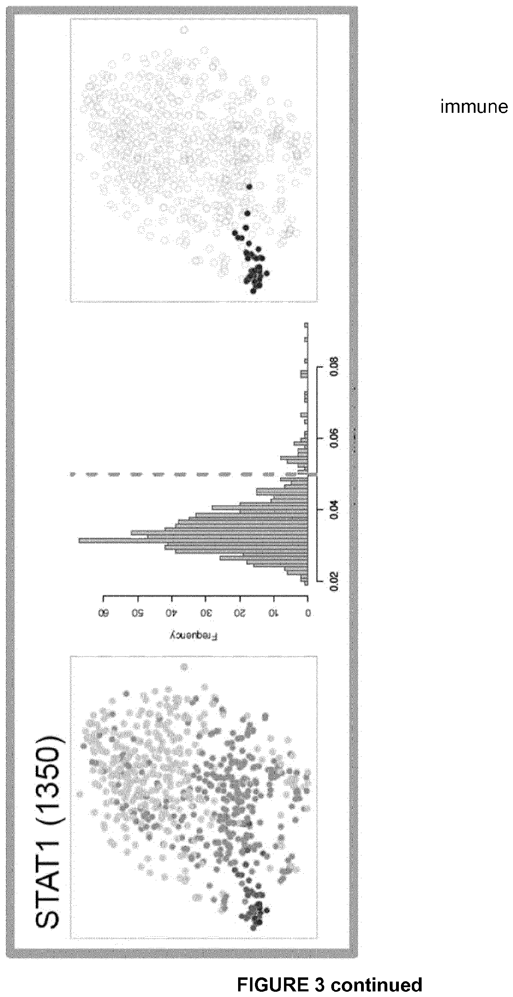

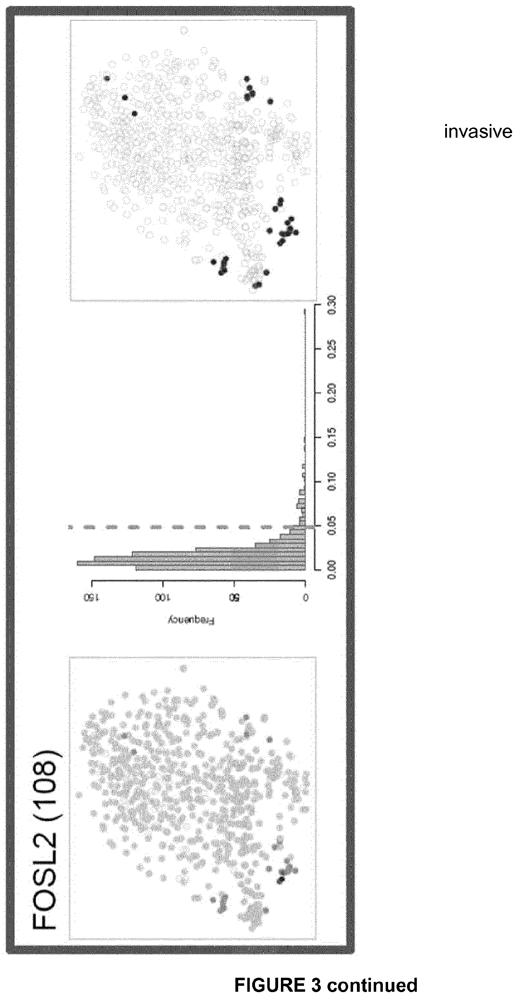

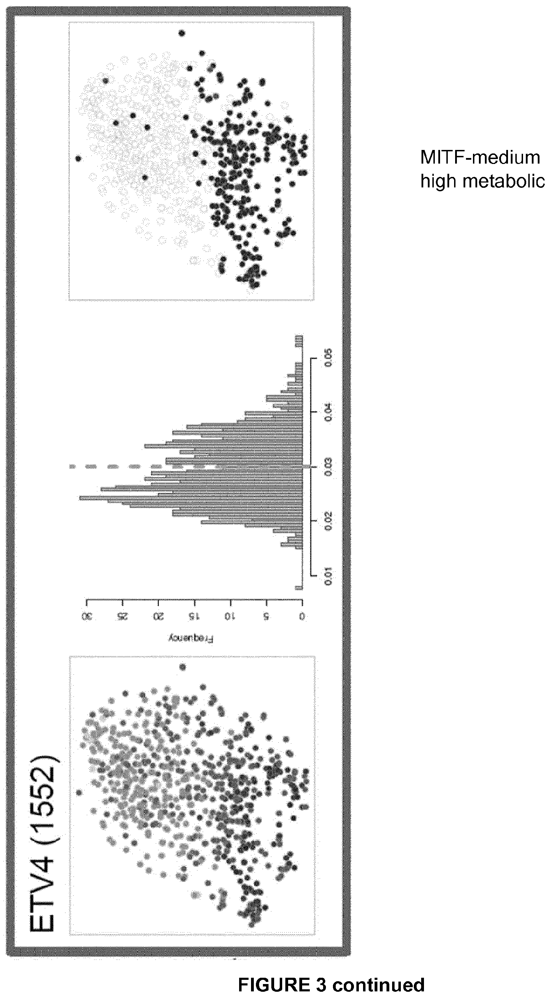

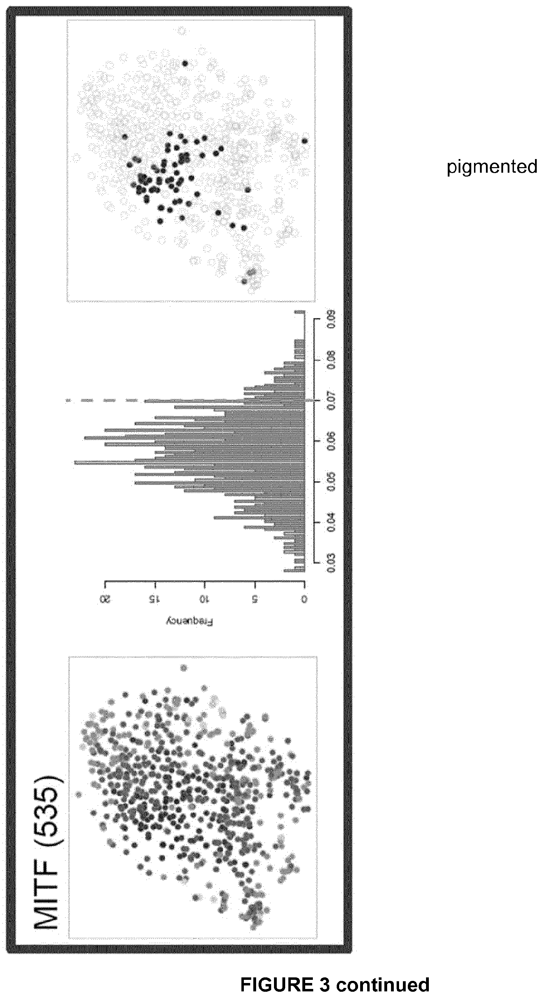

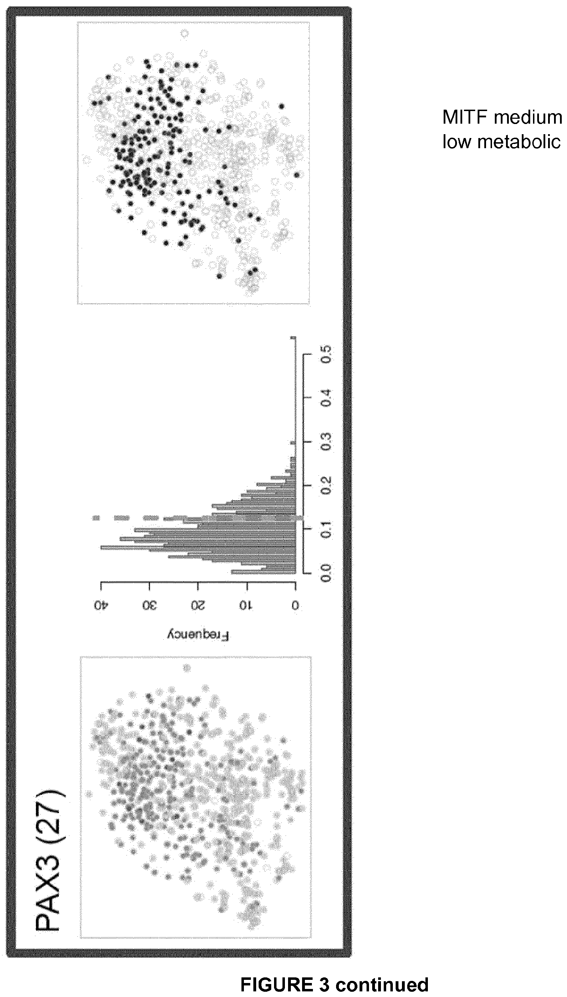

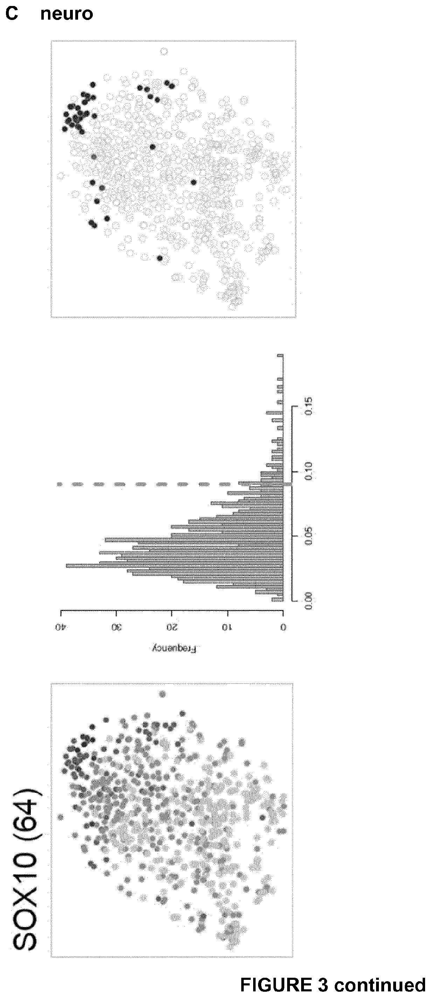

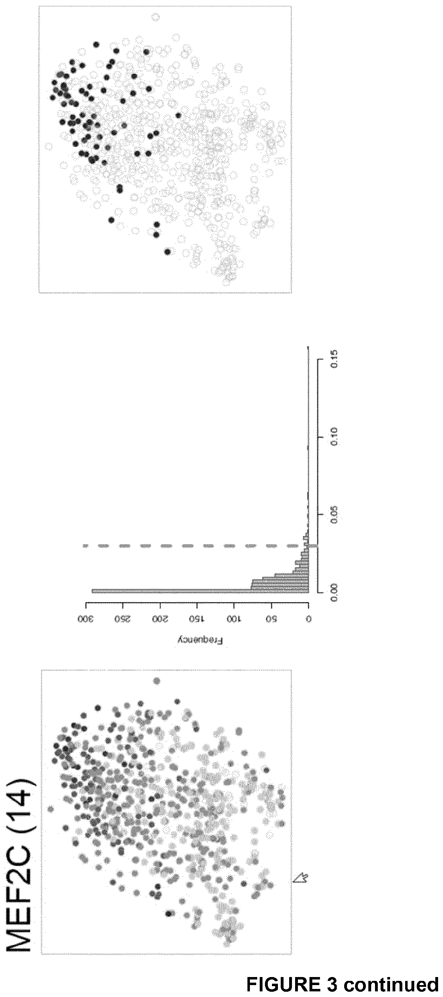

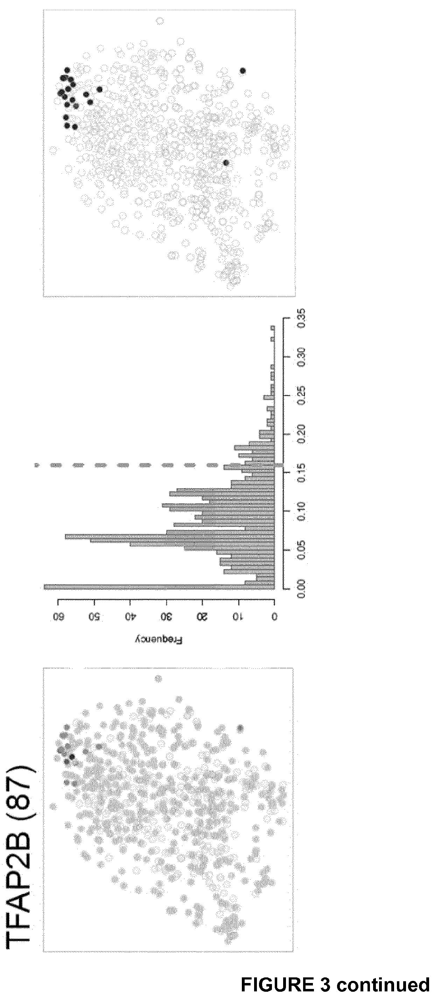

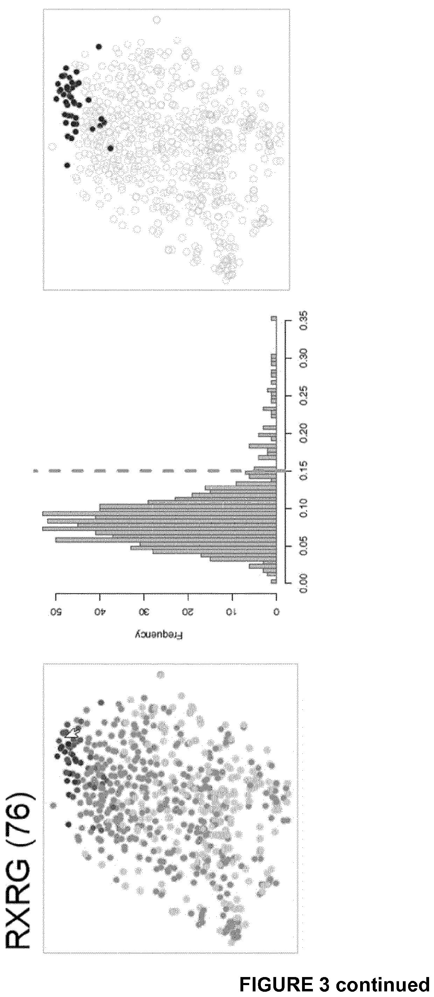

**FIGURE 3****: Gene regulatory network analysis identifies critical nodes driving the NDTC state**
   A) tSNE shows cells colored by state-identity (SCENIC approach). The identities are inferred by the binary activities of the TF regulons. Cell identities inferred by SCENIC are largely overlapping with the SCDE approach (***p = overlap by chance).
   B) Regulons of best predicted transcription factors per state are shown. In the first column, AUC values are used to color the cells of the tSNE plot. The second column shows the distribution plot of AUC values together with the chosen cut-off (orange dashed line). The tSNE plot in the third column shows cells being in a higher state compared to the rest (blue). These are the cells to the right of the dashed line in the histogram. This selection constitutes the binary activity matrix.
   C) SCENIC analysis predicts TFs such as SOX10, MEF2C, TFAP2B and RXRG as central hubs governing the NDTC state. TF regulon activities are quantified by the AUCell score.

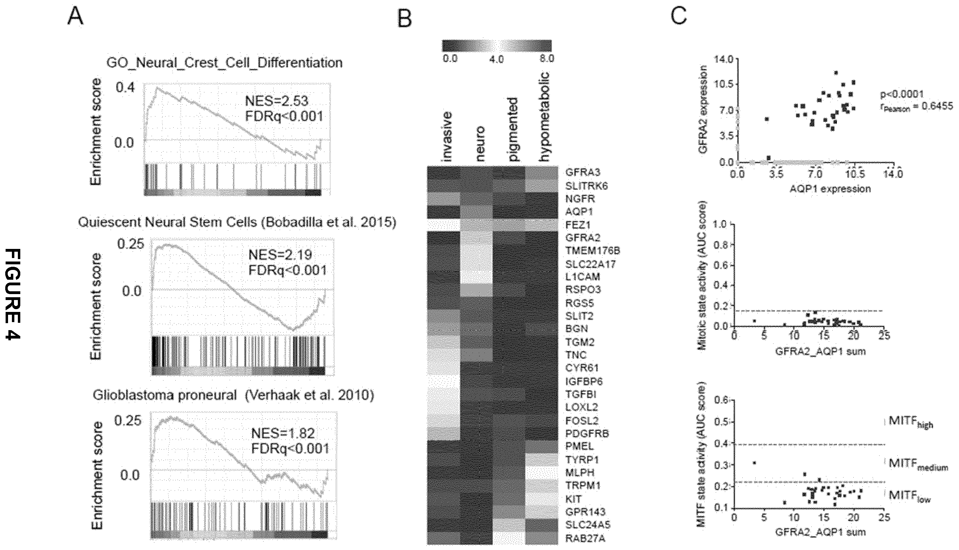

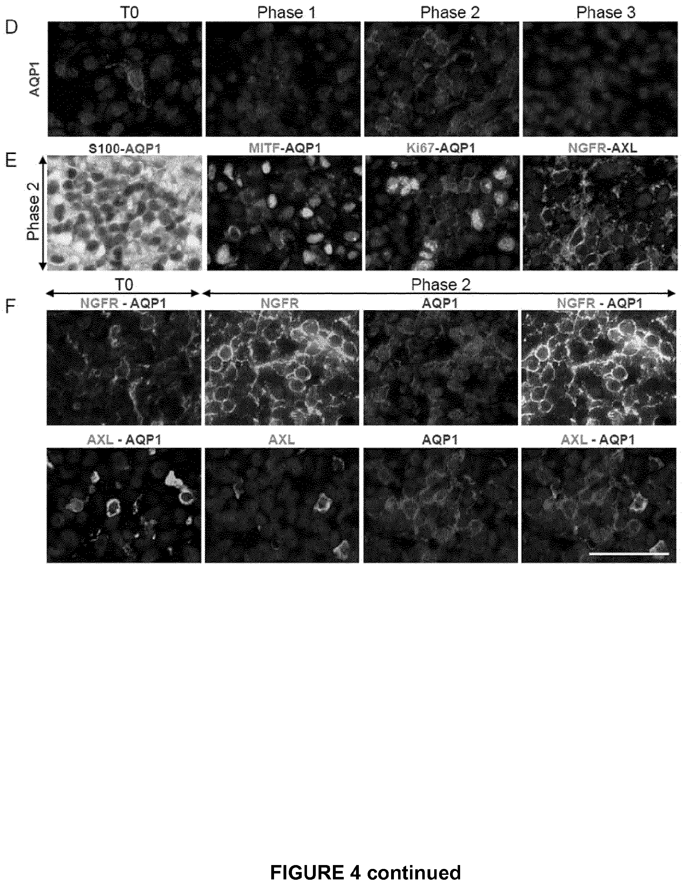

**FIGURE 4****: MAPK-inhibition induces the surge of a (rare -preexisting) neural crest stem cell-like subpopulation that is distinct from the "invasive" AXL+ subpopulation**
   A) Gene set enrichment analysis (GSEA) shows enrichment of gene sets related to neural crest differentiation, quiescent neural stem cells and proneural glioblastoma across the melanoma neuro-state. NES, normalized enrichment score; FDR, false discovery rate.
   B) Representative genes (group averages) for the invasive, neuro and pigmented single-cell state.
   C) AQP1 and GFRA2 are significantly coexpressed in single cells. Cells that express both markers are non-dividing and in a MITF-low state.
   D) AQP1 expression by immunohistochemistry at different time points: T0, phase 1, phase 2 and phase 3.
   E) Immunofluorescence analysis only at phase 2 showing co-expression of S100 and AQP1 and mutual exclusivity for MITF-AQP1, Ki67-AQP1 and NGFR-AXL.
   F) Immunofluorescence analysis comparing T0 and phase for NGFR-AQP1 and AXLAQP1. Scale bar 50 µm.

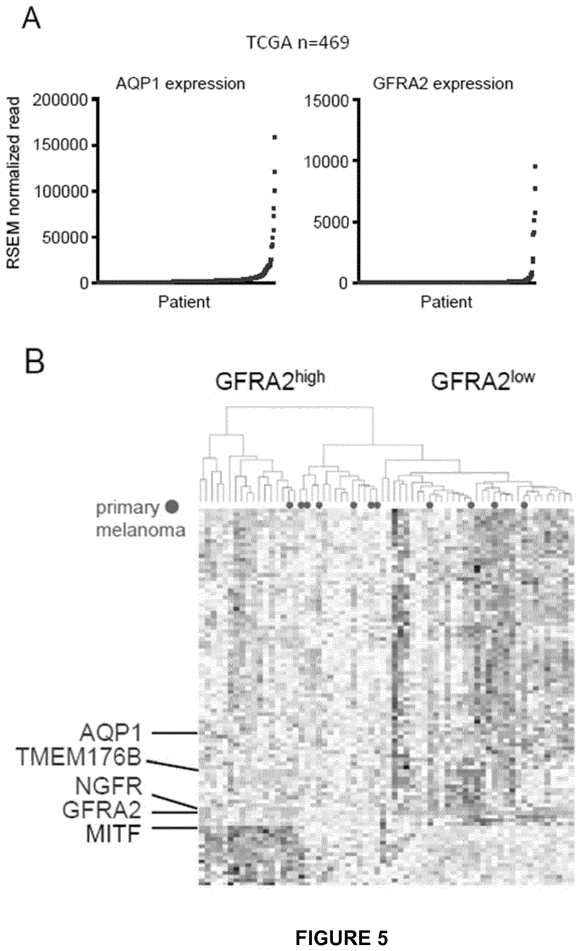

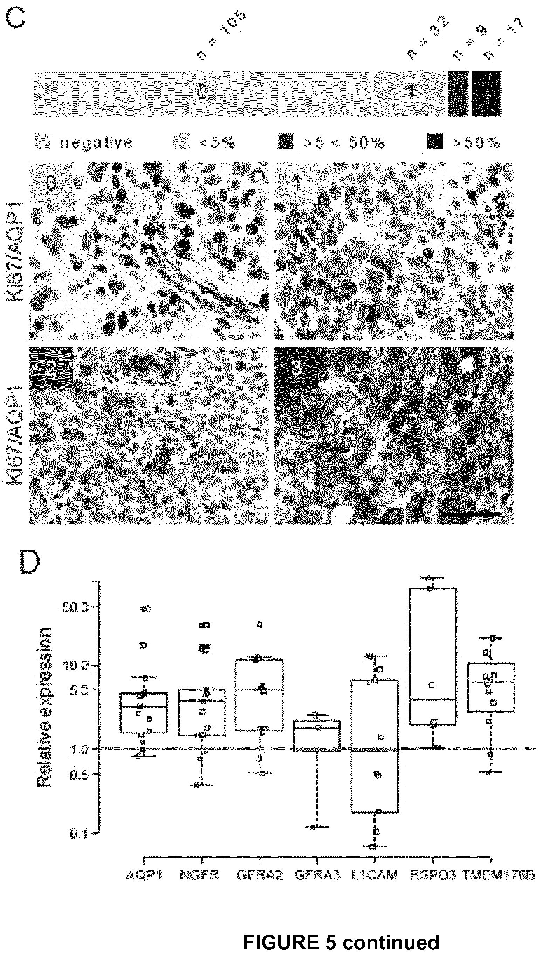

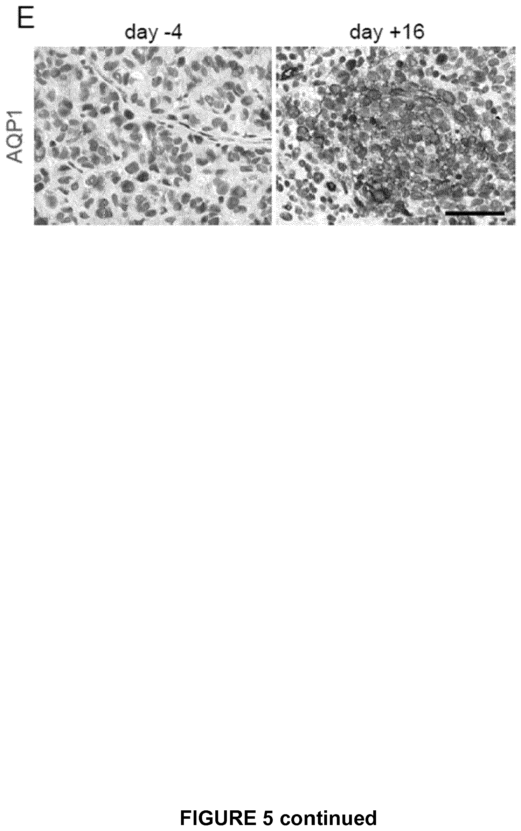

**FIGURE 5****: AQP1/NDTCs are enriched in treated human tumors/biopsies**
   A) AQP1 and GFRA2 expression is only detectable in a small fraction of human melanoma patients (TCGA).
   B) Additional neural genes are coexpressed with GFRA2 and anticorrelated with MITF as suggested by differential gene expression analysis of n=32 GFRA2high vs. n=32 low melanoma patients.
   C) Tissue microarray of 163 melanoma patients double stained for AQP1 (red) and Ki67 (brown). Representation of samples that have no, <5%, >5% and >50% melanoma cells expressing AQP1 (red). AQP1 positive endothelial cells constitute as an internal positive control in the AQP1 negative sample. Scale bar 50 µm.
   D) AQP1, NGFR, GFRA2, GFRA3, L1CAM, RSPO3, TMEM176B expression in patients undergoing BRAF inhibitor-based targeted therapy (RT-qPCR). Biopsies were taken pre-treatment and early on treatment with BRAFi or BRAFi + MEKi under DFCI Protocol 11-181 (PI: Boland). AQP1, NGFR, GFRA2, GFRA3, L1CAM, RSPO3, TMEM176B expression in patients undergoing targeted therapy (RT-qPCR).
   E) AQP1 expression (red) in patient 34 before and during treatment with dabrafenib/trametinib; scale bar 50 µm.

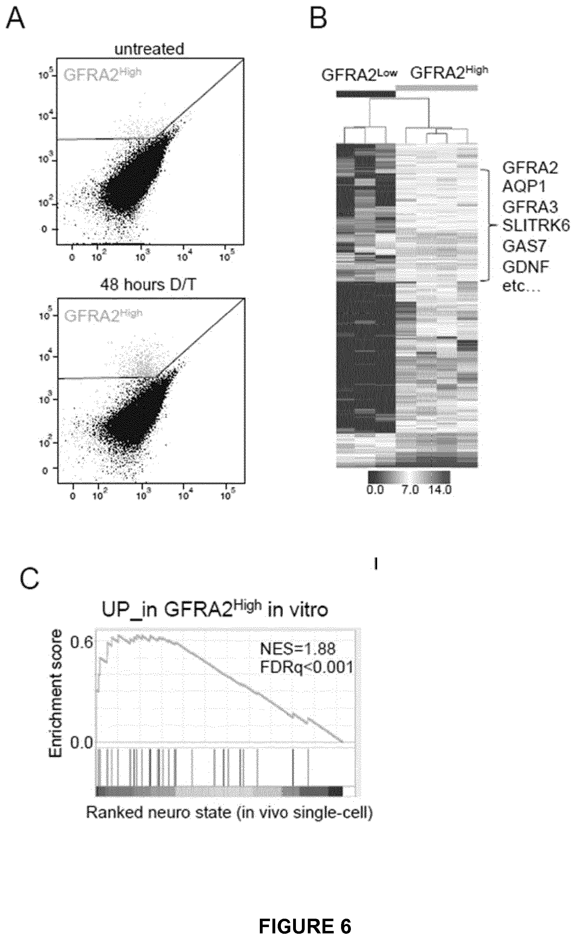

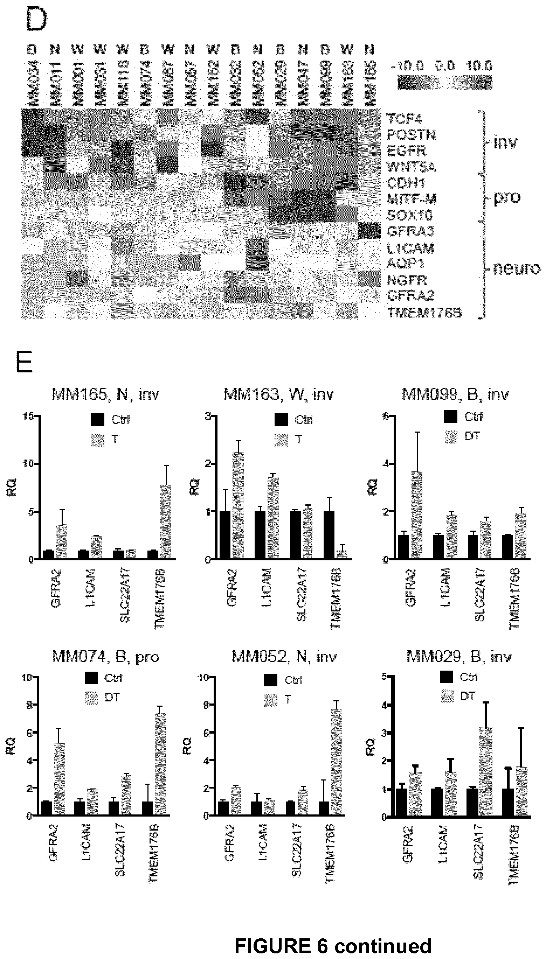

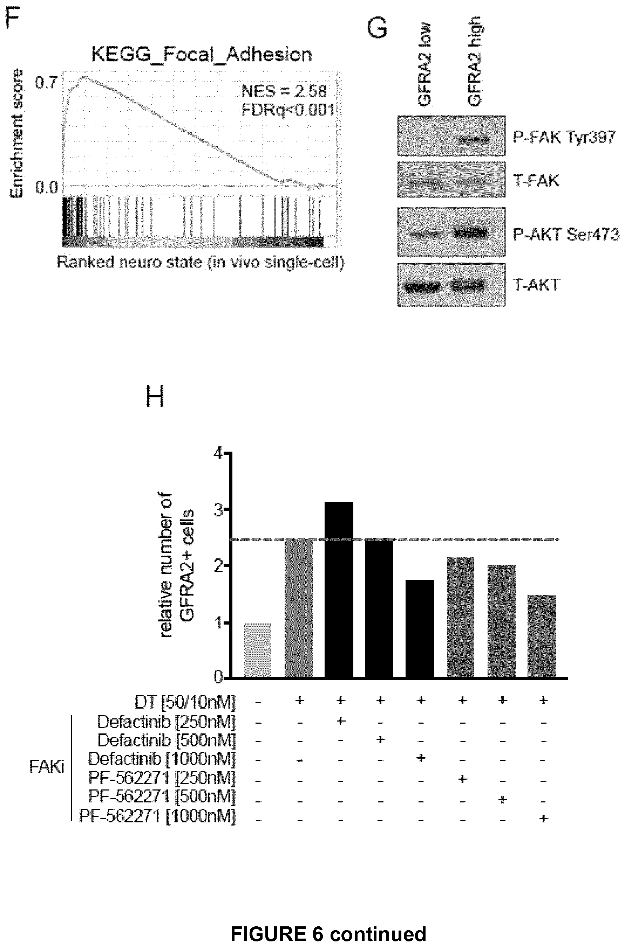

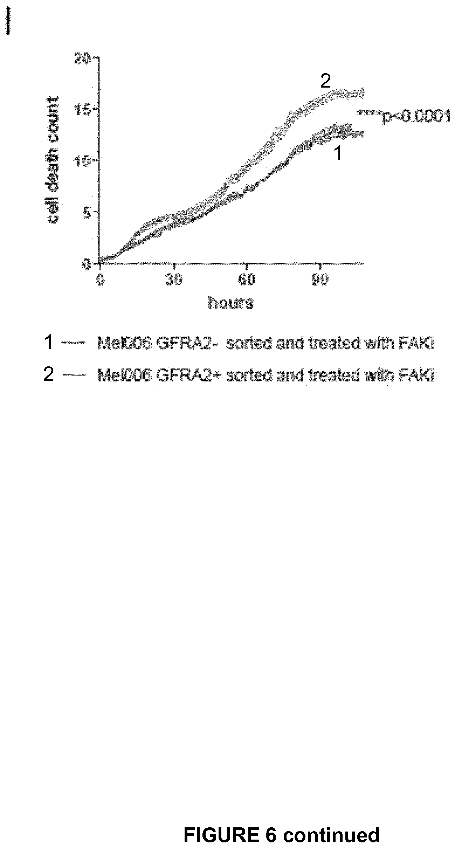

**FIGURE 6****: NDTC state is inducible *in vitro* and governed by RXR**
   A) Representative FACS profiles of GFRA2 expression. GFRA2 expression is induced in MEL006 cells upon treatment with BRAF&MEK inhibition.
   B) Heatmap shows gene expression profile (RNAseq) of FACS-sorted GFRA2high vs. low cells after ten days of treatment. Additional NDTC markers are coexpressed with GFRA2.
   C) GSEA plot shows significant enrichment for the top100 upregulated genes in FACS-sorted GFRA2high cells across *in vivo* single-cell NDTC state.
   D) Heatmap shows a selection of inasive, proliferative and NDTC markers across different melanoma short term cultures. Gene expression was measured using RTqPCR. (B=BRAFmut, N=NRASmut, W=neither B nor N).
   E) Treatment induced upregulation of GFRA2 expression and other NDTC markers in different melanoma short term cultures (RT-qPCR, pro=proliferative, inv=invasive molecular phenotype).
   F) GSEA plot shows significant enrichment for the KEGG_Focal_Adhesion geneset across the in vivo single-cell NDTC state.
   G) Western blot analysis of FACS-sorted GFRA2high and low MEL006 cells shows increased phosphorylation of FAK, AKT and ERK in GFRA2high cells.
   H) GFRA2high cells are sensitive to FAK inhibitors. MEL006 cells were treated with DT and or FAK inhibitors (defactinib, PF531). After treatment, cells were FACS sorted for GFRA2 expression.
   I) Cell death count (overlapping apoptosis and necrosis marker, incucyte) is significantly higher in FACS-sorted GFRA2high cells (pretreated with DT) upon FAK inhibition.

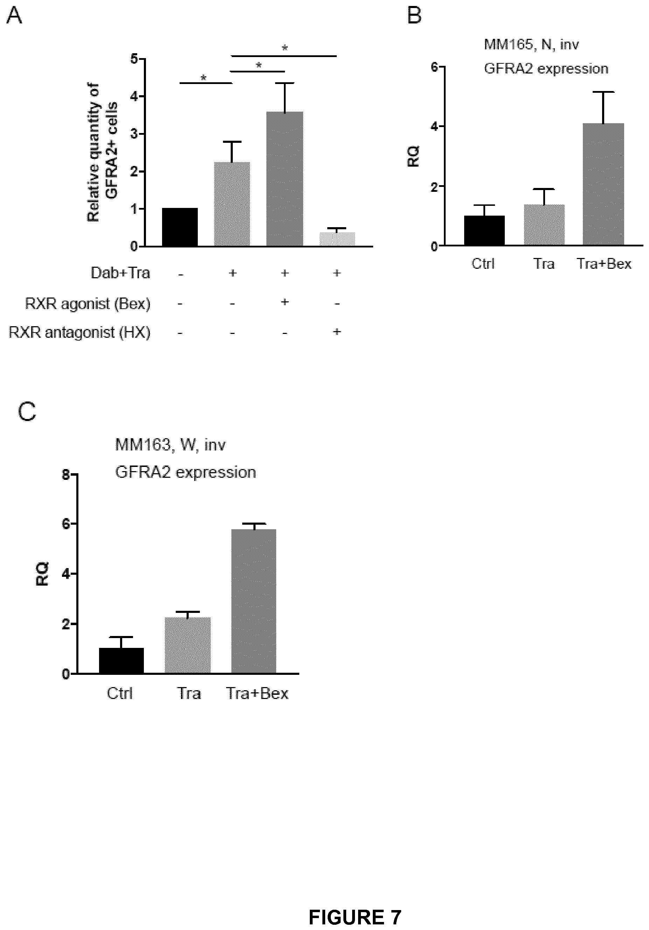

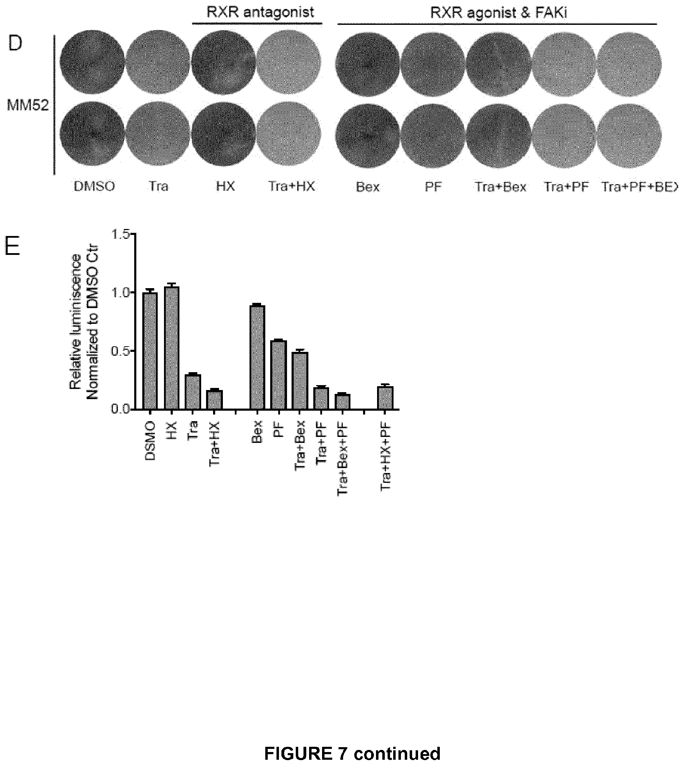

**FIGURE 7****: NDTC state is targetable by RXR modulation and FAK inhibition**
   A) Relative number of GFRA2+ cells are shown using FACS-sorting after treatment of MEL006 cells with either BRAFi&MEKi and/or RXR antagonist.
   B) Increase of GFRA2 expression (RT-qPCR) upon BRAF&MEK inhibition and RXR agonist treatment (bexarotene) in NRAS-mutant cells (MM165) and
   C) wild-type cells (MM163).
   D) Colony assay performed over 7 days.
   E) and Cell Titer Glo assay performed after 6 days after incubation of the short culture melanoma line MM052 with MEK inhibitor trametinib (TRA; 2nM), RXRG agonist bexarotene (BEX; 1uM), FAK inhibitor PF-562271 (1uM) and RXRG antagonist HX531 (2uM)

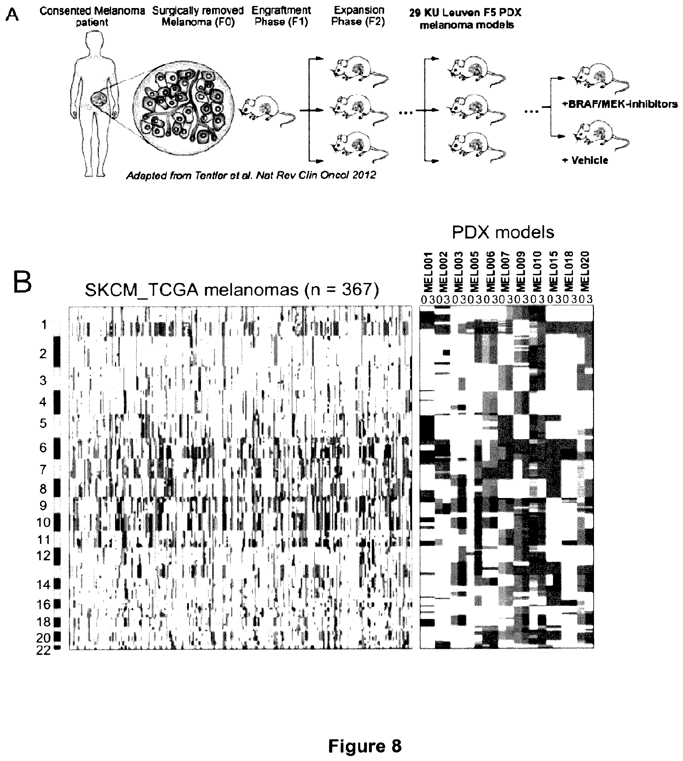

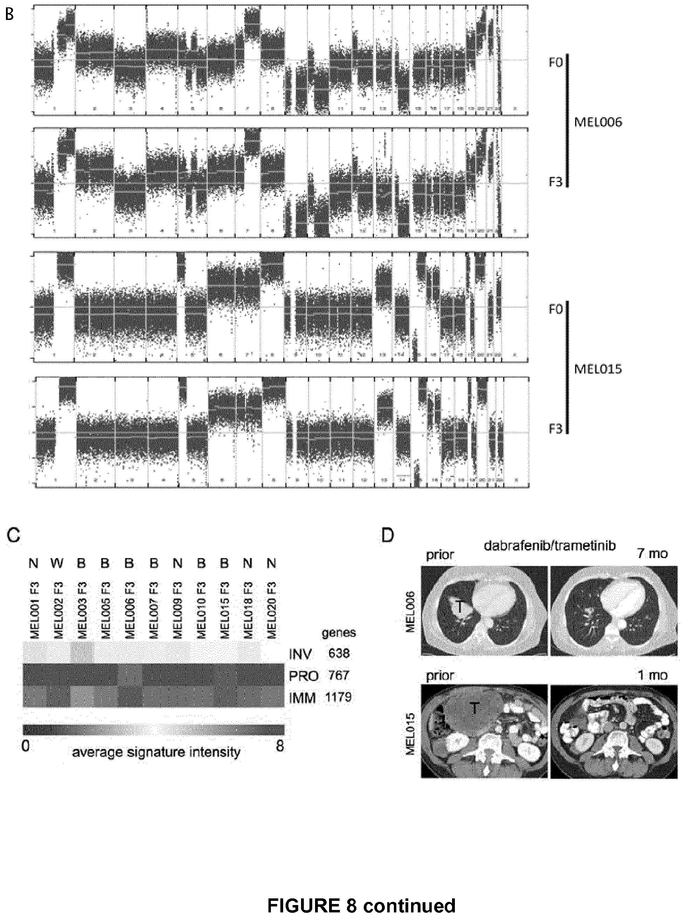

**FIGURE 8****: Establishment of melanoma PDX models to study drug tolerance *in vivo.***
   A) Twenty-nine patient-derived melanoma xenografts were established of which two models were challenged with BRAF & MEK inhibitors.
   B) Eleven PDX models (F0 and F3) were sequenced on the DNA level to establish copy-number profiles. Obtained copy number profiles mimic those of TCGA melanoma patients.
   C) Gene expression profiles of eleven (F3) PDX models were classified according their molecular phenotype in either invasive (INV), proliferative (PRO) and immune (IMM) based on already established gene sets (Verfaille et al. 2015). Largely, the eleven PDX models are of a proliferative phenotype.
   D) Patient MEL006 achieved an almost complete response after 7 months of treatment with dabrafenib-trametinib, illustrated here is a lung metastasis. Patient MEL015 had a deep partial response after 1 month of treatment with DT (double therapy; dabrafenib+trametinib). Computed tomography (CT) depicting a lung and intra-abdominal metastasis respectively.

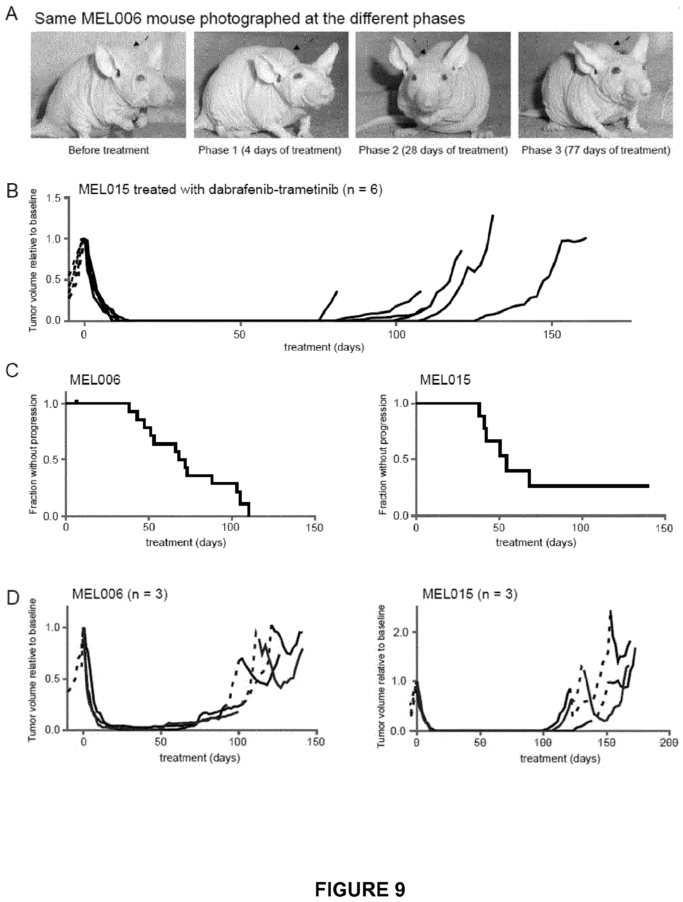

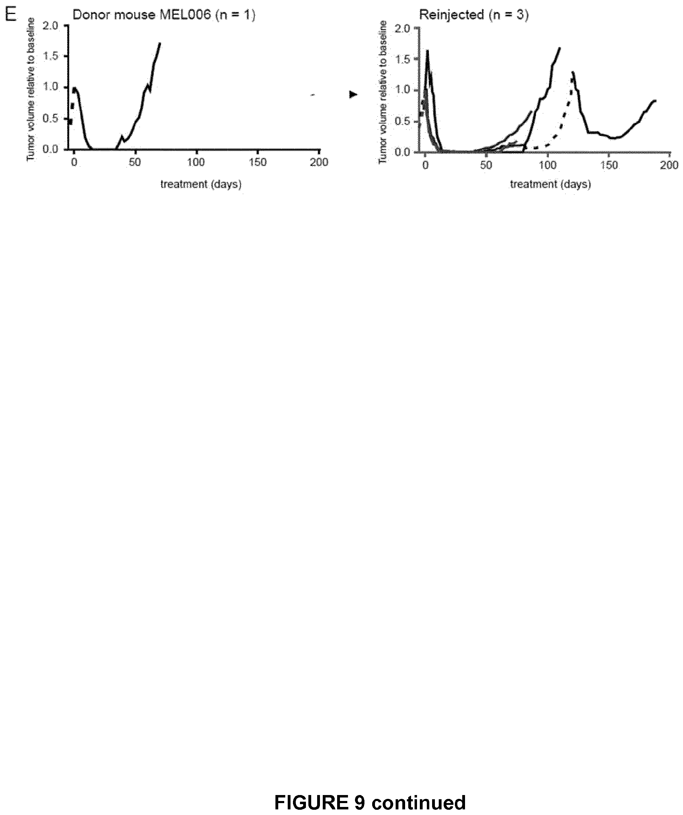

**FIGURE 9****: Tumor evolution following continuous and interrupted RAF/MEKinhibition**
   A) Photographs at different time points: same MEL006 mouse during the treatment phases with dabrafenib-trametinib: before treatment (T0 - 998 mm³), phase 1 (after 4 days of treatment - 396 mm³), phase 2 (after 28 days of treatment 12 mm³) and phase 3 (resistance after 77 days of treatment - 273 mm³).
   B) MEL015 six mice treated with dabrafenib-trametinib; dotted line denotes "off treatment".
   C) Kaplan-Meier estimate (fraction without progression): median time to progression for MEL006 70 days, median time to progression for MEL015 109 days.
   D) MEL006 (n = 3) and MEL015 (n = 3) treated until resistance, followed by a therapy-free interval, followed by rechallenge.
   E) MEL006 mouse treated until resistance; the resistant tumor was dissociated and reinjected into 3 mice: all three mice responded again, one mouse was rechallenged a second time (black line) and briefly responded a third time.

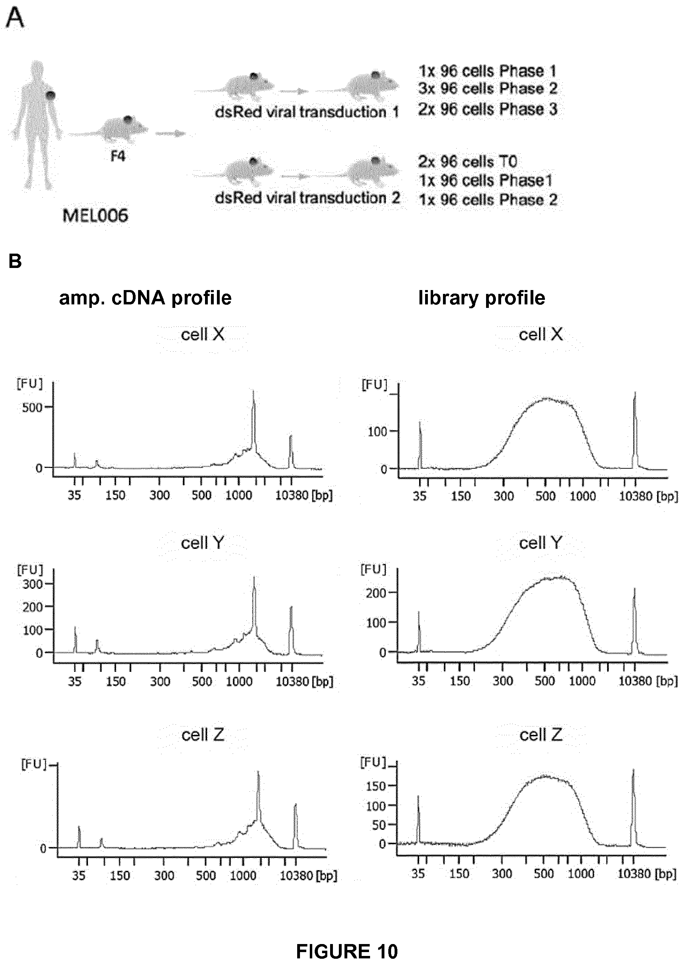

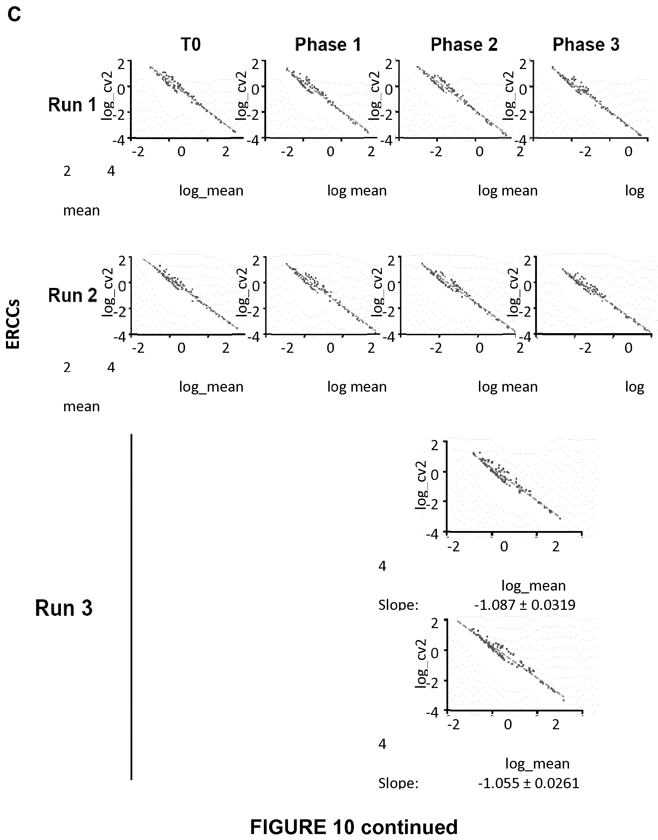

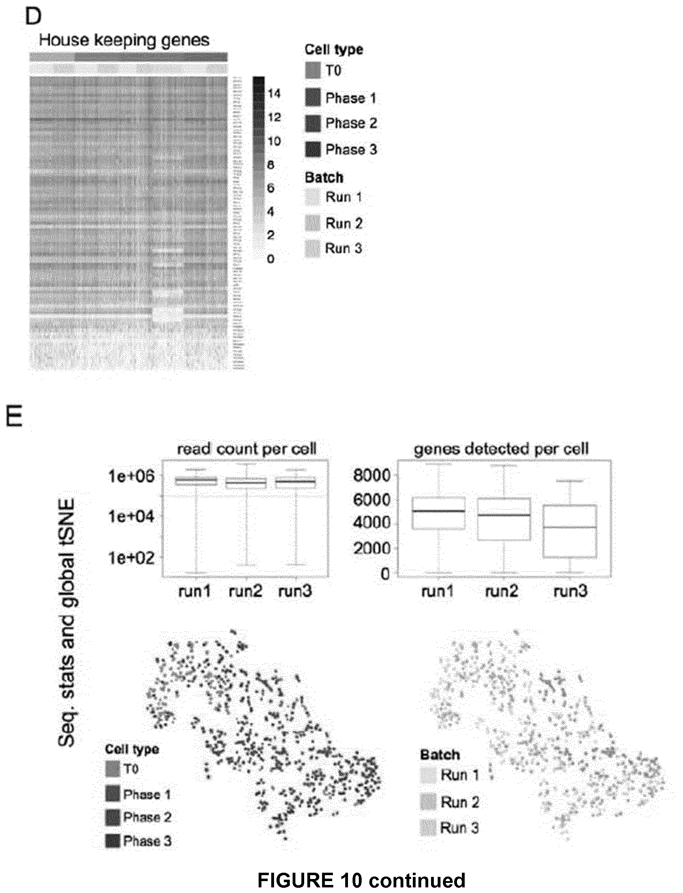

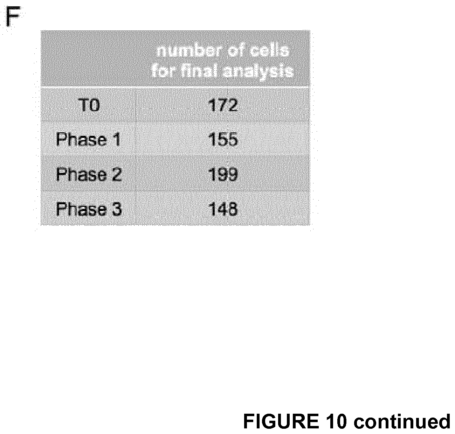

**FIGURE 10****: Single-cell RNA sequencing details and quality controls**
   A) SMARTseq2 based single-cell RNA sequencing was performed in a 96 well format. In total, ten 96 well plates, containing FACS-sorted single cells were prepared from six different animals over four time points. NexteraXT libraries were prepared subsequently. Sequencing was performed in three batches on the Nextseq500 platform by multiplexing up to 4 x 96 single cells.
   B) Representative single-cell Bioanalyzer profiles of amplified cDNA and respective NexteraXT libraries are shown.
   C) To control for amplification biases or other batch effects we spiked-in ERCCs to each single cell. After plotting variation (CV2) over mean expression of all ERCCs per library we observed comparable variation among all ten 96 well experiments.
   D) Heatmap of 85 housekeeping genes shows stable expression amongst single cells irrespective of time and sequencing batch.
   E) Number of aligned reads per cell and number of genes detected per cell of the three individual sequencing runs. tSNE plot of all cells based on global gene expression color coded by time point (stage) and sequencing run (batch).
   F) Number single cells for final analysis after filtering out low-quality cells based on library size, number of genes expressed, ERCC spike-ins and mitochondrial genes (Lun et al. 2016, F1000Research 5:2122).
**FIGURE 11****: Single-cell RNAseq data analysis.**
   A) Schematic of the applied Single-cell RNAseq data analysis workflow per time point. At first, highly variable genes are identified in an unsupervised manner. Then cells are clustered based on the highly variable genes using non-negative matrix factorization (NMF) allowing for up to 10 ranks (best fit chosen based on cophenetic correlation). Single cell differential expression analysis (SCDE) between NMF clusters generates Z-score ranked gene lists, which are analyzed for enrichment using different tools. After establishing characteristic gene signatures their activities are quantified in each single cell using the AUCell algorithm.
   B) NMF-clustering per time point based on highly variable genes. SCDE analysis of NMF clusters per time point generates ranked gene lists based on cZ-scores.
   C) Functional enrichment analysis of top100 gene lists using Ingenuity Pathway Analysis (IPA) and i-cisTarget to predict regulatory features and cis-regulatory modules. Six gene signatures are established after enrichment analysis.
   D) NMF clustering of MITF-medium cells (TO-phase 3) based on all highly variable genes (TO-phase 3) generates two clusters of cells. SCDE analysis of the two NMF clusters results in a top300 gene list (ranked by cZ-score), which is used for enrichment analysis.
**FIGURE 12****: AUC distributions of different gene signatures**
   AUC (Area Under the recovery Curve) represents the proportion of expressed genes in the signature, and their relative expression value compared to the other genes within the cell. The distribution plots show the number of cells (Frequency) per AUCell score. Thresholds (red dashed line) delineate cells being in a higher or lower state. These cells are color coded accordingly.
**FIGURE 13****: Cell state dynamics during BRAF&MEK inhibition.**
   A) tSNE plots show different states during treatment. Cells being in a higher state are colored according to AUCell (Figure 12).
   B) Cells in a higher invasive and neuro state are quantified relatively per time point.
   C) Diffusion map of invasive, neuro, pigmented and MITF-medium cells.
**FIGURE 14****: TF regulon activities for additional states and the NDTC gene regulatory network.**
   A) tSNE shows cells colored by state-identity (SCENIC approach). The identities are inferred by the binary activities of the TF regulons. Cell identities inferred by SCENIC are largely overlapping with the SCDE approach (***p = overlap by chance).
   B) Additional regulons of predicted transcription factors per state are shown. In the first column, AUC values are used to color the cells of the tSNE plot. The second column shows the distribution plot of AUC values together with the chosen cut-off (orange dashed line). The tSNE plot in the third column shows cells being in a higher state compared to the rest (blue). These are the cells to the right of the dashed line in the histogram. This selection constitutes the binary activity matrix.
   C) Gene regulatory network analysis using SCENIC identifies critical nodes driving the NDTC state. The predicted TFs and their target genes are shown.

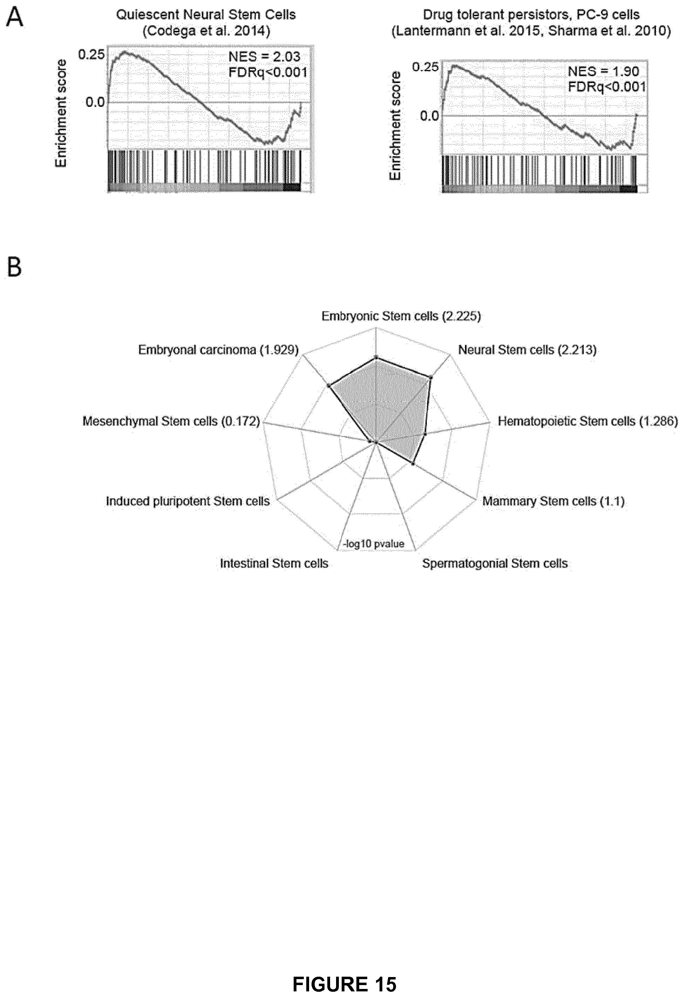

**FIGURE 15****: Drug-tolerant melanoma cells exhibit a neural stem cell-like transcriptional program.**
   A) GSEA plots show significant enrichment for quiescent Neural stem cells and drug tolerant persistors across the single-cell *in vivo* NDTC state.
   B) Top200 NDTC-genes were analyzed with stemchecker (Pinto et al. 2015). The spiderchart shows enrichment for embryonic and neural Stem cells.

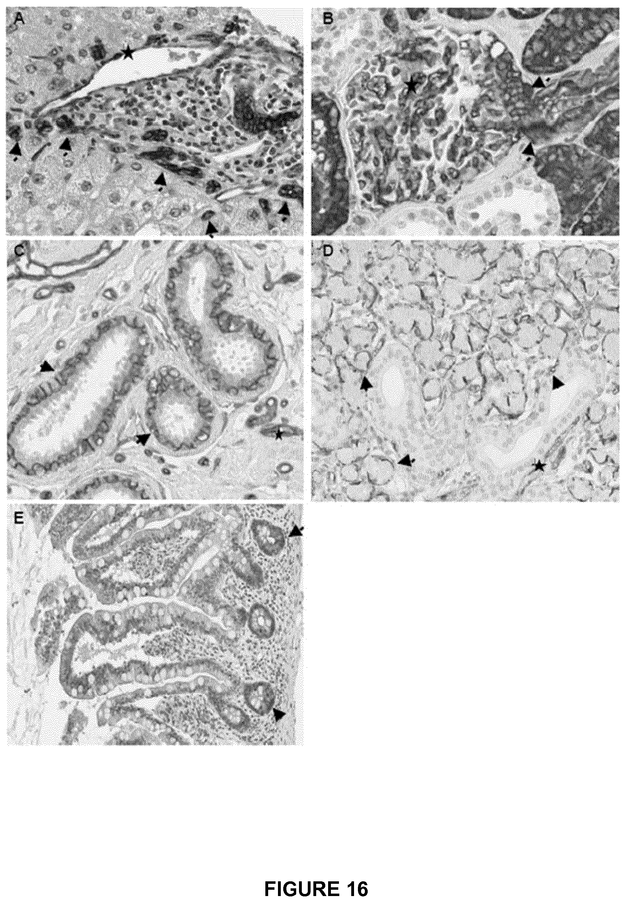

**FIGURE 16****: AQP1 is a marker of various human stem/progenitor cell compartments.**
   IHC for AQP1 (from the Protein Atlas) in normal liver (A), kidney (B), breast (C), salivary gland (D) and small intestine (E). Immunoreactivity is confined to endothelial cells (highlighted with asterisks) and reservoir cell compartments (arrows) such as canals of Hering (A), the junction between urinary dpace in the glomerulus and proximal tubule (B), the outer ring of myo-epithelial cells around ducts and ductules (C) and around acinar lobules (D) and scattered epithelial cells in the deepest parts of the crypts (E).

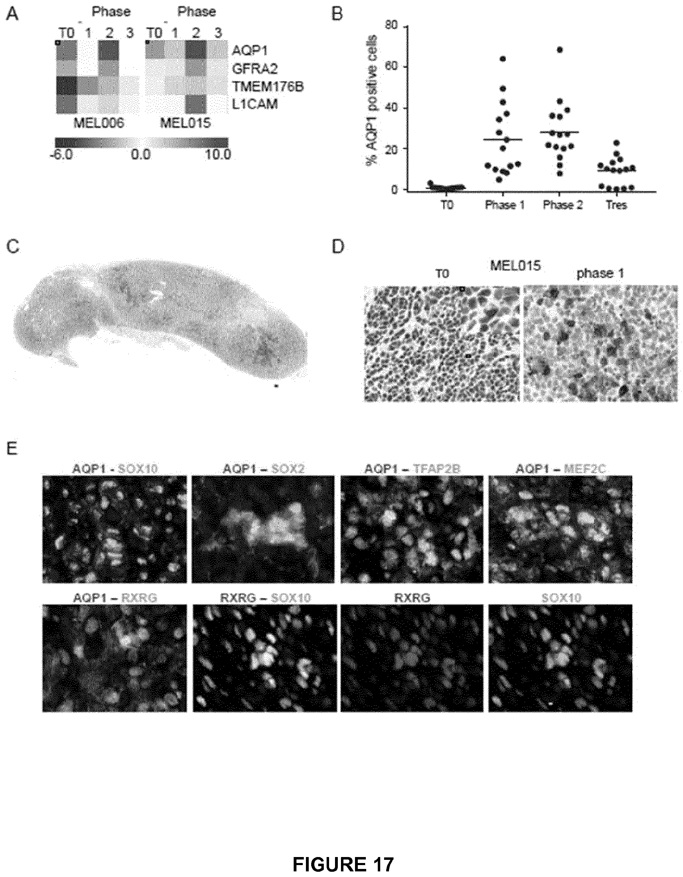

**FIGURE 17****: Expression of selected NDTC markers in drug-exposed PDX melanoma samples.**
   A) Expression of NDTC markers on the bulk level during BRAF&MEK inhibition in two PDX models (RT-qPCR analysis).
   B) Quantification of average AQP1+ cells on immunohistochemistry at T0, phase 1, phase 2 and Tres.
   C) AQP1 immunostaining of a representative Phase 2 MEL006 section showing occurrence of AQP1-positive in clusters. Scale bar, 1 mm.
   D) AQP1 immunostaining of a representative T0 and Phase 1 MEL015 sections. Scale bar, 1mm.
   E) Immunostainings demonstrating AQP1 positive cells express SOX10, SOX2, TFAP2B, MEF2C and RXRG. RXRG and SOX10 colocalize at phase 2. Scale bar 50 µm.

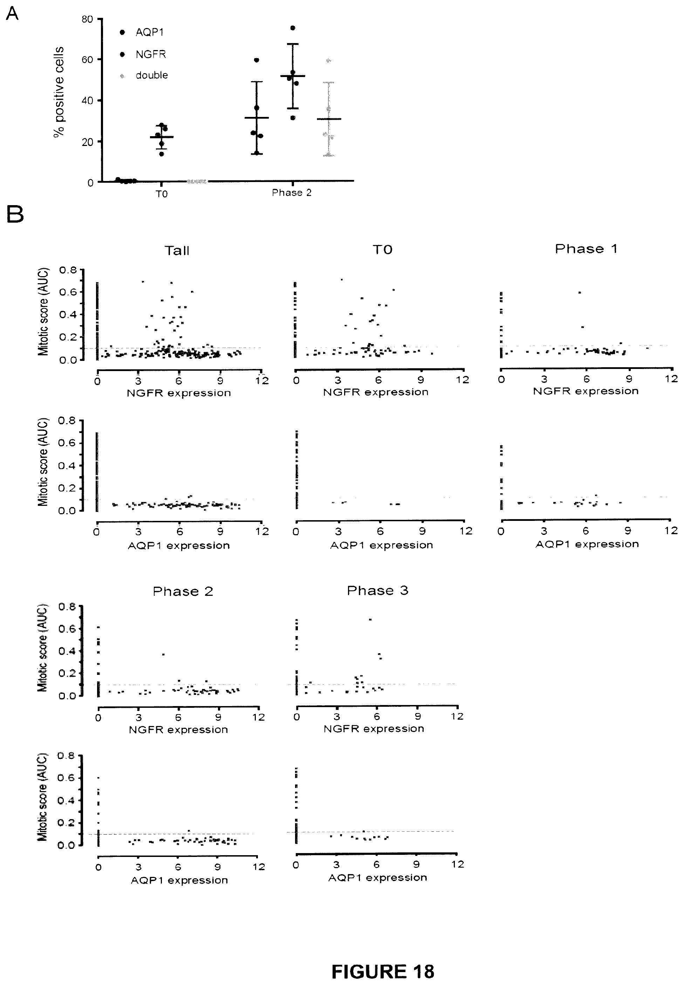

**FIGURE 18****: Monitoring expression of AQP1 and NGFR in drug-exposed PDX melanoma samples.**
   A) Quantification of average AQP1+, NGFR+ and AQP1/NGFR-double positive cells by IHC at the indicated time points.
   B) NGFR and AQP1 expression levels in individual melanoma cells were correlated with their corresponding mitotic state activity (score) during BRAF&MEK inhibition. The inferred mitotic state is compatible with NGFR, but not AQP1, expression.

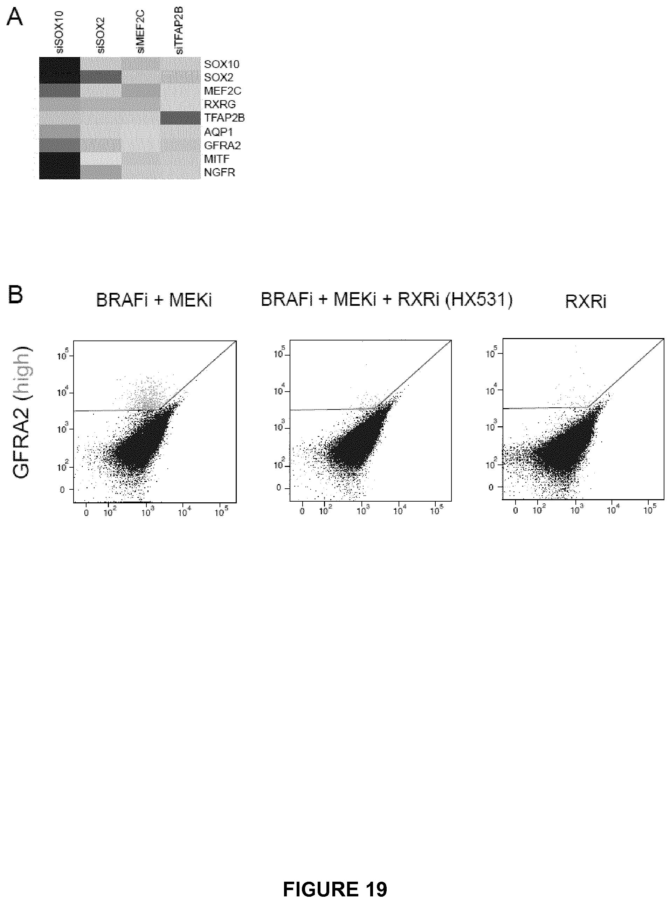

**FIGURE 19****: Perturbation of gene regulatory networks**
   A) Heatmap shows expressional changes after knock down of different transcription factors in presence of BRAF&MEK inhibitors using siRNAs (MEL006 in vitro, 48h).
   B) Representative FACS profiles of GFRA2 positive cells after DT (dabrafenib-trametinib) and/or RXRi (HX531) treatment.

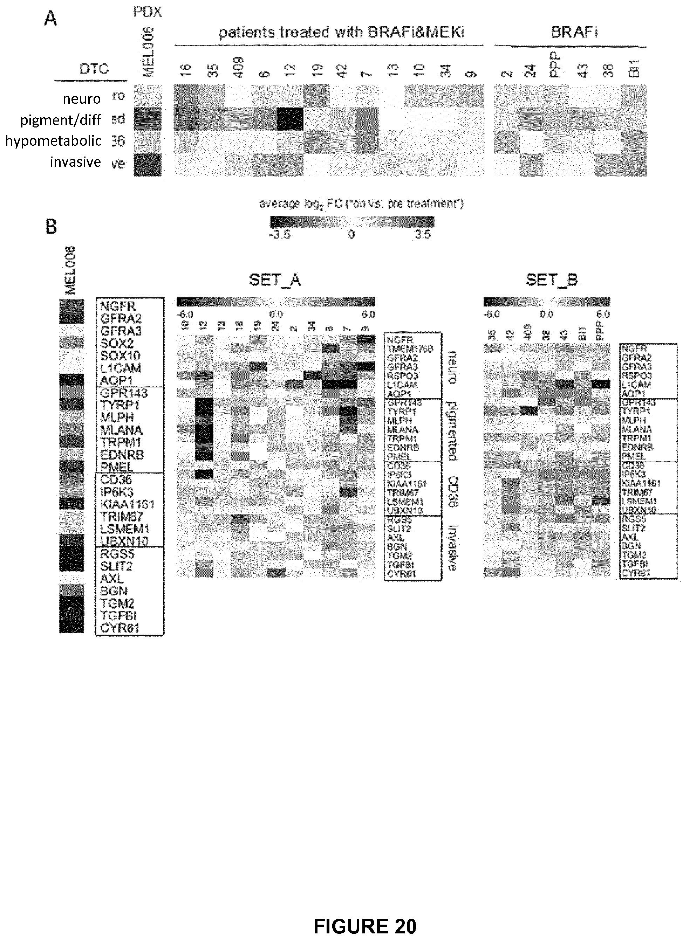

**FIGURE 20****: Gene expression analysis in melanoma tumors.**
   **(A)** Comparison of gene expression signatures specific for the indicated drug-tolerant cell (DTC) states in a PDX mouse model (MEL006), in patients treated with combination of BRAF and MEK inhibitors, and in patients treated with BRAF inhibitors only. The comparison was made based on bulk RNA analysis on a biopsy taken before start of treatment compared to a biopsy taken on-treatment and upon reaching of or during the residual disease phase.
   **(B)** Same as in (A) but at the individual gene level. The combined information of the individual gene expression data of (B) is merged to arrive at the scores for the drug-tolerant cell (DTC) states indicated in (A).

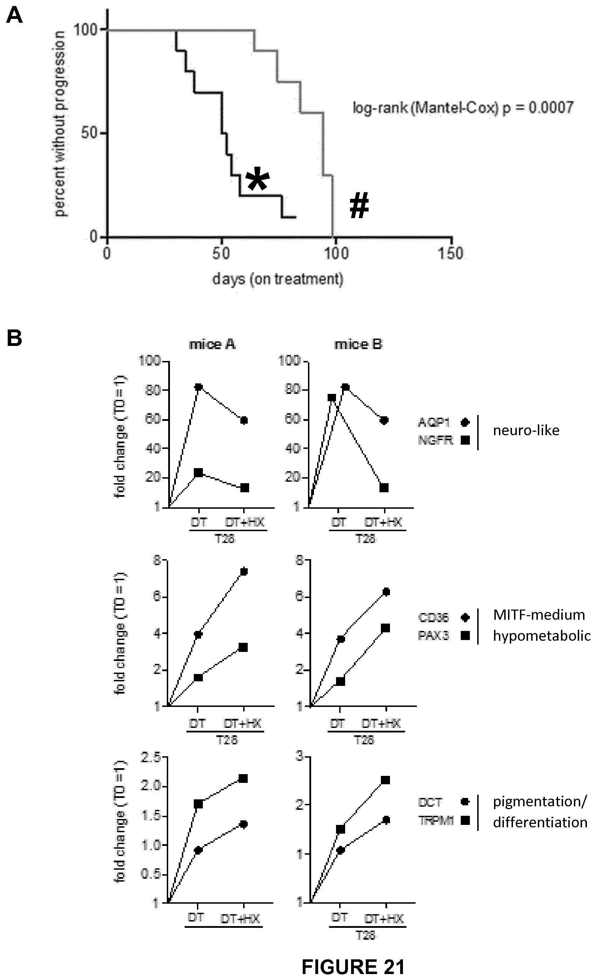

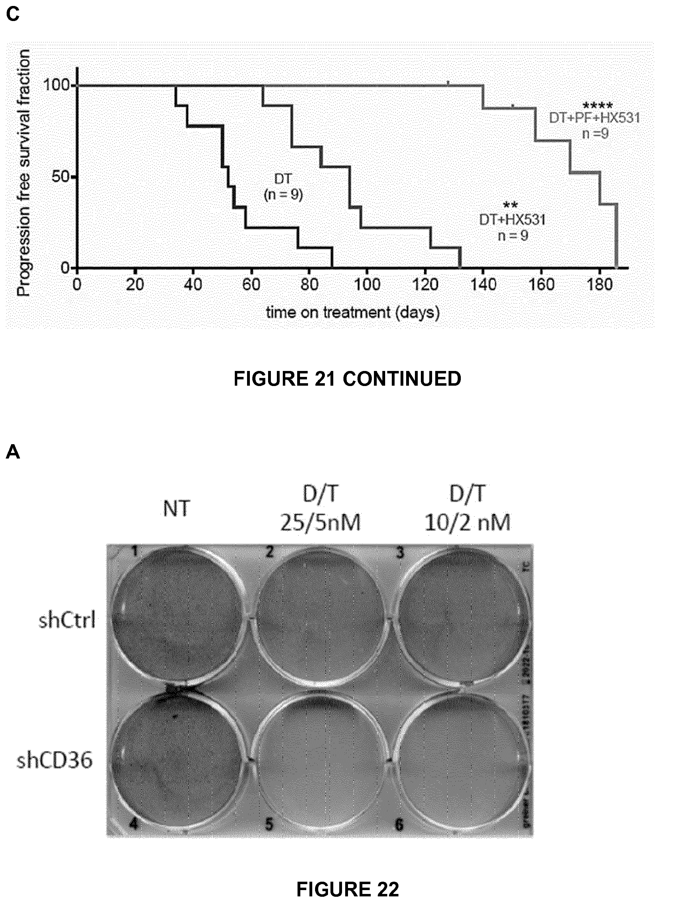

**FIGURE 21****. In vivo efficacy of RXR antagonist in PDX mouse melanoma model.**
   **(A)** Kaplan-Meier estimate for time to progression. Comparison in time to progression between PDX melanoma (MEL006) mice treated with BRAF/MEK inhibitors (marked with *) and PDX melanoma (MEL006) mice treated with BRAF/MEK/RXR inhibitors (marked with #).
   **(B)** RT-qPCR analysis of the indicated genes selected from the gene expression signatures specific for the indicated drug-tolerant cell (DTC) states. DT = double therapy/dabrafenib+trametinib; HX = RXR antagonist HX531.
   (C) Similar to (A) mice treated with BRAF/MEK inhibitors (DT), PDX melanoma (MEL006) mice treated with BRAF/MEK/RXR inhibitors (DT+HX531), and PDX melanoma (MEL006) mice treated with BRAF/MEK/RXR/FAK inhibitors (DT+PF+HX531).

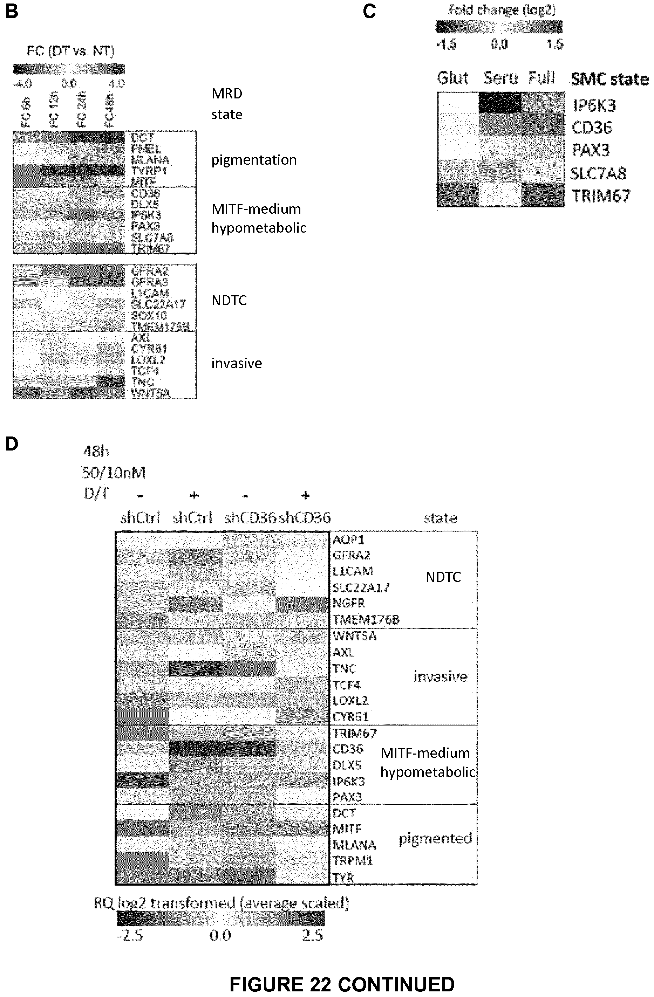

**FIGURE 22****: NDTC state is targetable by CD36 inhibition**
   A) Colony assay performed over 14 days (see Example 2.9).
   B) Induction of the 4 different minimal residual disease subpopulations, and increase of CD36 expression, as measured by RT-qPCR of the indicated genes upon BRAF&MEK inhibition in cultured Mel006 cells ("DT"), expressed as fold change (FC) relative to untreated Mel006 cells ("NT").
   C) Starvation of cultured Mel006 cells induces the MITFmedium hypometabolic residual disease cell population.
   D) CD36 inhibition by shRNA (shCD36) suppresses emergence of NDTCs, MITFmedium hypometabolic cells and invasive cells, but induces pigmented cells in cultured Mel006 cells treated with dabrafinib and trametinib (DT), relative to control shRNA (shCtrl), as determined by RT-qPCR of expression of the indicated marker genes specific for each of the 4 different minimal residual disease subpopulations.

### DETAILED DESCRIPTION TO THE INVENTION

The invention is set out in the appended set of claims.

In view of the development of acquired resistance to therapy, and the (possibility of) reversible tolerance to therapy before acquiring genetic resistance, there is still a great medical need for improved cancer patient stratification in the clinical setting and in- and outside clinical trials, such as (but not limited thereto) for melanoma patients treated with concurrent RAF/MEK-inhibition, which has become a standard of care for BRAFV600E mutated melanoma patients (Larkin et al. 2014, NEJM 371:1867-1876; Long et al. 2014, NEJM 371:1877-1888; Robert et al. 2015, NEJM 372:30-39).

In work leading to the invention, it was observed that therapeutic pressure on melanoma cells kills the majority of melanoma cells but leaves behind a heterogeneous population of residual melanoma tumor cells that remain viable and are resistant to the applied therapeutic pressure. By means of analysis of single-cell RNA/transcriptome sequencing data, it became apparent that no less than 4 (four) different melanoma tumor cell subpopulations can be present during the minimal residual disease (MRD) phase. This invention is based on further analysis of these four subpopulations.

Although the therapy selecting for these 4 MRD-stage subpopulations as applied in the Examples herein relies on combined inhibition of BRAF and MEK kinases, it can be envisaged that other therapeutic modalities also give rise to the emergence of the same or some of these 4 MRD-stage subpopulations. For instance, although the study by Riaz et al. 2017 (Cell 171:934-949) was not designed to assess MRD, analysis of the available RNASeq data indicated a trend for immunotherapy with the PD-1 inhibitor nivolumab to induce at least the pigmented state and MITFmedium-hypometabolic state MRD subpopulations as described hereinafter (comparing the patient subgroups SD + PD versus CR + PR; SD = stable disease, PD = progressive disease, CR = complete response, PR = partial response; results not shown). Likewise, targeted therapies of cancers other than melanoma can lead to a MRD phase during which one or more of the MRD subpopulations as identified herein for melanoma are present. Such targeted therapies include e.g. anti-VEGF (bevacizumab), anti-EGFR (cetuximab, erlotinib), mTOR inhibition (everolimus), Tyr-kinase inhibitor/anti-EGFR (gefitinib), Tyr-kinase inhibition/BCR-ABL inhibition (imatinib), anti-HER2/anti-EGFR (lapatinib), and Tyr-kinase inhibition (sorafenib, sunitinib).

On the one hand, as described above, different therapies targeting melanoma can be expected to induce all or part of the minimal residual disease tumor subpopulations as described herein. On the other hand, it can be envisaged that inhibition of the MAPK-pathway (with BRAF and/or MEK inhibitors) is inducing in cancers or tumors different from melanoma one or more minimal residual disease tumor subpopulations as described herein for melanoma. Clinical application of MAPK-inhibitors is indeed widespread in the oncology field, and includes treatment of colorectal cancer (e.g. Sanz-Garcia et al. 2017, Ann Oncol 28:2648-2657; Van Cutsem et al. 2018, Gastrointestinal Cancers Symposium, Abstract 627; Corcoran RB et al. 2015, J Clin Oncol 33:4023-4031), non-small cell lung cancer (e.g. Anguera & Majem 2018, J Thorac Dis 10:589-592), thyroid cancer (e.g. Subbiah et al. 2017, J Clin Oncol 36:7-13), cholangiocarcinoma (e.g. Lavingia et al. 2016, J Gastrointest Oncol 7:E98-E102), ameloblastoma (e.g. Abe et al. 2018, Chin J Cancer Res 30:677-678; Clinical Trial NCT02367859), glioma (e.g. Kaley et al. 2018, J Clin Oncol 36:3477-3484), glioblastoma (e.g. Ceccon et al. 2018, Int J Mol Sci 19:1090), biliary tract cancer and adenocarcinoma of the small intestine (e.g. https://www.onclive.com/web-exclusives/dabrafenib-plus-trametinib-demonstrates-activity-in-gi-cancers), neuroblastoma (e.g. Johnsen et al. 2018, Pharmacol Res 131:164-176), acute myeloid leukemia (e.g. Wander et al. 2017, Precision Oncology DOI: 10.1200/PO.16.00032), chronic myeloid leukemia (e.g. Andrews et al. 2015, Clin Cancer Res 21:5222-5234), and hairy cell leukemia (e.g. Vergote et al. 2014, Annals of Hematology 93:2087-2089).

The four MRD-stage cell subpopulations have been characterized in terms of on-treatment gene expression changes compared to a reference expression level (in this case pre-treatment gene expression in tumor cells) and based thereon "gene feature sets" or "gene expression signatures" were conceived (see Example 2.5). Increased expression of one or more of the genes of a gene expression signature or gene feature set allows, by analyzing bulk RNA, identification of patients with a tumor harboring any one of these 4 tumor cell subpopulations (Example 2.5; Figure 20).

Based hereon, and after introducing some information on melanoma and its different disease stages, the invention is defined in the following aspects and embodiments, and described in more detail hereafter. The aspects include methods for tumor analysis (allowing tumor disease stratification, in particular when progressing to, at, or during residual disease), such as for determining tumor cell heterogeneity during cancer treatment. Such methods are helpful in selecting or optimizing the tumor or cancer therapy, or in predicting responses to therapy. Knowledge on the tumor cell subpopulations in residual disease also allows for targeted screening for cytotoxic or cytostatic compounds targeting one or more of the heterogeneous tumor cell populations occurring such as during therapy. In particular, the tumor or cancer is melanoma.

### Melanoma

Melanoma, or malignant melanoma, is a cancer developing from pigment-containing cells/melanocytes. High exposure to UV-light is one of the major causes of cutaneous melanoma, the most aggressive form of skin cancer (non-melanoma skin cancers include squamous cell carcinoma and basal cell carcinoma which are rarely a cause of death). Skin melanomas are the predominant form of melanoma (95% of cases). Melanomas can, however, also develop in the mucous membranes of mouth, nose, anus, vagina, and intestine. Uveal melanomas can arise from melanocytes residing in iris, ciliary body or choroid. Histological melanoma subtypes include superficial spreading melanoma, nodular melanoma, acral lentiginous melanoma, and lentigo maligna melanoma. Patients with localized melanoma have a good prognosis upon adequate surgical excision whereas metastatic melanoma is largely resistant to current therapies, or is relatively rapidly acquiring resistance to current therapies. Melanoma incidences vary 100-fold between countries worldwide, with the highest rates in Caucasian populations. More than 80% of the estimated new cases and close to 65% of the melanoma cancer deaths occurred in Oceania, Europe, and North America. Australia and New Zealand are the countries with the highest melanoma incidence rates, being 2 times than those in any other country. The above and further information on melanoma can be found in e.g. the WHO World Cancer Report 2014, pp 495-502.

Melanosomes, the organelles within melanocytes synthesizing, storing, and/or transporting the melanin pigment, contribute to sequestration of cytotoxic drugs and melanosome-mediated drug export, a process involving e.g. the folate receptor alpha in case of the antifolate methotrexate (amethopterin; inhibitor of dihydrofolate reductase) (Saez-Ayala et al. 2012, Exp Cell Res 318:1146-1159).

### Melanoma treatment: disease stages

The residual disease stage or refractory disease stage can be defined as the stage of a disease in which clinical symptoms have disappeared or have largely disappeared (such as during treatment or after treatment) but at which a fraction of the cells/a small number of cells originating from the treated diseased cells have developed tolerance, in particular reversible tolerance, to the treatment or to the drug(s) used in the treatment, and remain viable. These viable cells can be the origin of relapse (upon ceasing treatment) or can, upon continued treatment, evolve to acquire stable resistance to the treatment or to the drug(s) used in the treatment, and cause relapse. Roughly, as illustrated in Figure 1B for melanoma, three phases or stages can be defined: a first phase during which the melanoma responds to the applied therapy and during which the number of melanoma cells is drastically reduced; a second phase apparently disease-free, but during which drug-resistant (reversible) melanoma cells are maintained; and, upon continued treatment with the same applied therapy, a third relapse phase usually accompanied by acquired (genetic) drug resistance (irreversible unless sensitizers to the applied therapy are applied). Whereas drug tolerance is a transient and reversible property of the cells originating from treated diseased cells, acquired resistance is a stable property (stably acquired resistance) gained by diseased cells upon continued treatment. The residual disease stage or refractory disease stage can alternatively be defined as the disease stage in which the cells originating from the treated diseased cells have adapted, in particular have reversibly adapted to the treatment, i.e., have obtained adaptive resistance to the treatment. Induced tolerance stage or induced drug-tolerance stage are further alternatives for describing the residual or refractory disease stage. The concept of reversible drug tolerance is described in more detail in Sharma et al. (2010, Cell 141:69-80).

The initial site where a cancer starts to develop gives rise to the primary cancer. When cancer cells break away from the primary cancer ("seed"), they can move (e.g. via blood and/or lymph fluid) to another site even remote from the initial site. If the other site allows settlement and growth of these moving cancer cells, a new cancer, called secondary cancer, can emerge ("soil"). The process leading to secondary cancer is also termed metastasis, and secondary cancers are also termed metastases. This is a further stage of melanoma disease.

An alternative way of staging melanoma is based on clinical signs. Such stages refer to thickness, depth of penetration, and the degree to which a melanoma has spread. The staging is used to determine treatment. Early melanomas (Stages 0 and I) are localized: Stage 0 tumors are in situ, meaning they are noninvasive and have not penetrated below the outer layer of the skin (the epidermis). Stage I tumors have invaded below the epidermis into the skin's next layer (the dermis), but are small and have no other traits such as ulceration that put them at high risk of spreading (metastasizing) to nearby lymph nodes or beyond. Stage II tumors, though localized, are larger (generally over 1 mm. thick) and/or may have other traits such as ulceration that put them at high risk of spreading to the nearby lymph nodes or beyond. They are considered intermediate or "high-risk" melanomas. More advanced melanomas (Stages III and IV) have metastasized to other parts of the body. There are also subdivisions within stages. (https://www.skincancer.org/skin-cancer-information/melanoma/the-stages-of-melanoma).

In one aspect, the invention therefore relates to methods for analysis of a human tumor, wherein such methods comprise determining or detecting in a biological sample from the human tumor or in a biological sample comprising human tumor nucleic acid, the expression level, or change in the expression level:
- of one or more (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, or all 37) genes selected from gene signature A1, wherein gene signature A1 consists of the genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; and/or
- of one or more (i.e. 1, 2, 3, 4, 5, 6, or all 7) genes selected from gene signature A2, wherein gene signature A2 consists of the genes NGFR, GFRA2, GFRA3, RSPO3, L1CAM, AQP1, TMEM176B; and/or
- of one or more (i.e. 1, 2, 3, 4, 5 or all 6) genes selected from gene selected from gene signature A3, wherein gene signature A3 consists of the genes NGFR, GFRA2, L1CAM, AQP1, TMEM176B, SLC22A17; and/or
- of one or more (i.e. 1, 2, 3, 4, 5, 6, 7, 8, or all 9) genes selected from gene signature B1, wherein gene signature B1 consists of the genes SLC7A8, DLX5, TRIM67, CD36, PAX3, IP6K3, UBXN10, KIAA1161, LSMEM1; and/or
- of one or more (i.e. 1, 2, 3, 4, 5, 6, 7, or all 8) genes selected from gene signature B2, wherein gene signature B2 consists of the genes CD36, IP6K3, KIAA1161, TRIM67, LSMEM1, UBXN10, PAX3, SLC7A8; and/or
- of one or more (i.e. 1, 2, 3, 4, or all 5) genes selected from gene signature B3, wherein gene signature B3 consists of the genes DLX5, CD36, IP6K3, TRIM67, PAX3; and/or
- of one or more (i.e. 1, 2, 3, 4, 5, 6, 7, 8, 9, 10, 11, 12, 13, 14, 15, 16 or all 17) genes selected from gene signature C1, wherein gene signature C1 consists of the genes SLC24A5, PMEL, FABP7, SLC45A2, KIT, EDNRB, TRPM1, APOE, MLANA, MLPH, TYRP1, GPR143, TYR, RAB27A, SNAI2, MITF, DCT; and/or
- of one or more (i.e. 1, 2, 3, 4, 5, 6, or all 7) genes selected from gene signature C2, wherein gene signature C2 consists of the genes GPR143, TYRP1, MLPH, MLANA, TRPM1, EDNRB, PMEL; and/or
- of one or more (i.e. 1, 2, 3, 4, or all 5) genes selected from gene signature C3, wherein gene signature C3 consists of the genes DCT, MITF, TYR, MLANA, TRPM1; and/or
- of one or more (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or all 52) genes selected from gene signature D1, wherein gene signature D1 consists of VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4; and/or
- of one or more (i.e. 1, 2, 3, 4, 5, or all 7) genes selected from gene signature D2, wherein gene signature D2 consists of the genes RGS5, SLIT2, AXL, BGN, TGM2, TGFBI, CYR61; and/or
- of one or more (i.e. 1, 2, 3, 4, 5 or all 6) genes selected from gene signature D3, wherein gene signature D3 consists of the genes WNT5A, AXL, TNC, TCF4, LOXL2, CYR61.

Information on the genes of the gene signatures, on determining or detecting (changes in) gene expression levels and on reference expression levels is included further hereinafter.

In one embodiment, in such methods for analysis of a human tumor, the expression level is determined or detected:
- of one or more genes selected from gene signature A1; and/or
- of one or more or more genes selected from gene signature A2; and/or
- of one or more or more genes selected from gene signature A3; and/or
- of one or more or more genes selected from gene signature B1; and/or
- of one or more or more genes selected from gene signature B2; and/or
- of one or more or more genes selected from gene signature B3.

More in particular, the expression level of 1 or more genes selected from gene signature A1, A2 or A3 may be determined. Alternatively, the expression level of one or more genes selected from gene signature B1, B2 or B3 may be determined. Alternatively, the expression level of one or more genes selected from gene signature A1 and the expression level of one or more genes selected from gene signature B1 may be determined. Alternatively, the expression level of one or more genes selected from gene signature A1 and the expression level of one or more genes selected from gene signature B2 may be determined. Alternatively, the expression level of one or more genes selected from gene signature A1 and the expression level of one or more genes selected from gene signature B3 may be determined. Alternatively, the expression level of one or more genes selected from gene signature A2 and the expression level of one or more genes selected from gene signature B1 may be determined. In particular, the expression level of one or more genes selected from gene signature A2 and the expression level of one or more genes selected from gene signature B2 may be determined. Alternatively, the expression level of one or more genes selected from gene signature A2 and the expression level of one or more genes selected from gene signature B3 may be determined. Alternatively, the expression level of one or more genes selected from gene signature A3 and the expression level of one or more genes selected from gene signature B1 may be determined. In particular, the expression level of one or more genes selected from gene signature A3 and the expression level of one or more genes selected from gene signature B2 may be determined. Alternatively, the expression level of one or more genes selected from gene signature A3 and the expression level of one or more genes selected from gene signature B3 may be determined.

Added to the above embodiments are methods further comprising determining or detecting in the biological sample from the human tumor the expression:
- of one or more genes selected from gene signature C1; and/or
- of one or more genes selected from gene signature C2; and/or
- of one or more genes selected from gene signature C3; and/or
- of one or more genes selected from gene signature D1; and/or
- of one or more genes selected from gene signature D2; and/or
- of one or more genes selected from gene signature D3.

In particular, the expression level of one or more genes selected from gene signature C1, C2 or C3 may be further determined. Alternatively, the expression level of one or more genes selected from gene signature D1, D2 or D3 may be further determined. Alternatively, the expression level of one or more genes selected from gene signature C1, C2 or C3 is further determined, and the expression level of one or more genes selected from gene signature D1, D2 or D3 is further determined.

In above methods wherein the (change in) expression level of at least one gene of gene signature A1 or at least one gene of gene signature D1 different from COL1A1 and/or VCAN is determined when the expression level of COL1A1 and/or the expression level of VCAN is determined as part of the selection of genes of gene signature A1 and of genes of gene signature D1.

In above methods wherein the (change in) expression level of AQP1 is determined (gene selected from gene signature A1, A2 or A3), then the expression level at least a second selected gene is determined, i.e. the expression level of 1 or more (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or all 36) further genes selected from ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, or SEMA3B, is further determined. In another embodiment, when the expression level of AQP1 is determined, then the expression level at least a second selected gene is determined, i.e. the expression level is determined of 1 or more (i.e. 1, 2, 3, 4, 5, or all 6) further genes selected from NGFR, GFRA2, GFRA3, RSPO3, L1CAM, or TMEM176B; of 1 or more (i.e. 1, 2, 3, 4, 5, 6, or all 7) further genes selected from NGFR, GFRA2, GFRA3, RSPO3, L1CAM, SLC22A17, or TMEM176B; or of 1 or more (i.e. 1, 2, 3, 4, or all 5) further genes selected from NGFR, GFRA2, L1CAM, SLC22A17, or TMEM176B. In further embodiments, when the expression level of either one of NGFR, GFRA2 or L1CAM is determined, then the expression level of at least a second selected gene is determine, i.e. the expression level is determined of 1 or more further gene, similarly as outlined for AQP1. In yet further embodiments, expression levels of AQP1 and NGFR, of AQP1 and GFRA2, of AQP1 and L1CAM, of NGFR and GFRA2, of NGFR and L1CAM, or of GFRA2 and L1CAM are determined, possibly in combination with determining expression levels of at least a third selected gene. In yet further embodiments, expression levels of AQP1, NGFR and GFRA2, of AQP1, NGFR and L1CAM, of AQP1, GFRA2 and L1CAM, or of NGFR, GFRA2 and L1CAM are determined, possibly in combination with determining expression levels of at least a fourth selected gene. In yet further embodiments, expression levels of AQP1, NGFR, GFRA2 and L1CAM are determined, possibly in combination with determining expression levels of at least a fifth selected gene. In yet a further embodiment, increased expression levels of 4 to 6, 4 to 7, 4 to 8, 4, 5, 6, 7 or 8 genes selected from AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, or SEMA3B is determined or detected in order to identify or detect the emergence or presence of the NDTC subpopulation.

In above methods wherein the expression level of CD36 or PAX3 is determined (gene selected from gene signature B1, B2 or B3), then the expression level at least a second selected gene is determined, i.e. the expression level of 1 or more (i.e. 1, 2, 3, 4, 5, 6, 7, or all 8) genes selected from SLC7A8, DLX5, TRIM67, PAX3, IP6K3, UBXN10, KIAA1161, and LSMEM1 (in case of CD36 as first selected gene) or selected from CD36, SLC7A8, DLX5, TRIM67, IP6K3, UBXN10, KIAA1161, and LSMEM1 (in case of PAX3 as first selected gene) is further determined. In a further embodiment, the expression levels of CD36 and PAX3 are determined, optionally in combination with determining the expression level at least a third selected gene is determined, i.e. the expression level of 1 or more (i.e. 1, 2, 3, 4, 5, 6, or all 7) genes selected from SLC7A8, DLX5, TRIM67, IP6K3, UBXN10, KIAA1161, and LSMEM1 is determined. In yet a further embodiment, increased expression levels of 4 to 6, 4 to 7, 4 to 8, 4, 5, 6, 7 or 8 genes selected from SLC7A8, DLX5, TRIM67, CD36, PAX3, IP6K3, UBXN10, KIAA1161, and LSMEM1 is determined or detected in order to identify or detect the emergence or presence of the HMTC subpopulation.

In above methods wherein identification or detection of the emergence or presence of the pigmented subpopulation is envisaged, the expression level of 1 or more genes selected from the gene signatures C1, C2, or C3 can be relied on. In a particular embodiment, increased expression levels of 4 to 6, 4 to 7, 4 to 8, 4, 5, 6, 7 or 8 genes selected from SLC24A5, PMEL, FABP7, SLC45A2, KIT, EDNRB, TRPM1, APOE, MLANA, MLPH, TYRP1, GPR143, TYR, RAB27A, SNAI2, DCT, or MITF is determined or detected in order to identify or detect the emergence or presence of the pigmented cell subpopulation.

In above methods wherein identification or detection of the emergence or presence of the invasive subpopulation is envisaged, the expression level of 1 or more genes selected from the gene signatures D1, D2, or D3 can be relied on. In a particular embodiment, increased expression levels of 4 to 6, 4 to 7, 4 to 8, 4, 5, 6, 7 or 8 genes selected from VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4 is determined or detected in order to identify or detect the emergence or presence of the invasive cell subpopulation.

In the above methods and embodiments, the tumor may have a wild-type MAPK-pathway and/or PI3K-pathway or may have a mutant MAPK-pathway and/or PI3K-pathway; or the tumor in particular has a mutation in the MAPK-pathway and/or in the PI3K-pathway. Such mutation may be further defined as a mutation in the BRAF kinase gene or a mutation in the NRAS gene. Further particularly, the mutation in the BRAF kinase gene may result in BRAF V600E, BRAF V600R or BRAF V600K mutant kinase protein, or the mutation in the NRAS gene may result in NRAS Q61K protein. In particular, the tumor is melanoma, but other tumor types treatable with MAPK-inhibitors frequently accumulate the same mutations and occurrence of NDTCs in these cancers other than melanoma therefore is also expected to be independent of their genetic background (e.g. wild-type or mutant MAPK pathway). Such cancers other than melanoma include colorectal cancer, non-small cell lung cancer, thyroid cancer, cholangiocarcinoma, ameloblastoma, glioma, glioblastoma, biliary tract cancer and adenocarcinoma of the small intestine, neuroblastoma, acute myeloid leukemia, chronic myeloid and hairy cell leukemia.

In the above methods and embodiments, the subject having the tumor may have been or may be on therapy. Such therapy may include treatment with an inhibitor of the MAPK pathway, wherein the inhibitor of the MAPK pathway may be a BRAF-inhibitor, an inhibitor of BRAF-mutant kinase, a MEK-inhibitor, an inhibitor of MEK-mutant kinase or any combination in any way of a BRAF-inhibitor and a MEK-inhibitor. In particular, the inhibitor of the MAPK pathway may be chosen from sorafenib, vemurafenib, dabrafenib, regorafenib, LY-3009120, HM95573, LXH-254, MLN2480, BeiGene-283, RXDX-105, BAL3833, encorafenib (LGX818), GDC-0879, XL281, ARQ736, PLX3603, RAF265, selumetinib, trametinib, cobimetinib, pimasertib, refametinib, binimetinib, CI-1040 (PD184352), GDC-0623, PD-0325901, and BI-847325, or a pharmaceutically acceptable salt of any thereof; or may be a compound specifically inhibiting the MAPK pathway and is chosen from an antisense oligonucleotide, a gapmer, a siRNA, a shRNA, a zinc-finger nuclease, a meganuclease, a TAL effector nuclease, a CRISPR-Cas effector, an antibody or a fragment thereof, an alpha-body, a nanobody, an intrabody, an aptamer, a DARPin, an affibody, an affitin, an anticalin, or monobody; or may be chosen from any combination of any of the foregoing.

As explained above, the knowledge of which tumor cell populations are present during residual disease/minimal residual disease (MRD) (following a positive response to a treatment) is instrumental for deciding on further therapy, and in particular for switching between treatment modalities such that the occurrence of acquired resistance to a particular therapy can be inhibited or delayed.

The above methods and their embodiments are therefore particularly useful in selecting therapy for a patient having a tumor, in optimizing therapy of a patient having a tumor, or in predicting response to therapy of a patient having a tumor. In particular, the tumor is melanoma.

In further assisting such selection of a (further) therapy, optimization of a (further) therapy or predicting response to a (further) therapy, the above methods may include one or more additional steps as outlined hereafter.

One additional step may be comparing the expression level determined in the tumor sample for a selected gene (selected from a gene expression signature) with the reference expression level of the selected gene. In particular, the methods may determine the expression level determined in the tumor sample for a selected genes to be increased compared to the reference expression level of the selected gene.

A further additional or alternative additional step may be selection of a therapy for a patient having a tumor wherein the selected therapy is normalizing the expression levels of a selected gene determined to be increased in the tumor sample compared to the reference expression level of the selected gene.

A further additional or alternative additional step may be determining the presence of one or more cell populations in the tumor, wherein:
- compared to reference expression levels, increased expression levels of the one or more genes selected from gene signature A1, from gene signature A2 or from gene signature A3 is indicative for the presence of a population of neuro-like /NDTCcells in the tumor;
- compared to reference expression levels, increased expression levels of the one or more genes selected from gene signature B1, from gene signature B2 or from gene signature B3 is indicative for the presence of a population of hypometabolic/HMTC cells in the tumor;
- compared to reference expression levels, increased expression levels of the one or more genes selected from gene signature C1, from gene signature C2 or from gene signature C3 is indicative for the presence of a population of pigmentation/differentiation cells in the tumor;
- compared to reference expression levels, increased expression levels of the one or more genes selected from gene signature D1, from gene signature D2 or from gene signature D3 is indicative for the presence of a population of invasive cells in the tumor.

With "population" is meant any number of cells that is detectable by complying with the characteristic of displaying expression of 1 or more (as indicated at least 1, and up to the maximum number dependent on the gene expression signature) of the listed genes.

A further additional or alternative additional step may be selecting a therapy for a patient having a tumor wherein the therapy is chosen from:
- a compound that is cytotoxic or cytostatic for the population of cells in the tumor with, compared to reference expression levels, increased expression levels of the one or more genes selected from gene signature A1, from gene signature A2, or from gene signature A3; and/or
- a compound that is cytotoxic or cytostatic for the population of cells in the tumor with, compared to reference expression levels, increased expression levels of the one or more genes selected from gene signature B1, from gene signature B2, or from gene signature B3; and/or
- a compound that is cytotoxic or cytostatic for the population of cells in the tumor with, compared to reference expression levels, increased expression levels of the one or more genes selected from gene signature C1, from gene signature C2, or from gene signature C3; and/or
- a compound that is cytotoxic or cytostatic for the cells in the tumor with, compared to reference expression levels, increased expression levels of the one or more genes selected from gene signature D1, from gene signature D2, or from gene signature D3.

In particular herein:
- the compound that is cytotoxic or cytostatic for the population of cells in the tumor with, compared to reference expression levels, increased expression levels of the 1 or more genes selected from gene signature A1, from gene signature A2 or from gene signature A3 may be an antagonist of retinoid X receptor, a CD36 antagonist, an antagonist of retinoid X receptor or CD36 combined with a FAK-inhibitor;
- the compound that is cytotoxic or cytostatic for the population of cells in the tumor with, compared to reference expression levels, increased expression levels of the 1 or more genes selected from gene signature B1, from gene signature B2 or from gene signature B3 may be an inhibitor of CD36 or inhibitor of PAX3;
- the compound that is cytotoxic or cytostatic for the population of cells in the tumor with, compared to reference expression levels, increased expression levels of the 1 or more genes selected from gene signature C1, from gene signature C2 or from gene signature C3 may be a CD36 inhibitor, an antifolate drug, a melanocyte-directed enzyme prodrug, an antibody drug conjugate wherein the antibody is targeting GPNMB, nelfinavir, or a combination of any thereof (e.g. a CD36 antagonist combined with nelfinavir, a CD36 inhibitor combined with an antifolate drug);
- a compound that is cytotoxic or cytostatic for the cells in the tumor with, compared to reference expression levels, increased expression levels of the 1 or more genes selected from gene signature D1, from gene signature D2 or from gene signature D3 may be an inhibitor of AXL.

It will be clear that in any of the above methods and their embodiments, the biological sample may be from a human subject having a tumor, such as melanoma, such as cutaneous melanoma. In particular, the biological sample is a tumor tissue sample is a sample comprising tumor nucleic acid(s).

In the above methods and their embodiments, the expression level may be an mRNA expression level or a protein expression level. In case of an mRNA expression level, it may be determined by RNA-sequencing, PRC, RT-PCR, gene expression profiling, serial analysis of gene expression, microarray analysis, whole genome sequencing, or is determined based on at least one of an amplification reaction, a sequencing reaction, a melting reaction, a hybridization reaction or a reverse hybridization reaction. Aspects of gene expression and function and their determination are explained hereinafter.

In particular to the above methods and their embodiments the expression level of at most 250 genes is determined. Further in particular, the expression level of at most 225, 200, 175, 150, 125, 111, 110, 105, 100, 95, 90, 85, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20,19, 18, 17, 16, 15, 14, 13, 12, 11, 10, 9, 8, 7, 6, 5 or genes is determined.

With the knowledge of the tumor cell populations present in residual disease/the residual disease stage, it becomes possible to engage in screening campaigns in order to find compounds, in particular cytotoxic or cytostatic compounds with selectivity for at least one, possibly two or more, of the populations. In fact, inhibition of CD36 was shown to inhibit or suppress at least 3 of these populations, and to enhance the 4^{th} population, which leads to a combination of e.g. a CD36 antagonist and e.g. an AXL antagonist potentially inhibiting or suppressing all 4 subpopulations.

As such, the invention further relates to methods for screening for cytotoxic or cytostatic compounds (in particular for cytotoxic or cytostatic compounds specific to one or more of the tumor cell subpopulations occurring during progression to, at, or during residual disease) such methods comprising the steps of:
- culturing tumor cells;
- applying a therapy to the cultured tumor cells, wherein the therapy induces the occurrence of one or more populations of tumor cells that are reversibly resistant to the therapy, and wherein the populations are one or more of:
   - a population with increased expression of one or more genes selected from gene signature A1, wherein gene signature A1 consists of the genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; and/or
   - a population with increased expression of one or more genes selected from gene signature A2, wherein gene signature A2 consists of the genes NGFR, GFRA2, GFRA3, RSPO3, L1CAM, AQP1, TMEM176B; and/or
   - a population with increased expression of one or more genes selected from gene selected from gene signature A3, wherein gene signature A3 consists of the genes NGFR, GFRA2, L1CAM, AQP1, TMEM176B; and/or
   - a population with increased expression of one or more genes selected from gene signature B1, wherein gene signature B1 consists of the genes SLC7A8, DLX5, TRIM67, CD36, PAX3, IP6K3, UBXN10, KIAA1161, LSMEM1; and/or
   - a population with increased expression of one or more genes selected from gene signature B2, wherein gene signature B2 consists of the genes CD36, IP6K3, KIAA1161, TRIM67, LSMEM1, UBXN10, PAX3, SLC7A8; and/or
   - a population with increased expression of one or more genes selected from gene signature B3, wherein gene signature B3 consists of the genes DLX5, CD36, IP6K3, TRIM67, PAX3; and/or
   - a population with increased expression of one or more genes selected from gene signature C1, wherein gene signature C1 consists of the genes SLC24A5, PMEL, FABP7, SLC45A2, KIT, EDNRB, TRPM1, APOE, MLANA, MLPH, TYRP1, GPR143, TYR, RAB27A, SNAI2, MITF,DCT; and/or
   - a population with increased expression of one or more genes selected from gene signature C2, wherein gene signature C2 consists of the genes GPR143, TYRP1, MLPH, MLANA, TRPM1, EDNRB, PMEL; and/or
   - a population with increased expression of one or more genes selected from gene signature C3, wherein gene signature C3 consists of the genes DCT, MITF, TYR, MLANA, TRPM1; and/or
   - a population with increased expression of one or more genes selected from gene signature D1, wherein gene signature D1 consists of VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4; and/or
   - a population with increased expression of one or more genes selected from gene signature D2, wherein gene signature D2 consists of the genes RGS5, SLIT2, AXL, BGN, TGM2, TGFBI, CYR61; and/or
   - a population with increased expression of one or more genes selected from gene signature D3, wherein gene signature D3 consists of the genes WNT5A, AXL, TNC, TCF4, LOXL2, CYR61;
   wherein the increased expression of a selected gene is determined compared to a reference expression level of the selected gene;
- contacting the one or more populations of tumor cells that are reversibly resistant to the therapy with a compound that is a candidate compound cytotoxic or cytostatic to one or more of the populations of tumor cells that are reversibly resistant to the therapy;
- identifying a compound cytotoxic or cytostatic to one or more of the populations of tumor cells that are reversibly resistant to the therapy.
The scope of "one or more genes" for which the increased expression is to be detected has been explained in detail hereinabove.

Alternative methods for screening for cytotoxic or cytostatic compounds (in particular for cytotoxic or cytostatic compounds specific to a target specific for one or more of the tumor cell subpopulations occurring during progression to, at, or during residual disease), include methods comprising the steps of:
- culturing tumor cells;
- applying a therapy to the cultured tumor cells, wherein the therapy induces the occurrence of one or more populations of tumor cells that are reversibly resistant to the therapy, and wherein the populations are one or more of:
   - a population with increased expression of one or more genes selected from gene signature A1, wherein gene signature A1 consists of the genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; and/or
   - a population with increased expression of one or more genes selected from gene signature A2, wherein gene signature A2 consists of the genes NGFR, GFRA2, GFRA3, RSPO3, L1CAM, AQP1, TMEM176B; and/or
   - a population with increased expression of one or more genes selected from gene selected from gene signature A3, wherein gene signature A3 consists of the genes NGFR, GFRA2, L1CAM, AQP1, TMEM176B; and/or
   - a population with increased expression of one or more genes selected from gene signature B1, wherein gene signature B1 consists of the genes SLC7A8, DLX5, TRIM67, CD36, PAX3, IP6K3, UBXN10, KIAA1161, LSMEM1; and/or
   - a population with increased expression of one or more genes selected from gene signature B2, wherein gene signature B2 consists of the genes CD36, IP6K3, KIAA1161, TRIM67, LSMEM1, UBXN10, PAX3, SLC7A8; and/or
   - a population with increased expression of one or more genes selected from gene signature B3, wherein gene signature B3 consists of the genes DLX5, CD36, IP6K3, TRIM67, PAX3; and/or
   - a population with increased expression of one or more genes selected from gene signature C1, wherein gene signature C1 consists of the genes SLC24A5, PMEL, FABP7, SLC45A2, KIT, EDNRB, TRPM1, APOE, MLANA, MLPH, TYRP1, GPR143, TYR, RAB27A, SNAI2, MITF, DCT; and/or
   - a population with increased expression of one or more genes selected from gene signature C2, wherein gene signature C2 consists of the genes GPR143, TYRP1, MLPH, MLANA, TRPM1, EDNRB, PMEL; and/or
   - a population with increased expression of one or more genes selected from gene signature C3, wherein gene signature C3 consists of the genes DCT, MITF, TYR, MLANA, TRPM1; and/or
   - a population with increased expression of one or more genes selected from gene signature D1, wherein gene signature D1 consists of VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4; and/or
   - a population with increased expression of one or more genes selected from gene signature D2, wherein gene signature D2 consists of the genes RGS5, SLIT2, AXL, BGN, TGM2, TGFBI, CYR61; and/or
   - a population with increased expression of one or more genes selected from gene signature D3, wherein gene signature D3 consists of the genes WNT5A, AXL, TNC, TCF4, LOXL2, CYR61; wherein the increased expression of a selected gene is determined compared to a reference expression level of the selected gene;
- contacting the one or more populations of tumor cells that are reversibly resistant to the therapy with a compound that is a candidate compound for modifying expression or function of a gene selected from any of gene signatures A1, A2, A3, B1, B2, B3, C1, C2, C3, D1, D2 or D3;
- identifying as cytotoxic or cytostatic compound a compound that is modifying expression or function a gene selected from any of gene signatures A1, A2 A3, B1, B2, B3, C1, C2, C3, D1, D2 or D3.
In particular in these methods the compound may be inhibiting, blocking, or antagonizing expression or function of a gene selected from any of gene signatures A1, A2, A3, B1, B2, B3, C1, C2, C3, D1, D2 or D3.

A further aspect of the invention relates to compounds for use in treating a tumor, in inhibiting, delaying or suppressing tumor progression, in inhibiting, delaying or suppressing tumor relapse, in inhibiting, delaying or suppressing tumor metastasis, in reducing tumor cell heterogeneity in the residual disease phase, or for use in inhibiting, delaying or suppressing acquisition of resistance to a therapy, comprising:
- determining or detecting in a biological sample from the subject having the tumor or in a biological sample comprising human tumor nucleic acids the presence of a cell population with an increased expression level:
   - of one or more genes selected from gene signature A1, wherein gene signature A1 consists of the genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; and/or
   - of one or more genes selected from gene signature A2, wherein gene signature A2 consists of the genes NGFR, GFRA2, GFRA3, RSPO3, L1CAM, AQP1, TMEM176B; and/or
   - of one or more genes selected from gene selected from gene signature A3, wherein gene signature A3 consists of the genes NGFR, GFRA2, L1CAM, AQP1, TMEM176B; and/or
   - of one or more genes selected from gene signature B1, wherein gene signature B1 consists of the genes SLC7A8, DLX5, TRIM67, CD36, PAX3, IP6K3, UBXN10, KIAA1161, LSMEM1; and/or
   - of one or more genes selected from gene signature B2, wherein gene signature B2 consists of the genes CD36, IP6K3, KIAA1161, TRIM67, LSMEM1, UBXN10, PAX3, SLC7A8; and/or
   - of one or more genes selected from gene signature B3, wherein gene signature B3 consists of the genes DLX5, CD36, IP6K3, TRIM67, PAX3; and/or
   - of one or more genes selected from gene signature C1, wherein gene signature C1 consists of the genes SLC24A5, PMEL, FABP7, SLC45A2, KIT, EDNRB, TRPM1, APOE, MLANA, MLPH, TYRP1, GPR143, TYR, RAB27A, SNAI2, MITF, DCT; and/or
   - of one or more genes selected from gene signature C2, wherein gene signature C2 consists of the genes GPR143, TYRP1, MLPH, MLANA, TRPM1, EDNRB, PMEL; and/or
   - of one or more genes selected from gene signature C3, wherein gene signature C3 consists of the genes DCT, MITF, TYR, MLANA, TRPM1; and/or
   - of one or more genes selected from gene signature D1, wherein gene signature D1 consists of VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4; and/or
   - of one or more genes selected from gene signature D2, wherein gene signature D2 consists of the genes RGS5, SLIT2, AXL, BGN, TGM2, TGFBI, CYR61; and/or
   - of one or more genes selected from gene signature D3, wherein gene signature D3 consists of the genes WNT5A, AXL, TNC, TCF4, LOXL2, CYR61;
   wherein the increased expression of a selected gene is determined compared to a reference expression level of the selected gene; and
- administering a therapeutically effective amount of a compound to the subject, wherein the compound is selected to target one or more of the populations detected to be present in the subject, and is chosen from a compound that:
   - is cytotoxic or cytostatic for the population of cells in the tumor with, compared to reference expression levels, increased expression levels of the 1 or more genes selected from gene signature A1, A2 or A3, and is chosen from compounds inhibiting, blocking, or antagonizing expression or function of a gene selected from the gene signatures A1, A2 or A3;
   - is cytotoxic or cytostatic for the population of cells in the tumor with, compared to reference expression levels, increased expression levels of the 1 or more genes selected from gene signature B1, B2 or B3, and is chosen from compounds inhibiting, blocking, or antagonizing expression or function of a gene selected from the gene signatures B1, B2 or B3;
   - is cytotoxic or cytostatic for the population of cells in the tumor with, compared to reference expression levels, increased expression levels of the 1 or more genes selected from gene signature C1, C2 or C3, and is chosen from compounds inhibiting, blocking, or antagonizing expression or function of a gene selected from the gene signatures C1, C2 or C3; and/or
   - is cytotoxic or cytostatic for the cells in the tumor with, compared to reference expression levels, increased expression levels of the 1 or more genes selected from gene signature D1, D2 or D3 , and is chosen from compounds inhibiting, blocking, or antagonizing expression or function of a gene selected from the gene signatures D1, D2 or D3.

The scope of "one or more genes" for which the increased expression is to be detected has been explained in detail hereinabove. In particular the tumor is melanoma, and the tumor cells are melanoma tumor cells.

Examples of such compounds have been included and specified hereinabove.

In particular to the final aspect, the therapy may further comprise an inhibitor of the MAPK pathway.

### Genes of gene signature A1, A2, A3

AQP1 (aliases: Aquaporin 1 (Colton Blood Group); Aquaporin 1 (Channel-Forming Integral Protein, 28kDa, CO Blood Group); Water Channel Protein For Red Blood Cells And Kidney Proximal Tubule; Urine Water Channel; Aquaporin-CHIP; CHIP28; Cotton Blood Group Antigen; AQP-CHIP, CO); NCBI reference mRNA sequences:NM_001329872.1, NM_198098.3.
ITGA1 (aliases: Integrin Subunit Alpha 1; CD49 Antigen-Like Family Member A; CD49a; Laminin And Collagen Receptor; VLA1; Very Late Activation Protein 1; Integrin Alpha 1); human mRNA sequence: NCBI reference mRNA sequences: NM_181501.1.
L1CAM (aliases: L1 Cell Adhesion Molecule; Antigen Identified By Monoclonal Antibody R1; Neural Cell Adhesion; Molecule L1; NCAM-L1;CAML1; MIC5; CD171 Antigen; CD171; HSAS1; MASA; HSAS; SPG1; S10; axonal glycoprotein belonging to the immunoglobulin supergene family): NCBI reference mRNA sequences:NM_000425.4, NM_001143963.2, NM_001278116.1, NM_024003.3.
NLGN3 (aliases: Neuroligin 3; Gliotactin Homolog; KIAA1480; HNL3; NL3); NCBI reference mRNA sequences:NM_001166660.1; NM_001321276.1; NM_018977.3; NM_181303.1; XM_006724662.3; XM_006724663.3; XM_011530974.2; XM_017029597.1
S100A4 (aliases: S100 Calcium Binding Protein A4; Placental Calcium-Binding Protein; Fibroblast-Specific Protein-1; Protein Mts1; MTS1; CAPL; Leukemia Multidrug Resistance Associated Protein; Malignant Transformation Suppression 1; Protein S100-A4; Calvasculin; Metastasin; PEL98; FSP1; 18A2; P9KA; 42A); NCBI reference mRNA sequences:NM_002961.2, NM_019554.2.
IL1RAP (aliases: Interleukin 1 Receptor Accessory Protein; IL-1 Receptor Accessory Protein; Interleukin-1 Receptor 3; IL-1RAcP; C3orf13; IL1R3; Interleukin-1 Receptor Accessory Protein Beta); NCBI reference mRNA sequences:NM_001167928.1, NM_001167929.1, NM_001167930.1, NM_001167931.1, NM_002182.3, NM_134470.3, XM_017006347.1, XM_017006348.1.
COL4A1 (aliases: Collagen Type IV Alpha 1 Chain; Collagen Of Basement Membrane, Alpha-1 Chain; Collagen IV, Alpha-1 Polypeptide; Collagen, Type IV, Alpha 1; Collagen Alpha-1(IV) Chain; COL4A1 NC1 Domain; EC 6.3.1.2; EC 3.4.23; Arresten; RATOR; BSVD); NCBI reference mRNA sequences:NM_001303110.1, NM_001845.5, XM_011521048.2.
THBS2 (aliases: Thrombospondin 2; TSP2); NCBI reference mRNA sequences: NM_003247.3.
SLITRK6 (aliases: SLIT And NTRK Like Family Member 6; SLIT And NTRK-Like Protein 6; Slit And Trk Like Gene 6; 4832410J21Rik; DFNMYP); NCBI reference mRNA sequences: NM_032229.2.
CADM1 (aliases: Cell Adhesion Molecule 1; Tumor Suppressor In Lung Cancer 1; Spermatogenic Immunoglobulin Superfamily; Immunoglobulin Superfamily Member 4; Synaptic Cell Adhesion Molecule; Nectin-Like Protein 2; Nectin-Like 2; Necl-2; SgIGSF; IGSF4A; SYNCAM; TSLC-1; IGSF4; Immunoglobulin Superfamily, Member 4D Variant 1; Immunoglobulin Superfamily, Member 4D Variant 2; TSLC1/Nectin-Like 2/IGSF4; Truncated CADM1 Protein; STSLC-1; SynCAM1; RA175; ST17; BL2); NCBI reference mRNA sequences: NM_001098517.1, NM_001301043.1, NM_001301044.1, NM_001301045.1, NM_014333.3, XM_005271494.2, XM_017017457.1, XM_017017458.1, XM_017017459.1, XM_017017460.1, XM_017017461.1.
NRXN1 (aliases: Neurexin 1; Hs.22998; KIAA0578; PTHSL2; SCZD17); NCBI reference mRNA sequences: NM_001135659.2, NM_001320156.3, NM_001320157.3, NM_001330077.1, NM_001330078.1, NM_001330079.1, NM_001330081.1, NM_001330082.1, NM_001330083.1, NM_001330084.1, NM_001330085.1, NM_001330086.1, NM_001330087.1, NM_001330088.1, NM_001330089.1, NM_001330090.1, NM_001330091.1, NM_001330092.1, NM_001330093.1, NM_001330094.1, NM_001330095.1, NM_001330096.1, NM_001330097.1, NM_004801.5, NM_138735.4, XM_005264642.3, NM_001330082, XM_006712137.3, XM_006712140.3, XM_011533167.2, NM_001330078, XM_011533172.2, NM_001330077, XM_011533175.2, XM_011533177.2, XM_011533178.2, XM_011533180.2, XM_011533183.1, XM_017005303.1, XM_017005304.1, XM_017005305.1, XM_017005306.1, XM_017005307.1, XM_017005308.1, XM_017005309.1, XM_017005310.1, XM_017005311.1, NM_001330093, NM_001330086, XM_017005314.1, XM_017005315.1, XM_017005316.1, NM_001330094, XM_017005318.1,_NM_001330085, XM_017005320.1, XM_017005321.1, XM_017005322.1, NM_001330084, XM_017005324.1, XM_017005325.1, XM_017005326.1, XM_017005327.1, NM_001330095, XM_017005329.1, NM_001330083, NM_001330088, NM_001330087, NM_001330096, XM_017005334.1, NM_001330092, NM_001330091, NM_001330097.
A2M (aliases: Alpha-2-Macroglobulin; C3 And PZP-Like Alpha-2-Macroglobulin Domain-Containing Protein 5; Alpha-2-M; CPAMD5; FWP007; S863-7; A2MD); NCBI reference mRNA sequences: NM_000014.5, NM_001347423.1, NM_001347424.1, NM_001347425.1, XM_006719056.2.
PRIMA1 (aliases: Proline Rich Membrane Anchor 1; PRIMA; Acetylcholinesterase Membrane Anchor Precursor PRiMA; Membrane Anchor Of Acetylcholinesterase); NCBI reference mRNA sequences: NM_178013.3, XM_011536456.2.
GFRA2 (aliases: GDNF Family Receptor Alpha 2; TGF-Beta-Related Neurotrophic Factor Receptor 2; Neurturin Receptor Alpha; GDNF Receptor Beta; GDNFR-Alpha-2; RET Ligand 2; NRTNR-ALPHA; GDNFR-Beta; GDNFRB; RETL2; TRNR2; Glial Cell Line Derived Neurotrophic Factor Receptor, Beta; PI-Linked Cell-Surface Accessory Protein; TRN Receptor, GPI-Anchored; GFR-Alpha 2; NTNRA); NCBI reference mRNA sequences: NM_001165038.1, NM_001165039.1, NM_001495.4, XM_006716327.3, XM_011544484.2.
MPZ (aliases: Myelin Protein Zero; Charcot-Marie-Tooth Neuropathy 1B; Myelin Peripheral Protein; MPP; Myelin Protein P0; CMTDI3; CMTDID; HMSNIB; CMT4E; CMT2I; CMT2J; CMT1B; CMT1; CHM; DSS; P0); NCBI reference mRNA sequences: NM_000530.7, NM_001315491.1, XM_017001321.1.
ADAMTS4 (aliases: ADAM Metallopeptidase With Thrombospondin Type 1 Motif 4; A Disintegrin-Like And Metalloprotease (Reprolysin Type) With Thrombospondin Type 1 Motif, 4; Aggrecanase-1; EC 3.4.24.82; ADAM-TS 4; ADAMTS-4; ADMP-1; A Disintegrin And Metalloproteinase With Thrombospondin Motifs 4; EC 3.4.24; KIAA0688); NCBI reference mRNA sequences: NM_001320336.1, NM_005099.5.
GFRA1 (aliases: GDNF Family Receptor Alpha 1; TGF-Beta-Related Neurotrophic Factor Receptor 1; GDNFR-Alpha-1; RET Ligand 1; GFR-ALPHA-1; GDNFRA; RETL1; TRNR1; Glial Cell Line-Derived Neurotrophic Factor Receptor Alpha; PI-Linked Cell-Surface Accessory Protein; GPI-Linked Anchor Protein; GDNF Receptor Alpha-1; GDNFR; RET1L); NCBI reference mRNA sequences: NM_001145453.2, NM_001348096.1, NM_001348097.1, NM_001348098.1, NM_001348099.1, NM_005264.5, NM_145793.4, NM_001348098, XM_011539634.1.
RSPO3 (aliases: R-Spondin 3; Thrombospondin Type-1 Domain-Containing Protein 2; Thrombospondin, Type I, Domain Containing 2; Protein With TSP Type-1 Repeat; Roof Plate-Specific Spondin-3; THSD2; PWTSR; R-Spondin 3 Homolog; CRISTIN1; HPWTSR; HRspo3); NCBI reference mRNA sequences: NM_032784.4, XM_017011378.1, XM_017011379.1.
GFRA3 (aliases: GDNF Family Receptor Alpha 3; GDNFR-Alpha-3; GFR-Alpha-3; Glial Cell Line-Derived Neurotrophic Factor Receptor Alpha-3; GPI-Linked Receptor; GDNFR3); NCBI reference mRNA sequences: NM_001496.3.
LAMC1 (aliases: Laminin Subunit Gamma 1; Laminin, Gamma 1 (Formerly LAMB2); Laminin-1 Subunit Gamma ; Laminin-2 Subunit Gamma; Laminin-3 Subunit Gamma; Laminin-4 Subunit Gamma; Laminin-6 Subunit Gamma; Laminin-7 Subunit Gamma; Laminin-8 Subunit Gamma; Laminin-9 Subunit Gamma; Laminin-10 Subunit Gamma; Laminin-11 Subunit Gamma; S-Laminin Subunit Gamma; Laminin B2 Chain; S-LAM Gamma; LAMB2); NCBI reference mRNA sequences: NM_002293.3.
ANXA1 (aliases: Annexin A1; Phospholipase A2 Inhibitory Protein; Chromobindin-9; Calpactin II; Calpactin-2; Annexin-1; ANX1; LPC1; Lipocortin I; P35); NCBI reference mRNA sequences:NM_000700.2, XM_011518609.1, XM_017014657.1.
SYT11 (aliases: Synaptotagmin 11; Synaptotagmin XI; SytXI; Synaptotagmin; KIAA0080; SYT12); NCBI reference mRNA sequences: NM_152280.4, XM_005245014.2, XM_017000759.1.
MATN2 (aliases: Matrilin 2; Testis Tissue Sperm-Binding Protein Li 94mP); NCBI reference mRNA sequences: NM_001317748.1, NM_002380.4, NM_030583.3, XM_005250920.1, XM_017013417.1, XM_017013418.1.
ATP1B2 (aliases: ATPase Na+/K+ Transporting Subunit Beta 2; Sodium-Potassium ATPase Subunit Beta 2 (Non-Catalytic); ATPase, Na+/K+ Transporting, Beta 2 Polypeptide; Sodium Pump Subunit Beta-2; Adhesion Molecule In Glia; AMOG; Sodium/Potassium-Transporting ATPase Beta-2 Chain; Sodium/Potassium-Dependent ATPase Beta-2 Subunit; Na, K-ATPase Beta-2 Polypeptide; Adhesion Molecule On Glia); NCBI reference mRNA sequences: NM_001303263.1, NM_001678.4.
ADGB (aliases: androglobin; Calpain-7-Like Protein; C6orf103; Chromosome 6 Open Reading Frame 103; CAPN16; CAPN7L); NCBI reference mRNA sequences: NM_024694.3, XM_006715566.3, XM_011536125.2, XM_011536126.1, XM_011536127.2, XM_011536128.2, XM_011536130.2, XM_011536131.2, XM_011536132.2, XM_011536134.2, XM_011536135.2, XM_017011314.1, XM_017011315.1).
CNN3 (aliases: Calponin 3; Calponin, Acidic Isoform; Calponin 3, Acidic; DJ639P13.2.2 (Acidic Calponin 3)); NCBI reference mRNA sequences: NM_001286055.1, NM_001286056.1, NM_001839.4, XM_017000245.1.
COL1A1 (aliases: Collagen Type I Alpha 1 Chain; Alpha-1 Type I Collagen; Collagen Of Skin, Tendon And Bone, Alpha-1 Chain; Collagen Alpha-1(I) Chain Preproprotein; Type I Procollagen Alpha 1 Chain; Collagen Alpha 1 Chain Type I; Pro-Alpha-1 Collagen Type 1; Collagen Alpha-1(I) Chain; Alpha1(I) Procollagen; Type I Proalpha 1; EDSC; OI1; OI2; OI3; OI4); NCBI reference mRNA sequences: NM_000088.3, XM_005257058.3, XM_005257059.4, XM_011524341.1.
TMEM176B (aliases: Transmembrane Protein 176B; LR8; LR8-Like Protein; Protein LR8; MS4B2): NCBI reference mRNA sequences: NM_001101311.1, NM_001101312.1, NM_001101314.1, NM_014020.3, XM_006715933.3.
PLAT (aliases: Plasminogen Activator, Tissue Type; T-Plasminogen Activator; EC 3.4.21.68; Alteplase; Reteplase; T-PA; TPA; Tissue-Type Plasminogen Activator; Plasminogen Activator, Tissue; Plasminogen/Activator Kringle; EC 3.4.21); NCBI reference mRNA sequences: NM_000930.4, NM_001319189.1, NM_033011.3.
PDGFB (aliases: Platelet Derived Growth Factor Subunit B; Becaplermin; Platelet-Derived Growth Factor Beta Polypeptide (Simian Sarcoma Viral (V-Sis) Oncogene Homolog); Platelet-Derived Growth Factor Beta Polypeptide; Platelet-Derived Growth Factor B Chain; Proto-Oncogene C-Sis; PDGF Subunit B; PDGF2; SIS; Platelet-Derived Growth Factor, Beta Polypeptide (Oncogene SIS); Platelet-Derived Growth Factor 2; PDGF, B Chain; Oncogene SIS; IBGC5; C-Sis; SSV); NCBI reference mRNA sequences: NM_002608.3, NM_033016.3.
SLC22A17 (aliases: Solute Carrier Family 22 Member 17; Neutrophil Gelatinase-Associated Lipocalin Receptor; Brain-Type Organic Cation Transporter; Lipocalin-2 Receptor; 24p3 Receptor; NGALR; 24p3R; BOCT; BOIT; Solute Carrier Family 22 (Organic Cation Transporter), Member 17; Potent Brain Type Organic Ion Transporter; Solute Carrier Family 22, Member 17; NGAL Receptor; NGALR2; NGALR3; HBOIT); NCBI reference mRNA sequences: NM_001289050.1, NM_016609.4, NM_020372.3, XM_005267747.4, XM_005267748.4, XM_017021361.1, XM_017021362.1.
ITGA6 (aliases: Integrin Subunit Alpha 6; CD49 Antigen-Like Family Member F; VLA-6; Integrin Alpha-6; Integrin Alpha6B; CD49f Antigen; ITGA6B; CD49f); NCBI reference mRNA sequences: NM_000210.3, NM_001079818.2, NM_001316306.1, XM_006712510.1, XM_006712511.1, XM_017004005.1, XM_017004006.1, XM_017004007.1, XM_017004008.1.
NGFR (aliases: Nerve Growth Factor Receptor; Low Affinity Neurotrophin Receptor P75NTR; TNFR Superfamily, Member 16; NGF Receptor; Gp80-LNGFR; TNFRSF16; P75 ICD; Nerve Growth Factor Receptor (TNFR Superfamily, Member 16); Tumor Necrosis Factor Receptor Superfamily Member 16; Low Affinity Nerve Growth Factor Receptor; CD271 Antigen; P75(NTR); P75NTR; CD271); NCBI reference mRNA sequences: NM_002507.3.
VCAN (aliases: Versican; Chondroitin Sulfate Proteoglycan Core Protein 2; Chondroitin Sulfate Proteoglycan 2; Glial Hyaluronate-Binding Protein; Large Fibroblast Proteoglycan; Versican Proteoglycan; CSPG2; GHAP; PG-M; Versican Core Protein; ERVR; WGN1; WGN); NCBI reference mRNA sequences: NM_001126336.2, NM_001164097.1, NM_001164098.1, NM_004385.4.
ATP1A2 (aliases: ATPase Na+/K+ Transporting Subunit Alpha 2; Sodium Pump Subunit Alpha-2; Sodium/Potassium-Transporting ATPase Subunit Alpha-2; Sodium-Potassium ATPase Catalytic Subunit Alpha-2; Na(+)/K(+) ATPase Alpha-2 Subunit; EC 3.6.3.9; ATPase, Na+/K+ Transporting, Alpha 2 (+) Polypeptide; Sodium/Potassium-Transporting ATPase Alpha-2 Chain; Na+/K+ ATPase, Alpha-A(+) Catalytic Polypeptide; ATPase Na+/K+ Transporting Alpha 2 Polypeptide; Na+/K+ ATPase, Alpha-B Polypeptide; Migraine, Hemiplegic 2; KIAA0778; EC 3.6.3; FHM2; MHP2); NCBI reference mRNA sequences: NM_000702.3.
IGF1 (aliases: Insulin Like Growth Factor 1; Insulin-Like Growth Factor 1 (Somatomedin C); Mechano Growth Factor; Somatomedin-C; IGF-I; MGF; Insulin-Like Growth Factor IB; Somatomedin C; IBP1; IGFI); NCBI reference mRNA sequences: NM_000618.4, NM_001111283.2, NM_001111284.1, NM_001111285.2, XM_017019259.1, NM_001111285, XM_017019261.1, XM_017019262.1, XM_017019263.1.
SEMA3B (aliases: Semaphorin 3B; Sema Domain, Immunoglobulin Domain (Ig), Short Basic Domain, Secreted, (Semaphorin) 3B; Semaphorin-V; Sema A(V); SEMAA; SEMA5; Semaphorin A; LuCa-1; Sema V; SemaV; SemA); NCBI reference mRNA sequences: NM_001005914.2, NM_001290060.1, NM_001290061.1, NM_001290062.1, NM_001290063.1, NM_004636.3.

### Genes of gene signature B1, B2, B3

SLC7A8 (aliases: Solute Carrier Family 7 Member 8; Solute Carrier Family 7 (Amino Acid Transporter Light Chain, L System), Member 8; L-Type Amino Acid Transporter 2; LAT2; Solute Carrier Family 7 (Cationic Amino Acid Transporter, Y+ System), Member 8; Solute Carrier Family 7 (Amino Acid Transporter, L-Type), Member 8; Large Neutral Amino Acids Transporter Small Subunit 2; Integral Membrane Protein E16H; LPI-PC1; HLAT2); NCBI reference mRNA sequences: NM_001267036.1, NM_001267037.1, NM_012244.3, NM_182728.2.
DLX5 (aliases: Distal-Less Homeobox 5; Distal-Less Homeo Box 5; Split Hand/Foot Malformation Type 1 With Sensorineural Hearing Loss; Homeobox Protein DLX-5; SHFM1D); NCBI reference mRNA sequences: NM_005221.5, XM_005250185.3, XM_017011803.1.
TRIM67 (aliases: Tripartite Motif Containing 67; TNL; Tripartite Motif-Containing Protein 67; TRIM9-Like Protein TNL; TRIM9-Like Protein); NCBI reference mRNA sequences: NM_001004342.3, NM_001300889.1, XM_011544192.2, XM_017001323.1.
CD36 (aliases: CD36 Molecule; CD36 Antigen (Collagen Type I Receptor, Thrombospondin Receptor); CD36 Molecule (Thrombospondin Receptor); Leukocyte Differentiation Antigen CD36; Platelet Glycoprotein IV; Fatty Acid Translocase; Glycoprotein Illb; PAS IV; GPIIIB; GP3B; GPIV; FAT; GP4; Scavenger Receptor Class B, Member 3; Platelet Collagen Receptor; Platelet Glycoprotein 4; Thrombospondin Receptor; Cluster Determinant 36; PAS-4 Protein; CD36 Antigen; BDPLT10; SCARB3; CHDS7; PASIV; PAS-4); NCBI reference mRNA sequences: NM_000072.3, NM_001001547.2, NM_001001548.2, NM_001127443.1, NM_001127444.1, NM_001289908.1, NM_001289909.1, NM_001289911.1, XM_005250713.1, XM_005250714.1, XM_005250715.4.
PAX3 (aliases: Paired Box 3; HUP2; Paired Box Gene 3 (Waardenburg Syndrome 1); Transcriptional Factor PAX3; Paired Box Homeotic Gene 3; Paired Box Protein Pax-3; Paired Domain Gene HuP2; Waardenburg Syndrome 1; Paired Domain Gene 3; CDHS; WS3; WS1); NCBI reference mRNA sequences: NM_000438.5, NM_001127366.2, NM_013942.4, NM_181457.3, NM_181458.3, NM_181459.3, NM_181460.3, NM_181461.3.
IP6K3 (aliases: Inositol Hexakisphosphate Kinase 3; Inositol Hexaphosphate Kinase 3; InsP6 Kinase 3; EC 2.7.4.21; IHPK3; ATP:1D-Myo-Inositol-Hexakisphosphate Phosphotransferase; INSP6K3); NCBI reference mRNA sequences: NM_001142883.1, NM_054111.4, XM_005248842.3, XM_005248843.3, XM_011514295.2.
UBXN10 (aliases: UBX Domain Protein 10; UBX Domain-Containing Protein 3; UBX Domain Containing 3; UBXD3; UBX Domain-Containing Protein 10); NCBI reference mRNA sequences: NM_152376.4, XM_005245742.3, XM_011540699.2.
KIAA1161 (aliases: MYORG; Myogenesis Regulating Glycosidase (Putative); Uncharacterized Family 31 Glucosidase KIAA1161; EC 3.2.1.-; NET37); NCBI reference mRNA sequences: NM_020702.4, XM_011517966.2, XM_017014930.1.
LSMEM1 (aliases: Leucine-Rich Single-Pass Membrane Protein 1; C7orf53; Chromosome 7 Open Reading Frame 53); NCBI reference mRNA sequences: NM_001134468.1, NM_182597.2, XM_011516074.1, XM_011516075.2, XM_011516076.2, XM_017012028.1.

### Genes of gene signature C1, C2, C3

SLC24A5 (aliases: Solute Carrier Family 24 Member 5; Solute Carrier Family 24 (Sodium/Potassium/Calcium Exchanger), Member 5; Oculocutaneous Albinism 6 (Autosomal Recessive); Na(+)/K(+)/Ca(2+)-Exchange Protein 5; NCKX5; JSX; Sodium/Potassium/Calcium Exchanger 5; Solute Carrier Family 24, Member 5; Ion Transporter JSX; SHEP4; OCA6); NCBI reference mRNA sequences: NM_205850.2, XM_017022079.1, XM_017022080.1.
PMEL (aliases: Premelanosome Protein; Melanocytes Lineage-Specific Antigen GP100; Melanoma-Associated ME20 Antigen; Silver Locus Protein Homolog; Melanocyte Protein Pmel 17; D12S53E; ME20-M; PMEL17; ME20M; P100; SILV; P1; Melanosomal Matrix Protein17; Silver (Mouse Homolog) Like; Melanocyte Protein Mel 17; Silver, Mouse, Homolog Of; Melanocyte Protein PMEL; Silver Homolog (Mouse); Gp100; ME20; SIL; SI); NCBI reference mRNA sequences: NM_001200053.1, NM_001200054.1, NM_001320121.1, NM_001320122.1, NM_006928.4, XM_006719569.1, XM_011538685.1.
FABP7 (aliases: Fatty Acid Binding Protein 7; Brain-Type Fatty Acid-Binding Protein; Brain Lipid-Binding Protein; B-FABP; FABPB; BLBP; MRG; Mammary-Derived Growth Inhibitor-Related; Mammary-Derived Growth Inhibitor Related; Hypothetical Protein DKFZp547J2313; Brain Lipid Binding Protein); NCBI reference mRNA sequences:NM_001319039.1, NM_001319041.1, NM_001319042.1, NM_001446.4.
SLC45A2 (aliases: Solute Carrier Family 45 Member 2; Melanoma Antigen AIM1; Protein AIM-1; AIM1; MATP; Membrane-Associated Transporter Protein; Solute Carrier Family 45, Member 2; Membrane Associated Transporter; Underwhite; SHEP5; OCA4; 1A1); NCBI reference mRNA sequences: NM_001012509.3, NM_001297417.2, NM_016180.4.
KIT (aliases: KIT Proto-Oncogene Receptor Tyrosine Kinase; V-Kit Hardy-Zuckerman 4 Feline Sarcoma Viral Oncogene Homolog; Tyrosine-Protein Kinase Kit; Piebald Trait Protein; Proto-Oncogene C-Kit; EC 2.7.10.1; P145 C-Kit; SCFR; PBT; V-Kit Hardy-Zuckerman 4 Feline Sarcoma Viral Oncogene-Like Protein; Proto-Oncogene Tyrosine-Protein Kinase Kit; Mast/Stem Cell Growth Factor Receptor Kit; Soluble KIT Variant 1; C-Kit Protooncogene; Piebald Trait; CD117 Antigen; EC 2.7.10; C-Kit; CD117); NCBI reference mRNA sequences: NM_000222.2, NM_001093772.1, XM_005265740.1, XM_005265741.1, XM_005265742.2, XM_017008178.1, XM_017008179.1, XM_017008180.1.
EDNRB (aliases: Endothelin Receptor Type B; Endothelin Receptor Non-Selective Type; ET-BR; ET-B; ETRB; Endothelin Receptor Subtype B1; ABCDS; HSCR2; ETB1; ETBR; WS4A; HSCR; ETB); NCBI reference mRNA sequences: NM_000115.4, NM_001122659.2, NM_001201397.1, NM_003991.3
TRPM1 (aliases: Transient Receptor Potential Cation Channel Subfamily M Member 1; Long Transient Receptor Potential Channel 1; Melastatin-1; LTRPC1; MLSN1; Transient Receptor Potential Melastatin Family; Melastatin 1; CSNB1C; MLSN); NCBI reference mRNA sequences: NM_001252020.1, NM_001252024.1, NM_001252030.1, NM_002420.5.
APOE (aliases: Apolipoprotein E; APO-E; Alzheimer Disease 2 (APOE*E4-Associated, Late Onset); Apolipoprotein E3; LDLCQ5; ApoE4; LPG; AD2); NCBI reference mRNA sequences: NM_000041.3, NM_001302688.1, NM_001302689.1, NM_001302690.1, NM_001302691.1.
MLANA (aliases: Melan-A; Antigen LB39-AA; Antigen SK29-AA; Protein Melan-A; MART1; Melanoma Antigen Recognized by T-Cells 1); NCBI reference mRNA sequences: NM_005511.1.
MLPH (aliases: Melanophilin; Slp Homolog Lacking C2 Domains A; Synaptotagmin-Like Protein 2a; Exophilin-3; SLAC2A); NCBI reference mRNA sequences: NM_001042467.2, NM_001281473.1, NM_001281474.1, NM_024101.6, XM_006712737.1, XM_006712739.1, XM_006712740.1, XM_011511812.1, XM_017004893.1, XM_017004894.1.
TYRP1 (aliases: Tyrosinase Related Protein 1; Melanoma Antigen Gp75; Glycoprotein 75; DHICA Oxidase; Catalase B; CAS2; TRP1; TYRP; TRP; 5,6-Dihydroxyindole-2-Carboxylic Acid Oxidase; EC 1.14.18.1; EC 1.14.18.-; EC 1.14.18; B-PROTEIN; TYRRP; CATB; GP75; OCA3); NCBI reference mRNA sequences: NM_000550.2.
GPR143 (aliases: G Protein-Coupled Receptor 143; Ocular Albinism Type 1 Protein; Ocular Albinism 1; OA1; Ocular Albinism 1 (Nettleship-Falls); NYS6); NCBI reference mRNA sequences: NM_000273.2, XM_005274541.3.
TYR (aliases: Tyrosinase; Oculocutaneous Albinism IA; Tumor Rejection Antigen AB; Monophenol Monooxygenase; EC 1.14.18.1; LB24-AB; SK29-AB; OCA1A; OCAIA; SHEP3; CMM8; OCA1; ATN); NCBI reference mRNA sequences: NM_000372.4, XM_011542970.2.
RAB27A (aliases: RAB27A, Member RAS Oncogene Family; GTP-Binding Protein Ram; Rab-27; RAB27; Mutant Ras-Related Protein Rab-27A; Ras-Related Protein Rab-27A; HsT18676; RAM; GS2); NCBI reference mRNA sequences: NM_004580.4, NM_183234.2, NM_183235.2, NM_183236.2, XM_005254576.4, XM_011521852.1, XM_011521854.1, XM_011521855.2, XM_011521856.2.
SNAI2 (aliases: Snail Family Transcriptional Repressor 2; Snail Family Zinc Finger 2; Protein Snail Homolog 2; SLUGH; SLUG; Slug Homolog, Zinc Finger Protein (Chicken); Slug (Chicken Homolog), Zinc Finger Protein; Neural Crest Transcription Factor SLUG; Neural Crest Transcription Factor Slug; Snail Homolog 2 (Drosophila); Zinc Finger Protein SNAI2; Snail Homolog 2; SLUGH1; SNAIL2; WS2D); NCBI reference mRNA sequences: NM_003068.4.
MITF (aliases: Melanocyte Inducing Transcription Factor, Microphthalmia-Associated Transcription Factor, Melanogenesis Associated Transcription Factor, Class E Basic Helix-Loop-Helix Protein 32, BHLHe32, Homolog Of Mouse Microphthalmia, Waardenburg Syndrome Type 2A, BHLHE32, COMMAD, CMM8, WS2A, WS2, MI); NCBI reference mRNA sequences: NM_000248.3, NM_001184967.1, NM_001184968.1, NM_001354604.1, NM_001354605.1, NM_001354606.1, NM_001354607.1, NM_001354608.1, NM_006722.2, NM_198158.2, NM_198159.2, NM_198177.2, NM_198178.2.
DCT (aliases: (L-)Dopachrome Tautomerase, (L-)Dopachrome (Delta-)lsomerase, Tyrosine Related Protein 2, EC 5.3.3.12, TYRP2, TRP-2, TRP2, DT, Tyrosinase Related Protein 2); NCBI reference mRNA sequences: NM_001129889.2, NM_001322182.1, NM_001322183.1, NM_001322184.1, NM_001322185.1, NM_001322186.1, NM_001922.4.
**Genes of gene signature D1, D2, D3** VCAN (aliases: Versican; Chondroitin Sulfate Proteoglycan Core Protein 2; Chondroitin Sulfate Proteoglycan 2; Glial Hyaluronate-Binding Protein; Large Fibroblast Proteoglycan; Versican Proteoglycan; CSPG2; GHAP; PG-M; Versican Core Protein; ERVR; WGN1; WGN); NCBI reference mRNA sequences: NM_001126336.2, NM_001164097.1, NM_001164098.1, NM_004385.4.
COL1A1 (aliases: Collagen Type I Alpha 1 Chain; Alpha-1 Type I Collagen; Collagen Of Skin, Tendon And Bone, Alpha-1 Chain; Collagen Alpha-1(I) Chain Preproprotein; Type I Procollagen Alpha 1 Chain; Collagen Alpha 1 Chain Type I; Pro-Alpha-1 Collagen Type 1; Collagen Alpha-1(I) Chain; Alpha1(I) Procollagen; Type I Proalpha 1; EDSC; OI1; OI2; OI3; OI4); NCBI reference mRNA sequences: NM_000088.3, XM_005257058.3, XM_005257059.4, XM_011524341.1.
TNC (aliases: Tenascin C; Glioma-Associated-Extracellular Matrix Antigen; Deafness, Autosomal Dominant 56; Hexabrachion (Tenascin); Myotendinous Antigen; Neuronectin; GP 150-225; Cytotactin; GMEM; TN-C; HXB; TN; JI; Hexabrachion (Tenascin C, Cytotactin); Tenascin-C Additional Domain 1; Tenascin-C Isoform 14/AD1/16; Hexabrachion; Tenascin-C; Tenascin; 150-225; DFNA56; GP); NCBI reference mRNA sequences: NM_002160.3, XM_005251972.3, XM_005251973.3, XM_005251974.3, XM_005251975.3, XM_006717096.3, XM_006717097.3, XM_006717098.3, XM_006717101.3, XM_011518625.2, XM_011518626.2, XM_011518628.2, XM_011518629.2, XM_017014678.1, XM_017014679.1, XM_017014680.1, XM_017014681.1.
BCAT1 (aliases: Branched Chain Amino Acid Transaminase 1; Branched Chain Aminotransferase 1, Cytosolic; EC 2.6.1.42; ECA39; BCT1; Branched-Chain-Amino-Acid Aminotransferase, Cytosolic; Branched Chain Amino-Acid Transaminase 1, Cytosolic; Placental Protein 18; Protein ECA39; PNAS121; BCAT(C); MECA39; BCATC; PP18); NCBI reference mRNA sequences: NM_001178091.1, NM_001178092.1, NM_001178093.1, NM_001178094.1, NM_005504.6, XM_017019768.1;
FOSL2 (aliases: FOS Like 2, AP-1 Transcription Factor Subunit; FOS Like Antigen 2; FRA-2; FRA2; FOS Like 2, AP-1 Transcription Factor Subunit; Fos-Related Antigen 2); NCBI reference mRNA sequences: NM_005253.3, XM_005264231.3, XM_006711976.2, XM_006711977.3, XM_017003737.1.
UNC5B (aliases: Unc-5 Netrin Receptor B; P53-Regulated Receptor For Death And Life Protein 1; Protein Unc-5 Homolog 2; Protein Unc-5 Homolog B; P53RDL1; UNC5H2; Transmembrane Receptor Unc5H2; Netrin Receptor UNC5B; Unc-5 Homolog 2; Unc-5 Homolog B); NCBI reference mRNA sequences: NM_001244889.1, NM_170744.4, XM_011539453.1, XM_017015834.1, XM_017015835.1.
CCL2 (aliases: C-C Motif Chemokine Ligand 2; Monocyte Chemotactic And Activating Factor; Monocyte Secretory Protein JE; Small Inducible Cytokine A2 (Monocyte Chemotactic Protein 1, Homologous To Mouse Sig-Je); Small Inducible Cytokine Subfamily A (Cys-Cys), Member 2; Monocyte Chemoattractant Protein-1; Chemokine (C-C Motif) Ligand 2; Monocyte Chemotactic Protein 1; Small-Inducible Cytokine A2; SCYA2; MCP-1; MCAF; HC11; MCP1; Homologous To Mouse Sig-Je; C-C Motif Chemokine 2; HSMCR30; GDCF-2; SMC-CF); NCBI reference mRNA sequences: : NM_002982.3.
SH2B3 (aliases: SH2B Adaptor Protein 3; Lymphocyte-Specific Adapter Protein Lnk; Signal Transduction Protein Lnk; LNK; Lymphocyte Adaptor Protein; Lymphocyte Adapter Protein; SH2B Adapter Protein 3; IDDM20); NCBI reference mRNA sequences: NM_001291424.1, NM_005475.2, XM_005253818.4, XM_005253819.4, XM_006719180.3, XM_011537719.2, XM_011537720.2, XM_011537721.2.
MGP (aliases: Matrix Gla Protein; Cell Growth-Inhibiting Gene 36 Protein; MGLAP; GIG36; NTI); NCBI reference mRNA sequences: NM_000900.4, NM_001190839.2.
VEGFA (aliases: Vascular Endothelial Growth Factor A; Vascular Permeability Factor; VEGF; VPF; Vascular Endothelial Growth Factor A121; Vascular Endothelial Growth Factor A165; Vascular Endothelial Growth Factor; VEGF-A; MVCD1); NCBI reference mRNA sequences: NM_001025366.2, NM_001025367.2, NM_001025368.2, NM_001025369.2, NM_001025370.2, NM_001033756.2, NM_001171622.1, NM_001171623.1, NM_001171624.1, NM_001171625.1, NM_001171626.1, NM_001171627.1, NM_001171628.1, NM_001171629.1, NM_001171630.1, NM_001204384.1, NM_001204385.1, NM_001287044.1, NM_001317010.1, NM_003376.5.
LOX (aliases: Lysyl Oxidase; EC 1.4.3.13; Protein-Lysine 6-Oxidase; AAT10); NCBI reference mRNA sequences:NM_001178102.2, NM_001317073.1, NM_002317.6.
FGF1 (aliases: Fibroblast Growth Factor 1; Heparin-Binding Growth Factor 1; Endothelial Cell Growth Factor, Alpha; Endothelial Cell Growth Factor, Beta; Fibroblast Growth Factor 1 (Acidic); HBGF-1; FGF-1; AFGF; FGFA; ECGF; Beta-Endothelial Cell Growth Factor; Acidic Fibroblast Growth Factor; ECGF-Beta; FGF-Alpha; GLIO703; ECGFA; ECGFB; HBGF1); NCBI reference mRNA sequences: NM_000800.4, NM_001144892.2, NM_001144934.1, NM_001144935.1, NM_001257205.1, NM_001257206.1, NM_001257207.1, NM_001257208.1, NM_001257209.1, NM_001257210.1, NM_001257211.1, NM_001257212.1, NM_001354955.1, NM_001354956.1, NM_001354957.1, NM_001354958.1, NM_001354959.1, NM_001354961.1, NM_001354963.1, NM_001354964.1, NM_033136.3, NM_033137.3, XM_005268390.4, NM_001354958.
PDGFRB (aliases: Platelet Derived Growth Factor Receptor Beta; Beta-Type Platelet-Derived Growth Factor Receptor; Platelet-Derived Growth Factor Receptor 1; CD140 Antigen-Like Family Member B; EC 2.7.10.1; PDGFR-Beta; PDGFR-1; PDGFR1; PDGFR; Beta Platelet-Derived Growth Factor Receptor; Activated Tyrosine Kinase PDGFRB; CD140b Antigen; NDEL1-PDGFRB; EC 2.7.10; CD140B; IBGC4; JTK12; PENTT; IMF1; KOGS); NCBI reference mRNA sequences: NM_001355016.1, NM_001355017.1, NM_002609.3, NM_001355016, XM_011537658.1, XM_011537659.1.
IGFBP5 (aliases: Insulin Like Growth Factor Binding Protein 5; IGF-Binding Protein 5; IGFBP-5; IBP-5; IBP5); NCBI reference mRNA sequences: NM_000599.3.
ERRFI1 (aliases: ERBB Receptor Feedback Inhibitor 1; Mitogen-Inducible Gene 6 Protein; MIG6; Receptor-Associated Late Transducer; GENE-33; RALT); NCBI reference mRNA sequences: NM_018948.3, XM_005263477.2, XM_006710697.2, XM_011541596.2.
PRDX1 (aliases: Peroxiredoxin 1; Thioredoxin-Dependent Peroxide Reductase 2; Natural Killer Cell-Enhancing Factor A; Proliferation-Associated Gene Protein; Thioredoxin Peroxidase 2; EC 1.11.1.15; NKEF-A; TDPX2; PAGA; PAGB; PAG; Natural Killer-Enhancing Factor A; Proliferation-Associated Gene A; EC 1.11.1; MSP23; NKEFA; PRX1; PRXI); NCBI reference mRNA sequences: NM_001202431.1, NM_002574.3, NM_181696.2, NM_181697.2.
TGFBI (aliases: Transforming Growth Factor Beta Induced; RGD-Containing Collagen-Associated Protein; Kerato-Epithelin; Beta Ig-H3; RGD-CAP; BIGH3; Transforming Growth Factor-Beta-Induced Protein Ig-H3; Transforming Growth Factor Beta-Induced 68kDa; Betaig-H3; CDGG1; CDB1; CDG2; EBMD; CSD1; CSD2; CSD3; LCD1; CSD); NCBI reference mRNA sequences: NM_000358.2.
IL13RA2 (aliases: Interleukin 13 Receptor Subunit Alpha 2; Interleukin 13 Receptor, Alpha 2; IL-13 Receptor Subunit Alpha-2; Cancer/Testis Antigen 19; IL-13R Subunit Alpha-2; IL-13R-Alpha-2; IL-13RA2; Interleukin 13 Receptor Alpha 2 Chain; Interleukin 13 Binding Protein; CD213a2 Antigen; CD213A2; IL-13R; IL13BP; IL13R; CT19); NCBI reference mRNA sequences: NM_000640.2.
SOX4 (aliases: SRY (Sex Determining Region Y)-Box 4; Ecotropic Viral Integration Site 16; SRY-Related HMG-Box Gene 4; Transcription Factor SOX-4; SRY Box 4; EVI16); *REFSEQ mRNA* :NM_003107.2.
NES (aliases: Nestin; Nbla00170); NCBI reference mRNA sequences: NM_006617.1.
LOXL2 (aliases: Lysyl Oxidase Like 2; Lysyl Oxidase-Related Protein WS9-14; Lysyl Oxidase-Related Protein 2; Lysyl Oxidase-Like Protein 2; Lysyl Oxidase-Like 2 Delta E13; Lysyl Oxidase-Like 2 Protein; Lysyl Oxidase Homolog 2; Lysyl Oxidase Related 2; EC 1.4.3.13; EC 1.4.3; WS9-14; LOR2; LOR); NCBI reference mRNA sequences: NM_002318.2.
SPRY2 (aliases: Sprouty RTK Signaling Antagonist 2; Sprouty (Drosophila) Homolog 2; Sprouty Homolog 2 (Drosophila); Protein Sprouty Homolog 2; HSPRY2; Spry-2; IGAN3); NCBI reference mRNA sequences: NM_001318536.1, NM_001318537.1, NM_001318538.1, NM_005842.3.
CDH13 (aliases: Cadherin 13; T-Cadherin; H-Cadherin (Heart); Heart Cadherin; T-Cad; CDHH; P105; Cadherin 13, H-Cadherin (Heart); Truncated Cadherin); NCBI reference mRNA sequences: NM_001220488.1, NM_001220489.1, NM_001220490.1, NM_001220491.1, NM_001220492.1, NM_001257.4, XM_011522804.2, XM_017022848.1, XM_017022849.1.
LMO4 (aliases: LIM Domain Only 4; LIM Domain Only Protein 4; Breast Tumor Autoantigen; LMO-4; LIM Domain Transcription Factor LMO4; LIM-Only 4 Protein); NCBI reference mRNA sequences: NM_006769.3, XM_005271291.3.
RGS5 (aliases: Regulator Of G Protein Signaling 5; MSTP032; MSTP092; MSTP106; MSTP129; MST092; MST106; MST129); NCBI reference mRNA sequences: NM_001195303.2, NM_001254748.1, NM_001254749.1, NM_003617.3.
RGS16 (aliases: Regulator Of G Protein Signaling 16; Retinally Abundant Regulator Of G-Protein Signaling; Retinal-Specific RGS; A28-RGS14P; HRGS-R; RGS-R; A28-RGS14; RGSR); NCBI reference mRNA sequences: NM_002928.3.
DLX1 (aliases: Distal-Less Homeobox 1); NCBI reference mRNA sequences: NM_001038493.1, NM_178120.4.
SLIT2 (aliases: Slit Guidance Ligand 2; Slit-2; SLIL3; Slit (Drosophila) Homolog 2; Slit Homolog 2 (Drosophila); Slit Homolog 2 Protein); NCBI reference mRNA sequences: NM_001289135.2, NM_001289136.2, NM_004787.3, XM_005248211.3, XM_006713986.3, XM_011513909.2, XM_011513910.2, XM_017008845.1.
GPC3 (aliases: Glypican 3; Intestinal Protein OCI-5; Glypican Proteoglycan 3; GTR2-2; MXR7; Heparan Sulphate Proteoglycan; Secreted Glypican-3; SGBS1; DGSX; SGBS; SDYS; OCI5; SGB); NCBI reference mRNA sequences: NM_001164617.1, NM_001164618.1, NM_001164619.1, NM_004484.3, XM_017029413.1.
ADM (aliases: Adrenomedullin; AM; Proadrenomedullin N-20 Terminal Peptide; Preproadrenomedullin; PAMP); NCBI reference mRNA sequences: NM_001124.2.
EDNRA (aliases: Endothelin Receptor Type A; HET-AR; ETA-R; ET-A; ETRA; ETA; Endothelin-1-Specific Receptor; Endothelin Receptor Subtype A; G Protein-Coupled Receptor; Endothelin-1 Receptor; ETAR; MFDA); NCBI reference mRNA sequences: NM_001166055.1, NM_001354797.1, NM_001957.3, NM_001256283.1.
CYSLTR2 (aliases: Cysteinyl Leukotriene Receptor 2; G-Protein Coupled Receptor GPCR21; G-Protein Coupled Receptor HG57; CYSLT2R; HGPCR21; CYSLT2; HPN321; Cysteinyl Leukotriene CysLT2 Receptor; PSEC0146; KPG_011; CysLTR2; GPCR21; HG57); NCBI reference mRNA sequences: NM_001308465.2, NM_001308467.2, NM_001308468.2, NM_001308469.2, NM_001308470.2, NM_001308471.2, NM_001308476.2, NM_020377.4.
DDAH1 (aliases: Dimethylarginine Dimethylaminohydrolase 1; Dimethylargininase-1; EC 3.5.3.18; DDAH-1; DDAHI; DDAH; N(G),N(G)-Dimethylarginine Dimethylaminohydrolase 1; NG, NG-Dimethylarginine Dimethylaminohydrolase; Epididymis Secretory Protein Li 16; HEL-S-16); NCBI reference mRNA sequences: NM_001134445.1, NM_001330655.1, NM_012137.3, XM_005270707.2, XM_005270710.2, XM_011541158.1, XM_017000889.1, NM_001330655.
PLXDC1 (aliases: Plexin Domain Containing 1; Tumor Endothelial Marker 3; Tumor Endothelial Marker 7; TEM3; TEM7; Tumor Endothelial Marker 7 Precursor; Plexin Domain-Containing Protein 1; 2410003I07Rik); NCBI reference mRNA sequences: NM_020405.4.
VSNL1 (aliases: Visinin Like 1; Hippocalcin-Like Protein 3; VILIP; VLP-1; HLP3; Visinin-Like Protein 1; HUVISL1; VILIP-1; HPCAL3; VISL1); NCBI reference mRNA sequences: NM_003385.4.
COL1A2 (aliases: Collagen Type I Alpha 2 Chain; Collagen Of Skin, Tendon And Bone, Alpha-2 Chain; Collagen I, Alpha-2 Polypeptide; Alpha-2 Type I Collagen; Alpha 2(I)-Collagen; Type I Procollagen; Osteogenesis Imperfecta Type IV; Alpha 2 Type I Procollagen; Collagen Alpha-2(I) Chain; Alpha-2 Collagen Type I; Collagen Type I Alpha 2; Alpha 2(I) Procollagen; OI4); NCBI reference mRNA sequences: NM_000089.3.
DLC1 (aliases: DLC1 Rho GTPase Activating Protein; StAR-Related Lipid Transfer (START) Domain Containing 12; Rho-Type GTPase-Activating Protein 7; START Domain-Containing Protein 12; Deleted In Liver Cancer 1 Protein; ARHGAP7; STARD12; StAR-Related Lipid Transfer Protein 12; Deleted In Liver Cancer 1 Variant 2; Deleted In Liver Cancer Variant 4; Rho GTPase-Activating Protein 7; Deleted In Liver Cancer 1; P122-RhoGAP; HP Protein; KIAA1723; DLC-1; HP); NCBI reference mRNA sequences: NM_001164271.1, NM_001316668.1, NM_001348081.1, NM_001348082.1, NM_001348083.1, NM_001348084.1; NM_006094.4; NM_024767.3; NM_182643.2; XM_005273374.1; NM_001348081; NM_001348083.
AXL (aliases: AXL Receptor Tyrosine Kinase; AXL Oncogene; EC 2.7.10.1; UFO; Tyrosine-Protein Kinase Receptor UFO; AXL Transforming Sequence/Gene; EC 2.7.10; JTK11; Tyro7; ARK); NCBI reference mRNA sequences: NM_001278599.1, NM_001699.5, NM_021913.4.
ANGPTL4 (aliases: Angiopoietin Like 4; Hepatic Fibrinogen/Angiopoietin-Related Protein; Peroxisome Proliferator-Activated Receptor (PPAR) Gamma Induced Angiopoietin-Related Protein; Hepatic Angiopoietin-Related Protein; PPARG Angiopoietin Related Protein; Fasting-Induced Adipose Factor; Angiopoietin-Related Protein 4; HFARP; Arp4; PGAR; Angiopoietin-Like Protein 4; UNQ171; Pp1158; TGQTL; HARP; FIAF; NL2); NCBI reference mRNA sequences: NM_001039667.2, NM_139314.2, NM_016109.2, XM_005272484.3, XM_005272485.3.
IGFBP6 (aliases: Insulin Like Growth Factor Binding Protein 6; IGFBP-6; IBP-6; IBP6; IGF Binding Protein 6); NCBI reference mRNA sequences: NM_002178.2, XM_017019264.1.
COL3A1 (aliases: Collagen Type III Alpha 1 Chain; Ehlers-Danlos Syndrome Type IV, Autosomal Dominant; Collagen, Type III, Alpha 1; Collagen Alpha-1(III) Chain; Alpha-1 Type III Collagen; Alpha1 (III) Collagen; Collagen, Fetal; EDS4A); NCBI reference mRNA sequences: NM_000090.3.
FABP4 (aliases: Fatty Acid Binding Protein 4; Adipocyte-Type Fatty Acid-Binding Protein; Adipocyte Fatty Acid Binding Protein; Adipocyte Lipid-Binding Protein; A-FABP; AFABP; ALBP; Fatty Acid Binding Protein 4, Adipocyte; Fatty Acid-Binding Protein, Adipocyte; Epididymis Secretory Protein Li 104; HEL-S-104; AP2); NCBI reference mRNA sequences: NM_001442.2.
CDH2 (aliases: Cadherin 2; Cadherin 2, Type 1, N-Cadherin (Neuronal); Neural Cadherin; N-Cadherin; CDw325; NCAD; CDHN; Calcium-Dependent Adhesion Protein, Neuronal; CD325 Antigen; N-Cadherin 1; CD325); NCBI reference mRNA sequences: NM_001308176.1, NM_001792.4, XM_011525788.1, XM_017025514.1.
PTGER4 (aliases: Prostaglandin E Receptor 4; Prostaglandin E Receptor 4 (Subtype EP4); PGE2 Receptor EP4 Subtype; Prostanoid EP4 Receptor; Prostaglandin E2 Receptor EP4 Subtype; PGE Receptor, EP4 Subtype; PGE Receptor EP4 Subtype; PTGER2; EP4R; EP4); NCBI reference mRNA sequences: NM_000958.2, XM_017009656.1, XM_017009657.1, XM_017009658.1, XM_017009659.1.
NDNF (aliases: Neuron Derived Neurotrophic Factor; C4orf31; Fibronectin Type-III Domain-Containing Protein C4orf31; Chromosome 4 Open Reading Frame 31; Protein NDNF; NORD); NCBI reference mRNA sequences: NM_024574.3.
NR2F1 (aliases: Nuclear Receptor Subfamily 2 Group F Member 1; V-ErbA-Related Protein 3; COUP-TF1; TFCOUP1; ERBAL3; EAR-3; EAR3; Transcription Factor COUP 1 (Chicken Ovalbumin Upstream Promoter 1, V-Erb-A Homolog-Like 3); Chicken Ovalbumin Upstream Promoter-Transcription Factor I; COUP Transcription Factor 1; COUP-TFI; TCFCOUP1; BBSOAS; BBOAS; NR2F2; SVP44); NCBI reference mRNA sequences: NM_005654.5, XM_017009797.1.
BGN (aliases: Biglycan; SLRR1A; PG-S1; Dermatan Sulphate Proteoglycan I; Small Leucine-Rich Protein 1A; Bone/Cartilage Proteoglycan I; Biglycan Proteoglycan; DSPG1; SEMDX; MRLS; PGI); NCBI reference mRNA sequences: NM_001711.5, XM_017029724.1.
TGM2 (aliases: Transglutaminase 2; C Polypeptide, Protein-Glutamine-Gamma-Glutamyltransferase; Tissue Transglutaminase; Transglutaminase C; Transglutaminase H; EC 2.3.2.13; TGase C; TGase H; TGase-2; TG(C); TGC; Transglutaminase 2 (C Polypeptide, Protein-Glutamine-Gamma-Glutamyltransferase); Protein-Glutamine Gamma-Glutamyltransferase 2; Transglutaminase-2); NCBI reference mRNA sequences: NM_001323316.1, NM_001323317.1, NM_001323318.1, NM_004613.3, NM_198951.2, XM_011529028.1.
TMSB4X (aliases: Thymosin Beta 4, X-Linked; Thymosin, Beta 4, X Chromosome; T Beta-4; TMSB4; TB4X; FX; Prothymosin Beta-4; Thymosin Beta-4; PTMB4; THYB4); NCBI reference mRNA sequences: NM_021109.3.
CYR61 (aliases: Cysteine Rich Angiogenic Inducer 61; Insulin-Like Growth Factor-Binding Protein 10; IGF-Binding Protein 10; CCN Family Member 1; IGFBP10; IBP-10; CCN1; GIG1; Cysteine-Rich Heparin-Binding Protein 61; Protein CYR61; Protein GIG1); NCBI reference mRNA sequences: NM_001554.4.
WNT5A (aliases: Wnt Family Member 5A, Wingless-Type MMTV Integration Site Family Member 5A, Protein Wnt-5a, WNT-5A Protein, Epididymis Secretory Sperm Binding Protein, HWNT5A); NCBI reference mRNA sequences: NM_001256105.1, NM_003392.4.
TCF4 (aliases: Transcription Factor 4, Class B Basic Helix-Loop-Helix Protein 19, Immunoglobulin Transcription Factor 2, SL3-3 Enhancer Factor 2, BHLHb19, ITF2, SEF2, FECD3, E2-2, PTHS); NCBI reference mRNA sequences: NM_001083962.1, NM_001243226.2, NM_001243227.1, NM_001243228.1, NM_001243230.1, NM_001243231.1, NM_001243232.1, NM_001243233.1, NM_001243234.1, NM_001243235.1, NM_001243236.1, NM_001306207.1, NM_001306208.1, NM_001330604.2, NM_001330605.2, NM_001348211.1, NM_001348212.1, NM_001348213.1, NM_001348214.1, NM_001348215.1, NM_001348216.1, NM_001348217.1, NM_001348218.1, NM_001348219.1, NM_001348220.1, NM_003199.2.

### Activation/inactivation of a target

This section applies to all targets herein described, such as, but not limited to RXR, MAPK pathway components such as BRAF, CRAF, MEK and ERK, FAK, AXL, CD36 and so on.

The term "antagonist of a target" as used herein refers to inhibitors of function, inhibitors of expression and inhibitors of upstream or downstream signaling pathway (components) of the target. Antagonists of a target may also be compounds binding to a target (e.g. tumor) cell and causing its killing; examples of such antagonists include e.g. antibody-(cytotoxic) drug-conjugates or antibodies capable of causing ADCC. Activation of effector immune cells such as natural killer cells (NKs), neutrophils, macrophages or eosinophils can lead to the process of antibody-dependent cellular cytotoxicity or antibody-dependent cell-mediated cytotoxicity (abbreviated as ADCC). Activation of Fc-receptors on effector immune cells by antibodies (e.g. IgGs in case of NK cells, IgEs in case of eosinophils) results in lysis of the target cells (coated with antibodies binding to (a) target cell antigen(s)). It was in fact demonstrated that the therapeutic potential of blockbuster antibodies such as trastuzumab and rituximab depends at least in part from ADCC as tumors in mice with myeloid cells deficient in activation antibody receptors (FcyRIII) were much less susceptible to antibody therapy than mice with myeloid cells deficient in inhibitory (FcyRIIB) antibody receptors (Clynes et al. 2000, Nature Med 6:443-446). Interchangeable alternatives for "antagonist" include inhibitor, repressor, suppressor, inactivator, and blocker.

The term "agonist of a target" as used herein refers to enhancers of function, enhancers of expression and enhancers of upstream or downstream signaling pathway (components) of the target. Interchangeable alternatives for "agonist" include enhancer, stimulator, promoter, and activator.

An antagonist of expression of a target is referring to a compound negatively influencing or decreasing expression of the target at the mRNA and/or protein level. An antagonist of the function of a target is referring to a compound negatively influencing or decreasing stability and/or a biological function of the target protein. Herein, the antagonist can act directly, meaning that the inactivation of expression of the target is through an event at the level of the target gene itself (inactivation of gene expression or transcription) or at the level of the target mRNA (inactivation of protein expression or translation); or meaning that the inactivation of the function of target is through an event at the level of the target protein (e.g. protein destabilization, post-translational modification, intracellular trafficking) itself. In case of indirect inactivation, the inactivation signal may act e.g. upstream of a direct inactivation signal, but eventually leads to the direct inactivation signal - indirect inactivation in other words leads through an intermediate event not at the level of the target gene, mRNA or protein itself to direct inactivation of the target gene, mRNA or protein. An agonist obviously has the opposite effect of an antagonist (in the above: positively influencing instead of negatively influencing; increasing instead of decreasing; activation instead of inactivation; stabilization instead of destabilization, etc.).

In relation to (in)activation or (in)activation of any target or protein herein described, the (in) activation or (ant)agonistic effect can be transient, inducible (or alternatively conditional), or can be transient after induction (or alternatively transient after conditional start of the (in)activation). The (in)activation of target can be triggered by a pharmacologic or pharmaceutical compound (such as a small molecule, (in)organic molecule, (in)organic compound), a biopharmacologic or biopharmaceutical compound (such as a peptide or (poly)protein, modified peptide or (poly)proteins, antibody and their fragments and the like), or by a nucleic acid or nucleic acid comprising compound or alternatively a gene therapeutic compound or alternatively by gene therapy or nucleic acid therapy (used interchangeably); or by any combination of 2 or more of a pharmacologic compound, biopharmacologic compound, and nucleic acid therapy.

A single administration of a pharmacologic compound in general leads to a transient effect due to its gradual removal from the cell, organ and/or body and is reflected in the pharmacokinetic/-dynamic behavior of the compound. Depending on the desired level of (in)activation, two or more (multiple) administrations of the pharmacologic compound may be required. (In)activation by gene or nucleic acid therapy or by a gene therapeutic compound (nucleic acid or nucleic acid comprising compound) can be inducible when controlled by a promoter responsive to a to be administered signal not normally present in the target cell, -organ, or -body. As such, the (in)activation by gene or nucleic acid therapy may be transient (e.g. upon removal or disappearance of the administered signal from the target cell, -organ, or -body). In case of a nucleic acid or nucleic acid comprising compound degrading once inside the target cell, -organ, or -body (e.g. in case when not integrated in the genome), the effect of the compound generally is transient.

An "antagonist" thus refers to a molecule that decreases, blocks, inhibits, abrogates, or interferes with target expression, activation or function, whereas an "agonist" refers to a molecule that increases, promotes, enhances, or stimulates target expression, activation or function. In a particular embodiment for an (ant)agonist being a pharmaceutical or biopharmaceutical compound, an (ant)agonist has a binding affinity (dissociation constant) to its target of about 1000 nM or less, a binding affinity to target of about 100 nM or less, a binding affinity to target of about 50 nM or less, a binding affinity to target, of about 10 nM or less, or a binding affinity to target of about 1 nM or less.

In a particular embodiment, an antagonist inhibits target signaling or function with an IC50 of 1000 nM or less, with an IC50 of 500 nM or less, with an IC50 of 100 nM or less, with an IC50 of 50 nM or less, with an IC50 of 10 nM or less, or with an IC50 of 1 nM or less.

In a particular embodiment, an agonist enhances target signaling or function with an IC50 of 1000 nM or less, with an IC50 of 500 nM or less, with an IC50 of 100 nM or less, with an IC50 of 50 nM or less, with an IC50 of 10 nM or less, or with an IC50 of 1 nM or less.

Downregulating of expression of a gene encoding a target is feasible through gene therapy (e.g., by administering siRNA, shRNA or antisense oligonucleotides to the target gene). Biopharmaceutical and gene therapeutic antagonists include such entities as antisense oligonucleotides, gapmers, siRNA, shRNA, zinc-finger nucleases, meganucleases, TAL effector nucleases, CRISPR-Cas effectors, antibodies or fragments thereof, alpha-bodies, nanobodies, intrabodies, aptamers, DARPins, affibodies, affitins, anticalins, and monobodies (general description of these compounds included hereinafter). In some instances such entities can have agonist activity instead of antagonist activity.

Inactivation of a process as envisaged in the current invention refers to different possible levels of inactivation, e.g., at least 10%, at least 20%, at least 30%, at least 40%, at least 50%, at least 60%, at least 70%, at least 80%, at least 90%, at least 95%, or even 100% or more if inactivation (compared to a normal situation). The nature of the inactivating compound is not vital/essential to the invention as long as the process envisaged is inactivated such as to treat or inhibit tumor growth or such as to inhibit relapse of tumor growth or such as to reduce tumor cell heterogeneity at or during residual disease.

Activation of a process as envisaged in the current invention refers to different possible levels of activation, e.g., at least 10%, at least 20%, at least 30%, at least 40%, at least 50%, at least 60%, at least 70%, at least 80%, at least 90%, at least 95%, or even 100% (200%, 300% or more) of activation (compared to a normal situation). The nature of the activating compound is not vital/essential to the invention as long as the process envisaged is activated such as to treat or inhibit tumor growth or such as to inhibit relapse of tumor growth or such as to reduce tumor cell heterogeneity at or during residual disease.

### CD36

CD36 (aliases: CD36 Molecule; CD36 Antigen (Collagen Type I Receptor, Thrombospondin Receptor); CD36 Molecule (Thrombospondin Receptor); Leukocyte Differentiation Antigen CD36; Platelet Glycoprotein IV; Fatty Acid Translocase; Glycoprotein Illb; PAS IV; GPIIIB; GP3B; GPIV; FAT; GP4; Scavenger Receptor Class B, Member 3; Platelet Collagen Receptor; Platelet Glycoprotein 4; Thrombospondin Receptor; Cluster Determinant 36; PAS-4 Protein; CD36 Antigen; BDPLT10; SCARB3; CHDS7; PASIV; PAS-4); NCBI reference mRNA sequences include: NM_000072.3, NM_001001547.2, NM_001001548.2, NM_001127443.1, NM_001127444.1, NM_001289908.1, NM_001289909.1, NM_001289911.1, XM_005250713.1, XM_005250714.1, XM_005250715.4.

CD36 is implicated in a wide variety of normal and abnormal biological functions, including angiogenesis, atherosclerosis, phagocytosis, inflammation, lipid metabolism, and removal of apoptotic cells (Febbraio et al. 2001, J Clin Invest 108:785-791). The anti-CD36 monoclonal antibody (mAb) against the oxLDL binding site (clone JC63.1 IgA mouse; Cayman Chemical, Ann Arbor, MI) or the anti-CD36mAb against the TSP-1-binding site (clone FA6-152 IgG1 mouse; Beckman Coulter, Fullerton, CA) were shown to exacerbate inflammatory corneal neovascularization (Mwaikombo et al. 2006, Invest Ophthalmol Vis Sci 47:4356-4364). Anti-CD36 clone JC63.1 was reported to inhibit metastasis of oral squamous cell carcinoma (Pascual et al. 2017, Nature 541:41-45). The apolipoprotein Al-mimetic peptide 5A was shown to be an inhibitor of CD36. Peptide 5A is a 37-residue amphipathic peptide with amino acid sequence DWLKAFYDKVAEKLKEAF-P-DWAKAAYDKAAEKAKEAA (SEQ ID NO:1) two amphipathic helices separated by a proline). (Souza et al. 2016, Kidney Int 89:809-822). Small molecule/pharmacologic inhibitors of CD36 include AP5055 and AP5258 (Geloen et al. 2012, PLoS ONE 7:e37633; WO 2011/073165) and further salvianolic acid B (lithospermic acid B or tanshinoate B; Bao et al. 2012, Atherosclerosis 223:152-159), sodium danshensu (DSS) and rosmarinic acid (RA) (Wang et al. 2010, J Biomol Screening 15:239-250), 3-cinnamoyl indole and 13 pentyl berberine (Xu et al. 2010, Anal Biochem 400: 207-212), Sulfo-N-succinimidyl oleate (Kuda et al. 2013, J Biol Chem 288:15547-15555), hexarelin or EP80317 (Marleau et al. 2005, FASEB J 19:1869-1871; Avallone et al. 2006, Mol Endocrinol 20:3165-3178), and synthetically engineered nanoblockers (Chnari et al. 2006, Biomacromolecules 7:1796-1805). Some statins, flavonoids such as fisetin, morin and myricetin and other antioxidants such as alpha-tocopherol (vitamin E) and antioxidant SS peptides (e.g. SS31) are able to attenuate CD36 expression (Zhao et al. 2004, J Biol Chem 279:34682-34690; Cho et al. 2007, J Biol Chem 282:4643-4642; Lian et al. 2008, Biochim Biophys Acta 1781:601-609; Venugopal et al. 2004, Atherosclerosis 175:213-220; Ricciarelli et al. 2000, Circulation 102: 82-87, Fuhrman et al. 2002, Atherosclerosis 164:179-185). Exemplary CD36-inhibitory siRNAs include siRNA with the sequences defined in SEQ ID NO:2 (GAACCUAUUGAUGGAUUAATT), SEQ ID NO:3 (CCUUCACUAUCAGUUGGAATT), and SEQ ID NO:4 (GCAACAUUCAAGUUAAGCATT) (WO2014/033130). Exemplary shRNAs targeting CD36 are those including one of the following nucleotide sequences (target sequence underlined):
TRCN0000056998 (SEQ ID NO:5)
   5'-CCGG-GAAGTTACATATTAGGCCATA-CTCGAG-TATGGCCTAATATGTAACTTC-TTTTTG-3'
TRCN0000056999 (SEQ ID NO:6)
   5'-CCGG-CCGACGTTAATCTGAAAGGAA-CTCGAG-TTCCTTTCAGATTAACGTCGG-TTTTTG-3'
TRCN0000057000 (SEQ ID NO:7)
   5'-CCGG-GCCATAATCGACACATATAAA-CTCGAG-TTTATATGTGTCGATTATGGC-TTTTTG-3'
TRCN0000057001 (SEQ ID NO:8)
   5'-CCGG-CCTGCTTATCCAGAAGACAAT-CTCGAG-ATTGTCTTCTGGATAAGCAGG-TTTTTG-3'
TRCN0000057002 (SEQ ID NO:9)
   5'-CCGG-CCATTGGTGATGAGAAGGCAA-CTCGAG-TTGCCTTCTCATCACCAATGG-TTTTTG-3'

### MAPK (mitogen-activated protein kinase) and PI3K (phosphatidylinositol-3-kinase) signaling pathway and inhibitors/antagonists

BRAF (aliases: B-Raf Proto-Oncogene; Serine/Threonine Kinase; V-Raf Murine Sarcoma Viral Oncogene Homolog B1; V-Raf Murine Sarcoma Viral Oncogene Homolog B; Proto-Oncogene B-Raf; BRAF1; RAFB1; B-Raf Proto-Oncogene Serine/Threonine-Protein Kinase (P94); Murine Sarcoma Viral (V-Raf) Oncogene Homolog B1; Serine/Threonine-Protein Kinase B-Raf; B-Raf Serine/Threonine-Protein; 94 KDa B-Raf Protein; EC 2.7.11.1; B-RAF1, B-Raf; NS7; P94); NCBI reference mRNA sequences include: NM_001354609.1, NM_004333.5, NM_001354609, XM_017012558.1, XM_017012559.1
MEK1 (aliases: Mitogen-Activated Protein Kinase Kinase 1; ERK Activator Kinase 1; MAPK/ERK Kinase 1; EC 2.7.12.2; MAPKK 1; PRKMK1; MAP2K1; MEK1; MKK1; Mitogen-Activated, Kinase 1; Dual Specificity Mitogen-Activated Protein Kinase Kinase 1; MAP Kinase Kinase 1; MAPKK; CFC3); NCBI reference mRNA sequences include: NM_002755.3, XM_011521783.2, XM_017022411.1, XM_017022412.1, XM_017022413.1
MEK2 (aliases: Mitogen-Activated Protein Kinase Kinase 2, ERK Activator Kinase 2, MAP Kinase Kinase 2, MAPK/ERK Kinase 2, EC 2.7.12.2, PRKMK2, MAP2K2, MKK2, Dual Specificity Mitogen-Activated Protein Kinase Kinase 2, MAPKK2, CFC4); NCBI reference cDNA sequences include NM_030662.3, XM_006722799.2, XM_017026989.1, XM_017026990.1, XM_017026991.1.
ERK1 (Mitogen-Activated Protein Kinase 3; Extracellular Signal-Regulated Kinase 1; Microtubule-Associated Protein 2 Kinase; Insulin-Stimulated MAP2 Kinase; MAP Kinase Isoform P44; EC 2.7.11.24; P44-ERK1; P44-MAPK; PRKM3; ERK-1; MAPK3; ERT2; Extracellular Signal-Related Kinase 1; MAP Kinase 3; EC 2.7.11; HS44KDAP; HUMKER1A; P44ERK1; P44MAPK; MAPK 1; MAPK 3); NCBI reference mRNA sequences include: NM_001040056.2, NM_001109891.1, NM_002746.2
ERK2 (Mitogen-Activated Protein Kinase 1; Extracellular Signal-Regulated Kinase 2; Mitogen-Activated Protein Kinase 2; MAP Kinase Isoform P42; MAP Kinase 1; MAP Kinase 2; EC 2.7.11.24; MAPK 2; PRKM1; PRKM2; ERK-2; ERT1; Protein Tyrosine Kinase ERK2; EC 2.7.11; P42MAPK; P41mapk; MAPK 1; MAPK2; P38; P40; ERK; P41); NCBI reference mRNA sequences include: NM_138957.3, NM_002745.4

The term "MAPK signaling pathway" refers to the mitogen-activated protein kinase signaling pathway (e.g., the RAS/RAF/MEK/ERK signaling pathway) which encompasses a family of conserved serine/threonine protein kinases (e.g., the mitogen-activated protein kinases (MAPKs)). Abnormal regulation of the MAPK pathway contributes to uncontrolled proliferation, invasion, metastases, angiogenesis, and diminished apoptosis. The RAS family of GTPases includes KRAS, HRAS, and NRAS. Exemplary MAPKs within the RAS/RAF/MEK/ERK signaling pathway include the RAF family of serine/threonine protein kinases (such as ARAF, BRAF, and CRAF (RAF1)) and the extracellular signal-regulated kinase 1 and 2 (i.e., ERK1 and ERK2). The importance of the MAPK pathway in melanoma is recognized in the art. It is estimated that about 40-60% of melanomas carry a mutation in this MAPK pathway which leads to constitutive activation of the MAPK pathway. The mutation is often in the BRAF gene, more particularly BRAF V600E and V600K (20% of MAPK mutations in melanoma), or V600R (7% of MAPK mutations in melanoma). Exclusive to BRAF mutations, mutations in the N-RAS gene occur (20% of MAPK mutations in melanoma; one N-RAS mutant in melanoma results in NRAS Q61K mutant kinase protein). Mutations in cKIT, GNAQ and GNA11 are not frequent in cutaneous melanoma but cKIT (mast/stem cell growth factor receptor (SCFR), proto-oncogene c-Kit, tyrosine-protein kinase Kit, CD117) mutations occur with a 20-30% incidence in mucosal melanomas, and mutations in GNAQ (guanine nucleotide-binding protein G(q) subunit alpha) and GNA11 (GNAQ paralogue) with a 85% incidence in uveal melanoma.(Manzano et al. 2016, Ann Transl Med 4:237). Furthermore, MEK1 (MAP2K1) mutants include MEK P124L identified in a melanoma patient and may confer cross-resistance to B-RAF inhibition (Emery et al. 2009, Proc Natl Acad Sci USA 106:20411-20416). Other BRAF mutations include BRAF G593S, BRAF L597R and BRAF K601E, whereas other MEK1 mutations include F53L, P124S, E203K and N382H, and MEK2 mutations include S154F (Nikolaev et al. 2012, Nature Genet 44:133-139). A comprehensive review of BRAF mutations in melanoma and other cancers is provided by Dankner et al. 2018 (Oncogene 37:3183-3199).

The term "inhibitor of MAPK pathway", "MAPK signaling inhibitor", "MAPK pathway inhibitor" or "MAPK pathway signaling inhibitor" (wherein "inhibitor" may be exchanged for "antagonist") refers to a molecule that decreases, blocks, inhibits, abrogates, or interferes with signal transduction through the MAPK pathway (e.g., the RAS/RAF/MEK/ERK pathway). In some embodiments, a MAPK signaling inhibitor may inhibit the activity of one or more proteins involved in the activation of MAPK signaling. In some embodiments, a MAPK signaling inhibitor may activate the activity of one or more proteins involved in the inhibition of MAPK signaling. MAPK signaling inhibitors include, but are not limited to, MEK inhibitors (e.g., MEK1 inhibitors, MEK2 inhibitors, and inhibitors of both MEK1 and MEK2), RAF inhibitors (e.g., ARAF inhibitors, BRAF inhibitors, CRAF inhibitors, and pan- RAF inhibitors (i.e., RAF inhibitors that are inhibiting more than one member of the RAF family (i.e., two or all three of ARAF, BRAF, and CRAF)), and ERK inhibitors (e.g., ERK1 inhibitors and ERK2 inhibitors).

The term "inhibitor of BRAF or CRAF", "inhibitor of BRAF or CRAF kinase", or "BRAF or CRAF inhibitor" (wherein "inhibitor" may be exchanged for "antagonist") refers to molecule that decreases, blocks, inhibits, abrogates, or interferes with BRAF or CRAF activation or function.

Sorafenib (a tyrosine kinase inhibitor, TKI) blocks wild-type BRAF, whereas vemurafenib (Zelboraf^{®}) and dabrafenib (Tafinlar^{®}) block mutant BRAF (B-RAF kinase) protein. Phase 3 trial data indicated a rapid response of BRAF-mutant melanoma to vemurafenib but of a short duration. BRAF-inhibitors are also referred to as RAF-inhibitors. Other examples of BRAF inhibitors include, without limitation, encorafenib (LGX818), GDC-0879, XL281, ARQ736, PLX3603, and RAF265, or a pharmaceutically acceptable salt thereof, or a pharmaceutically acceptable salt of sorafenib, vermurafenib, or dabrafenib. BRAF inhibitors may inhibit only BRAF or may inhibit BRAF and one or more additional targets. BRAF inhibitors are described in e.g. WO 2005/062795, WO 2007/002325, WO 2007/002433, WO 2008/079903, and WO 2008/079906. Examples of CRAF include, without limitation, sorafenib, semapimod (Messoussi et al. 2014, Chem Biol 21: 1433-1443), or a pharmaceutically acceptable salt thereof. CRAF inhibitors may inhibit only CRAF or may inhibit CRAF and one or more additional targets.

Exemplary BRAF-specific inhibitory short-hairpin RNAs (shRNAs) include, but are not limited to (wherein the underlined sequence is the target sequence):

TRCN0000006289:5'-CCGG-GCAGATGAAGATCATCGAAAT-CTCGAG-ATTTCGATGATCTTCATCTGC-
TTTTT-3' (SEQ ID NO:10),

TRCN0000006290:5'-CCGG-CCGCTGTCAAACATGTGGTTA-CTCGAG-TAACCACATGTTTGACAGCGG-
TTTTT-3'(SEQ ID NO:11),

TRCN0000006291:5'-CCGG-GCTGGTTTCCAAACAGAGGAT-CTCGAG-ATCCTCTGTTTGGAAACCAGC-
TTTTT-3'(SEQ ID NO:12),

TRCN0000006292:5'-CCGG-CAGCAGTTACAAGCCTTCAAA-CTCGAG-TTTGAAGGCTTGTAACTGCTG-
TTTTT-3'(SEQ ID NO:13), and

TRCN0000006293:5'-CCGG-CTATGAAGAATACACCAGCAA-CTCGAG-TTGCTGGTGTATTCTTCATAG-
TTTTT-3'(SEQ ID NO:14).

The term "pan-RAF inhibitor" (wherein "inhibitor" may be exchanged for "antagonist") refers to a molecule that decreases, blocks, inhibits, abrogates, or interferes with the activation or function of two or more RAF family members (e.g., two or more of ARAF, BRAF, and CRAF). In one embodiment, the pan-RAF inhibitor inhibits all three RAF family members (i.e., ARAF, BRAF, and CRAF) to some extent. Examples of pan-RAF inhibitors include, without limitation, LY-3009120, HM95573, LXH-254, MLN2480, BeiGene-283, RXDX-105, BAL3833, regorafenib, and sorafenib, or a pharmaceutically acceptable salt thereof. Pan-RAF inhibitors are described in e.g. WO2013/100632, WO2014/151616, and WO2015/075483. Pan-RAF inhibitors may inhibit ARAF, BRAF, and/or CRAF and one or more additional targets.

The term "ERK inhibitor" (wherein "inhibitor" may be exchanged for "antagonist") refers to molecule that decreases, blocks, inhibits, abrogates, or interferes with ERK (e.g., ERK1 and/or ERK2) activation or function. ERK kinase inhibitors include SCH772984 (shown to inhibit proliferation of cell resistant to BRAF- inhibitor or to BRAF/MEK-inhibitor; Morris et al. 2013, Cancer Discov 3:742-750) and VTX11E, or a pharmaceutically acceptable salt thereof. Further examples of ERK inhibitors include, without limitation, ravoxertinib (GDC- 0994) and ulixertinib (BVD-523), or a pharmaceutically acceptable salt (e.g., a besylate salt (e.g., a besylate salt of ravoxertinib)) thereof. ERK inhibitors are described in e.g. WO 2013/130976, WO 2012/1 18850, WO 2013/020062, WO 2015/154674, WO 2015/085007, WO2015/032840, WO 2014/036015, WO 2014/060395, WO 2015/103137, and WO 2015/103133. ERK inhibitors may inhibit only ERK or may inhibit ERK and one or more additional targets.

The *MEK* (mitogen-activated extracellular signal regulated kinase) gene works together with the *BRAF* gene, so drugs that block MEK proteins can also help treat melanomas with BRAF gene changes. Alone, MEK inhibitors seem less effective compared to BRAF inhibitors. A common approach becoming standard of care, is to combine BRAF- and MEK-inhibitors. Such combination often is effective over a longer time and seems to reduce the frequency of side effects such as the development of squamous cell skin cancers. The term "MEK inhibitor" (wherein "inhibitor" may be exchanged for "antagonist") refers to molecule that decreases, blocks, inhibits, abrogates, or interferes with MEK (e.g., MEK1 and/or MEK2) activation or function. MEK inhibitors include selumetinib, trametinib (Mekinist^{®}), cobimetinib (Cotellic^{®}, hemifumarate salt of cobimetinib), pimasertib, refametinib, binimetinib, and CI-1040 (PD184352), or a pharmaceutically acceptable salt thereof. Other examples of MEK inhibitors include, without limitation, GDC-0623, PD-0325901, and BI-847325, or a pharmaceutically acceptable salt thereof. MEK inhibitors are described in e.g. WO 2007/044515, WO 2008/024725, WO 2008/024724, WO 2008/067481, WO 2008/157179, WO 2009/085983, WO 2009/085980, WO 2009/082687, WO 2010/003025, and WO 2010/003022. MEK inhibitors may inhibit only MEK or may inhibit MEK and one or more additional targets.

BRAF-inhibitor resistant melanoma cells express increased levels of YAP1 (YES-associated protein 1, YAP or YAP65), TAZ (transcriptional co-activator with PDZ-binding motif; encoding the tafazzin protein) and TEAD (TEA domain transcription factors), all components of the Hippo-pathway, and increase survival of the melanoma cells. Verteporfin (an inhibitor of YAP) sensitizes BRAF-mutant BRAF-inhibitor-resistant melanoma to the BRAF-inhibitor and reduces tumor growth (Fisher et al. 2017, Oncotarget 8:110257-110272). Expression of YAP can be countered by administration of YAP-inhibitors such as antiparasitic macrocyclic lactones (e.g. ivermectin, milbemycin D) (Nishio et al. 2016, Proc Natl Acad Sci USA 113:E71-E80), porphyrin- and dipyrrin-related derivatives (such as verteporfin) (Gibault et al. 2017, Chem Med Chem12:954-961), or statins (e.g. simvastatin) (Wang et al. 2014, Proc Natl Acad Sci USA 111:E89-E98).

Mechanisms leading to BRAF inhibitor resistance are summarized by e.g. Manzano et al. (2016, Ann Transl Med 4:237). The PI3K (phosphatidylinositol-3-kinase) /AKT (protein kinase B) /mTOR (mechanistic target of rapamycin) pathway is an intracellular signaling pathway (natural inhibitor: PTEN; PTEN loss occurs in 10-30% of melanomas, exclusive to NRAS mutation and can coexist with BRAF mutations; Manzano et al. 2016, Ann Transl Med 4:237) also involved in cell cycle regulation. Mutations in this pathway may contribute for instance to resistance to BRAF inhibitors. A possible solution is to combine BRAF and/or MEK inhibitors with inhibitors of PI3K (e.g. pictilisib, copanlisib, taselisib, idelalisib, buparlisib, alpelisib), with inhibitors of mTOR (temsirolimus, sirolimus, everolimus, ridaforolimus and other rapamycin derivatives) (Massacesi et al. 2016, Oncotargets Ther9:203-210), with inhibitors of CDK4-6 (cell cycle dependent kinase) such as PD 0332991 (Flaherty et al. 2012, Clin Cancer Res 18:568-576) or ribociclib or palbociclib, with mTOR/PI3K dual inhibitors (e.g. dactolisib, BGT226, SF1126, PKI-587, NVPBE235), with mTORC1/mTORC2 dual inhibitors (e.g. sapanisertib, AZD8055, and AZD2014), with inhibitors of AKT (e.g. MK-2206 is 8-[4-(1-aminocyclobutyl)phenyl]-9-phenyl-1,2,4-triazolo[3,4-f] [1,6]naphthyridin-3(2H)-one hydrochloride; Hirai et al. 2010, Mol Cancer Ther 9:1956-1967), with anti-MET therapy (e.g. crizotinib (Wilson et al. 2012, Nature 487:505-510) or GNE-A (Liederer et al. 2011, Xenobiotica 41:327-339)) or cabozantinib or foretinib or tivantinib or AMG 458 or JNJ-38877605 or PF-04217903 or MK-2461 (see e.g., Underiner et al. 2010, Anti-Cancer Agents Med Chem 10:7-27; Katz et al. 2011, J Med Chem 54:4092-4108). A comprehensive review of BRAF mutations in melanoma and other cancers, as well as of available treatment options, is provided by Dankner et al. 2018 (Oncogene 37:3183-3199).

In any of the foregoing aspects and embodiments of the invention, the inhibitor of the MAPK pathway may be a BRAF-inhibitor, an inhibitor of BRAF-mutant kinase, a MEK-inhibitor, an inhibitor of MEK-mutant kinase or any combination in any way of a BRAF-inhibitor and a MEK-inhibitor. In particular, the inhibitor of the MAPK pathway may be chosen from sorafenib, vemurafenib, dabrafenib, regorafenib, LY-3009120, HM95573, LXH-254, MLN2480, BeiGene-283, RXDX-105, BAL3833, encorafenib (LGX818), GDC-0879, XL281, ARQ736, PLX3603, RAF265, selumetinib, trametinib, cobimetinib, pimasertib, refametinib, binimetinib, CI-1040 (PD184352), GDC-0623, PD-0325901, and BI-847325, or a pharmaceutically acceptable salt of any thereof; or may be a compound specifically inhibiting the MAPK pathway and is chosen from an antisense oligonucleotide, a gapmer, a siRNA, a shRNA, a zinc-finger nuclease, a meganuclease, a TAL effector nuclease, a CRISPR-Cas effector, an antibody or a fragment thereof, an alpha-body, a nanobody, an intrabody, an aptamer, a DARPin, an affibody, an affitin, an anticalin, or monobody; or may be chosen from any combination of any of the foregoing.

In any of the foregoing, the CD36 antagonist may be a pharmaceutical compound, a biopharmaceutical compound, a nucleic acid compound, or may be a combination of any of the foregoing. In particular, the CD36 antagonist may be chosen from apolipoprotein Al-mimetic peptide 5A, AP5055, AP5258, salvianolic acid B, sodium danshensu (DSS), rosmarinic acid, 3-cinnamoyl indole, 13 pentyl berberine, sulfo-N-succinimidyl oleate, hexarelin, EP80317, a statin, a flavonoid, alpha-tocopherol, vitamin E, an antioxidant SS peptide, SS31, or a pharmaceutically salt of any thereof, or is chosen from any combination of any of the foregoing; or may be a compound specifically inhibiting CD36 and chosen from an antisense oligonucleotide, a gapmer, a siRNA, a shRNA, a zinc-finger nuclease, a meganuclease, a TAL effector nuclease, a CRISPR-Cas effector, an antibody or a fragment thereof, an alpha-body, a nanobody, an intrabody, an aptamer, a DARPin, an affibody, an affitin, an anticalin, or monobody; or may be chosen from any combination of any of the foregoing.

In the context of any of the foregoing, the tumor, such as melanoma, may at any disease stage be or have been treated with a compound sensitizing the tumor, such as melanoma, to an inhibitor of the MAPK pathway, treated by surgery, treated by radiation, treated by chemotherapy, treated by immunotherapy, treated by immune checkpoint therapy, treated with any other anticancer agent, or be or have been treated by any combination of any of the foregoing. In particular, the other anticancer agent or compound sensitizing the tumor, in particular melanoma, to an inhibitor of the MAPK pathway may be chosen from nelfinavir, atazanavir, fulvestrant, telmisartan, terazosin, mifepristone, spironol acetone/spironolactone, WP1066, cyclophosphamide, an GPNMB antibody conjugated to a cytotoxic drug, nivolumab, prembrolizumab, ipilumab, varlilumab, CDX-301, bemcentinib, BPI-9016M, LY2801653, amuvatinib, bosutinib, glesatinib, MGCD516, ASP2215, cabozantinib, foretinib, SGI-7079, TP-0903, ASLAN002, erlotinib, crizotinib, BMS-777607, gilteritinib, cytarabine, an AXL antibody conjugated to a cytotoxic drug, an antibody drug conjugate with the antibody targeting e.g. GPNMB, an inhibitor of JNK, an inhibitor of FAK, an inhibitor of Src, an inhibitor of BET protein, an ERK inhibitor, a PI3K inhibitor, an mTOR inhibitor, an inhibitor of CDK4-6, an AKT inhibitor, a MET-inhibitor, a YAP-inhibitor, verteporfin, dacarbazine, an antifolate drug, an AXL inhibitor, a melanocyte-directed enzyme prodrug, or a pharmaceutically acceptable salt of any thereof, or any combination in any way of any thereof.

In any of the foregoing, the tumor may be melanoma, such as cutaneous melanoma. In any of the foregoing, the subject may be a mammalian subject. The group of mammals includes, besides humans, mammals such as primates, cattle, horses, sheep, goats, pigs, rabbits, mice, rats, guinea pigs, Ilama's, dromedaries and camels, as well as to mammalian pet animals (dogs, cats, gerbils, hamsters, chinchillas, ferrets etc.).

### Retinoid X receptors (RXRs)

Retinoid X receptors (RXR) belong to the NR2B nuclear receptor family and are common binding partners to many other nuclear receptors, including RARs (retinoic acid receptor), PPARs (peroxisome proliferator activated receptors), liver X receptors (LXRs), farnesoid X receptor (FXR), and vitamin D receptors (VDRs). There are three RXR subtypes: alpha (RXRA), beta (RXRB) and gamma (RXRG). NCBI reference -mRNA sequences for the retinoid X Receptor Gamma (aliases: RXRG; Nuclear Receptor Subfamily 2 Group B Member 3; NR2B3; Retinoic Acid Receptor RXR-Gamma; Retinoid X Receptor, Gamma; RXRC) include NM_001256570.1, NM_001256571.1, and NM_006917.4. RXRG is involved in retinoic acid (RA) signaling. Mice in which the RXRG gene is knocked-down survive and appear normal (Krezel et al. 1996, Proc Natl Acad Sci USA 93:9010-9014). In humans, RXRG is implied in down-regulation of human lipoprotein lipase (LPL) and a point mutation (Gly14Ser) was identified as even stronger LPL repressor. Genetic variation in RXRG may therefore play a role in genetic dyslipidemia, such as familial combined hyperlipidemia (FCHL) (Nohara et al. 2009, J Atheroscler Thromb 16:303-318).

The term "antagonist of retinoid X receptor" as used herein refers to inhibitors of function, inhibitors of expression and inhibitors of downstream signaling pathway (components) of RXR.

Pharmacologic inhibitors of RXR at least block or inhibit RXRG, but can be promiscuous as to other nuclear receptors such as RXRalpha and/or RXRbeta and/or PPAR (peroxisomal proliferator-activated receptor). Development of pharmacologic (small molecule-type or -like) RXR-subtype-selective rexinoids (agonists or activators; antagonists or inhibitors) has been challenging due to the conserved lipid biding pocket. A rexinoid is a (any) synthetic agent that specifically binds to a retinoid X receptor. Pharmacologic RXR antagonists include HX 531 (a pan-RXR antagonist inhibiting activation of RAR-RXR heterodimers), HX 630 (pan-RXR agonist, with weak RAR-antagonizing properties), and HX711 (Ebisawa et al. 1999, Chem Pharm Bull 47:1778-1786). Other RXR antagonist are listed in e.g. Table 4 of Dawson & Xia 2012 (Biochim Biophys Acta 1821:21-26).

Exemplary RXRG shRNAs can be found via https://portals.broadinstitute.org/gpp/public/clone/search (Broad Institute Genomic Perturbations Platform and the RNAi Consortium) and include (with TRCN barcode reference) the following sequences (wherein the target sequence is underlined):
TRCN0000021639 (SEQ ID NO:15)
   5'-CCGG-CGGGATTGGAAACATGAACTA-CTCGAG-TAGTTCATGTTTCCAATCCCG-TTTTT-3'
TRCN0000021640 (SEQ ID NO:16)
   5'-CCGG-GAGTCCTAACTGAGCTGGTTT-CTCGAG-AAACCAGCTCAGTTAGGACTC-TTTTT-3'
TRCN0000021641 (SEQ ID NO:17)
   5'-CCGG-GCCTACACCAAGCAGAAGTAT-CTCGAG-ATACTTCTGCTTGGTGTAGGC-TTTTT-3'
TRCN0000021642 (SEQ ID NO:18)
   5'-CCGG-CTATCAGAAGTGCCTTGTCAT-CTCGAG-ATGACAAGGCACTTCTGATAG-TTTTT-3'
TRCN0000021643 (SEQ ID NO:19)
   5'-CCGG-GCGAGCCATTGTACTCTTTAA-CTCGAG-TTAAAGAGTACAATGGCTCGC-TTTTT-3'

The term "agonist of retinoid X receptor" as used herein refers to enhancers or stimulators of function, enhancers or stimulators of expression and enhancers or stimulators of downstream signaling pathway (components) of RXR.

RXR-activating rexinoids include bexarotene, a potent and selective pan-RXR agonist (EC₅₀ values are 24, 25 and 33 nM for RXRβ, RXRy and RXRα, respectively), and exhibiting a >300-fold selectivity for RXR over RAR receptors. SR 11237 (BMS 649) is a pan-RXR agonist that is devoid of any RAR activity. Aliretinoin (9-cis-RA) is an RXR-agonist approved for topical treatment of Kaposi's sarcoma and systemic treatment of refractory chronic hand eczema.

### Focal Adhesion Kinase (FAK) and inhibitors

FAK (PTK2; Protein Tyrosine Kinase 2; Protein Phosphatase 1 Regulatory Subunit 71; Focal Adhesion Kinase-Related Nonkinase; EC 2.7.10.2; Pp125FAK; PPP1R71; P125FAK; FADK 1; FAK1; FRNK; Focal Adhesion Kinase Isoform FAK-Del33; FAK-Related Non-Kinase Polypeptide; Focal Adhesion Kinase 1; EC 2.7.10; FADK); NCBI reference mRNA sequences include: NM_001199649.1, NM_001316342.1, NM_001352694.1, NM_001352695.1, NM_001352696.1

FAK is involved in cellular adhesion and spreading. Inhibitors of FAK include PF-573,228 (PF-228), PF-562,271 (PF-271, VS-6062), NVP-226, Y15 (1,2,4,5-benzenetetraamine tetrahydrochloride), PND-1186, GSK2256098, defactinib (VS-6063, PF-04554878), VS-4718 (PND-1186), TAE226, and daurinol (e.g. Dunn et al. 2010, Anti-Cancer Agents Med Chem 10:722-34; Woo et al. 2017, Oncotarget 8:57058-57071).

### Antifolate drugs

Antifolate drugs, antifolates, folate antagonists, or antifols, include aminopterin, methotrexate, trimetrexate, fluorouracil, lometrexol, raltitrexed, pemetrexed, plevitrexed, nolatrexed, OSI-7904L (Ricart et al. 2008, Clin Cancer Res 14:7947-7955), ZD9331 (Benepal & Judson 2005, Anticancer Drugs 16:1-9) or BGC 945 (ONX-0801; Gibbs et al. 2005, Cancer Res 65:11721-11728), or a pharmaceutically acceptable drug thereof.

### AXL inhibitors

AXL (aliases: AXL Receptor Tyrosine Kinase; AXL Oncogene; EC 2.7.10.1; UFO; Tyrosine-Protein Kinase Receptor UFO; AXL Transforming Sequence/Gene; EC 2.7.10; JTK11; Tyro7; ARK); NCBI reference mRNA sequences include: NM_001278599.1, NM_001699.5, NM_021913.4.

Signaling pathways activated downstream of AXL include PI3K-AKT-mTOR, MEK-ERK, NF-kB, and JAK/STAT. Selective pharmacological inhibition of AXL is feasible with bemcentinib (BGB324 or R428) or BPI-9016M. Other RTK-inhibitors also inhibiting AXL include LY2801653, amuvatinib (MP-470; inhibitor of c-Kit, FLT3, RET, PDGFRbeta and AXL), bosutinib (SKI-606), glesatinib (MGCD 265; MET/AXL inhibitor), MGCD516, ASP2215, cabozantinib (XL184; multi-kinase inhibitor), foretinib (GSK1363089/XL880), SGI-7079, TP-0903, ASLAN002, erlotinib, crizotinib, BMS-777607, and the dual FLT3-AXL inhibitor gilteritinib. Chemotherapeutics targeting AXL include cytarabine. (Gay et al. 2017, Br J Cancer 116:415-423; Levin et al. 2016, J Thorac Oncol 11:1357-1362). Biopharmaceuticals targeting AXL include a monoclonal antibody (YW327.6S2; Ye et al. 2010, Oncogene 29:5254-5264) and an RNA-based aptamer (GL21.T; Cerchia et al. 2012, Mol Ther 20:2291-2303). AXL-inhibitors have been applied in overcoming resistance to PI3K inhibitors, in sensitizing tumors to PARP inhibition, and in overcoming or delaying resistance to EGFR inhibitors (reviewed by Gay et al. 2017, Br J Cancer 116:415-423). Exemplary shRNAs targeting AXL are listed hereafter (target sequence underlined):
TRCN0000000572 (SEQ ID NO:20)
   5'-CCGG-CTTTAGGTTCTTTGCTGCATT-CTCGAG-AATGCAGCAAAGAACCTAAAG-TTTTT-3'
TRCN0000000573 (SEQ ID NO:21)
   5'-CCGG-GCGGTCTGCATGAAGGAATTT-CTCGAG-AAATTCCTTCATGCAGACCGC-TTTTT-3'
TRCN0000000574 (SEQ ID NO:22)
   5'-CCGG-CGAAAGAAGGAGACCCGTTAT-CTCGAG-ATAACGGGTCTCCTTCTTTCG-TTTTT-3'
TRCN0000000575 (SEQ ID NO:23)
   5'-CCGG-CGAAATCCTCTATGTCAACAT-CTCGAG-ATGTTGACATAGAGGATTTCG-TTTTT-3'
RCN0000000576 (SEQ ID NO:24)
   5'-CCGG-GCTGTGAAGACGATGAAGATT-CTCGAG-AATCTTCATCGTCTTCACAGC-TTTTT-3'

### Melanocyte-directed enzyme prodrugs

Tyrosinase is mainly or only present in melanocytes and melanoma cells where it is involved in melanin synthesis. It therefore is a unique target for treatment of melanoma, which is at the origin of different melanocyte-directed enzyme prodrug therapies (Rooseboom et al. 2004, Pharmacol Rev 56:53-102). Prodrugs are inactive forms of active drugs that are designed such that they are converted to their active counterpart preferentially at *the required site of action (thus potentially adding to stability and/or reducing systemic toxicity of the drug). TMECG (3-O-(3,4,5-trimethoxybenzoyl)-(-)-epicatechin) and TMCG (3-O-(3,4,5-trimethoxybenzoyl)-(-)-catechin) are antifolate prodrugs activated by melanocyte-specific tyrosinase into a quinone methide inhiting dihyrdofolate reductase (DHFR) (Saez-Ayala et al. 2011, ChemMedChem 6:440-449). Methotrexate (MTX), in driving melanocytes away from the invasive phenotype, was reported to induce differentiation-associated expression of tyrosinase in proliferating melanocytes, therewith further sensitizing MTX-treated melanocytes to the action of TMECG, this independent of *BRAF-, MEK-* or p53-status (Saez-Ayala et al. 2013, Cancer Cell 24:105-119).

### JNK

JNK1 (aliases: Mitogen-Activated Protein Kinase 8; Stress-Activated Protein Kinase 1c; C-Jun N-Terminal Kinase 1; JUN N-Terminal Kinase; MAP Kinase 8; EC 2.7.11.24; JNK-46; SAPK1c; PRKM8; SAPK1; JNK1; Mitogen-Activated Protein Kinase 8 Isoform JNK1 Alpha1; Mitogen-Activated Protein Kinase 8 Isoform JNK1 Beta2; Stress-Activated Protein Kinase JNK1; Stress-Activated Protein Kinase 1; JNK21B1/2; EC 2.7.11; JNK1A2; MAPK 8); NCBI reference mRNA sequences include: NM_001278547.1, NM_001278548.1, NM_001323302.1, NM_001323320.1, NM_001323321.1, NM_001323322.1, NM_001323323.1, NM_001323324.1, NM_001323325.1, NM_001323326.1, NM_001323327.1, NM_001323328.1, NM_001323329.1, NM_001323330.1, NM_001323331.1, NM_139046.3, NM_139049.3. Other JNK family members include JNK2 and JNK3. JNK antagonists may inhibit one or more of the JNK paralogues, and include first generation ATP-competitive JNK inhibitors such as SP600125 and CEP-1347 (KT7515), second-generation ATP-competitive inhibitors such as CC-401, and further antagonists include CC-930, peptide D-JNKI-1 (XG-102, AM-111). PGL5001 (bentamapimod, AS601245) is an ATP-competitive inhibitor which inhibits JNK1, JNK2, and JNK3 with an IC50 of 80 nM, 90 nM, and 230 nM, respectively. ATP-noncompetitive small molecule JNK antagonists include BI-78D3, BI-87G3 (Messoussi et al. 2014, Chem Biol 21:1433-1443; Bogoyevitch et al. 2010, Biochim Biophys Acta 1804:463-475)

### Src/SRC

Src (aliases: SRC Proto-Oncogene, Non-Receptor Tyrosine Kinase; V-Src Avian Sarcoma (Schmidt-Ruppin A-2) Viral Oncogene Homolog; Proto-Oncogene C-Src; EC 2.7.10.2; P60-Src; SRC1; Proto-Oncogene Tyrosine-Protein Kinase Src; Protooncogene SRC, Rous Sarcoma; Tyrosine-Protein Kinase SRC-1; Tyrosine Kinase Pp60c-Src; Pp60c-Src; EC 2.7.10; C-SRC; THC6; ASV); NCBI reference mRNA sequences include: NM_005417.4, NM_198291.2, XM_011529013.2, XM_017028024.1, XM_017028025.1, XM_017028026.1, XM_017028027.1. SRC antagonists include KX2-391, bosutinib, saracatinib, quercetin, and dasatinib.

### BET proteins

BET proteins are reviewed by Tanigeuchi 2016 (Int J Mol Sci 17:1849), and include human BRD2, BRD3, BRD4 and BRDT. A BET antagonist may inhibit one or more of the paralogous BET proteins. At least five BET-antagonistic pharmacologic compounds are in clinical trials: RVX-208, I-BET762 (GSK525762A), OTX 015, CPI0610 and TEN-010. Other small molecule BET antagonists include JQ1, I-BET151, I-BET, CPI203, RVX2135, dinaciclib, PFI-1, and RVX-208 (Fu et al. 2015, Oncotarget 6:5501-5516).

### Combination therapy/sensitization to MAPK-pathway inhibitors

Combination in any way is meant to refer to any sequence (consequent or separated from each other for any amount of time) of 2 or more therapeutic modalities and/or, in case of therapeutic compounds, any formulation of 2 or more therapeutic modalities (e.g. individually provided in separate vials, combination of 2 or more therapeutic modalities in the same vial, combination of both).

A number of combinations of therapeutic modalities have been outlined hereinabove. Some of these combinations may comprise a compound sensitizing tumor cells, in particular melanoma tumor cells, otherwise resistant thereto, to MAPK-inhibitors.

Reduction of PAX3 expression sensitizes melanoma cells to MEK inhibitors. Based hereon, the PAX3-MITF axis was targeted to counteract MITF-driven drug tolerance, and seven FDA-approved drugs were identified of which nelfinavir mesylate had the strongest effect (the others being atazanavir, fulvestrant, telmisartan, terazosin, mifepristone, and spironol acetone/spironolactone). Nelfinavir was shown to sensitize BRAF-mutant melanoma to BRAF and MEK inhibitors, to overcome NRAS-mediated acquired resistance to BRAF inhibition, and to sensitize NRAS-mutant melanoma to MEK inhibitors. Decreasing PAX3 expression was also obtained by overexpression of SKI, SMAD2 or SMAD4. Smith et al. 2016 (Cancer Cell 29:270-284) therewith position nelfinavir, via inhibition of MITF expression, as enhancer of BRAF and MEK inhibitors. More information on the applicability of nelfinavir in cancer therapy can be found in Koltai 2015 (F1000Research 4:9).

Silencing of the Stat3-PAX3 signaling (by shRNA targeting Stat3 or PAX3, or by pharmacologic inhibition of Stat3 with WP1066) was shown to inhibit growth of BRAF V600E mutant melanoma cells with acquired resistance to BRAF-inhibition (Liu et al. 2013, J Invest Dermatol 133:2041-2049). WP1066 was also shown to enhance antitumor activity of cyclophosphamide in mice xenografted with melanoma (Hatiboglu et al. 2012, Int J Cancer 131:8-17).

Increased MITF expression (such as upon treatment with BRAF- and/or MEK-inhibitors) induces expression of melanosomal differentiation genes such as cell-surface transmembrane glycoprotein NMB (GPNMB) which is required for melanin production, and is associated with poor survival. Glembatumumab vedotin (CDX-011; CR011-vcMMAE) is an antibody-drug conjugate (ADC) that targets cells expressing GPNMB wherein the antibody is monoclonal antibody glembatumumab (CR011) and the drug is monomethyl auristatin E (MMAE). Glembatumumab vedotin was shown to inhibit MAPK-pathway inhibitor induced pigmentation and melanoma growth, especially in combination with BRAF- and MEK-inhibitors, which underlies the proposal to combine CDX-011 with an intermittent MAPK-pathway inhibitor dosing regimen (Rose et al. 2016, Clin Cancer Res 22:6088).

Whereas high MITF expression contributes to drug resistance (see above; and also to ERK-inhibition), a contrary observation was disclosed by Müller et al. 2014 (Nature Commun 5:5712) as, within cultured melanoma cell-lines, two drug-resistant (to BRAF inhibitor, to ERK inhibitor) populations were characterized: MITFhigh and MITFlow. The MITFlow population was further characterized as having a low MITF/RTK ratio (RTK: receptor tyrosine kinase; kinase identified: AXL, EGFR and PDGFRbeta) and being correlated to increased invasiveness. Inhibition of multiple, not single, RTKs sensitized drug-resistant (BRAF-mutant or NRAS-mutant) melanoma cells to BRAF-inhibition or BRAF/MEK-inhibition. A similar observation was reported by Konieczkowski et al. 2014 (Cancer Discov 4:816-827) linking MITFlow with high NF-kappa B and high AXL. The effect of inhibition of AXL (pharmacologic or via shRNA) on reversing BRAF-inhibitor resistance appeared minimal. MAPK-pathway inhibitor-induced expression of EGFR and PDGFRbeta was also reported by Sun et al. 2014 (Nature 508:118-122), including a minimal effect of combination of BRAF- and EGFR-inhibition, but a beneficial effect of combining BRAF- and PI3K-inhibitors. An AXL antibody-drug conjugate in conjunction with MAPK-pathway inhibition was shown to be able to cooperatively eliminate drug-resistant BRAF-mutant and NRAS mutant melanomas enriched for AXL-positive cells (Boshuizen et al. 2018, Nature Med doi:10.1038/nm.4472).

In studying the effect of the BRAF inhibitor vemurafenib on 2 melanoma cell lines, Fallahi-Sichani et al. 2017 (Mol Systems Biol 13:905) identified 2 drug-induced populations in 1 out of the 2 melanoma cell lines and characterized these as NGFRhigh and NGFRlow. The NGFRhigh population was further characterized by upregulation of the neurogenesis genes S100B, CNTN6, L1CAM, FYN, MAP2 and NCAM1. The NGFRhigh population displayed reversible resistance to vemurafenib, but was sensitive to combinations of on the one hand vemurafenib or dabrafenib plus trametinib, with, on the other hand, either one of an inhibitor of FAK-kinase, an inhibitor of c-Jun N-terminal kinase (JNK), an inhibitor of Src family kinases (acting downstream of FAK-kinase), or an inhibitor of Bromodomain and extraterminal domain (BET) protein. The upregulation of NGFR (CD271) during drug exposure, as well as under hypoxia and low glucose conditions was previously reported by Menon et al. 2015 (Oncogene 34:4448-4459). During maintained drug exposure, NGFR (CD271) expression is transiently upregulated followed by a profound decrease indicative of the appearance of a disease stage permanently resistant to the drug (Menon et al. 2015 Oncogene 34:4448-4459).

The therapeutic modality of the current invention (be it a (bio)pharmacologic compound, nucleic acid, or nucleic acid comprising compound) can be combined (simultaneously or in any order) with one or more other antitumor, anticancer or antineoplastic therapy in a combination therapy. Several types of antitumor, anticancer or antineoplastic therapy are listed hereunder. It will be clear, however, that none of these lists is meant to be exhaustive and is included merely for illustrative purposes.

Without being exhaustive, antitumor, anticancer or antineoplastic agents include alkylating agents (nitrogen mustards: melphalan, cyclophosphamide, ifosfamide; nitrosoureas; alkylsulfonates; ethyleneimines; triazene; methyl hydrazines; platinum coordination complexes: cisplatin, carboplatin, oxaliplatin), antimetabolites (folate antagonists: methotrexate; purine antagonists; pyrimidine antagonists: 5-fluorouracil, cytarabibe), natural plant products (Vinca alkaloids: vincristine, vinblastine; taxanes: paclitaxel, docetaxel; epipodophyllotoxins: etoposide; camptothecins: irinotecan), natural microorganism products (antibiotics: doxorubicin, bleomycin; enzymes: L-asparaginase), hormones and antagonists (corticosteroids: prednisone, dexamethasone; estrogens: ethinyloestradiol; antiestrogens: tamoxifen; progesteron derivative: megestrol acetate; androgen: testosterone propionate; antiandrogen: flutamide , bicalutamide; aromatase inhibitor: letrozole, anastrazole; 5-alpha reductase inhibitor: finasteride; GnRH analogue: leuprolide, buserelin; growth hormone, glucagon and insulin inhibitor: octreotide). Other antineoplastic or antitumor agents include hydroxyurea, imatinib mesylate, epirubicin, bortezomib, zoledronic acid, geftinib, leucovorin, pamidronate, and gemcitabine.

Without being exhaustive, antitumor, anticancer or antineoplastic antibodies (antibody therapy) include rituximab, bevacizumab, ibritumomab tiuxetan, tositumomab, brentuximab vedotin, gemtuzumab ozogamicin, alemtuzumab, adecatumumab, labetuzumab, pemtumomab, oregovomab, minretumomab, farletuzumab, etaracizumab, volociximab, cetuximab, panitumumab, nimotuzumab, trastuzumab, pertuzumab, mapatumumab, denosumab, and sibrotuzumab.

A particular class of antitumor, anticancer or antineoplastic agents are designed to stimulate the immune system (immune checkpoint or other immunostimulating therapy). These include so-called immune checkpoint inhibitors or inhibitors of co-inhibitory receptors and include PD-1 (Programmed cell death 1) inhibitors (e.g. pembrolizumab, nivolumab, pidilizumab), PD-L1 (Programmed cell death 1 ligand) inhibitors (e.g. atezolizumab, avelumab, durvalumab), CTLA-4 (Cytotoxic T-lymphocyte associated protein 4; CD152) inhibitors (e.g. ipilimumab, tremelimumab) (e.g. Sharon et al. 2014, Chin J Canc 33:434-444). PD-1 and CTLA-4 are members of the immunoglobulin superfamily of co-receptors expressed on T-cells. Inhibition of other co-inhibitory receptors under evaluation as antitumor, anticancer or antineoplastic agents include inhibitors of Lag-3 (lymphocyte activation gene 3), Tim-3 (T cell immunoglobulin 3) and TIGIT (T cell immunoglobulin and ITM domain) (Anderson et al. 2016, Immunity 44:989-1004). Stimulation of members of the TNFR superfamily of co-receptors expressed on T-cells, such as stimulation of 4-1BB (CD137), OX40 (CD134) or GITR (glucocorticoid-induced TNF receptor family-related gene), is also evaluated for antitumor, anticancer or antineoplastic therapy (Peggs et al. 2009, Clin Exp Immunol 157:9-19).

Further antitumor, anticancer or antineoplastic agents include immune-stimulating agents such as - or neo-epitope cancer vaccines (neo-antigen or neo-epitope vaccination; based on the patient's sequencing data to look for tumor-specific mutations, thus leading to a form of personalized immunotherapy; Kaiser 2017, Science 356:112; Sahin et al. 2017, Nature 547:222-226) and some Toll-like receptor (TLR) ligands (Kaczanowska et al. 2013, J Leukoc Biol 93:847-863).

Yet further antitumor, anticancer or antineoplastic agents include oncolytic viruses (oncolytic virus therapy) such as employed in oncolytic virus immunotherapy (Kaufman et al. 2015, Nat Rev Drug Discov 14:642-662), any other cancer vaccine (cancer vaccine administration; Guo et al. 2013, Adv Cancer Res 119:421-475), and any other anticancer nucleic acid therapy (wherein "other" refers to it being different from therapy with a nucleic acid or nucleic acid comprising compound already specifically envisaged in the current invention).

Therefore, in any of the aspects and embodiments of the invention, the therapeutic modality of the current invention may be further combined with another therapy against the tumor, cancer or neoplasm. Such other therapies include for instance surgery, radiation, chemotherapy, immunotherapy, immune checkpoint or other immunostimulating therapy, neo-antigen or neo-epitope vaccination, cancer vaccine administration, oncolytic virus therapy, antibody therapy, any anticancer agent, any other nucleic acid therapy targeting the tumor, cancer or neoplasm, or any combination of any of the foregoing.

### Treatment / therapeutically effective amount

"Treatment"/"treating" refers to any rate of reduction, delaying or retardation of the progress of the disease or disorder, or a single symptom thereof, compared to the progress or expected progress of the disease or disorder, or singe symptom thereof, when left untreated. This implies that a therapeutic modality on its own may not result in a complete or partial response (or may even not result in any response), but may, in particular when combined with other therapeutic modalities, contribute to a complete or partial response (e.g. by rendering the disease or disorder more sensitive to therapy). More desirable, the treatment results in no/zero progress of the disease or disorder, or singe symptom thereof (i.e. "inhibition" or "inhibition of progression"), or even in any rate of regression of the already developed disease or disorder, or singe symptom thereof. "Suppression/suppressing" can in this context be used as alternative for "treatment/treating". Treatment/treating also refers to achieving a significant amelioration of one or more clinical symptoms associated with a disease or disorder, or of any single symptom thereof. Depending on the situation, the significant amelioration may be scored quantitatively or qualitatively. Qualitative criteria may e.g. by patient well-being. In the case of quantitative evaluation, the significant amelioration is typically a 10% or more, a 20% or more, a 25% or more, a 30% or more, a 40% or more, a 50% or more, a 60% or more, a 70% or more, a 75% or more, a 80% or more, a 95% or more, or a 100% improvement over the situation prior to treatment. The time-frame over which the improvement is evaluated will depend on the type of criteria/disease observed and can be determined by the person skilled in the art.

A "therapeutically effective amount" refers to an amount of a therapeutic agent to treat or prevent a disease or disorder in a mammal. In the case of cancers, the therapeutically effective amount of the therapeutic agent may reduce the number of cancer cells; reduce the primary tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, e.g., be measured by assessing the duration of survival (e.g. overall survival), time to disease progression (TTP), response rates (e.g., complete response and partial response, stable disease), length of progression-free survival, duration of response, and/or quality of life. The term "effective amount" refers to the dosing regimen of the agent (e.g. antagonist as described herein) or composition comprising the agent (e.g. medicament or pharmaceutical composition). The effective amount will generally depend on and/or will need adjustment to the mode of contacting or administration. The effective amount of the agent or composition comprising the agent is the amount required to obtain the desired clinical outcome or therapeutic effect without causing significant or unnecessary toxic effects (often expressed as maximum tolerable dose, MTD). To obtain or maintain the effective amount, the agent or composition comprising the agent may be administered as a single dose or in multiple doses. The effective amount may further vary depending on the severity of the condition that needs to be treated; this may depend on the overall health and physical condition of the mammal or patient and usually the treating doctor's or physician's assessment will be required to establish what is the effective amount. The effective amount may further be obtained by a combination of different types of contacting or administration.

The aspects and embodiments described above in general may comprise the administration of one or more therapeutic compounds to a mammal in need thereof, i.e., harboring a tumor, cancer or neoplasm in need of treatment. In general a (therapeutically) effective amount of (a) therapeutic compound(s) is administered to the mammal in need thereof in order to obtain the described clinical response(s). "Administering" means any mode of contacting that results in interaction between an agent (e.g. a therapeutic compound) or composition comprising the agent (such as a medicament or pharmaceutical composition) and an object (e.g. cell, tissue, organ, body lumen) with which said agent or composition is contacted. The interaction between the agent or composition and the object can occur starting immediately or nearly immediately with the administration of the agent or composition, can occur over an extended time period (starting immediately or nearly immediately with the administration of the agent or composition), or can be delayed relative to the time of administration of the agent or composition. More specifically the "contacting" results in delivering an effective amount of the agent or composition comprising the agent to the object.

### Nucleic acid or gene therapy compounds

One process of modulating/downregulating expression of a gene of interest (such as a MAPK-signaling pathway component or RXRG) relies on antisense oligonucleotides (ASOs), or variants thereof such as gapmers. An antisense oligonucleotide (ASO) is a short strand of nucleotides and/or nucleotide analogues that hybridizes with the complementary mRNA in a sequence-specific manner via Watson-Crick base pairing. Formation of the ASO-mRNA complex ultimately results in downregulation of target protein expression (Chan et al. 2006, Clin Exp Pharmacol Physiol 33:533-540; this reference also describes some of the software available for assisting in design of ASOs). Modifications to ASOs can be introduced at one or more levels: phosphate linkage modification (e.g. introduction of one or more of phosphodiester, phosphoramidate or phosphorothioate bonds), sugar modification (e.g. introduction of one or more of LNA (locked nucleic acids), 2'-O-methyl, 2'-O-methoxy-ethyl, 2'-fluoro, S-constrained ethyl or tricyclo-DNA and/or non-ribose modifications (e.g. introduction of one or more of phosphorodiamidate morpholinos or peptide nucleic acids). The introduction of 2'-modifications has been shown to enhance safety and pharmacologic properties of antisense oligonucleotides. Antisense strategies relying on degradation of mRNA by RNase H requires the presence of nucleotides with a free 2'-oxygen, i.e. not all nucleotides in the antisense molecule should be 2'-modified. The gapmer strategy has been developed to this end. A gapmer antisense oligonucleotide consists of a central DNA region (usually a minimum of 7 or 8 nucleotides) with (usually 2 or 3) 2'-modified nucleosides flanking both ends of the central DNA region. This is sufficient for the protection against exonucleases while allowing RNAseH to act on the (2'-modification free) gap region. Antidote strategies are available as demonstrated by administration of an oligonucleotide fully complementary to the antisense oligonucleotide (Crosby et al. 2015, Nucleic Acid Ther 25:297-305).

Another process to modulate expression of a gene of interest (such as a MAPK-signaling pathway component or RXRG) is based on the natural process of RNA interference. It relies on double-stranded RNA (dsRNA) that is cut by an enzyme called Dicer, resulting in double stranded small interfering RNA (siRNA) molecules which are 20-25 nucleotides long. siRNA then binds to the cellular RNA-Induced Silencing Complex (RISC) separating the two strands into the passenger and guide strand. While the passenger strand is degraded, RISC is cleaving mRNA specifically at a site instructed by the guide strand. Destruction of the mRNA prevents production of the protein of interest and the gene is 'silenced'. siRNAs are dsRNAs with 2 nt 3' end overhangs whereas shRNAs are dsRNAs that contains a loop structure that is processed to siRNA. shRNAs are introduced into the nuclei of target cells using a vector (e.g. bacterial or viral) that optionally can stably integrate into the genome . Apart from checking for lack of cross-reactivity with non-target genes, manufacturers of RNAi products provide guidelines for designing siRNA/shRNA. siRNA sequences between 19-29 nt are generally the most effective. Sequences longer than 30 nt can result in nonspecific silencing. Ideal sites to target include AA dinucleotides and the 19 nt 3' of them in the target mRNA sequence. Typically, siRNAs with 3' dUdU or dTdT dinucleotide overhangs are more effective. Other dinucleotide overhangs could maintain activity but GG overhangs should be avoided. Also to be avoided are siRNA designs with a 4-6 poly(T) tract (acting as a termination signal for RNA pol III), and the G/C content is advised to be between 35-55%. shRNAs should comprise sense and antisense sequences (advised to each be 19-21 nt in length) separated by loop structure, and a 3' AAAA overhang. Effective loop structures are suggested to be 3-9 nt in length. It is suggested to follow the sense-loop-antisense order in designing the shRNA cassette and to avoid 5' overhangs in the shRNA construct. shRNAs are usually transcribed from vectors, e.g. driven by the Pol III U6 promoter or H1 promoter. Vectors allow for inducible shRNA expression, e.g. relying on the Tet-on and Tet-off inducible systems commercially available, or on a modified U6 promoter that is induced by the insect hormone ecdysone. A Cre-Lox recombination system has been used to achieve controlled expression in mice. Synthetic shRNAs can be chemically modified to affect their activity and stability. Plasmid DNA or dsRNA can be delivered to a cell by means of transfection (lipid transfection, cationic polymer-based nanoparticles, lipid or cell-penetrating peptide conjugation) or electroporation. Viral vectors include lentiviral, retroviral, adenoviral and adeno-associated viral vectors.

Ribozymes (ribonucleic acid enzymes) are another type of molecules that can be used to modulate expression of a target gene. They are RNA molecules capable of catalyzing specific biochemical reactions, in the current context capable of targeted cleavage of nucleotide sequences. Examples of ribozymes include the hammerhead ribozyme, the Varkud Satellite ribozyme, Leadzyme and the hairpin ribozyme. Besides the use of the inhibitory RNA technology, modulation of expression of a gene of interest can be achieved at DNA level such as by gene therapy to knock-out or disrupt the target gene. As used herein, a "gene knock-out" can be a gene knockdown or the gene can be knocked out by a mutation such as, a point mutation, an insertion, a deletion, a frameshift, or a missense mutation by techniques such as described hereafter, including, but not limited to, retroviral gene transfer. Another way in which genes can be knocked out is by the use of zinc finger nucleases. Zinc-finger nucleases (ZFNs) are artificial restriction enzymes generated by fusing a zinc finger DNA-binding domain to a DNA-cleavage domain. Zinc finger domains can be engineered to target desired DNA sequences, which enable zinc-finger nucleases to target unique sequence within a complex genome. By taking advantage of the endogenous DNA repair machinery, these reagents can be used to precisely alter the genomes of higher organisms. Other technologies for genome customization that can be used to knock out genes are meganucleases and TAL effector nucleases (TALENs, Cellectis bioresearch). A TALEN^{®} is composed of a TALE DNA binding domain for sequence-specific recognition fused to the catalytic domain of an endonuclease that introduces double strand breaks (DSB). The DNA binding domain of a TALEN^{®} is capable of targeting with high precision a large recognition site (for instance 17bp). Meganucleases are sequence-specific endonucleases, naturally occurring "DNA scissors", originating from a variety of single-celled organisms such as bacteria, yeast, algae and some plant organelles. Meganucleases have long recognition sites of between 12 and 30 base pairs. The recognition site of natural meganucleases can be modified in order to target native genomic DNA sequences (such as endogenous genes). Another recent genome editing technology is the CRISPR/Cas system, which can be used to achieve RNA-guided genome engineering. CRISPR interference is a genetic technique which allows for sequence-specific control of gene expression in prokaryotic and eukaryotic cells. It is based on the bacterial immune system-derived CRISPR (clustered regularly interspaced palindromic repeats) pathway. Recently, it was demonstrated that the CRISPR-Cas editing system can also be used to target RNA. It has been shown that the Class 2 type VI-A CRISPR-Cas effector C2c2 can be programmed to cleave single stranded RNA targets carrying complementary protospacers (Abudayyeh et al. 2016 Science353/science.aaf5573). C2c2 is a single-effector endoRNase mediating ssRNA cleavage once it has been guided by a single crRNA guide toward the target RNA.

Methods for administering nucleic acids include methods applying non-viral (DNA or RNA) or viral nucleic acids (DNA or RNA viral vectors). Methods for non-viral gene therapy include the injection of naked DNA (circular or linear), electroporation, the gene gun, sonoporation, magnetofection, the use of oligonucleotides, lipoplexes (e.g. complexes of nucleic acid with DOTAP or DOPE or combinations thereof, complexes with other cationic lipids), dendrimers, viral-like particles, inorganic nanoparticles, hydrodynamic delivery, photochemical internalization (Berg et al. 2010, Methods Mol Biol 635:133-145) or combinations thereof.

Many different vectors have been used in human nucleic acid therapy trials and a listing can be found on http://www.abedia.com/wiley/vectors.php. Currently the major groups are adenovirus or adeno-associated virus vectors (in about 21% and 7% of the clinical trials), retrovirus vectors (about 19% of clinical trials), naked or plasmid DNA (about 17% of clinical trials), and lentivirus vectors (about 6% of clinical trials). Combinations are also possible, e.g. naked or plasmid DNA combined with adenovirus, or RNA combined with naked or plasmid DNA to list just a few. Other viruses (e.g. alphaviruses) are used in nucleic acid therapy and are not excluded in the context of the current invention.

Administration may be aided by specific formulation of the nucleic acid e.g. in liposomes (lipoplexes) or polymersomes (synthetic variants of liposomes), as polyplexes (nucleic acid complexed with polymers), carried on dendrimers, in inorganic (nano)particles (e.g. containing iron oxide in case of magnetofection), or combined with a cell penetrating peptide (CPP) to increase cellular uptake. Organ- or cellular-targeting strategies may also be applied to the nucleic acid (nucleic acid combined with organ- or cell-targeting moiety); these include passive targeting (mostly achieved by adapted formulation) or active targeting (e.g. by coupling a nucleic acid-comprising nanoparticle with any compound (e.g. an aptamer or antibody or antigen binding molecule) binding to a target organ- or cell-specific antigen) (e.g. Steichen et al. 2013, Eur J Pharm Sci 48:416-427).

CPPs enable translocation of the drug of interest coupled to them across the plasma membrane. CPPs are alternatively termed Protein Transduction Domains (TPDs), usually comprise 30 or less (e.g. 5 to 30, or 5 to 20) amino acids, and usually are rich in basic residues, and are derived from naturally occurring CPPs (usually longer than 20 amino acids), or are the result of modelling or design. A non-limiting selection of CPPs includes the TAT peptide (derived from HIV-1 Tat protein), penetratin (derived from Drosophila Antennapedia - Antp), pVEC (derived from murine vascular endothelial cadherin), signal-sequence based peptides or membrane translocating sequences, model amphipathic peptide (MAP), transportan, MPG, polyarginines; more information on these peptides can be found in Torchilin 2008 (Adv Drug Deliv Rev 60:548-558) and references cited therein. CPPs can be coupled to carriers such as nanoparticles, liposomes, micelles, or generally any hydrophobic particle. Coupling can be by absorption or chemical bonding, such as via a spacer between the CPP and the carrier. To increase target specificity an antibody binding to a target-specific antigen can further be coupled to the carrier (Torchilin 2008, Adv Drug Deliv Rev 60:548-558). CPPs have already been used to deliver payloads as diverse as plasmid DNA, oligonucleotides, siRNA, peptide nucleic acids (PNA), proteins and peptides, small molecules and nanoparticles inside the cell (Stalmans et al. 2013, PloS One 8:e71752).

Any other modification of the DNA or RNA to enhance efficacy of nucleic acid therapy is likewise envisaged to be useful in the context of the applications of the nucleic acid or nucleic acid comprising compound as outlined herein. The enhanced efficacy can reside in enhanced expression, enhanced delivery properties, enhanced stability and the like. The applications of the nucleic acid or nucleic acid comprising compound as outlined herein may thus rely on using a modified nucleic acid as described above. Further modifications of the nucleic acid may include those suppressing inflammatory responses (hypoinflammatory nucleic acids).

### Biopharmaceutical agents

Interfering with structure, which can result in inhibition or activation of function, can be achieved by e.g. binding moieties binding to the protein of interest (such as a MAPK-signaling pathway component or RXRG). Non-limiting examples are (monoclonal) antibodies or antigen-binding fragments thereof, alphabodies, nanobodies, intrabodies (antibodies binding and/or acting to intracellular target; this typically requires the expression of the antibody within the target cell, which can be accomplished by gene therapy), aptamers, DARPins, affibodies, affitins, anticalins, monobodies, phosphatases (in case of phosphorylated target) and kinases (in case of a phosphorylatable target).

The term "antibody" as used herein refers to any naturally occurring format of antibody or antigen binding protein the production of which is induced by an immune system (immunoglobulins or IgGs). It is clear, however, that not all antibodies are naturally occurring as e.g. some antigens are problematic in the sense that they are poor or not at all immunogenic, or are not recognized by the immune system (e.g. self-antigens); artificial tricks may be required to obtain antibodies against such antigens (e.g. knock-out mice: e.g. Declercq et al. 1995, J Biol Chem 270:8397-8400; DNA immunization for e.g. transmembrane antigens; e.g. Liu et al. 2016, Emerg Microbes Infect 5:e33). "Conventional" antibodies comprise two heavy chains linked together by disulfide bonds and two light chains, one light chain being linked to each of the heavy chains by disulfide bonds. Each heavy chain has at one end a variable domain (VH) followed by a number of constant domains (three or four constant domains, CH1, CH2, CH3 and CH4, depending on the antibody class). Each light chain has a variable domain (VL) at one end and a constant domain (CL) at its other end; the constant domains of the light chains each align with the first constant domains of the heavy chains, and the light chain variable domains each align with the variable domains of the heavy chains. This type of antibodies exist in camels, dromedaries and llamas along with an "unconventional" naturally occurring type of antibodies consisting of only two heavy chains, and thus being devoid of light chains. Other "unconventional" naturally occurring antibodies exist in in the serum of nurse sharks (Ginglymostomatidae) and wobbegong sharks (Orectolobidae). These latter antibodies are called Ig new antigen receptors (IgNARs). They are disulfide-bonded homodimers consisting of five constant domains (CNAR) and one variable domain (VNAR). There is no light chain, and the individual variable domains are independent in solution and do not appear to associate across a hydrophobic interface (Greenberg et al. 1995, Nature 374:168- 173; Nuttall et al. 2001, Mol Immunol 38:313-326; Diaz et al. 2002, Immunogenetics 54:501- 512; Nuttall et al. 2003, Eur J Biochem 270:3543-3554). Due to the heavy chain dimer structure characteristic of camelid and shark antibodies, these are sometimes termed "Heavy-Chain Mini- Antibodies" (mnHCAbs) or simply "Mini- Antibodies" (mnAbs) (Holliger & Hudson 2005, Nature Biotechnol 23:1126-1136). Without the light chain, these heavy-chain antibodies bind to their antigens by one single domain, the variable antigen binding domain of the heavy-chain immunoglobulin, referred to as Vab (camelid antibodies) or V-NAR (shark antibodies). These smallest intact and independently functional antigen binding fragment Vab is referred to as nano-antibody or nanobody (Muyldermans 2001, J Biotechnol 74:277-302). Multivalent (etc. divalent, trivalent, tetravalent and pentavalent) Vab and/or V-NAR domains may be preferred in some instances due to their potentially higher cellular intake and retention and may be made by recombinant technology or by chemical means, such as described in WO 2010/033913. The variable domains of the light and/or heavy chains are involved directly in binding the antibody to the antigen. An antibody, or antibody fragment as described hereafter, may also be part of a multivalent and/or multispecific antigen binding molecule. An overview of e.g. available bispecific formats (around 100) is provided in Brinkmann & Kontermann 2017 (mAbs 9:182-212). The term "antibody fragment" refers to any molecule comprising one or more fragments (usually one or more CDRs) of an antibody (the parent antibody) such that it binds to the same antigen to which the parent antibody binds. Antibody fragments include Fv, Fab, Fab', Fab'-SH, singlechain antibody molecules (such as scFv), F(ab') 2, single variable VH domains, and single variable VL domains (Holliger & Hudson 2005, Nature Biotechnol 23:1126-1136), Vab and V-NAR. The term further includes microantibodies, i.e. the minimum recognition unit of a parent antibody usually comprising just one CDR (Heap et al. 2005, J Gen Virol 86:1791-1800). Any of the fragments can be incorporated in a multivalent and/or multispecific larger molecule, e.g. mono-or bi-specific Fab 2, mono-or tri-specific Fab 3, bis-scFv (mono- or bispecific), diabodies (mono-or bi- specific), triabodies (e.g. trivalent monospecific), tetrabodies (e.g. tetravalent monospecific), minibodies and the like (Holliger & Hudson 2005, Nature Biotechnol 23:1126-1136). Any of the fragments can further be incorporated in e.g. V-NAR domains of shark antibodies or VhH domains of camelid antibodies (nanobodies). All these are included in the term "antibody fragment".

Alphabodies are also known as Cell-Penetrating Alphabodies andare small 10 kDa proteins engineered to bind to a variety of antigens.

Aptamers have been selected against small molecules, toxins, peptides, proteins, viruses, bacteria, and even against whole cells. DNA/RNA/XNA aptamers are single stranded and typically around 15-60 nucleotides in length although longer sequences of 220nt have been selected; they can contain nonnatural nucleotides (XNA) as described for antisense RNA. A nucleotide aptamer binding to the vascular endothelial growth factor (VEGF) was approved by FDA for treatment of macular degeneration. Variants of RNA aptamers are spiegelmers are composed entirely of an unnatural L-ribonucleic acid backbone. A Spiegelmer of the same sequence has the same binding properties of the corresponding RNA aptamer, except it binds to the mirror image of its target molecule. Peptide aptamers consist of one (or more) short variable peptide domains, attached at both ends to a protein scaffold, e.g. the Affimer scaffold based on the cystatin protein fold. A further variation is described in e.g. WO 2004/077062 wherein e.g. 2 peptide loops are attached to an organic scaffold. Phage-display screening of such peptides has proven to be possible in e.g. WO 2009/098450.

DARPins stands for designed ankyrin repeat proteins. DARPin libraries with randomized potential target interaction residues, with diversities of over 10^12 variants, have been generated at the DNA level. From these, DARPins can be selected for binding to a target of choice with picomolar affinity and specificity. Affitins, or nanofitins, are artificial proteins structurally derived from the DNA binding protein Sac7d, found in Sulfolobus acidocaldarius. By randomizing the amino acids on the binding surface of Sac7d and 5 subjecting the resulting protein library to rounds of ribosome display, the affinity can be directed towards various targets, such as peptides, proteins, viruses, and bacteria.

Anticalins are derived from human lipocalins which are a family of naturally binding proteins and mutation of amino acids at the binding site allows for changing the affinity and selectivity towards a 10 target of interest. They have better tissue penetration than antibodies and are stable at temperatures up to 70°C.

Monobodies are synthetic binding proteins that are constructed starting from the fibronectin type III domain (FN3) as a molecular scaffold.

In the above, the molecules are specific to their intended target, which is referring to the fact that the molecules are acting at the level of the intended target and not at the level of target different from the intended target. Specificity can be ascertained by e.g. determining physical interaction of the molecules to their intended target.

### Gene expression level

The term "level of expression" or "expression level" generally refers to the amount of a biomarker in a biological sample. "Expression" generally refers to the process by which information (e.g., gene- encoded and/or epigenetic information) is converted into the structures present and operating in the cell. Therefore, as used herein, "expression" may refer to transcription into a polynucleotide, translation into a polypeptide, or even polynucleotide and/or polypeptide modifications (e.g., posttranslational modification of a polypeptide). Fragments of the transcribed polynucleotide, the translated polypeptide, or polynucleotide and/or polypeptide modifications (e.g., posttranslational modification of a polypeptide) are also regarded as expressed whether they originate from a transcript generated by alternative splicing or a degraded transcript, or from a post-translational processing of the polypeptide, e.g., by proteolysis. "Expressed genes" include those that are transcribed into a polynucleotide as mRNA and then translated into a polypeptide, and also those that are transcribed into RNA but not translated into a polypeptide (for example, transfer and ribosomal RNAs).

"Increased expression," "increased expression level," "increased levels," "elevated expression," "elevated expression levels," or "elevated levels" refers to an increased expression or increased levels of a biomarker in an individual relative to a control, such as an individual or individuals who do not have the disease or disorder (e.g., cancer), an internal control (e.g., a housekeeping biomarker), a median expression level of the biomarker in samples from a group/population of patients, or relative to an expression level of the biomarker in samples taken before onset of a certain therapy.

The term "detection" includes any means of detecting, including direct and indirect detection. The term "biomarker" as used herein refers to an indicator molecule or set of molecules (e.g., predictive, diagnostic, and/or prognostic indicator), which can be detected in a sample. The biomarker may be a predictive biomarker and serve as an indicator of the likelihood of sensitivity or benefit of a patient having a particular disease or disorder (e.g., a proliferative cell disorder (e.g., cancer)) to treatment. Biomarkers include, but are not limited to, polynucleotides (e.g., DNA and/or RNA (e.g., mRNA)), polynucleotide copy number alterations (e.g., DNA copy numbers), polypeptides, polypeptide and polynucleotide modifications (e.g., post-translational modifications), carbohydrates, and/or glycolipidbased molecular markers. In some embodiments, a biomarker is a gene. The "amount" or "level" of a biomarker, as used herein, is a detectable level in a biological sample. These can be measured by methods known to one skilled in the art and also disclosed herein.

Any gene detection or gene expression detection method is starting from an analyte nucleic acid and may be defined as comprising one or more of, for instance,
- a step of isolating RNA from a biological sample;
- a step of reverse transcribing the RNA obtained from the biological sample into DNA;
- a step of amplifying the isolated DNA; and/or
- a step of quantifying the isolated RNA, the DNA obtained after reverse transcription, or the amplified DNA.

In case an amplified DNA is quantified, this quantification step can be performed concurrent with the amplification of the DNA, or is performed after the amplification of the DNA.

The quantification of gene expression or the determination of gene expression levels may be based on at least one of an amplification reaction, a sequencing reaction, a melting reaction, a hybridization reaction or a reverse hybridization reaction.

### Detection and quantification of gene expression

The invention covers methods for detecting the presence of nucleic acids corresponding to one or more gene(s) as defined herein (gene expression signatures specific for the tumor cell subpopulations defined herein) in a biological sample and/or methods for determining or detecting the expression level of one or more gene(s) as defined herein, wherein said methods comprise the step of detecting the presence of a gene of interest nucleic acid or expression level of a gene of interest. In any of these methods the detection can comprise a step such as a nucleic acid amplification reaction, a nucleic acid sequencing reaction, a melting reaction, a hybridization reaction to a nucleic acid, or a reverse hybridization reaction to a nucleic acid, or a combination of such steps.

Often one or more artificial, man-made, non-naturally occurring oligonucleotide is used in such method. In particular, such oligonucleotides can comprise besides ribonucleic acid monomers or deoxyribonucleic acid monomers: one or more modified nucleotide bases, one or more modified nucleotide sugars, one or more labeled nucleotides, one or more peptide nucleic acid monomers, one or more locked nucleic acid monomers, the backbone of such oligonucleotide can be modified, and/or non-glycosidic bonds may link two adjacent nucleotides. Such oligonucleotides may further comprise a modification for attachment to a solid support, e.g., an amine-, thiol-, 3-'propanolamine or acrydite-modification of the oligonucleotide, or may comprise the addition of a homopolymeric tail (for instance an oligo(dT)-tail added enzymatically via a terminal transferase enzyme or added synthetically) to the oligonucleotide. If said homopolymeric tail is positioned at the 3'-terminus of the oligonucleotide or if any other 3'-terminal modification preventing enzymatic extension is incorporated in the oligonucleotide, the priming capacity of the oligonucleotide can be decreased or abolished. Such oligonucleotides may also comprise a hairpin structure at either end. Terminal extension of such oligonucleotide may be useful for, e.g., specifically hybridizing with another nucleic acid molecule (e.g. when functioning as capture probe), and/or for facilitating attachment of said oligonucleotide to a solid support, and/or for modification of said tailed oligonucleotide by an enzyme, ribozyme or DNAzyme. Such oligonucleotides may be modified in order to detect (the levels of) a target nucleotide sequence and/or to facilitate in any way such detection. Such modifications include labeling with a single label, with two different labels (for instance two fluorophores or one fluorophore and one quencher), the attachment of a different 'universal' tail to two probes or primers hybridizing adjacent or in close proximity to each other with the target nucleotide sequence, the incorporation of a target-specific sequence in a hairpin oligonucleotide (for instance Molecular Beacontype primer), the tailing of such a hairpin oligonucleotide with a 'universal' tail (for instance Sunrise-type probe and Amplifluor TM -type primer). A special type of hairpin oligonucleotide incorporates in the hairpin a sequence capable of hybridizing to part of the newly amplified target DNA. Amplification of the hairpin is prevented by the incorporation of a blocking nonamplifiable monomer (such as hexethylene glycol). A fluorescent signal is generated after opening of the hairpin due to hybridization of the hairpin loop with the amplified target DNA. This type of hairpin oligonucleotide is known as scorpion primers (Whitcombe et al. 1999, Nat Biotechnol 17:804-807). Another special type of oligonucleotide is a padlock oligonucleotide (or circularizable, open circle, or C-oligonucleotide) that are used in RCA (rolling circle amplification). Such oligonucleotides may also comprise a 3'-terminal mismatching nucleotide and/or, optionally, a 3'-proximal mismatching nucleotide, which can be particularly useful for performing polymorphism-specific PCR and LCR (ligase chain reaction) or any modification of PCR or LCR. Such oligonucleotide may can comprise or consist of at least and/or comprise or consist of up to 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200 or more contiguous nucleotides.

The analyte nucleic acid, in particular the analyte nucleic acid of a gene of interest (a gene from a gene expression signature specific for the tumor cell subpopulations defined herein and which is used for detection or which is used to determine the relative expression level) can be any type of nucleic acid, which will be dependent on the manipulation steps (such as isolation and/or purification and/or duplication, multiplication or amplification) applied to the nucleic acid of the gene of interest in the biological sample; as such it can be DNA, RNA, cDNA, may comprise modified nucleotides, or may be hybrids of DNA and/or RNA and/or modified nucleotides, and can be single- or double-stranded or may be a triplex-forming nucleic acid.

The artificial, man-made, non-naturally occurring oligonucleotide(s) as applied in the above detection methods can be probe(s) or a primer(s), or a combination of both.

A probe capable of specifically hybridizing with a target nucleic acid is an oligonucleotide mainly hybridizing to one specific nucleic acid sequence in a mixture of many different nucleic acid sequences. Specific hybridization is meant to result, upon detection of the specifically formed hybrids, in a signal-to-noise ratio (wherein the signal represents specific hybridization and the noise represents unspecific hybridization) sufficiently high to enable unambiguous detection of said specific hybrids. In a specific case specific hybridization allows discrimination of up to a single nucleotide mismatch between the probe and the target nucleic acids. Conditions allowing specific hybridization generally are stringent but can obviously be varied depending on the complexity (size, GC-content, overall identity, etc.) of the probe(s) and/or target nucleic acid molecules. Specificity of a probe in hybridizing with a nucleic acid can be improved by introducing modified nucleotides in said probe.

A primer capable of directing specific amplification of a target nucleic acid is the at least one oligonucleotide in a nucleic acid amplification reaction mixture that is required to obtain specific amplification of a target nucleic acid. Nucleic acid amplification can be linear or exponential and can result in an amplified single nucleic acid of a single- or double-stranded nucleic acid or can result in both strands of a double-stranded nucleic acid. Specificity of a primer in directing amplification of a nucleic acid can be improved by introducing modified nucleotides in said primer. The fact that a primer does not have to match exactly with the corresponding template or target sequence to warrant specific amplification of said template or target sequence is amply documented in literature (for instance: Kwok et al. 1990, Nucl Acids Res 18:999-1005. Primers as short as 8 nucleotides in length have been applied successfully in directing specific amplification of a target nucleic acid molecule (e.g. Majzoub et al. 1983, J Biol Chem 258:14061-14064).

A nucleotide is meant to include any naturally occurring nucleotide as well as any modified nucleotide wherein said modification can occur in the structure of the nucleotide base (modification relative to A, T, G, C, or U) and/or in the structure of the nucleotide sugar (modification relative to ribose or deoxyribose). Any of the modifications can be introduced in a nucleic acid or oligonucleotide to increase/decrease stability and/or reactivity of the nucleic acid or oligonucleotide and/or for other purposes such as labeling of the nucleic acid or oligonucleotide. Modified nucleotides include phophorothioates, alkylphophorothioates, methylphosphonate, phosphoramidate, peptide nucleic acid monomers and locked nucleic acid monomers, cyclic nucleotides, and labeled nucleotides (i.e. nucleotides conjugated to a label which can be isotopic (<32>P, <35>S, etc.) or non-isotopic (biotin, digoxigenin, phosphorescent labels, fluorescent labels, fluorescence quenching moiety, etc.)). Other modifications are described higher (see description on oligonucleotides).

Nucleotide acid amplification is meant to include all methods resulting in multiplication of the number of a target nucleic acid. Nucleotide sequence amplification methods include the polymerase chain reaction (PCR; DNA amplification), strand displacement amplification (SDA; DNA amplification), transcription-based amplification system (TAS; RNA amplification), self-sustained sequence replication (3SR; RNA amplification), nucleic acid sequence-based amplification (NASBA; RNA amplification), transcription-mediated amplification (TMA; RNA amplification), Qbeta-replicase-mediated amplification and run-off transcription. During amplification, the amplified products can be conveniently labeled either using labeled primers or by incorporating labeled nucleotides.

The most widely spread nucleotide sequence amplification technique is PCR. The target DNA is exponentially amplified. Many methods rely on PCR including AFLP (amplified fragment length polymorphism), IRS-PCR (interspersed repetitive sequence PCR), iPCR (inverse PCR), RAPD (rapid amplification of polymorphic DNA), RT-PCR (reverse transcription PCR) and real-time PCR. RT-PCR can be performed with a single thermostable enzyme having both reverse transcriptase and DNA polymerase activity (Myers et al. 1991, Biochem 30:7661-7666). Alternatively, a single tube-reaction with two enzymes (reverse transcriptase and thermostable DNA polymerase) is possible (Cusi et al. 1994, Biotechniques 17:1034-1036).

Solid phases, solid matrices or solid supports on which molecules, e.g., nucleic acids, analyte nucleic acids and/or oligonucleotides as described hereinabove, may be bound (or captured, absorbed, adsorbed, linked, coated, immobilized; covalently or non-covalently) comprise beads or the wells or cups of microtiter plates, or may be in other forms, such as solid or hollow rods or pipettes, particles, e.g., from 0.1 mu m to 5 mm in diameter (e.g. "latex" particles, protein particles, or any other synthetic or natural particulate material), microspheres or beads (e.g. protein A beads, magnetic beads). A solid phase may be of a plastic or polymeric material such as nitrocellulose, polyvinyl chloride, polystyrene, polyamide, polyvinylidene fluoride or other synthetic polymers. Other solid phases include membranes, sheets, strips, films and coatings of any porous, fibrous or bibulous material such as nylon, polyvinyl chloride or another synthetic polymer, a natural polymer (or a derivative thereof) such as cellulose (or a derivative thereof such as cellulose acetate or nitrocellulose). Fibers or slides of glass, fused silica or quartz are other examples of solid supports. Paper, e.g., diazotized paper may also be applied as solid phase. Clearly, molecules such as nucleic acids, analyte nucleic acids and/or oligonucleotides as described hereinabove, may be bound, captured, absorbed, adsorbed, linked or coated to any solid phase suitable for use in hybridization assay (irrespective of the format, for instance capture assay, reverse hybridization assay, or dynamic allele-specific hybridization (DASH)). Said molecules, such as nucleic acids, analyte nucleic acids and/or oligonucleotides as described hereinabove, can be present on a solid phase in defined zones such as spots or lines. Such solid phases may be incorporated in a component such as a cartridge of e.g. an assay device. Any of the solid phases described above can be developed, e.g. automatically developed in an assay device.

Quantification of amplified DNA can be performed concurrent with or during the amplification. Techniques include real-time PCR or (semi-)quantitative polymerase chain reaction (qPCR). One common method includes measurement of a non-sequence specific fluorescent dye (e.g. SYBR Green) intercalating in any double-stranded DNA. Quantification of multiple amplicons with different melting points can be followed simultaneously by means of following or analyzing the melting reaction (melting curve analysis or melt curve analysis; which can be performed at high resolution, see, e.g. Wittwer et al. 2003, Clin Chem 843-860; an alternative method is denaturing gel gradient electrophoresis, DGGE; both methods were compared in e.g. Tindall et al. 2009, Hum Mutat 30:857-859).

Another common method includes measurement of sequence-specific labelled probe bound to its complementary sequence; such probe also carries a quencher and the label is only measurable upon exonucleolytic release from the probe (hydrolysis probes such as TaqMan probes) or upon hybridization with the target sequence (hairpin probes such as molecular beacons which carry an internally quenched fluorophore whose fluorescence is restored upon unfolding the hairpin). This latter method allows for multiplexing by e.g. using mixtures of probes each tagged with a different label e.g. fluorescing at a different wavelength.

Exciton-controlled hybridization-sensitive fluorescent oligonucleotide (ECHO) probes also allow for multiplexing. The hybridization-sensitive fluorescence emission of ECHO probes and the further modification of probes have made possible multicolor RNA imaging in living cells and facile detection of gene polymorphisms (Okamoto 2011, Chem Soc Rev, 40:5815-5828).

Other methods of quantifying expression include SAGE (Serial Analysis of Gene Expression) and MPSS (Massively Parallel Signature Sequencing), each involving reverse-transcription of RNA.

With "assaying" or "determining" is meant that a biological sample, suspected of comprising a target nucleic acid (such as a nucleic acid of interest as described herein), is processed as to generate a readable signal in case the target nucleic acid is actually present in the biological sample. Such processing may include, as described above, a step of producing an analyte nucleic acid. Simple detection of a produced readable signal indicates the presence of a target or analyte nucleic acid in the biological sample. When in addition the amplitude of the produced readable signal is determined, this allows for quantification of levels of a target or analyte nucleic acid as present in a biological sample.

In particular, the readable signal may be a signal-to-noise ratio (wherein the signal represents specific detection and the noise represents unspecific detection) of an assay optimized to yield signal-to-noise ratios sufficiently high to enable unambiguous detection and/or quantification of the target nucleic acid. The noise signal, or background signal, can be determined e.g. on biological samples not comprising the target or analyte nucleic acid of interest, e.g. control samples, or comprising the required reference level of the target or analyte nucleic acid of interest, e.g referenced samples. Such noise or background signal may also serve as comparator value for determining an increase or decrease of the level of a target or analyte nucleic acid in the biological sample, e.g. in a biological sample taken from a subject suffering from a disease or disorder, further e.g. before start of a treatment and during treatment.

The readable signal may be produced with all required components in solution or may be produced with some of the required components in solution and some bound to a solid support. Said signals include, e.g., fluorescent signals, (chemi)luminescent signals, phosphorescence signals, radiation signals, light or color signals, optical density signals, hybridization signals, mass spectrometric signals, spectrometric signals, chromatographic signals, electric signals, electronic signals, electrophoretic signals, real-time PCR signals, PCR signals, LCR signals, Invader-assay signals, sequencing signals (by any method such as Sanger dideoxy sequencing, pyrosequencing, 454 sequencing, single-base extension sequencing, sequencing by ligation, sequencing by synthesis, "next-generation" sequencing (van Dijk et al. 2014, Trends Genet 30:418-426)), melting curve signals etc. An assay may be run automatically or semiautomatically in an assay device.

### Other Definitions

The present invention is described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., current Protocols in Molecular Biology (Supplement 100), John Wiley & Sons, New York (2012), for definitions and terms of the art. The definitions provided herein should not be construed to have a scope less than understood by a person of ordinary skill in the art.

The term "defined by SEQ ID NO:X" as used herein refers to a biological sequence consisting of the sequence of amino acids or nucleotides given in the SEQ ID NO:X. For instance, an antigen defined in/by SEQ ID NO:X consists of the amino acid sequence given in SEQ ID NO:X. A further example is an amino acid sequence comprising SEQ ID NO:X, which refers to an amino acid sequence longer than the amino acid sequence given in SEQ ID NO:X but entirely comprising the amino acid sequence given in SEQ ID NO:X (wherein the amino acid sequence given in SEQ ID NO:X can be located N-terminally or C-terminally in the longer amino acid sequence, or can be embedded in the longer amino acid sequence), or to an amino acid sequence consisting of the amino acid sequence given in SEQ ID NO:X.

The description of the genes as included hereinabove refer to "NCBI reference mRNA sequences", these can be found be searching e.g. GenBank. The listed reference mRNA sequences all refer to human sequences and neither of the listings is meant to be exhaustive. Based on the listed sequences, a skilled person will be able to retrieve e.g. genomic sequences, other mRNA sequences and encoded protein sequences either of human or other mammalian origin (e.g. by applying the BLAST tool publicly available via e.g. NCBI).

The following examples are provided to better illustrate particular embodiments. The application is limited only by the claims.

### EXAMPLES

### 1. MATERIAL and METHODS

### 1.1. Patient-derived xenografts (PDX)

In collaboration with TRACE (PDX platform at the University of Leuven), PDX models were established using tissue from patients undergoing surgery as part of standard-of-care melanoma treatment at the University Hospitals KU Leuven. Written informed consent was obtained from all patients and all procedures involving human samples were approved by the UZ Leuven Medical Ethical Committee (S54185/S57760/S59199) and carried out in accordance with the principles of the Declaration of Helsinki. All procedures involving animals were performed in accordance with the guidelines of the IACUC of KU Leuven and within the context of approved project applications P147/2012, P038/2015, P098/2015 and P035/2016. Fresh tumor tissue was collected in transport medium (RPMI1640 medium supplemented with 1% penicillin/streptomycin, 1 µg/mL amphotericin B and 50 µg/mL gentamicin; all from Thermo Fisher Scientific).

Tumor fragments were subsequently rinsed in phosphate-buffered saline (PBS; Thermo Fisher Scientific) supplemented with penicillin/streptomycin and amphotericin B and cut into small pieces of approximately 3 x 3 x 3 mm3. Tumor pieces were implanted subcutaneously in the intercapsular fat pad of female SCID-beige mice (Taconic). Sedation was performed using 75 mg/kg ketamine and 100 mg/kg medetomidine and reversed by 1 mg/kg atipamezole after the procedure. Mice also received 0.05 mg/kg buprenorphine for analgesia. After reaching generation 4 (F4), one mouse with a tumor of 1000 mm³ was sacrificed. This tumor was minced followed by dissociation using collagenase I & IV (Sigma) and trypsin (Life Technologies). Cells were resuspended in serum-free DMEM/F12 medium (Thermo Fisher Scientific) and 250 000 cells were injected in the interscapular fat pad of 8 - 16 week old female NMRI nude mice (Taconic).

For single cell RNA sequencing purposes, cells were transduced with a lentivirus carrying dsRed: 20 min at room temperature followed by 30 min infection at 32°C. Cells were washed four times before injecting into the interscapular fat pad.

For immunohistochemistry, non-dsRed-transduced tumors were used. For FACS, tumors were enzymatically dissociated using the same protocol.

### 1.2. Pharmacologic treatment of mice

Mice with tumors reaching 1000 mm³ were started on the BRAF-MEK combination via daily oral gavage (Instech Laboratories). BRAF inhibitor dabrafenib (Tafinlar capsules) and MEK inhibitor trametinib (MCE) were dissolved in DMSO at a concentration of 30 and 0.3 mg/mL respectively, aliquoted and stored at - 80°C.

Each day 900 µL PBS (Sigma) was added to a new aliquot. Mice were treated with a capped dose of 600 - 6 µg dabrafenib - trametinib respectively in 200 µL total volume. Tumor volume was monitored with a caliper and the volume was calculated using the following formula: V = (π/6)*length*width*height.

### 1.3. Immunohistochemistry

Tumor biopsies were formalin-fixed, paraffin embedded and cut in sections of about 5 µm. Samples were deparaffinized and dehydrated with xylene and graded alcohols, and subsequently rehydrated with demineralized water.

Immunostainings were performed with following antibodies: AQP1 AB2219 (Millipore) 1:5000 for light microscopy 1:4000 for fluorescence; AXL AF154 (R&D Systems) 1:45; Ki67 (clone sp6) RM-9106-S0 (Thermo Fisher Scientific) 1:200; MEFC HPA005533 (Sigma) 1:500; MelanA HPA048662 (Sigma) 1:200; MITF HPA003259 (Sigma) 1:100; NGFR #8238 (Cell Signaling Technology) 1:3000, phospho-ERK 4370 (Cell Signaling Technology) 1:200; RXRG ab15518 (Abcam) 1:300; S100 ready-to-use (Dako); SOX2 ab92494 (Abcam) 1:300; SOX10 sc-17342 (Santa Cruz) 1:75 and TFAP2B HPA034683 (Sigma), 1:300. Epitope retrieval was carried out at pH6 (citrate) with the exception of NGFR and AXL both at pH9 (for double stainings with these antibodies, pH9 was chosen). Light microscopy stainings were performed with Leica Bond automated stainer (AEC single and DAB/AEC double stainings). Fluorescent stainings were performed with following secondary antibodies: donkey anti-rabbit Alexa Fluor^{®} 488 and donkey anti-goat Alexa Fluor^{®} 594 (Thermo Fisher Scientific) with DAPI (Thermo Fisher Scientific) counterstain. AQP1 - NGFR double stainings were carried out with donkey anti-rabbit Fab fragment Alexa Fluor^{®} 488, followed by blocking with unconjugated Fab fragment donkey anti-rabbit, followed by Fab fragment Alexa Fluor^{®} 594 (Jackson Immunoresearch). Fontana-Masson silver method histochemistry was performed with working silver solution and fast red counterstain. Images were acquired on the Zeiss Axio Scan.Z1 using x20 and x40 objectives and ZEN 2 software. Light microscopy images were light and contrast adjusted, fluorescent images were brightness and contrast adjusted.

### 1.4. Cell culturing

Cells from dissociated MEL006 tumors were grown in 5% CO2 at 37°C in F10 (Gibco, Life Technologies) supplemented with 10% FBS (Gibco, Life Technologies) and 0.25% Glutamax^{®} (Gibco, Life Technologies).

### 1.5. FACS

Cells were incubated with GFRA2 antibody (AF429 R&D 3 µg/mL) for 45 minutes at room temperature, followed by anti-goat secondary antibody conjugated with Alexa Fluor^{®} 594 (ThermoFisherScientific) for 30 min at room temperature. Cells were resuspended in FACS sorting buffer (culture medium supplied with 1% EDTA 100 mM). FACS analyses were performed with FACSARIA III (BD) and images made with FlowJo^{®}.

### 1.6. Single cell sorting

Living (SYTOXblue negative, Thermofisher), dsRED positive single cells were sorted (FACS ARIA III, BD) in 96 well plates (VWR, DNAse, RNAse free) containing 2ul of lysis buffer (0.2% Triton-X100, 4U of RNase inhibitor, Takara) per well. Plates were properly sealed and spun down at 2000g for 1min before storing at -80°C.

### 1.7. SMARTseq2

Whole transcriptome amplification was performed with a modified SMART-seq2 protocol as described previously (Picelli et al. 2014, Nature Protocols 9:171-181), using 20 instead of 18 cycles of cDNA amplification. PCR purification was realized with a 0.8:1 ratio (ampureXP beads:DNA). Amplified cDNA quality was monitored with a high sensitivity DNA chip (Agilent) using the Bioanalyzer (Agilent).

### 1.8. Library preparation and RNA-sequencing

Sequencing libraries were performed using the Nextera XT kit (Illumina) as described previously (Picelli et al. 2014, Nature Protocols 9:171-181), using 1/4th of the recommended reagent volumes and 1/5th of input DNA with a tagmentation time of 9min and a clean-up. Library quality was monitored with a high sensitivity DNA chip (Agilent) using the Bioanalyzer (Agilent). Indexing was performed with the Nextera XT index Kit V2 (A-D). Up to 4x96 single cells were per sequencing lane. Samples were sequenced on the Illumina NextSeq 500 platform using 75bp single-end reads.

### 1.9. RNA-seq analysis

BAM files were converted to merged, demultiplexed FASTQ files, cleaned using fastq-mcf (ea-utils r819), and QC checked with FastQC (0.11.4). Reads were then mapped to the human genome (hg19) using STAR (2.4.1b) and quantified with Subread (1.4.6-p2). Cells with less than 100,000 reads and/or 1000 genes expressed were discarded. Furthermore, only cells with an average expression level >3 of a curated list of housekeeping genes (n=85) were kept (Tirosh et al 2016, Science 352:189-196). 760 of 937 sequenced cells passed these quality criteria. Subsequently, we applied an even more stringent workflow to detect low-quality cells based on: library size, genes expressed per cell, ERCCs, housekeeping gene expression and mitochondrial DNA reads (Lun et al 2016, F1000Research 5:2122). This resulted in 674 high-quality cells that were used for downstream analyses.

Next, we identified Highly Variable genes (HVGs) per time point following the Kharchenko pipeline (Kharchenko et al. 2014, Nature Meth 11:740-742) (Figure 11B): library size factor normalization (DESeq) and winsorization of the data prior plotting variation (logCV2) over mean expression (logmeans). After fitting a regression line to the data, genes were ranked and selected by their significance of deviation from the fit (adjusted p-value<1e-3). For each time point, single cells were clustered in an unsupervised manner based on the expression of corresponding HVGs using non-negative matrix factorization as dimension reduction approach (run=40, rank=10, in MeV 4.8.1). The "best fit" (numbers of clusters) was chosen based on the highest cophenetic correlation coefficient.

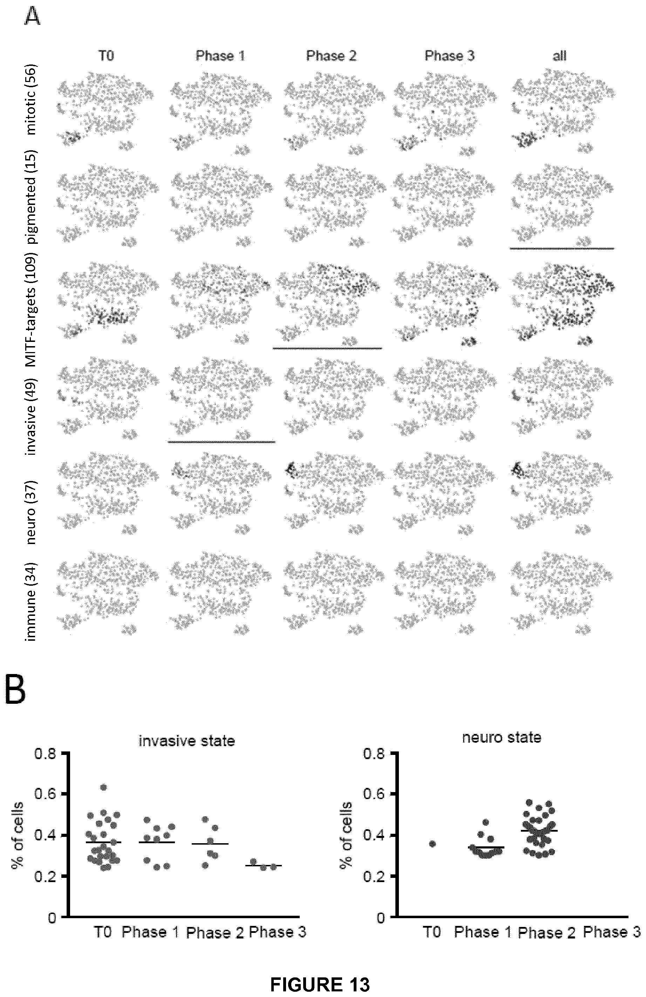

Next, Single-cell Differential Expression analysis (SCDE) was performed between the different NMF-clusters using the global gene expression matrix (Kharchenko et al 2014, Nature Meth 11:740-742). SCDE analysis generated a Z-score ranked gene list for each NMF cluster (n=10) of which the top 100 candidates were interpreted by Ingenuity Pathway Analysis (IPA) and i-cisTarget. Characteristic gene signatures per NMF cluster were established by combining genes of highly enriched IPA and/or i-cisTarget terms (Table 1) into 6 final gene signatures (Figure 11C, Table 2). To measure the activity of the 6 final gene signatures in each cell, we used the AUCell algorithm (Aibar et al. 2017, Nature Meth 14:1083-1086) (Figure 12). The activity of each of the 6 final signatures was visualized by i) projecting all 674 cells into two-dimensional space using t-distributed stochastic neighbor embedding (t-SNE, perplexity=30, initial_dims=10, max_iter=1000) based on the expression of all genes in the 6 final signatures (n=284 unique genes) and ii) coloring cells according to their binary AUCell score (Figure 13A). Table 2 provides the gene signatures specific to the 4 minimal residual disease cell subpopulations.

**TABLE 1. Enrichment results.**

| **Ingenuity Pathway Analysis - Diseases or Functions Annotation** |
|---|
| **Mitosis; p-value 1.1E-43** |
| ANLN,AURKB,BIRC5,BUB1,BUB1B,CCNA2,CCNB1,CCNB2,CDC20,CDCA5,CDCA8,CDK1,CENPA, CENPE, CENPF,CENPW,CKAP2,ESCO2,FOXM1,KIF11,KIF15,KIF18B,KIF2C,KIF4A,KIFC1,MAD2L1, MKI67,MYBL2, NDC80,NEK2,NUF2,NUSAP1,PLK1,PLK4,PTTG1,SGO1,SKA3,SPAG5,SPC25,STMN1, TACC3,TOP2A,TPX2, TTK,TUBB,UBE2C,ZWINT,CDC25B,COC25C,COKN3,OLGAP5,FBXO43,GMNN, KNL1,RAN,SKA1 |
| **disorder of pigmentation; p-value 1.27E-15** |
| EDNRB,FABP7,GPR143,MLANA,MLPH,PMEL,RAB27A,SLC24A5,SLC45A2,SNAI2,TRPM1,TYR,TYRP1, APOE,KIT |
| **differentiation of melanocytes; p-value 1.45E-07** |
| EDNRB,MLPH,RAB27A,TRPM1,TYRP1,KIT |
| **abnormal morphology of melanocytes; p-value 1.91E-09** |
| EDNRB,GPR143,MLANA,PMEL |
| **antiviral response; p-value 2.7E-26** |
| BCL3,BIRC3,BST2,CXCL10,HLA-A, IFI44,IFIH1,IFIT1,IFIT3,IFIT5,IFITM1,IFITM3,IRF7,ISG15,MX1, OAS1, OAS2,OAS3,OASL,PLSCR1,RSAD2,STAT1,STAT2,TRIM22 |
| **Interferon Signaling; p-value 9.40E-27** |
| IFIT1,IFITM3,IFI13,OAS1,MX1,IFI35,IFI6,STAT2,PSMB8,STAT1,TAP1,IFITM1,ISG15 |
| **Antigen Presentation Pathway; p-value 1.10E-17** |
| B2M,PSMB9,HLA-C,HLA-A,HLA-B,PSMB8,HLA-F,TAP1,HLA-E |
| **morphology of nervous system; p-value 2.63E-09** |
| A2M,ADAMTS4,ADGRG6,ANXA1,ATPIA2,ATPIB2,CNN3,COL1A1,GFRA1,GFRA2,GFRA3,IGF1,ITGA6, L1CAM,LAMC1,MPZ,NGFR,NLGN3,PDGFB,S100A4,SEMA3B,SLITRK6,TMEM176B,VCAN |
| **development of neurons; p-value 5.98E-09** |
| A2M,ADGRG6,CADM1,COL4A1,GFRA1,GFRA2,GFRA3,IGF1,IL1RAP,ITGA1,L1CAM,LAMC1,MATN2, MPZ,NGFR,NLGN3,NRXN1,PDGFB,PLAT,SLITRK6,THBS2,TNC |
| **morphology of head; p-value 1.67E-08** |
| ADAMTS4,ANXA1,AQP1,ATP1A2,ATP1B2,COL1A1,COL5A2,CTGF,IGF1,ITGA6,L1CAM,LAMC1,NGFR,NL GN3,PDGFA,PDGFB,PLAT,S100A4,SEMA3B,SLITRK6,SPARC,TECTB,TMEM176B |
| **migration of cells; p-value 3.32E-21** |
| ADM,ANGPTL4,AXL,BCAT1,BGN,CCL2,CDH13,CDH2,CEMIP,COL3A1,CYSLTR2,DLC1,DLX1,EDNRA, ERRFI1,F3,FABP4,FGF1,FGFR1,FOSL2,GNAI1,GNAS,GPC3,IGFBP5,IGFBP6,LMO4,LOX,LOXL2,MGP, MRC2,NDNF,NES,NFAT5,NR2F1,PDGFRB,PLXNB2,PRDX1,PTGER4,RARRES2,RGS16,SEMA3C,SH2B3,SLI T2, SPRY2,TGFBI,TGM2,TIMP3,TM4SF1,TMSB10/TMSB4X,UNC5B,VCAN,VEGFA,VSNL1 |
| **Angiogenesis; p-value 3.24E-18** |
| ADM,ANGPTL4,AXL,CCL2,CDH13,CDH2,COL1A2,COL3A1,CYSLTR2,DDAH1,EDNRA,F3,FABP4,FGF1, FGFR1,GPC3,IL13RA2,LOX,LOXL2,MGP,NDNF,NFAT5,PDGFRB,PLXDC1,PTGER4,RGS5,SEMA3C, SLIT2, SPRY2,TGFBI,TGM2,TIMP3,TM4SF1,UNC5B,VEGFA |
| **Metastasis; p-value 3.83E-12** |
| ADM,ANGPTL4,AXL,CCL2,CDH2,CEMIP,CPA4,DLC1,EDNRA,F3,FGF1,FGFR1,IGFBP5,LOX,LOXL2, MEST, NES,NR2F1,PDGFRB,SLC5A3,SLIT2,TGM2,TM4SF1,TMSB10/TMSB4X,TNFRSF10B, VCAN,VEGFA |
| **invasion of tumor cells; p-value 1.35E-11** |
| ADM,ANGPTL4,AXL,CCL2,CDH13,CDH2,DLC1,FGFR1,GNAI1,GNAS,IL13RA2,LOX,LOXL2,NES,NFAT5, SLl12,SOX4,SPRY2,TGFBI,TIM P3, TMSB10/TMSB4X, UNC5B, VCAN, VEGFA |
| **KEGG_metabolic pathways; p-value 6.25E-09** |
| NDUFA4L2,PGM1,B3GN12,TK1,GAPDH,PRDX6,AMD1,ACSL3,DHCR24,LDHA,RRM1,TYR,NDUFA4, PKM, RPE,PHGDH,BAAT,TUSC3,ENPP1,ACAT2,PGK1,ACSS2,ALDH1A3,TYMS,RPN2,ALDH1A1,LDHB |
| |
| **i-cisTarget analysis track** |
| **T0: MITF_501Mel_Davidson; NES score 6.20319** |
| **MITF ChIP-seq in 501Mel** |
| STX7,ASAH1,TYR,PTTG1IP,DCT,EDNRB,MBP,SLC45A2,TRIM2,CD59,MLPH,GPR56,MREG,AP1S2, ANXA5,C2orf88,APOD,GPNMB,MET,VAT1,SDCBP,TRIM63,MLANA,GJB1,TSPAN10,HAGHL,ARPC1B, CD63,SERPINF1,APCDD1,BCL2A1,PDP2,S100B,SLC24A5,SLC6A15,PYGL,DMKN,RILPL1,SRCIN1, PLEKHB1,PRAME,C1QTNF3,AMD1,ERBB3,TMEM117,CHRM1 |
| **T0: hocomoco__MITF_f1; NES score 3.78351** |
| CTSK,SLC45A2,DCT,TRIM63,CD63,GPNMB,TYR,SLC24A5,CD44,PROX1,MREG,APOD,CHRM1,GPR56, DIO2,SDCBP,PLP1,TMEM117,AP1S2,TMOD1,FYCO1,MLPH,SERPINF1,SORBS2,MBP,TRIM2,STX7, ITGAM,NINJ2,BCL2A1,ANXA5,ANKS1A,ASAH1,LYPLAL1,ESRP1,C1QTNF3,SAT1,CKM12,MET,AIF1L, PLEKHB1,MLANA,ADAMTS16,SLC6A15,AMD1,ERBB3,FAM196B,SRCIN1 |
| **Phase 1: MITF_501Mel_Davidson; NES score 6.63409** |
| **MITF ChIP-seq in 501Mel** |
| STX7,ASAH1,TYR,PTTG1IP,DCT,EDNRB,MBP,TNFRSF14,SLC45A2,CA14,MLPH,IRF4,TRPM1,MREG, KIAA1715,ANXA5,RAB27A,PTPLA,DEPDC1,VAT1,SDCBP,TRIM63,MLANA,HPGD,CD63,FBXO32, SLC29A1,MRPL44,MYO10,GPR143,BFAR,SLC24A5,SLC6A15,TPRN,PPARGC1A,DMKN,ZBTB37, PRAME, MDH1 |
| **Phase1: MITF(bHLH)/MastCells-MITF-ChIPSeq(GSE48085)/Homer; NES score 7.47041 Possible TFs: MITF** |
| CTSK,MSC,KIAA1715,CD63,SLC6A15,PPARGC1A,ASAH1,MLANA,DCT,SLC29A1,CA14,FBXO32, SLC45A2,NTF3,TYR,FAIM3,RAB27A,TRIM63,SDCBP,GPR143,MLPH,STX7,KIT,TRPM1,MREG, LGALS3,VAT1, DEPDC1,MBP,MYO10,GPRC5B,IRF4,SAT1,DAPL1,TYRP1,EDNRB,ZBTB37,PLP1 |
| **Phase 2: MITF[gene ID: "ENSG00000187098" species: "Homo sapiens" TF status: "direct" TF family: "bHLH" DBDs: "HLH"]; NES score 4.02839** |
| **Possible TFs: MITF** |
| DCT,C1orf51,PAX9,TYR,FAIM3,KIT,GPR158,FBXL14,NAT8L,TRPM1,CLCN5,SEMA3C,TYRP1,AGAP6, SDCBP,B3GNT5,DLGAP5,MLPH,GMPR,MBP,SNAI2,GPRIN3,FOXM1,VAV3,ST8SIA6,MXD3,ADRBK2, IRF4 |

**TABLE 2. Gene Signatures for melanoma cell populations in residual disease**

| |
|---|
| **Pigmentation state (15 genes)** |
| SLC24A5, PMEL, FABP7, SLC45A2, KIT, EDNRB, TRPM1, APOE, MLANA, MLPH, TYRP1, GPR143, TYR, RAB27A, SNAI2 |
| **Invasive state (50 genes)** |
| VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61 |
| **Neuro (37 genes)** |
| AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B |
| **MITF-medium hypometabolic (9 genes)** |
| SLC7A8, DLX5, TRIM67, CD36, PAX3, IP6K3, UBXN10, KIAA1161, LSMEM1 |

### 1.10. SCENIC analysis

The SCENIC analysis was run on the 760 cells as described in Aibar et al. (Aibar et al. 2017, Nature Meth 14:1083-1086; SCENIC version 0.1.5, which corresponds to GENIE3 0.99.3, RcisTarget 0.99.0 and AUCell 0.99.5) using the 20-thousand motifs database for RcisTarget (RcisTarget.hg19.motifDatabases.20k). The input matrix was the size-factor normalized expression matrix, from which 12255 genes passed the default filtering (rowSums > 5*0.03*760 and detected in at least 1% of the cells). From these genes, only the protein coding genes were kept in the co-expression modules from GENIE3 and analyzed for motif enrichment with RcisTarget.

Figure 17 shows the t-SNE on the binary regulon 920 activity matrix (253 regulons x 674 cells, run with Rtsne package (https://github.com/jkrijthe/Rtsne), using correlation as distance and 50 as perplexity).

### 1.11. Diffusion map

The diffusion maps were plotted through R/Bioconductor package destiny (version 2.0.8), centering and scaling the the size-factor normalized expression matrix including 557 cells (41 invasive, 44 neuro, 30 pigmented, and 442 MITFmedium) and the 1397 genes in any of the 4 signatures (units: log2(sfNormMat+1)).

### 1.12. RT-qPCR

Cells resuspended in QlAzol using an miRNeasy Kit and processed according to the manufacturer's instructions (QIAGEN) or in RA1 lysis buffer using the RNA NucleoSpin extraction kit (Macherey&Nagel). RNA was quantified using a NanoDrop 1000 (Thermo Scientific) and 500-2,000 ng was reverse transcribed with a High-Capacity cDNA Reverse Transcription Kit (Life Technologies). qPCRs were run using Fast SYBR Green Master Mix or SensiFast^{™} kit (Bioline) and a Roche LightCycler 384 (both from Life Technologies). Data processing with qbase+ 2.6 software (Biogazelle) relies on normalization with a minimum of 2 reference genes, indicated as RefGen below. RT-qPCR primers are listed in Table 3.

**TABLE 3. Primer sequences.**

| **Gene** | **forward 5'-3'** | **SEQ ID NO:** | **reverse 5'-3'** | **SEQ ID NO:** |
|---|---|---|---|---|
| AQP1 | CATGTACATCATCGCCCAGT | 25 | CACCATCAGCCAGGTCATT | 26 |
| NGFR | TCATCCCTGTCTATTGCTCCA | 27 | TGTTCTGCTTGCAGCTGTTC | 28 |
| GFRA2 | GACCGGGTGCCCAGCGAGTA | 29 | CAGCCGGGACCGACACAGG | 30 |
| GFRA3 | ATGCTGGAAGGGTTCTTCTC | 31 | TTTCATTCTGGTGTGCCATC | 32 |
| L1CAM | CTGCCTGCTTATCCAGATCC | 33 | CCTCACACTTGAGGCTGATG | 34 |
| RSPO3 | ACCTTGGAAAGTGCCTTGAC | 35 | CTCACAGTGCACAATACTGAC | 36 |
| TMEM176B | CCCTACCACTGGGTACAGATGGA | 37 | CTTCAAGACACAGACAGCCAGGA | 38 |
| SLC22A17 | CCTCTTCATCTTGGGCTTTG | 39 | AGCCCCTCCTACTCCACAG | 40 |
| GDNF | TGGGGCACCTGGAGTTAATG | 41 | ATCTTAAAGTCCCGTCCGGC | 42 |
| TFAP2B | CGAATGCCTCAATGCGTCT | 43 | CCCATTTTTCGATTTGGCTC | 44 |
| RXRG | CCCTTGAGGCCTACACCAAGC | 45 | CACACCTGCCCAGGGGTCATC | 46 |
| MEF2C | TCCACCAGGCAGCAAGAATACG | 47 | GGAGTTGCTACGGAAACCACTG | 48 |
| SOX2 | GGGAAATGGGAGGGGTGCAAAAGAGG | 49 | TTGCGTGAGTGTGGATGGGATTGGTG | 50 |
| SOX10 | CCAGTACCCGCACCTGCAC | 51 | CTTTCGTTCAGCAGCCTCCAG | 52 |
| MITF-M | CATTGTTATGCTGGAAATGCTAGAA | 53 | GGCTTGCTGTATGTGGTACTTGG | 54 |
| CDH1 | GGCTGGACCGAGAGAGTTTC | 55 | TGCTGTTGTGCTTAACCCCT | 56 |
| WNT5A | GGTGGTCGCTAGGTATGAATAACC | 57 | TCCACCTTCGATGTCGGAA | 58 |
| EGFR | TGCACCTACGGATGCACTG | 59 | CGATGGACGGGATCTTAGGC | 60 |
| POSTN | GTGGTAGCACCTTCAAAGAAATCC | 61 | GCAACTTCCTCACGGGTGTGTC | 62 |
| TCF4 | ATGGCAAATAGAGGAAGCGG | 63 | TGGAGAATAGATCGAAGCAAG | 64 |
| TBP | AATCTGTCATGCTGGTCTGCC | 65 | AGGAGATTTGTTTGGCGTGC | 66 |
| UBC | ATTTGGGTCGCGGTTCTTG | 67 | TGCCTTGACATTCTCGATGGT | 68 |
| YWHAZ | ACTTTTGGTACATTGTGGCTTCAA | 69 | CCGCCAGGACAAACCAGTAT | 70 |

### 1.13. Western Blot

Harvested cell culture pellets were resuspended in protein lysis buffer (25mM HEPES pH 7,5; 0,M NaCl; 1,5mM MgCl2; 2mM EDTA; 2mM EGTA; 1mM DTT; 1% Triton X-100; 10% Glycerol; phosphatase/protease inhibitor cocktail), incubated on ice (15min) and centrifuged (15min) at 4°C/13000 rpm. Tissue samples were additionally homogenized with a PreCellys in protein lysis buffer, prior to incubation on ice. Equal amounts of protein (Bradford quantification) were run on 4-12% Bis-TrisNuPageNovex gels (Invitrogen) and transferred to a nitrocellulose membrane with an iBlot dryblot system (Life Technologies). Membrane blocking (5% milk - TBS-0.2%Tween) is followed by incubation with the appropriate primary antibodies and HRP-conjugated secondary antibody (Cell Signaling Technology). Proteins were detected by enhanced chemiluminescence/western blotting (Thermo Scientific). The antibodies were from Cell Signaling Technology and diluted 1:1000 in 5% BSA in TBS-0.2% TWEEN; Phospho-FAK (Tyr397) D20B1 #8556; Total FAK (D2R2E) #13009; Phospho-AKT (S473) (D9E) #4060; Total AKT (40D4) #2920; Phospho-ERK1/2 (T202/Y204) #9106, Total ERK1/2 #9102.

### 1.14. Colony assay

Cells were grown to near confluency on 12well plates and treated with the indicated drug combinations for the indicated time period. Cells were washed once with PBS, stained with crystal violet (1% crystal violet w/v, 35% methanol v/v) for 15min, washed with PBS and destained in tap water.

### 1.15. Cell Titer Glo

2500 cells were plated onto a 96well plate and treated with the indicated drug combinations for the indicated time period. Cell Titer Glo assay was performed according to the manufacturer's instruction (Promega) and luminescence was measured on a VictorX3 (Perkin Elmer).

### 1.16. Live cell-analysis

Cell death was followed in real time using an IncuCyte ZOOM system (Essen BioScience). Sorted GFRA2 low and GFRA2 high Mel006 cells were seeded at a density of 4000 cells per well in a 384 well microplate, treated with DT x nM, CellEvent green (Life technologies) and Cytotox red (Essen BioScience). Phase contrast, green fluorescent and red fluorescent images were taken at 2 hour intervals for the duration of the experiments.

### 1.17. TCGA analysis

GFRA2 and AQP1 RNA expression levels were quantified (RSEM normalized reads) in 470 melanoma patients (TCGA_SKCM) using the RNAseq explorer (http://tcgabrowser.ethz.ch:3839/TEST/). Differential gene expression analysis between highest and lowest GFRA2 and AQP1 expressers (7th percentile) was performed, genes and patients were hierarchically clustered and a selection of co-differentially-expressed genes plotted as heatmap.

### 1.18. Gene set enrichment analysis

Gene set enrichment analysis (GSEA version 2.2.1) was performed by ranking SCDE genes based on their cZ-score as metric. Different ranked SCDE lists were used including MITF-medium comparison, neuro vs. invasive, and neuro vs. other comparison. The following gene signatures were analyzed for enrichment: proliferative melanoma (433 genes, FC>3; Verfaille et al. 2015, Nature Comm 6:6683), cancer cell metabolism gene database (2071 genes, (Kim et al. 2016, Nucl Acids Res 44:D959-968)), GO_neural crest_cell_differentiation (75 genes, MSigDB), quiescent neural stem cells (324 genes; Bobadilla et al. 2015, ), glioblastoma proneural (178 genes; Verhaak et al. 2010, Cancer Cell 17:98-110), KEGG_Focal adhesion (201 genes, MSigDB), quiescent neural stem cells (216 genes; Codega et al. 2014, Neuron 82:545-559), drug tolerant persistors PC-9 cells (233 genes, FC>3; Sharma et al. 2010, Cell 141:69-80), top 100 upregulated genes 992 in FACS sorted GFRA2+cells in vitro.

### 2. RESULTS

### 2.1. Establishment of an in vivo model of drug tolerance

PDX models maintain the essential properties of the original patient tumors, including intra-tumor heterogeneity, making them authentic experimental systems for studying the impact of heterogeneity in human cancer drug response (Byrne et al. 2017, Nature Rev Cancer 17:254-268). We derived several PDX models from various drug naïve melanoma lesions with distinct mutational backgrounds and confirmed their genetic stability over consecutive mouse-to-mouse passages (Figure 8A-B).

Transcriptome (bulk RNA-seq) analysis of 11 randomly selected F0-F3 lesions indicated that these tumors, despite their distinct genetic make-up, exhibited comparable bulk expression profiles (Figure 8C). All predominantly expressed the proliferative gene expression signatures previously described (Hoek et al. 2008, Cancer Res 68:650-656; Verfaillie et al. 2015, Nature Commun 6:6683). To establish an *in vivo* model of drug tolerance we chose to proceed with MEL006 and MEL015 because both patients from whom these models were derived exhibited the BRAFV600E mutation and developed metastatic lesions that showed marked responses to a combination of BRAF (i.e. dabrafenib) and MEK (i.e. trametinib) inhibitors (Figure 8D and Table 4).

**Table 4: Clinical history of patients from whom the MEL006 and MEL015 PDX models were established. PD, Progressive Disease; PR, Partial response**

| | MEL006 | MEL015 |
|---|---|---|
| PDX model derived from | In transit metastasis (arm) | Lymph node metastasis |
| BRAF mutation | V600E | V600E |
| Prior treatment | None | None |
| Treatment after establishing PDX model | Ipilimumab: PD; dabrafenibtrametinib:PR (PFS 33+ months) | Dabrafenib-trametinib: PR, (PFS 3 months) |

Before establishment of the experimental mouse cohorts, PDX lesions were dissociated and single cell suspensions were transduced with dsRed-encoding lentiviruses (Figure 1A). Importantly, this procedure did not affect the histopathological features of the resulting PDX melanoma lesions (data not shown). Once tumors reached a comparable size (1000 mm3), mice (n=29) were exposed to the dabrafenib/trametinib combination. All treated lesions rapidly shrunk to reach an impalpable size within approximatively 10 days. This is the first tumor regression phase with early adaptation and selection of the fittest cells. This is followed by a second "survival" or minimal residual disease phase (phase 2), which we referred thereafter as the drug tolerance phase (Figure 1B). Treatment interruption during this phase invariably led to appearance of rapidly growing melanoma lesions within a few days. About 50 days after continuous treatment, virtually all lesions re-grew on-treatment (Phase 3; Figure 1B). Recent clinical data showed that re-challenging with BRAF +/- MEK-inhibitors after drug interruption often led to significant anti-tumor responses in patients with relapsed disease (http://abstracts.asco.org/199/AbstView_199_180694.html).

Accordingly, all drug-resistant PDX lesions (n=9) responded to a second challenge with the BRAF/MEK-inhibitor combination following variable interruption treatment periods (Figure 9). These findings indicated that the transitions from one drug response phase to the next, including acquisition of the ability to grow on-treatment, are likely to be driven by non-mutational events. We concluded that this preclinical model is particularly suited to study the mechanisms of drug adaptation and tolerance in the relevant *in vivo* context.

### 2.2. Co-occurrence of residual subpopulations expressing distinct levels of MITF

Bulk RNA-sequencing indicated that the intensity of overlapping pigmentation/differentiation and MITF-driven gene expression signatures increased from T0 to phase 2 (Figure 1C). This increase is consistent with an increase in MITF levels and activity (Rose et al. 2016, Clin Cancer Res 22:6088-6098) and with this transcription factor driving drug tolerance in response to MAPK-inhibition (Smith et al. 2016, Cancer Cell 29:270-284). In contrast, both Hoek's and Verfaillie's invasive gene expression signatures decreased in response to drug exposure (Figure 1C).

To further visualize the MAPK-inhibition-dependent increase in MITF we analyzed its expression at single cell level by immunohistochemistry (IHC). MITF staining was rather uniform in drug-naïve melanoma lesions (T0) with most cells exhibiting both diffuse cytoplasmic and nuclear positivity. Consistent with the increase in MITF activity upon MAPK-inhibition, marked nuclear staining was seen in a fraction of the melanoma cells at phase 2 (Figure 1D). However, there was also a large fraction of cells that became completely devoid of any MITF staining (Figure 1D). Consistently, an increase in pigmentation, which is a likely consequence of increased MITF activity, was only seen in a small proportion of the drug-tolerant melanoma cells (Figure 1D). Similar results were obtained with the MEL015 PDX model (data not shown). These data indicated that the adaptive response to MAPK-inhibition is not uniform, as suggested previously (Lito et al. 2012, Cancer Cell 22:668-682; Smith et al. 2016, Cancer Cell 29:270-284; von Kriegsheim et al. 2009, Nature Cell Biol 11:1458-1464). Instead, distinct subpopulations that exhibit very different levels of MITF, can emerge *in vivo* within the same residual melanoma lesion.

### 2.3. Single-Cell RNA-sequencing identifies multiple co-existing drug-tolerant transcriptional states

In order to probe the extent of transcriptional state diversity and portray the dynamics of cell state transition during drug response we measured a thousand of transcriptomes from individual melanoma cells isolated at different time points (T0, Phase1, 2 and 3; Figure 10A). The Smart-Seq2 approach (Picelli et al. 2014, Nature Protocols 9:171-181) was chosen because it is the most appropriate method when the amount of starting material is limited, which is particularly the case at the minimal residual disease stage. Moreover, Smart-Seq2 has the highest sensitivity of all available methods to date, which is a major advantage when searching for rare cells and studying cell state transition (Ziegenhain et al. 2017, Mol Cell 65: 631-634).

SMARTseq2 generated high-quality amplified cDNA profiles and Nextera XT sequencing libraries (Figure 10B). ERCC spike-ins showed comparable amplification efficiencies between preparations (Figure 10C). 85 different housekeeping genes were stably expressed in all cells. The median library complexity was 5256 genes per cell. tSNE clustering (t-distributed stochastic neighbor embedding; van der Maaten & Hinton 2008, J Machine Learning Res 9:2579-2605) based on global expression did not show any obvious batch effect for either cell type (time-point) or sequencing run (Figure 10E). After filtering out low quality cells (Lun et al. 2016, F1000Research 5:2122), 674 single-cells passed stringent quality criteria (described in the Materials & Methods section) and were used for further analyses (Figure 10F). Single-cell data analysis was a multi-step process (Figure 11A), in which we first identified highly variable genes, likely to drive true biological heterogeneity (Brennecke et al. 2013, Nature Meth 10:1093-1095). We determined the number of distinct cellular subpopulations (clusters of cells) based on these highly variable genes, using Non-negative Matrix Factorization (NMF) as a method for unsupervised clustering (Figure 11B). For each cluster, gene signatures were defined by applying single cell differential expression analysis (Figure 11B) (Kharchenko et al. 2014, Nature Meth 11:740-742). These gene signatures were filtered and pruned based on a combination of Gene Ontology (GO), Pathway enrichment and transcription factor motif analyses (Figure 11C). The activity of these gene signatures was then measured in all single cells using AUCell (Aibar et al. 2017, Nature Meth 14:1083-1086) and the resulting clustering visualized in a tSNE plot (Figure 2A). Note that this pipeline is conceptually similar to PAGODA, a well-accepted single-cell RNA-seq analysis pipeline (Fan et al. 2016, Nature Meth 13:241-244). This procedure yielded six candidate melanoma transcriptional states.

The first state is a classical mitotic state (Figure 2A). As expected, the number of "mitotic" cells declined from T0 to phase 2 (Figure 2B and 13A), and rose again in phase 3.

A second state, referred to as the immune-like state, was characterized by high expression of IFN-inducible genes. This state was over-represented in phase 3, suggesting that cells in that particular state acquired the ability to grow in the presence of drugs (Figure 2B and 12A).

The third predicted state corresponded to the "pigmentation/differentiation" melanoma state. Consistent with the increased intensity in MITF nuclear immunostaining upon drug-exposure, a clear enrichment for cells in this particular state was observed at phase 1-2 (Figure 2B). Likewise, these cells showed high MITF transcriptional activity (Figure 2B-C and 13B).

The fourth state corresponded to the classical "invasive" cell state, which exhibits low MITF expression/activity and high expression of the previously described invasive melanoma markers (Hoek et al. 2008, Cancer Res 68:650-656; Verfaillie et al. 2015, Nature Comm 6:6683). Importantly, this subpopulation was not enriched upon drug-exposure (Figure 2B-C and 13A). In fact, the percentage of "invasive" melanoma cells dropped progressively from T0 to phase 1 and 2, indicating that in this particular *in vivo* model system drug tolerance is not driven by a proliferative to invasive phenotype switching event.

The fifth transcriptional state was characterized by high expression of a series of neural markers ("neuro" signature; Figure 2B-C). Just like cells in the invasive state, neuro-melanoma cells expressed low levels of MITF and its downstream targets. Nevertheless, this particular state clearly clustered away from the classical "invasive" state (Figure 2A). Moreover, in contrast to the invasive subpopulation the proportion of neuro-melanoma cells was dramatically increased upon drug exposure (Figure 2B and 12-13A-B). This population was present in minute amount (0,58%) in drug naïve lesions (T0) and was increased by 13-fold (7,74%) at phase 1, a time point at which the lesions had decreased by only 50% of their initial tumor volume. Given that cell proliferation drops abruptly during this first drug response phase (Figure 1D), this increase is unlikely to be solely due to an enrichment of a rare "neuro" subpopulation. In contrast, the data argue that melanoma cells are able to adopt this particular "neuro" phenotype through transcriptional reprogramming.

The remaining cells were initially grouped into a sixth melanoma transcriptional state that is characterized by MITF medium/moderate activity (Figure 2D). However, two distinct clusters of MITF-medium cells clearly emerged from the t-SNE plot indicating that these cells may in fact belong to two different transcriptional states. Consistent with this possibility, cells from phase 1 and 2, the growth of which is severely compromised by the treatment, clustered together. These cells clustered away from T0- and phase 3-cells, which are either drug-naïve or able to grow in the presence of MAPK therapeutics (Figure 2D). Gene Set Enrichment Analysis (GSEA) established that the TO-phase 3 MITF-medium cells expressed the classical "proliferative" gene signature and a large proportion of genes from the recently described cancer cell metabolism signature (Kim et al. 2016, Nucl Acids Res 44:D959-968) (Figure 2E). In contrast, the expression of these genes was drastically decreased in phase 1-and 2-cells, indicating that these cells exhibit low cancer cell metabolic activities. This "hypometabolic" cell state was particularly enriched at phase 2 and this enrichment may have occurred at the expense of the "proliferative" cell state that dramatically decreased upon drug-exposure (Figure 2F).

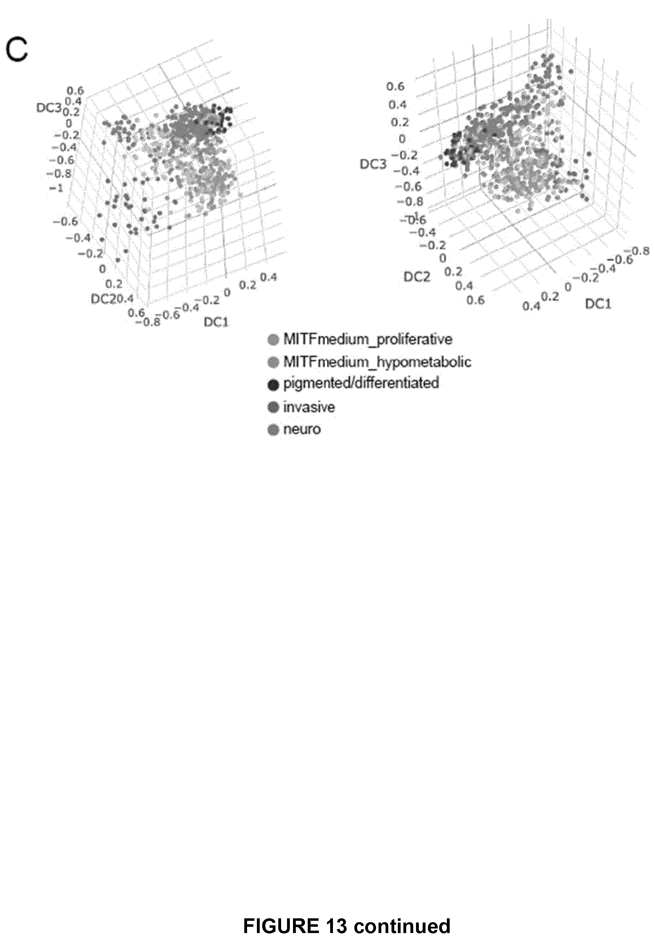

Destiny-based diffusion plots (Angerer et al. 2016, Bioinformatics 32:1241-1243) further confirmed that the neuro" versus "invasive" and "hypometabolic" versus "proliferative" melanoma cells belong to different subpopulations, and indicated that the two MITF-low states may represent distinct branches originating from the "proliferative" melanoma state in a diverging de-differentiation "trajectory" (Figure 13C). Importantly, none of the drug-induced cell populations (i.e. MITFhigh pigmentation, MITFmedium hypometabolic and MITFlow neuro cells) expressed mitotic markers (Figure 2G) indicating that acquisition of drug-tolerance requires cells to exit the cell cycle and enter into quiescence and/or dormancy. This is consistent with the absence of detectable tumor growth during phase 2.

In contrast to the number of mitotic cells, which increased as tumors gained the ability to grow in presence of the drugs, both neuro and hypometabolic subpopulations had decreased at this particular time point (phase 3; Figure 2B). This was particularly striking for the "neuro" subpopulation, which had disappeared from drug-resistant lesions (Figure 2B-C and 13B). This observation raised the possibility that these cells either do not directly contribute to drug resistance or can only do so following transcriptional reprogramming.

Altogether, these data demonstrated that adaptive 305 tolerance to MAPK-inhibition in an *in vivo* setting can occur via concomitant induction of at least three distinct and contrasting transcriptional states (i.e. MITFhigh pigmentation/differentiation, MITFmedium hypometabolic and MITFlow "neural" states), within the same residual melanoma lesion. Although divergent these transcriptional programs all instruct melanoma cells to enter into quiescence and/or dormancy.

### 2.4. Dissecting the gene regulatory networks underlying drug-tolerance diversity

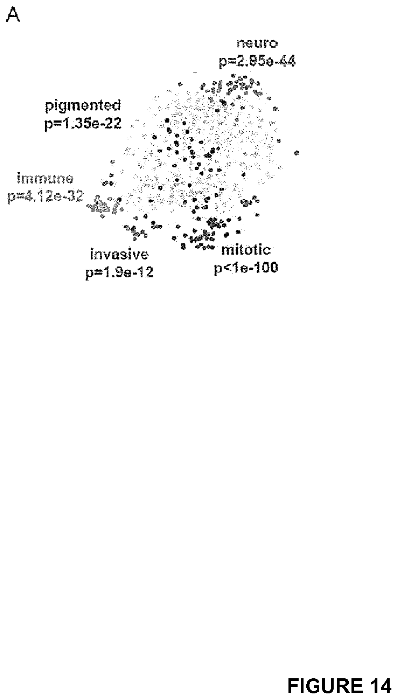

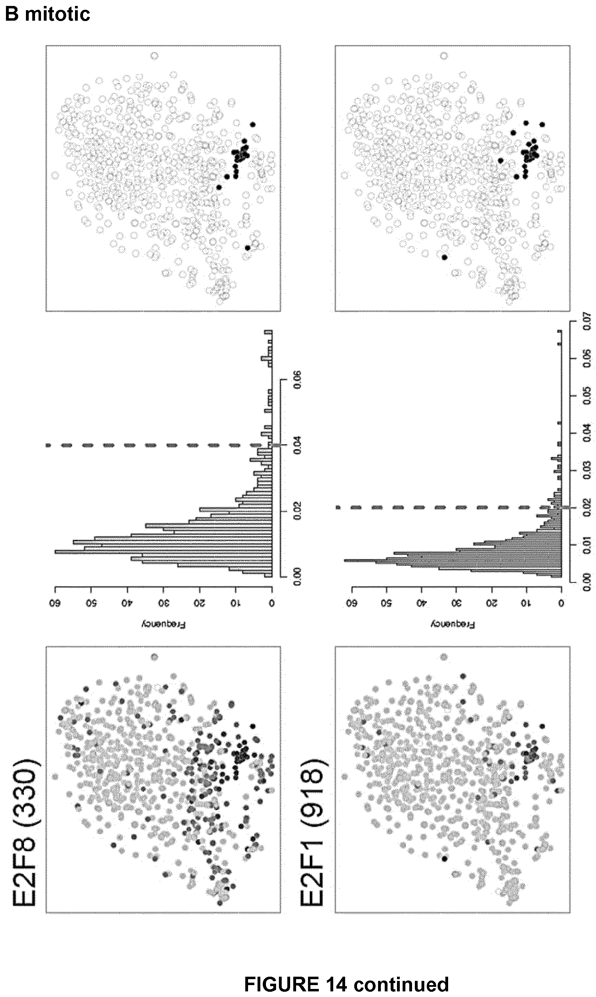

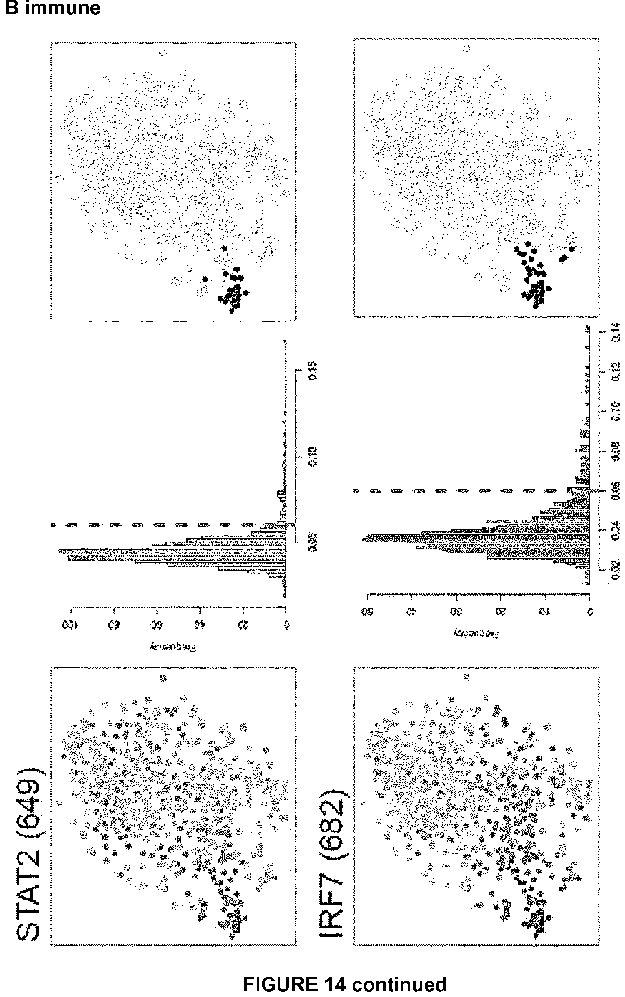

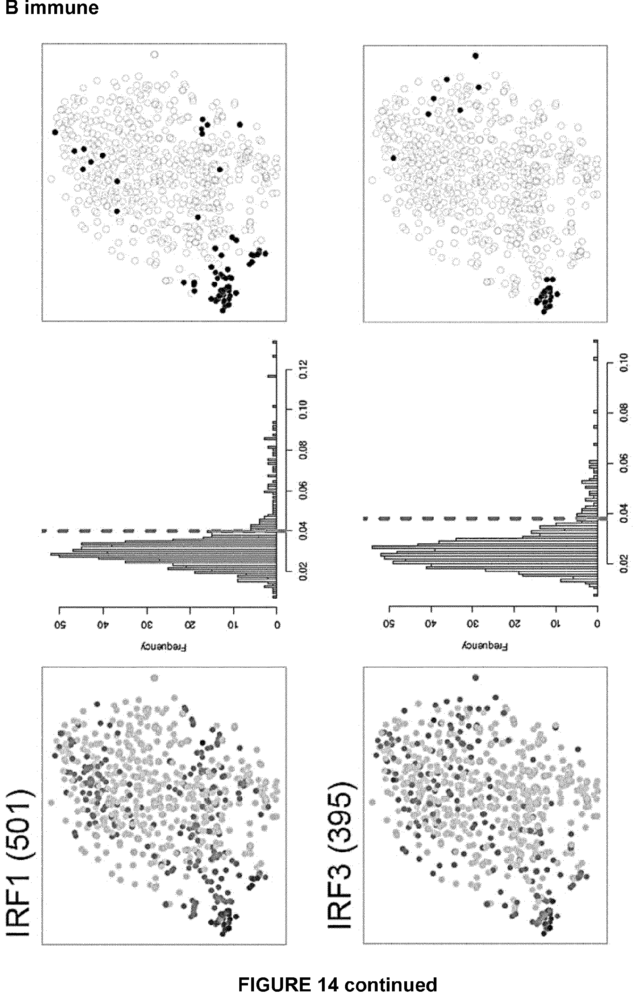

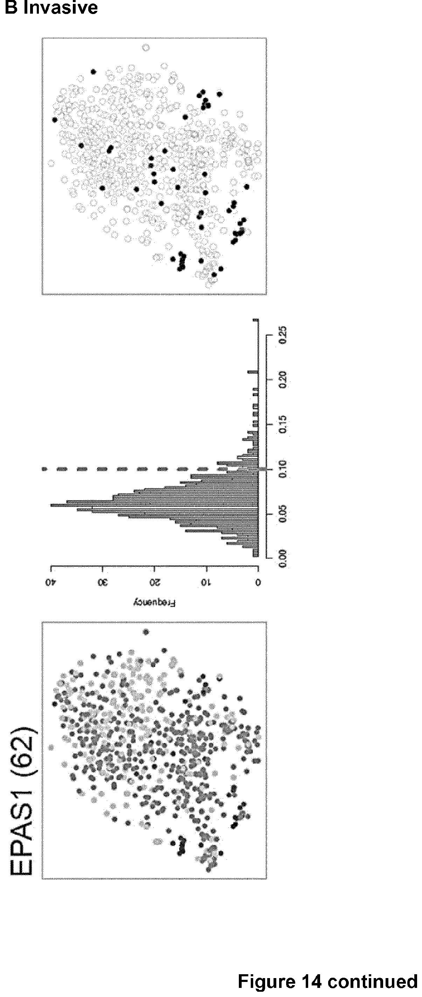

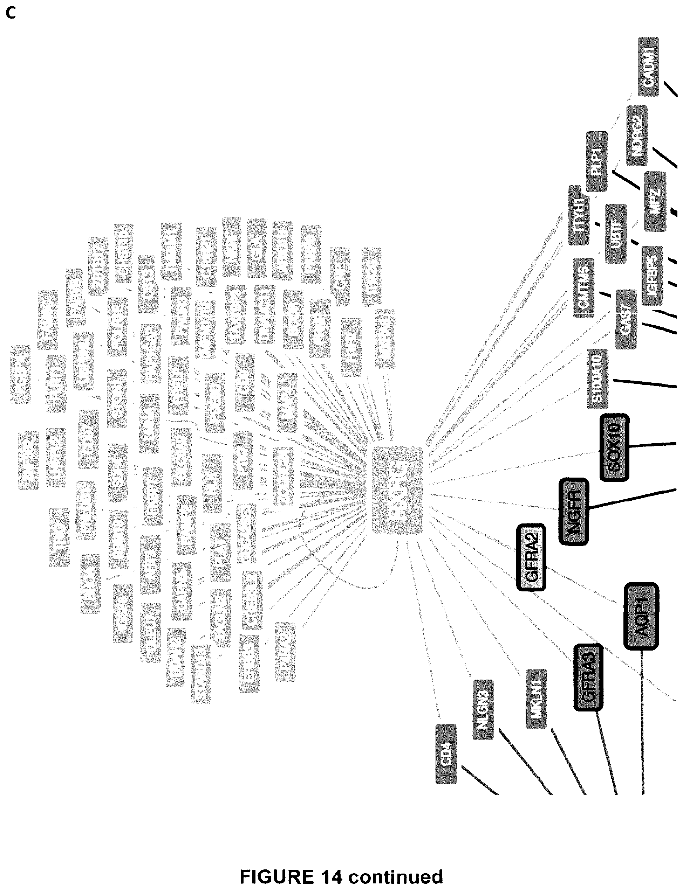

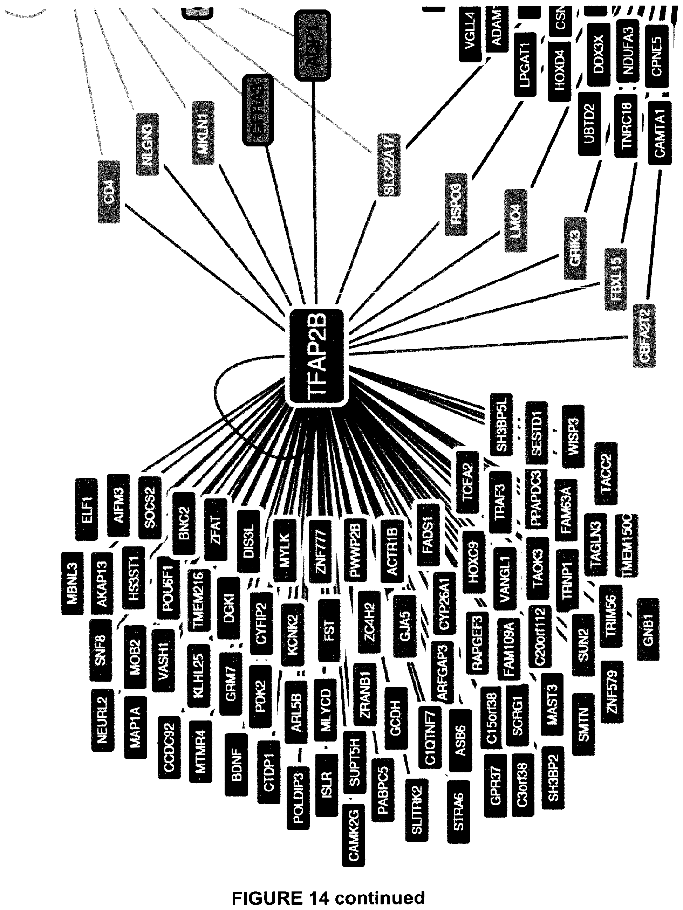

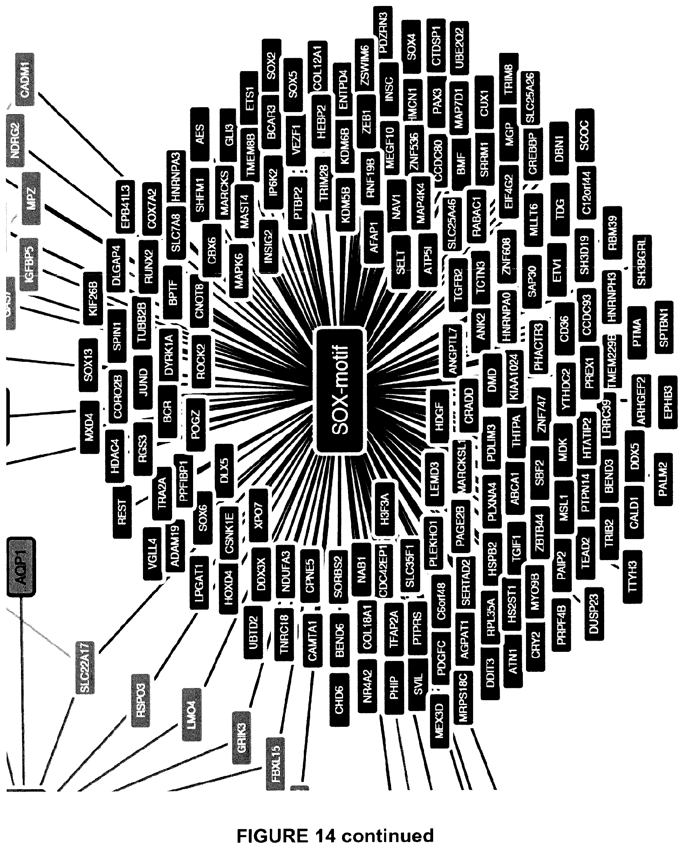

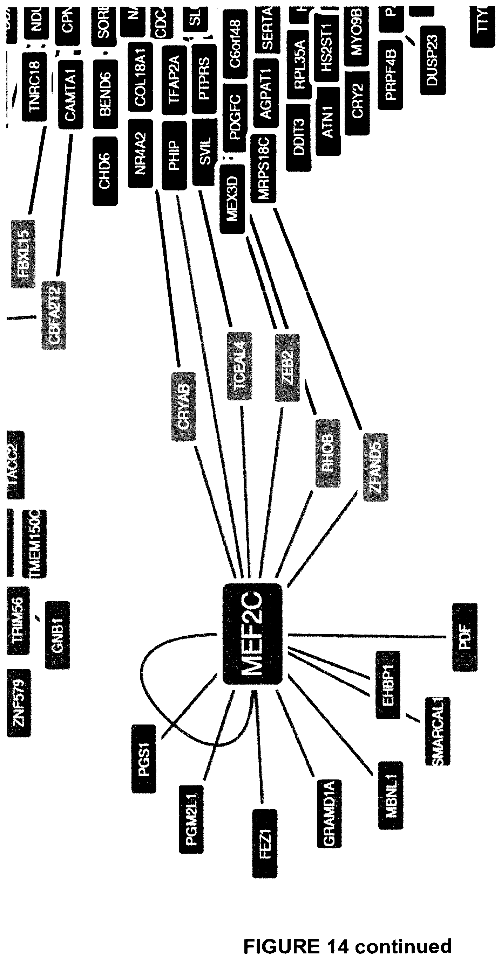

SCENIC was recently developed as a robust clustering method for the identification of stable cell states from scRNA-seq data based on the underlying gene regulatory networks (GRNs) (Aibar et al. 2017, Nature Meth 14:1083-1086). SCENIC confirmed the clear distinction between the mitotic, "immune", pigmentation, "neuro", "invasive", proliferative" and "hypometabolic" states and identified their underlying regulons, defined as a set of genes regulated by a common Transcription Factor (TF) (Figure 3A-B and 14). E2F family members (i.e. E2F1, E2F7 and E2F8) and STAT1, STAT2 and IRF factors (i.e. IRF1/2/3/7) were predicted drivers of the mitotic and immune states, respectively. AP-1 family members, such as FOSL2, and EPAS1 (or HIF-2alpha) were predicted drivers of the invasive state. The transcriptional activity of ETV4 (or PEA3), a member of the oncogenic subfamily of ETS TFs, was predicted to be high in the MITF-medium "proliferative" subpopulation. Equally expected was the identification of MITF as the main driver of the pigmentation/differentiation state.

Interestingly, the only clearly identifiable regulon in the MITF-medium hypometabolic subpopulation was under the control of PAX3 (Figure 3B), a TF previously implicated in the establishment of the melanocytic lineage and as putative driver of melanomagenesis (Seberg et al. 2017, Pigment Cell Melanoma Res 30:454-466). Strikingly, PAX3 overexpression was recently shown to induce resistance of melanoma to vemurafenib and, conversely, PAX3 silencing to inhibit the growth of melanoma that acquired resistance to the BRAF-inhibitor (Hartsough & Apli 2016, Clin Cancer Res 22:1550-1552; Liu et al. 2013, J Invest Dermatol 133:2041-2049). Together, these data indicate that PAX3 activation in a specific subset of drug-exposed cells may directly contribute to drug tolerance diversity in vivo.

As for the "neuro" transcriptional state, SCENIC predicted a complex regulatory network partly driven by SOX transcription factors, MEF2C and TFAP2B (Figure 3C and 14C). SOX10 was one of the SOX family members to exhibit predominant mRNA expression in the "neuro" cells as compared to other subpopulations (data not shown). Accordingly, its transcriptional activity was predicted to be high in these cells (Figure 3C). Consistent with SCENIC-predicted GRN, MEF2C is a direct transcription target and protein partner of SOX10 (Agarwal et al. 2011, Development 138:2555-2565). SOX10 and MEF2C physically interact and function cooperatively to activate the MEF2C gene in a feed-forward transcriptional circuit. In agreement with the "neural" identity of these cells, MEF2C was recently identified as an immediate transcriptional effector of neural crest development (Hu et al. 2015, Development 142:2775-2780). Similarly, TFAP2B (or AP-2beta) is also required for the development of the neural crest and its derivatives (Martino et al. 2016, Disease Models Mechanisms 9:849-861).

Another component of the SCENIC-predicted neuro regulatory network is the Retinoid X Receptor gamma (RXRG), a member of the nuclear receptor superfamily. The precise biological role of RXR remains largely unresolved (Evans & Mangelsdorf 2014, Cell 157:255-266) and RXR signaling has not yet been implicated into cutaneous melanoma biology. Interestingly, however, overwhelming evidence supports the importance of RXR heterodimer and its associated ligands in the clearance of toxic metabolites, endobiotics and other synthetic drugs (Willson & Kliewer 2002, Nature Rev Drug Discov 1:259-266). Moreover, a recent study indicated that RXR is capable of inducing a neuronal transcriptional program (Mounier et al. 2015, J Neurosci 35:11862-11876). Consistent with for RXR being a putative driver of the melanoma-neuro cell state, robust transcriptional activity of the RXR regulon was detected in the vast majority of these cells (Figure 3C).

Together, these data confirm that MITF is a key driver of drug tolerance in vivo. In addition, they also highlight the importance of other TFs, including PAX3 and RXR (and their underlying GRNs), as putative contributors of the concomitant emergence of distinct drug-tolerant states.

### 2.5. A neural stem cell gene activity program contributes to drug-tolerance

One of the drug-tolerant states strongly enriched in minimal residual disease (phase 2) was the MITFlow "neuro" transcriptional state. Neural crest cell differentiation was one of the most significantly enriched GO terms associated with the gene expression signature of these cells (Figure 4A). GSEA identified significant similarities between the neuro signature and quiescent neural stem cell profiles (Codega et al. 2014, Neuron 82:545-559; Llorens-Bobadilla et al. 2015, Cell Stem Cell 17:329-340) (Figure 4A and 15A).

Interestingly, there was also an overlap with other cancer stem cell profiles (Figure 15B) and a signature from Glioblastoma proneural (Figure 4A), one of the 375 GBM subtypes characterized by expression of several proneural development genes including, among others, the SOX genes (Verhaak et al. 2010, Cancer Cell 17:98-110). Notably, as opposed to the other GBM subtypes (i.e. classical and mesenchymal), proneural samples do not have a survival advantage when exposed to aggressive treatment protocols. These observations raise the possibility that drug-tolerance may be associated with common transcriptional, hence phenotypic traits, across tumor types. Consistent with this possibility, there were also significant similarities between the melanoma "neuro" transcriptome and the drug-persister signature from a NSLCLC-derived cell line (Sharma et al. 2010, Cell 141:69-80) (Figure 15A).

The MITFlow "neuro" melanoma cells, thereafter referred to as the Neural Drug Tolerant Cells (or NDTCs), expressed high levels of the Nerve Growth Factor Receptor NGFR (or CD271; Figure 4B). High levels of expression of this gene in melanoma has already been correlated with increased resistance to MAPK inhibition and acquisition of stem-like properties (Fallahi-Sichani et al. 2017, Mol Systems Biol 13:905; Menon et al. 2015, Oncogene 34:4545; Redmer et al. 2014, PLoS ONE 9:e92596; Shaffer et al. 2017, Nature 546:431-435). SLIT and NTRK like family member 6 (SLITRK6) is also selectively expressed in NDTCs (Figure 4B). This is a member of the neuronal Slitrk family of proteins, which are integral membrane proteins possessing a carboxy-terminal domain partially similar to that in the trk neurotrophin receptor proteins. RSPO3 expression is also elevated in NDTCs. RSPO3 is a member of the R-spondins family (RSPO1-4) of proteins, which together with their related receptors LGR4, 5 and 6 (LGR4-6) have emerged as a major ligand-receptor system with critical roles in (cancer) stem cell survival (Krausova & Korinek 2014, Cell Signal 26:570-579). Interestingly, two of the four family members of the glycosyl-phosphatidylinositol-anchored co-receptors, GFRA2 and GFRA3, were also highly and specifically expressed in these cells. These proteins are transmembrane receptors of the GDNF family ligands (GFLs) and essential transducers of GFLs-mediated activation of signaling pathways that promote survival, proliferation and differentiation of several neuronal populations in the CNS and PNS (Airaksinen & Saarma 2002, Nature Rev Neurosci 3:383-394; Paratcha & Ledda 2008, Trends Neurosci 31:384-391). Another discriminative marker for the NDTC subpopulation is AQP1, a small hydrophobic transmembrane protein with a predominant role in trans-cellular water transport (Figure 4B). Notably, AQP1 is expressed in endothelial cells and various stem/progenitor cell compartments (Figure 16). In agreement with the single cell profiling data, an increased expression of these markers at phase 2 was detected by bulk RT-qPCR in samples isolated from both MEL006 and MEL015 PDX models (Figure 17A). Importantly, further analysis of the single cell transcriptomics data confirmed that these markers (i.e. GFRA2 and AQP1) are co412 expressed within the same cells and that these are mitotically inactive and exhibit low MITF transcriptional activity (Figure 4C).

IHC confirmed the dramatic increase in the number of AQP1-positive cells at the dormancy phase (phase 2; Figure 4D and 17B). Notably, these cells did not appear to be randomly distributed within the lesions but rather occurred in clusters, raising the possibility that the NDTCs reside in specific niches (Figure 17C). Consistent with the scRNA-seq data, only rare AQP1-positive cells were detected at T0 (<1%) and in drug-resistant (phase 3) lesions (Figure 4D and 17B). This pattern of expression was not specific to MEL006 as a comparable increase in the number of AQP1-positive cells was also observed in drug-exposed MEL015 samples (Figure 17D).

Co-staining further confirmed that AQP-1-positive cells expressed undetectable levels of MITF and were of melanoma origin, as illustrated by their positivity for the marker S100 (Figure 4E). SCENIC identified SOX TFs as putative drivers of the NDTC state. scRNAseq indicated that SOX10, and to a lesser extent SOX2, expression was elevated in the NDTCs (data not shown). Importantly, these predictions could be validated by IHC (Figure 17E). Nuclear immunoreactivity was detected for both SOX10 and SOX2 in the vast majority (>90%) of AQP1-positive cells present at phase 2. These data are in line with the NDTC state being clearly distinct from the classical invasive melanoma state, the latter of which expresses low levels of both MITF and SOX10 (Hoek et al. 2008, Cancer Res 68:650-656; Verfaillie et al. 2015, Nature Commun 6:6683). IHC also confirmed that the majority (>80%) of AQP1-positive cells present at phase 2 express high nuclear levels of MEF2C and TFAP2B, two other TFs of the GRN architecture of the NDTC state (Figure 17E). Similarly, although not restricted to NDTCs RXR protein expression was detected at high levels in virtually all AQP-1-positive cells at phase 2 (Figure 17E).

Importantly, none of the AQP1+ cells expressed the proliferation marker KI67 (Figure 4E). This confirmed that NDTCs are not cycling and further supports the notion that the dramatic increase of this subpopulation at phase 2 is likely due to active phenotype switching rather than passive enrichment/selection. Consistent with the scRNA-seq data, most AQP1-positive cells also expressed NGFR at phase 2 (Figure 4F and 18A). Note that this overlap was only seen in the drug tolerance phase as all rare AQP1-positive cells present at T0 were NGFR-negative. Also note that there were many more NGFR-positive than AQP1-expressing cells at T0 (Figure 4F and 18B). In contrast to the rare AQP1-positive cells, many of the T0 NGFR were KI67-positive indicating that these cells exhibit proliferative capacity in drug-naive lesions (data not shown). This was further confirmed by the single-cell RNA-seq data (Figure 18B). We concluded that acquisition of NGFR expression by the AQP1-positive subpopulation is a drug-induced specific event. The data also indicated that although NGFR expression does increase upon drug exposure AQP1 is a more selective marker of drug response and tolerance than NGFR.

Consistent with the NDTC and "invasive" subpopulations being distinct, AQP1- and NGFR-positive cells were negative for the invasive marker AXL (Figure 3E-F). Whereas a fraction (10 to 15%) of melanoma cells was strongly positive for AXL at T0, their occurrence was much rarer (<5%) at phase 2 (Figure 3F). This is in keeping with the single-cell RNA-seq data showing a decrease in "invasive" melanoma cells upon drug-exposure.

The following signatures of increased gene expression (pre-treatment vs on-treatment with BRAF/MEK inhibitors and having reached the residual disease stage):
- neuro-like melanoma tumor cell subpopulation: genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; or, alternatively, genes NGFR, GFRA2, GFRA3, RSPO3, L1CAM, AQP1, TMEM176B; or, alternatively, genes NGFR, GFRA2, L1CAM, AQP1, TMEM176B (see Figure 22);
- MITF-medium hypometabolic melanoma tumor cell subpopulation: genes SLC7A8, DLX5, TRIM67, CD36, PAX3, IP6K3, UBXN10, KIAA1161, LSMEM1; or alternatively, genes CD36, IP6K3, KIAA1161, TRIM67, LSMEM1, UBXN10, PAX3, SLC7A8; or, alternatively, genes DLX5, CD36, IP6K3, TRIM67, PAX3 (see Figure 22);
- pigmentation/differentiation melanoma tumor cell subpopulation: genes SLC24A5, PMEL, FABP7, SLC45A2, KIT, EDNRB, TRPM1, APOE, MLANA, MLPH, TYRP1, GPR143, TYR, RAB27A, SNAI2; or alternatively, genes GPR143, TYRP1, MLPH, MLANA, TRPM1, EDNRB, PMEL; or, alternatively, genes DCT, MITF, TYR, MLANA, TRPM1 (see Figure 22); and
- invasive melanoma tumor cell subpopulation: genes VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61; or, alternatively, genes RGS5, SLIT2, AXL, BGN, TGM2, TGFBI, CYR61; or, alternatively, genes WNT5A, AXL, TNC, TCF4, LOXL2, CYR61 (see Figure 22).

Importantly, analysis of expression of one or more of the genes of each of the above-listed gene expression signatures allows identification of the presence or absence of the according melanoma tumor cell subpopulations in bulk RNA isolated from biopsies taken from different melanoma patients. Figure 20 illustrates the identification of residual disease melanoma tumor cell subpopulations from bulk RNA analysis from biopsies taken before start of treatment with BRAF and MEK inhibitors compared to biopsies taken upon reaching the residual disease stage. Overall, the presence of the 4 above-listed melanoma tumor cell subpopulations is confirmed. In individual patients different combinations of at least 2 of the above-listed melanoma tumor cell subpopulations can be discerned.

### 2.6. Emergence of NDTCs in biopsies from patients on treatment

To search for NDTCs in a large cohort of human clinical samples we inspected the TCGA (The Cancer Genome Atlas) bulk RNA-seq dataset (n=469). Detectable expression of AQP1 and GFRA2 was seen in a very small subset (<7%) of Skin Cutaneous Melanomas (SKCM; n=32; Figure 5A-B). Strikingly, expression of most NDTC markers, including AQP1, was elevated in all GFRA2high compared to GFRA2low samples (Figure 5B). In contrast, MITF levels were lower in the GFRA2high samples. Note that only very few of the TCGA patients have been exposed to targeted therapy (2 out of 469) or any other therapies (less than 10% in total). Given that only one of the 32 GFRA2high samples was isolated from a treated patient (i.e. autologous tumor cell vaccine), these data indicated that NDTCs are present in non-negligible amounts (i.e. sufficient to detect marker expression by bulk RNA-seq) in 5% of drug naïve melanoma lesions. Importantly, here was no significant association between the presence/detectability of these cells and the *BRAF, NRAS* and *NF1* mutational status (data not shown).

As TCGA data corresponds to tumor and stromal compartments, AQP1 expression was examined histologically in high density tissue microarrays (TMAs) containing collectively 501 cores, corresponding to 163 different cutaneous metastatic melanoma samples (van Kempen et al. 2016, Science Transl Med 8:369ra177). The majority of these samples either scored negative for AQP1 or contained less than 5 % of positive cells (score 0/1, 84%). Consistent with the TCGA data, a minority of samples contained between 5% and 50% of positive cells (score 2; 6%) or others more than 50% of AQP1-positive cells (score 3; 10%) (Figure 5C). Interestingly, there was a striking inverse correlation between AQP1 positivity and expression of the proliferation marker KI67, consistent with the notion that AQP1-positive cells are not cycling (Figure 5C).

We next assessed whether NDTCs are present/enriched in biopsies from patients exposed to the BRAF/MEK combination therapy. Strikingly, RT-qPCR showed upregulation of expression of the NDTC markers AQP1, GFRA2, GFRA3, NGFR, L1CAM, RSPO3 and TMEM176B in virtually all ON drug biopsies analyzed (n=12; obtained after 1-2 weeks of therapy) as compared to biopsies taken before drug exposure (Figure 5E). IHC confirmed the increase in the number of AQP1-positive cells, which occurred in clusters, in all ON-treatment biopsies analyzed (n=5; Figure 4F). Together, these data indicated that although NDTCs exist in varying amounts in drug naïve lesions, these cells are invariably accumulating in the midst of concurrent RAF/MEK-inhibition.

### 2.7. NDTCs require FAK signaling for growth and survival

To assess whether emergence of NDTCs upon MAPK inhibition is cancer cell intrinsic or depends on micro-environmental cues, we exposed 2D cultures of Mel006 to near IC25 concentrations of dabrafenib and trametinib for 10 days.

FACS analysis revealed a 10-fold increase in GFRA2-positive cells in the drug-exposed cultures (Figure 6A). Transcriptome analysis of FACS-sorted GFRA2high cells indicated a striking enrichment of NDTC markers in these cells as compared to the GFRA2low subpopulation (Figure 6B). GSEA established a significant overlap between the transcriptome of these *in vitro* drug-exposed GFRA2high cells and the NDTCs isolated from phase 2 PDX lesions (Figure 6C). These data indicated that in vitro cultures of BRAF-mutant melanoma cells, exposed to both BRAF and MEK-inhibitors, do produce NDTC-like cells.

To further investigate the generality of this effect, and in particular whether emergence of the NDTCs is dependent on the genetic background and/or the initial transcriptional state, we exposed a series of short-term melanoma cultures to concurrent BRAF and/or MEK-inhibition (Figure 6D-E). Critically, upregulation of NDTC markers was observed in all BRAF-mutant cultures, irrespective of whether they exhibited a "proliferative" (i.e. MM074) or an "invasive" profile (i.e. MM029 and MM099). A similar increase was observed in NRAS-mutant (i.e. MM052 and MM165) and triple wild-type (i.e. MM163) cultures upon exposure to a MEK-inhibitor (Figure 6D-E). Because expression of the NDTC markers was undetectable in some of these cultures (i.e. MM029), these data further support the possibility that melanoma cells can transit into the NDTC state through phenotype switching and thus irrespective of their initial transcriptional state (i.e. proliferative versus invasive) and driver mutations.

GFRA2 and GFRA3 transduce GFLs-mediated activation of survival pathways such as the FAK and PI3K pathways (Airaksinen & Saarma 2002, Nature Rev Neurosci 3:383-394; Paratcha & Ledda 2008, Trends Neurosci 31:384-391). Interestingly, GDNF, one of the GFRA ligands, is also upregulated in the GFRA2high cells (Figure 6B), raising the possibility that GFRA527 dependent signaling may actually be engaged in NDTCs in an autocrine fashion.

Note that a role for AQP1 as an inducer of FAK signaling has also recently been suggested (Tomita et al. 2017; Int J Mol Sci 18:299). Moreover, GSEA showed that the focal adhesion pathway is significantly activated in NDTCs (Figure 6F). Consistently, western blot analysis revealed a dramatic increase in phosphorylated levels of FAK and AKT in drug-induced GFRA2 high cells (Figure 6G). Importantly, exposure to the FAK inhibitors PF-562271 and defactinib diminished the drug-dependent emergence of the GFRA2-high cell population in a dose-dependent manner (Figure 7H). Moreover, FACS-sorted GFRA2-high cells were more sensitive to FAK-inhibition than GFRA2-low cells indicating that the survival of NDTCs dependent on FAK signaling (Figure 6I). Together, these indicated that pharmacological inhibition of FAK signaling may offer one potential therapeutic avenue to block the drug-dependent emergence of NDTCs in minimal residual disease and thereby decrease, at least to some extent, drug tolerance heterogeneity. This particular therapeutic approach would be particularly attractive if the NDTCs were to be the major source of cells from which resistant clone(s) emerge. However, as the NDTC state is only one of the identified drug tolerant states this approach may be insufficient to prevent relapse. We therefore searched for alternative therapeutic strategies that take into account the drug tolerance heterogeneity highlighted by our single cell transcriptomic experiments.

### 2.8. Limiting drug tolerance heterogeneity by targeting RXR signaling

SCENIC identified a relatively limited number of TFs as potential drivers of the NDTC state. Taking advantage of the in vitro culture system described above we aimed at validating these predictions using genetic and pharmacological perturbation experiments. Knock-down experiments in the BRAF-mutant MEL006 melanoma cultures exposed BRAF and MEK-inhibitors validated the importance of SOX10 and SOX2, and to a lesser degree of MEF2C and TFAP2B, as upstream regulators of the NDTC transcriptional program (Figure 19A). Further inspection of the SCENIC-based GRN, however, highlighted a putative prominent role for RXR in this network as most of the discriminative NDTC markers, including GFRA2, GFRA3, AQP1, NGFR and, importantly, SOX10 itself are predicted to be direct target of this TF (Figure 14C). To test whether RXR signaling contribute to the emergence of NDTCs, we exposed melanoma cells to the IC50 concentrations of the BRAF and MEK-inhibitors in the presence of HX531, a potent and selective RXR antagonist (Ebisawa et al. 1999, Chem Pharm Bull 47:1778-1786). Strikingly, pharmacological inhibition of RXR completely inhibited emergence of drug-dependent induction of GFRA2-positive cells (Figure 7A and 19B). These data indicated that RXR signaling is required for the reprogramming of melanoma cells into NDTCs.

RXR functions primarily by heterodimerizing with and regulating the activity of a dozen of nuclear receptors (Evans & Mangelsdorf 2014, Cell 157:255-266). In certain heterodimers, known as "permissive" dimers such as RXR/PPAR and RXR/LXR, ligand activation of RXR results in transcriptional activation. Certain natural lipids, such as 9-cis retinoic acid (RA), and synthetic compounds, such as the FDA-approved bexarotene, selectively and potently activate such transcriptional response. Given that RXR (including the three family members RXR-alpha, RXR-beta and RXR-gamma) is not only expressed in NDTCs and appears to play such a prominent role in the GRN underlying the NDTC state, we reasoned that pharmacological activation of this network may divert the fate of other drug-tolerant subpopulations into the NDTC state. Remarkably, exposure of MEL006 melanoma cultures to bexarotene either alone, or in addition with dabrafenib and trametinib, significantly increased the proportion of NDTCs, as evidenced by an increase in number of GFRA2-positive cells and expression of a series of NDTC markers, including GFRA2 and GFRA3 (Figure 7A and data not shown). A similar increase in GFRA2 expression levels was observed in NRAS-mutant and triple wild-type short-term melanoma cultures exposed to both trametinib and bexarotene (Figure 7B-C). These data indicated that liganded RXR, presumably through the formation of permissive dimers, can drive melanoma cells into the NDTC state, irrespective of their genetic background and initial transcriptional state. Together, these data establish RXR signaling as a key driver of the NDTC state and show that the NDTC GRN architecture can be rewired through modulation of this pathway. These data offer interesting therapeutic perspectives. One prediction is that combining HX531 (HX) with MAPK therapeutics may significantly limit the risk of relapse by diminishing the pool of drug-tolerant cells. To test this possibility, we used a short-term melanoma culture (MM52) that exhibits a robust increase in NDTCs in response to MAPK-inhibition (i.e trametinid; Tra). In keeping with the prediction, HX531 exposure sensitized these cells to trametinib (Figure 7D-E).

Furthermore, PDX-mice (with MEL006) were treated with dabrafenib (BRAF inhibitor) at 30 mg/kg/day combined with trametinib (MEK inhibitor) at 0.3 mg/kg/day; or were treated with dabrafenib (BRAF inhibitor) at 30 mg/kg/day combined with trametinib (MEK inhibitor) at 0.3 mg/kg/day, and further combined with HX531 (RXR antagonist) at 10 mg/kg/day. At the end-point of the Figure 21A, one (1) out of 10 mice was still treated with BRAF/MEK inhibitors, whereas four (4) out of 10 mice were still on treatment with BRAF/MEK inhibitors further combined with RXR antagonist. In a further experiment, MEL006 PDX melanoma mice were treated by daily oral gavage once the tumor size reached 1000mm³. The study included three treatment arms: i) DT : 600µg dabrafenib, 6µg trametinib per mouse (daily), ii) DT+HX531: 600µg dabrafenib, 6µg trametinib, 1mg HX531 (RXR antagonist) per mouse (daily), and iii) DT+HX531+PF-562271 (FAK inhibitor): 600µg dabrafenib, 6µg trametinib, 1mg HX531, 4.7mg PF-562271 (FAK inhibitor) per mouse (daily). Progression free survival was estimated between treatment arms using Kaplan-Meier analysis. Compared to DT alone, DT+HX531 (**p=0.12 ,log rank Mantel Cox) and even more strikingly DT+HX531+PF-562271 (****p<0.0001, log rank Mantel Cox) significantly delayed time to disease progression (Figure 21C).

RT-qPCR analysis of gene expression (Figure 21B) confirms the decrease in expression of genes from the neuro-like gene expression signature, with an apparent increase in expression of genes from the MITF-medium/hypometabolic and pigmented/differentiation gene expression signatures. Figure 21 clearly indicates a very significant delay in disease progression, i.e. the delay in the BRAF/MEK/RXR inhibitor combination treated group is about the double of the delay in the BRAF/MEK inhibitor combination treated group, which is underpinning the importance of dealing with the neuro-like melanoma tumor cell subpopulation emerging in the residual disease stage. This combination may therefore result in clinical benefit, especially if cells that eventually become drug-resistant have their origin in the NDTC pool.

In addition, an alternative therapeutic strategy may be proposed, one that reduces drug tolerance heterogeneity by directing distinct drug-tolerant subpopulations towards a therapeutically sensitive state. Treatment of the MM52 culture with bexarotene (Bex) alone did not induce measurable long-term growth inhibition (Figure 7D-E). Importantly, whereas bexarotene at concentrations as low as 10nM significantly decreased the sensitivity of MM52 cells to trametinib, presumably by promoting the NDTC state, it increased the sensitivity to a combination of trametinib/FAK-inhibitors (Figure 7D-E). This specific combination was particularly effective, with cell death occurring in close to 100% of the cells after 3 days of treatment (data not shown). Together, these data indicated that bexarotene-induced activation of the NDTC gene regulatory network may offer a unique opportunity to decrease drug tolerance heterogeneity by driving different subpopulation of persisters into a FAK-dependent state.

### 2.9. The importance of the neural drug tolerant cell (NDTC) subpopulation.

First, Mel006 melanoma cells were treated for 6, 12, 24, and 48h with 50 nM dabrafenib + 10 nM trametinib (D/T 50/10nM) and gene expression was assessed by RT-qPCR. Subsets of the marker genes characteristic for each residual disease cell subpopulation (see Table 2) were quantified. As depicted in Figure 22B, showing fold change (FC) of gene expression in cells treated with D/T 50/10nM compared to non-treated cells, expression of all analyzed genes, including CD36 gene expression, increased upon treatment.

Subsequently, in an *in vitro* setting similar as described in 2.8. (MEL-006 colony assay, see also 1.14), the effect of inhibition of CD36 (marker of the MITF-medium hypometabolic residual disease subpopulation, see Table 2) in combination with BRAF/MEK-inhibition was assessed. Mel006 melanoma cells were depleted for CD36 expression using lentiviral transduction of short hairpin RNAs directed against CD36 (shCD36). As control served the empty vectors (shCtrl). 100k cells were seeded per well in a 6-well plate. 24h after seeding, medium was renewed and was supplemented with either 0 nM dabrafenib + 0 nM trametinib (non-treated, NT); 25 nM dabrafenib + 5 nM trametinib (D/T 25/5nM); or 10 nM dabrafenib + 2 nM trametinib (D/T 10/2nM). Cells were incubated for 14 days and then stained with crystal violet (vitality stain; see 1.14 hereinabove). CD36-depleted cells were clearly more susceptible to BRAF + MEK inhibition by dabrafenib and trametinib, as depicted in Figure 22A.

As CD36 was identified herein as a marker specific for the MITF-medium hypometabolic residual disease cell subpopulation, Mel006 melanoma cells were subjected to starvation. Mel006 melanoma cells were starved by culturing the cells for 48h in either glutamine-free medium, serum-starved medium (2%), or full starved medium (10% complete medium). After starvation, gene expression was assessed using RT-qPCR on a subset of the genes characteristic for this cell subpopulation (see Table 2; results depicted in Figure 22C). Only full starvation was able to induce to recapitulate the gene expression signature of the MITF-medium hypometabolic residual disease cell subpopulation emerging upon therapeutic pressure (combined BRAF/MEK inhibition). This provides further clarification of the hypometabolic status of these cells, which is similar to a starvation-like phenotype.

Finally, the effect of CD36 inhibition on the 4 different melanoma residual disease cell subpopulation emerging after combined BRAF/MEK inhibition was assessed. Mel006 melanoma cells were depleted for CD36 expression using lentiviral transduction of short hairpin RNAs directed against CD36 (shCD36). As control served the empty vectors (shCtrl). Cells were treated with 50 nM dabrafenib + 10 nM trametinib (D/T 50/10nM) for 48h and gene expression was assessed by RT-qPCR. Subsets of the marker genes characteristic for each subpopulation (see Table 2) were quantified. Surprisingly, inhibition of CD36 suppressed the emergence (+shCtrl *versus* + shCD36) not only of the MITF-medium hypometabolic residual disease cell subpopulation, but also of the neural drug tolerant cell subpopulation and of the pigmented state cell subpopulation. In contrast, the invasive state cell subpopulation appeared as potential "escape" route of the tumor cells subjected to combined BRAF/MEK/CD36 inhibition (see Figure 22D).

### 2.10. Discussion

Targeting the non-mutational tolerance phase (also referred to as minimal residual disease or MRD), which precedes acquisition of stable resistance, has been proposed as a salvage strategy for targeted cancer therapy (Sharma et al. 2010, Cell 141: 69-80; Smith et al. 2016, Cancer Cell 29:270-284). Our single cell transcriptomics analysis, however, raises concerns about the feasibility of such an approach by demonstrating that targeted therapy exacerbates intra-tumor heterogeneity by promoting the emergence of contrasting drug-tolerant states within the same residual lesion. We show that in a given BRAF-mutant PDX melanoma lesion, both MITF high, medium and low/negative states are emerging upon concurrent RAF/MEK-inhibition. These data also illustrate the importance of performing such experiments at single cell level. Indeed, consistent with the Smith et al. study we also observed an increase in MITF levels and activity at drug tolerance in a bulk analysis. However, the concomitant increase in MITF-negative and medium cells would have been missed had we not performed the single cell experiments.

Our findings further illustrate the remarkable phenotypic plasticity of melanoma cells by their ability to escape the deleterious effect of the drug-combination through very distinct mechanisms. One mechanism relies on the activation of a robust MITF transcriptional program, instructing cells to differentiate into highly pigment-producing cells. Another causes drug-exposed cells to shut down most of their metabolic activities, a mechanism that is predicted to rely, at least partly, on activation of a PAX3-dependent transcriptional program. Interestingly, PAX3 activation has been shown to induce resistance of melanoma to vemurafenib (Hartsough & Aplin 2016, Clin Cancer Res 22:1550-1552; Liu et al. 2013, J Invest Dermatol 133:2041-2049). Whether PAX3 upregulation is causatively involved in metabolic reprogramming, and if so how, remains to be elucidated. Note that it has been proposed that PAX3 activation is a key triggering event in drug-induced MITF upregulation and, thereby, in MITF-mediated drug tolerance (Smith et al. 2016, Cancer Cell 29:270-284). Our findings, instead, highlight a role for PAX3 activation in cells that retain moderate MITF levels, and not in the MITFhigh pigmentation subpopulation, indicating that PAX3-induced drug tolerance may be independent of MITF. Interestingly, one of the SCENIC predicted PAX3 direct target gene is BRAF. Because numerous mechanisms that lead to reactivation of BRAF function and/or elevation of its expression, such as increased copy number (Xue et al. 2017, Nature Med 23:929-937), causes resistance to MAPK-therapeutics PAX3-dependent upregulation of BRAF may contributes, at least partly, to PAX3-induced drug tolerance.

A third, very distinct, mechanism of drug tolerance is activated in NDTCs, which exhibit no to very low MITF activity. These observations are consistent with the rheostat model stating that melanoma cells can adopt very distinct fates depending on the levels of MITF (Hoek & Goding 2010, Pigment Cell Melanoma Res 23:746-759). Importantly, however, the MITF-negative state described in the rheostat model, the classical/canonical "invasive" state, exhibits low levels of both MITF and SOX10. Unexpectedly, although this particular state was clearly identifiable in drug-naive PDX lesions its occurrence had decreased in drug tolerant lesions. In contrast, the NDTC state was identified as a (new) MITF negative state, one that was enriched upon drug exposure and that harbors a dormant neural stem cell-like transcriptional program driven, at least in part, by SOX10.

Importantly, although the NDTC and "invasive" states are both characterized by loss of MITF and differentiation markers, we provide evidence that these transcriptional states represent two distinct subpopulations of melanoma cells.

Note that the NDTC state is also clearly distinct from the (pre-) resistant state recently derived from *in vitro* cultured melanoma cells exposed to BRAF-inhibitor alone (Shaffer et al. 2017, Nature 546:431-435). In fact, this is only partly surprising as the gene expression signature of these "jackpot" cells significantly overlaps with the one described for the classical "invasive" cells. In contrast, the transcriptome of NDTCs presents some degree of similarity with the gene expression signatures obtained from bulk RNA-seq of drug-exposed melanoma cultures enriched for IDTCs (Menon et al. 2015, Oncogene 34:4545) and slow cycling NGFRhigh cells (Fallahi-Sichani et al. 2017, Mol Systems Biol 13:905). Collectively these data indicate that there exists, at least, two distinct MITF-negative "de-differentiated" states. An interesting hypothesis to explain the existence of these two distinct states is that cells, depending on the growth conditions and stress level, may initiate various adaptive responses and behaviors (Jimenez and Goding, review under consideration). An initial response to a stress situation may be to opt for an invasive/migratory behavior in quest of a more favorable environment. If stress is not resolved, similarly to the phenotypic-switch in bacteria from invasion to sporulation (Vlamakis et al. 2008, Genes Dev 22:945-953), cells may enter a dormancy state, paralleling the melanoma NDTC state described herein. This model may also help explain why different states were identified in the various studies described above. Composition of culture medium and concentrations of the drugs used for these experiments may influence their outcome. Exposure of *in vitro* cultured melanoma cells, which are often grown in rich medium supplemented with growth factors in excess, to a BRAF-inhibitor alone may favor the transition into an invasive cell state. In contrast, exposure of melanoma cells growing in a harsher *in vivo* microenvironment to the more clinically-relevant RAF/MEK combination may instead favor entry in the dormant NDTC state. SCENIC predicted RXR as a key driver of the NDTC state. Remarkably, inhibiting this pathway with a pan RXR antagonist completely blocked the emergence of NDTCs in drug-exposed melanoma cultures, thus validating the *in silico* predictions experimentally. Our collective findings indicate that RXR governs a survival signaling cascade that converges to the activation of FAK signaling and thereby contributes to the survival of drug-exposed NDTCs. FAK activation may be partly due to an autocrine mechanism involving RXR-dependent induction of expression of GFRA2 and GFRA3 and one of their ligands, GDNF. In turn, liganded GFRA2 and/or GFRA3 may contribute, at least partly, to FAK activation (Paratcha & Ledda 2008, Trends Neurosci 31:384-391). Although this possibility needs to be further tested experimentally, it may explain why NDTCs occur as small clusters of cells. GDNF may indeed function as an autocrine (and possibly paracrine) factor supporting the survival of NDTCs in a niche-like spatial organization. Note that RXR-dependent activation of AQP1 expression may also partly contribute to FAK activation (Tomita et al. 2017, Int J Mol Sci 18:299). Importantly, exposure to the FAK-inhibitors also blocked the drug-dependent emergence of the GFRA2-high cell population *in vitro.* This latter observation is consistent with recent findings showing that drugs targeting FAK signaling increases sensitivity of melanoma cells to RAF/MEK inhibitors (Fallahi-Sichani et al. 2017, Mol Systems Biol 13:905). Collectively, these findings indicated that combining MAPK-targeting agents with RXR antagonists alone (or further combined with e.g. FAK-inhibitors), prevents the emergence of NDTCs in minimal residual diseases and significantly delays the onset of resistance. This approach could in fact potentially completely prevent relapse if NDTCs were to be the only source of drug resistant cells. In support of NDTCs being an important reservoir of resistant cells, they exhibit a stem cell-like gene expression signature, which may endow them with self-renewal capacity and, importantly, ability to contribute to the formation of heterogeneous relapse lesions. Consistent with this possibility, AQP1-positive cells are found in reservoir compartments of normal human tissues and were observed in high numbers at various sites of recurrent melanoma lesions. Moreover, we observed their ability to generate mitotically active daughter cells upon drug withdrawal *in vitro* (data not shown).

It cannot be excluded, however, that NDTCs do not actually contribute to relapse at all. They may indeed represent an indefinitely/terminally dormant subpopulation of cells that are eventually outcompeted by other proliferating cells upon tumor regrow. Moreover, given the notorious plasticity of melanoma cells other drug-tolerant (i.e. MITFhigh pigmentation or MITFmedium hypometabolic) cells may also be reprogrammed into relapse initiating cells. In this context, one needs to envisage a more elaborated therapeutic strategy, one that diverts the fate of all/most distinct drug-tolerant persisters into a single (or limited numbers) of either permanently dormant and/or therapeutically sensitive state(s). Forcing cells to adopt a NDTC state is possible route towards this goal as these cells are dependent on FAK signaling for survival and are, by and large, absent from lesions that acquired resistance. Therefore, even if these cells contribute to relapse they can only do so following transcriptional reprogramming into mitotically active cells, an event that could be prevented by agents that maintain the cells in their dormant NDTC state. Remarkably, pharmacological activation of RXR-signaling was sufficient to enhance drug-induced entry into this particular state. The RXR agonist Bexarotene had a potent inducer effect on the ability of BRAF and MEK-inhibitors to promote conversion into the NDTC state. Consequently, this agent sensitized melanoma cells to the co-targeting of MAPK and FAK-signaling. These data therefore suggest a two-step therapeutic strategy in which MAPK-targeting is used to de-bulk the melanoma lesions and induce drug tolerance. In a second step, pharmacological activation of the GRN underlying the NDTC state is achieved by exposure to Bexarotene. This dormancy-directed strategy limits heterogeneity of drug tolerance by forcing DT cells to adopt a dormant NDTC phenotype. The cells may thus remain dormant for prolonged period of time and/or eventually be eradicated by taking advantage of their sensitivity to FAK-inhibitors. Important to note, bexarotene is FDA-approved and several FAK-inhibitors are already in advanced phases in various clinic trials.

Such an approach is therefore rapidly amenable to the clinical. Importantly, our findings also indicate that this approach may be applicable to a large spectrum of patients, and not only to patients harbouring BRAF-mutant melanomas. We indeed provide evidence that NDTCs are present in drug naïve NRAS-mutant melanomas and that expression levels of NDTC-specific markers increased in response to MEK-inhibition, an effect that was exacerbated upon addition to Bexarotene. It will therefore be interesting to assess the sensitivity of these cells to FAK-inhibitors and the long-term anti-tumor efficiency of coinhibition of MEK/FAK-signaling in the presence of Bexarotene both in preclinical models and eventually in patients.

The concept of directed phenotype switching as an antimelanoma strategy has already been suggested (Saez-Ayala et al., 2013). The previously proposed strategy, however, was very different. It was based on the observation that methotrexate (MTX) induces MITF levels and activity and therefore promotes melanoma differentiation and that MITF-high/differentiated cells are sensitive to a tyrosinase-processed antifolate prodrug TMECG. The MTX/TMECG combination delivered effective antimelanoma responses *in vitro* and mouse preclinical models. However, MTX is a chemotherapeutic agent, which causes severe advert events in patients. The therapeutic strategies we describe herein are not dependent on the use of such agents. Moreover, they take advantage of the well-proven efficacy of the RAF/MEK-inhibitor combination in patients and essentially aim at targeting a smaller pool of residual cells. It may, nevertheless, also be interesting in the future to test whether the addition of TMECG to the treatment regimen we propose above provide additional potential clinical benefit. Note that the targeting of MITFhigh/differentiated cells is also possible using a drug-conjugated antibody against GPNMB, a melanosomal antigen (Ott et al., 2017; Rose et al., 2016). Combined to the dormancy-directed approach these agents may prove useful to completely eradicate the MAPK-inhibitor refractory pool of cells and convert targeted antimelanoma therapy into a curative approach.

A further, and unexpected, observation related to the susceptibility of NDTC cells to CD36 inhibition. CD36 was identified herein as marker for a hypometabolic- or starvation-like-tye of MRD cells. In fact, inhibition of CD36 appeared to affect emergence of 3 out of the 4 melanoma MRD subpopulations (NDTCs, the MITFlow/hypometabolic-type cells, and the invasive-type cells), but to promote emergence of the pigmentation-type MRD subpopulation. Thus, a CD36 inhibitor, or a combination of an RXR antagonist with a CD36 inhibitor, potentially further with a FAK inhibitor and/or an agent targeting the pigmented cells (see above, targeting of MITFhigh/differentiated cells; TMECG, GPNMB, nelfinavir) provide further routes of targeted therapy of melanoma MRD. In any case, the unexpected effect of CD36 inhibition on NDTC type cells further underscores the importance of this cell type during MRD.

## Claims

1. A method of analysis of a human tumor, the method comprising detecting in a biological sample from the human tumor or in a biological sample comprising human tumor nucleic acids an increased expression level:
- of 4 to 8 genes selected from gene signature A1 consisting of the genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; and wherein detection of an increased expression level of the selected genes is indicating the emergence or presence of neural drug tolerant tumor cells (NDTCs); and
- of 4 to 8 genes selected from gene signature B1 consisting of the genes SLC7A8, DLX5, TRIM67, CD36, PAX3, IP6K3, UBXN10, KIAA1161, LSMEM1; and wherein detection of an increased expression level of the selected genes is indicating the emergence or presence of hypometabolic tumor cells (HMTCs); and
- of 4 to 8 genes selected from gene signature C1 consisting of the genes SLC24A5, PMEL, FABP7, SLC45A2, KIT, EDNRB, TRPM1, APOE, MLANA, MLPH, TYRP1, GPR143, TYR, RAB27A, SNAI2, MITF, DCT; and wherein detection of an increased expression level of the selected genes is indicating the emergence or presence of pigmented tumor cells; and
- of 4 to 8 genes selected from gene signature D1 consisting of VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4; and wherein detection of an increased expression level of the selected genes is indicating the emergence or presence of invasive tumor cells; and
wherein increased expression level of a selected gene is determined compared to a reference expression level of that selected gene.

2. The method according to claim 1 for use in selecting therapy or in optimizing therapy for a patient having a tumor, or for use in predicting the response of a tumor to therapy.

3. The method according to claim 2 further comprising the step of selecting therapy for a patient having a tumor wherein the selected therapy is normalizing the expression levels of a selected gene detected to be increased in the biological sample compared to the reference expression level of the selected gene.

4. The method according to claim 3 wherein the therapy is chosen from:
- a compound that is cytotoxic or cytostatic for the population of cells in the tumor with, compared to reference expression levels, increased expression levels of the 4 to 8 genes selected from gene signature A1; and/or
- a compound that is cytotoxic or cytostatic for the population of cells in the tumor with, compared to reference expression levels, increased expression levels of the 4 to 8 genes selected from gene signature B1; and/or
- a compound that is cytotoxic or cytostatic for the population of cells in the tumor with, compared to reference expression levels, increased expression levels of the 4 to 8 genes selected from gene signature C1; and/or
- a compound that is cytotoxic or cytostatic for the cells in the tumor with, compared to reference expression levels, increased expression levels of the 4 ot 8 genes selected from gene signature D1.

5. The method according to claim 4 wherein:
- the compound that is cytotoxic or cytostatic for the population of cells in the tumor with, compared to reference expression levels, increased expression levels of the 4 to 8 genes selected from gene signature A1, is an antagonist of retinoid X receptor gamma (RXRG), an antagonist of RXRG combined with a FAK-inhibitor, a CD36 antagonist, or a CD36 antagonist combined with an antifolate drug, a melanocyte-directed enzyme prodrug, an antibody drug conjugate wherein the antibody is targeting GPNMB, or nelfinavir;
- the compound that is cytotoxic or cytostatic for the population of cells in the tumor with, compared to reference expression levels, increased expression levels of the 4 to 8 genes selected from gene signature B1, is a CD36 antagonist, an inhibitor of PAX3, or a CD36 antagonist combined with an antifolate drug, a melanocyte-directed enzyme prodrug, an antibody drug conjugate wherein the antibody is targeting GPNMB, or nelfinavir;
- the compound that is cytotoxic or cytostatic for the population of cells in the tumor with, compared to reference expression levels, increased expression levels of the 4 to 8 genes selected from gene signature C1, is an antifolate drug, a melanocyte-directed enzyme prodrug, an antibody drug conjugate wherein the antibody is targeting GPNMB, or nelfinavir;
- a compound that is cytotoxic or cytostatic for the cells in the tumor with, compared to reference expression levels, increased expression levels of the 4 to 8 genes selected from gene signature D1, is an inhibitor of AXL.

6. The method according to any one of claims 1 to 5 wherein the tumor is melanoma.

7. The method according to any one of claims 1 to 6 wherein the expression level is an mRNA expression level.

8. The method according to claim 7 wherein the mRNA expression level is determined by RNA-sequencing, PCR, RT-PCR, gene expression profiling, serial analysis of gene expression, microarray analysis, whole genome sequencing, or is determined based on at least one of an amplification reaction, a sequencing reaction, a melting reaction, a hybridization reaction or a reverse hybridization reaction.

9. The method according to any one of claims 1 to 8 wherein the expression level of at most 250 genes is determined.

10. The method according to any one of claims 1 to 6 wherein the expression level is a protein expression level.

11. A method for screening for cytotoxic or cytostatic compounds, the method comprising:
- culturing tumor cells;
- applying a therapy to the cultured tumor cells, wherein the therapy induces the occurrence of one or more populations of tumor cells that are reversibly resistant to the therapy, and wherein the populations are one or more of:
- a population with increased expression of 4 to 8 genes selected from gene signature A1 consisting of the genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; and/or
- a population with increased expression of 4 to 8 genes selected from gene signature B1 consisting of the genes SLC7A8, DLX5, TRIM67, CD36, PAX3, IP6K3, UBXN10, KIAA1161, LSMEM1; and/or
- a population with increased expression of 4 to 8 genes selected from gene signature C1 consisting of the genes SLC24A5, PMEL, FABP7, SLC45A2, KIT, EDNRB, TRPM1, APOE, MLANA, MLPH, TYRP1, GPR143, TYR, RAB27A, SNAI2, MITF, DCT; and/or
- a population with increased expression of 4 to 8 genes selected from gene signature D1 consisting of VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4; wherein the increased expression of a selected gene is determined compared to a reference expression level of the selected gene;
- contacting the one or more populations of tumor cells that are reversibly resistant to the therapy with a compound that is a candidate compound cytotoxic or cytostatic to one or more of the populations of tumor cells that are reversibly resistant to the therapy;
- identifying a compound cytotoxic or cytostatic to one or more of the populations of tumor cells that are reversibly resistant to the therapy.

12. A method for screening for cytotoxic or cytostatic compounds, the method comprising:
- culturing tumor cells;
- applying a therapy to the cultured tumor cells, wherein the therapy induces the occurrence of one or more populations of tumor cells that are reversibly resistant to the therapy, and wherein the populations are one or more of:
- a population with increased expression of 4 to 8 genes selected from gene signature A1 consisting of the genes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B; and/or
- a population with increased expression of 4 to 8 genes selected from gene signature B1 consisting of the genes SLC7A8, DLX5, TRIM67, CD36, PAX3, IP6K3, UBXN10, KIAA1161, LSMEM1; and/or
- a population with increased expression of 4 to 8 genes selected from gene signature C1 consisting of the genes SLC24A5, PMEL, FABP7, SLC45A2, KIT, EDNRB, TRPM1, APOE, MLANA, MLPH, TYRP1, GPR143, TYR, RAB27A, SNAI2, MITF, DCT; and/or
- a population with increased expression of 4 to 8 genes selected from gene signature D1 consisting of VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4; wherein the increased expression of a selected gene is determined compared to a reference expression level of the selected gene;
- contacting the one or more populations of tumor cells that are reversibly resistant to the therapy with a compound that is a candidate compound for modifying expression or function of a gene selected from any of gene signatures A1, B1, C1, or D1;
- identifying as cytotoxic or cytostatic compound a compound that is modifying expression or function a gene selected from any of gene signatures A1, B1, C1, or D1.

## Patentansprüche

1. Verfahren zum Analysieren eines menschlichen Tumors, wobei das Verfahren ein Erfassen, in einer biologischen Probe von dem menschlichen Tumor oder in einer biologischen Probe, die Nukleinsäuren des humanen Tumors umfasst, eines erhöhten Expressionsniveaus:
- von 4 bis 8 Genen, die aus der Gensignatur A1 ausgewählt sind, die aus den Genen AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B besteht; und wobei eine Erfassung eines erhöhten Expressionsniveaus der ausgewählten Gene das Entstehen oder das Vorliegen von neuronalen arzneimitteltoleranten Tumorzellen (NDTZ) angibt; und
- von 4 bis 8 Genen, die aus der Gensignatur B1 ausgewählt sind, die aus den Genen SLC7A8, DLX5, TRIM67, CD36, PAX3, IP6K3, UBXN10, KIAA1161, LSMEM1 besteht; und wobei eine Erfassung eines erhöhten Expressionsniveaus der ausgewählten Gene das Entstehen oder das Vorliegen von hypometabolischen Tumorzellen (HMTZ) angibt; und
- von 4 bis 8 Genen, die aus der Gensignatur C1 ausgewählt sind, die aus den Genen SLC24A5, PMEL, FABP7, SLC45A2, KIT, EDNRB, TRPM1, APOE, MLANA, MLPH, TYRP1, GPR143, TYR, RAB27A, SNAI2, MITF, DCT besteht; und wobei eine Erfassung eines erhöhten Expressionsniveaus der ausgewählten Gene das Entstehen oder das Vorliegen von pigmentierten Tumorzellen angibt; und
- von 4 bis 8 Genen, die aus der Gensignatur D1 ausgewählt sind, die aus den Genen VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4 besteht; und wobei eine Erfassung eines erhöhten Expressionsniveaus der ausgewählten Gene das Entstehen oder das Vorliegen von invasiven Tumorzellen angibt, umfasst; und
wobei ein erhöhtes Expressionsniveau eines ausgewählten Gens im Vergleich zu einem Referenzexpressionsniveau jenes ausgewählten Gens bestimmt wird.

2. Verfahren nach Anspruch 1 zur Verwendung beim Auswählen einer Therapie oder beim Optimieren einer Therapie für einen Patienten mit einem Tumor oder zur Verwendung beim Vorhersagen des Ansprechens eines Tumors auf eine Therapie.

3. Verfahren nach Anspruch 2, ferner umfassend den Schritt eines Auswählens einer Therapie für einen Patienten mit einem Tumor, wobei die ausgewählte Therapie die Expressionsniveaus eines ausgewählten Gens normalisiert, von denen erfasst wurde, dass sie in der biologischen Probe im Vergleich zu dem Referenzexpressionsniveau des ausgewählten Gens erhöht sind.

4. Verfahren nach Anspruch 3, wobei die Therapie ausgewählt ist aus:
- einer Verbindung, die zytotoxisch oder zytostatisch für die Population von Zellen in dem Tumor mit im Vergleich zu Referenzexpressionsniveaus erhöhten Expressionsniveaus der 4 bis 8 Gene, die aus der Gensignatur A1 ausgewählt sind, ist; und/oder
- einer Verbindung, die zytotoxisch oder zytostatisch für die Population von Zellen in dem Tumor mit im Vergleich zu Referenzexpressionsniveaus erhöhten Expressionsniveaus der 4 bis 8 Gene, die aus der Gensignatur B1 ausgewählt sind, ist; und/oder
- einer Verbindung, die zytotoxisch oder zytostatisch für die Population von Zellen in dem Tumor mit im Vergleich zu Referenzexpressionsniveaus erhöhten Expressionsniveaus der 4 bis 8 Gene, die aus der Gensignatur C1 ausgewählt sind, ist; und/oder
- einer Verbindung, die zytotoxisch oder zytostatisch für die Zellen in dem Tumor mit im Vergleich zu Referenzexpressionsniveaus erhöhten Expressionsniveaus der 4 bis 8 Gene, die aus der Gensignatur D1 ausgewählt sind, ist.

5. Verfahren nach Anspruch 4, wobei:
- die Verbindung, die zytotoxisch oder zytostatisch für die Population von Zellen in dem Tumor mit im Vergleich zu Referenzexpressionsniveaus erhöhten Expressionsniveaus der 4 bis 8 Gene, die aus der Gensignatur A1 ausgewählt sind, ist, ein Antagonist von Retinoid-X-Rezeptor-Gamma (RXRG), ein Antagonist von RXRG in Kombination mit einem FAK-Inhibitor, ein CD36-Antagonist oder ein CD36-Antagonist in Kombination mit einem Antifolat-Arzneimittel, ein auf Melanozyten ausgerichtetes Enzym-Prodrug, ein Antikörper-Arzneimittel-Konjugat, wobei der Antikörper GPNMB targetiert, oder Nelfinavir ist;
- die Verbindung, die zytotoxisch oder zytostatisch für die Population von Zellen in dem Tumor mit im Vergleich zu Referenzexpressionsniveaus erhöhten Expressionsniveaus der 4 bis 8 Gene, die aus der Gensignatur B1 ausgewählt sind, ist, ein CD36-Antagonist, ein Inhibitor von PAX3 oder ein CD36-Antagonist in Kombination mit einem Antifolat-Arzneimittel, ein auf Melanozyten ausgerichtetes Enzym-Prodrug, ein Antikörper-Arzneimittel-Konjugat, wobei der Antikörper GPNMB targetiert, oder Nelfinavir ist;
- die Verbindung, die zytotoxisch oder zytostatisch für die Population von Zellen in dem Tumor mit im Vergleich zu Referenzexpressionsniveaus erhöhten Expressionsniveaus der 4 bis 8 Gene, die aus der Gensignatur C1 ausgewählt sind, ist, ein Antifolat-Arzneimittel, ein auf Melanozyten ausgerichtetes Enzym-Prodrug, ein Antikörper-Arzneimittel-Konjugat, wobei der Antikörper GPNMB targetiert, oder Nelfinavir ist;
- eine Verbindung, die zytotoxisch oder zytostatisch für die Zellen in dem Tumor mit im Vergleich zu Referenzexpressionsniveaus erhöhten Expressionsniveaus der 4 bis 8 Gene, die aus der Gensignatur D1 ausgewählt sind, ist, ein Inhibitor von AXL ist.

6. Verfahren nach einem der Ansprüche 1 bis 5, wobei der Tumor ein Melanom ist.

7. Verfahren nach einem der Ansprüche 1 bis 6, wobei das Expressionsniveau ein mRNA-Expressionsniveau ist.

8. Verfahren nach Anspruch 7, wobei das mRNA-Expressionsniveau durch RNA-Sequenzierung, PCR, RT-PCR, Genexpressionsprofilierung, serielle Analyse der Genexpression, Mikroarray-Analyse, Vollgenomsequenzierung bestimmt wird oder auf der Basis mindestens einer von einer Amplifikationsreaktion, einer Sequenzierungsreaktion, einer Schmelzreaktion, einer Hybridisierungsreaktion oder einer reversen Hybridisierungsreaktion bestimmt wird.

9. Verfahren nach einem der Ansprüche 1 bis 8, wobei das Expressionsniveau von höchstens 250 Genen bestimmt wird.

10. Verfahren nach einem der Ansprüche 1 bis 6, wobei das Expressionsniveau ein Proteinexpressionsniveau ist.

11. Verfahren zum Screenen auf zytotoxische oder zytostatische Verbindungen, wobei das Verfahren umfasst:
- Kultivieren von Tumorzellen;
- Anwenden einer Therapie auf die kultivierten Tumorzellen, wobei die Therapie das Auftreten einer oder mehrerer Populationen von Tumorzellen induziert, die gegen die Therapie reversibel resistent sind, und wobei die Populationen eine oder mehrere der folgenden sind:
- einer Population mit erhöhter Expression von 4 bis 8 Genen, die aus der Gensignatur A1 ausgewählt sind, die aus den Genen AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B besteht; und/oder
- einer Population mit erhöhter Expression von 4 bis 8 Genen, die aus der Gensignatur B1 ausgewählt sind, die aus den Genen SLC7A8, DLX5, TRIM67, CD36, PAX3, IP6K3, UBXN10, KIAA1161, LSMEM1 besteht; und/oder
- einer Population mit erhöhter Expression von 4 bis 8 Genen, die aus der Gensignatur C1 ausgewählt sind, die aus den Genen SLC24A5, PMEL, FABP7, SLC45A2, KIT, EDNRB, TRPM1, APOE, MLANA, MLPH, TYRP1, GPR143, TYR, RAB27A, SNAI2, MITF, DCT besteht; und/oder
- einer Population mit erhöhter Expression von 4 bis 8 Genen, die aus der Gensignatur D1 ausgewählt sind, die aus den Genen VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4 besteht;
wobei die erhöhte Expression eines ausgewählten Gens im Vergleich zu einem Referenzexpressionsniveau des ausgewählten Gens bestimmt wird;
- Inkontaktbringen der einen oder der mehreren Populationen von Tumorzellen, die gegen die Therapie reversibel resistent sind, mit einer Verbindung, die eine Kandidatenverbindung ist, die zytotoxisch oder zytostatisch für eine oder mehrere der Populationen von Tumorzellen, die gegen die Therapie reversibel resistent sind, ist;
- Identifizieren einer Verbindung, die zytotoxisch oder zytostatisch für eine oder mehrere der Populationen von Tumorzellen, die gegen die Therapie reversibel resistent sind, ist,

12. Verfahren zum Screenen auf zytotoxische oder zytostatische Verbindungen, wobei das Verfahren umfasst:
- Kultivieren von Tumorzellen;
- Anwenden einer Therapie auf die kultivierten Tumorzellen, wobei die Therapie das Auftreten einer oder mehrerer Populationen von Tumorzellen induziert, die gegen die Therapie reversibel resistent sind, und wobei die Populationen eine oder mehrere der folgenden sind:
- einer Population mit erhöhter Expression von 4 bis 8 Genen, die aus der Gensignatur A1 ausgewählt sind, die aus den Genen AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B besteht; und/oder
- einer Population mit erhöhter Expression von 4 bis 8 Genen, die aus der Gensignatur B1 ausgewählt sind, die aus den Genen SLC7A8, DLX5, TRIM67, CD36, PAX3, IP6K3, UBXN10, KIAA1161, LSMEM1 besteht; und/oder
- einer Population mit erhöhter Expression von 4 bis 8 Genen, die aus der Gensignatur C1 ausgewählt sind, die aus den Genen SLC24A5, PMEL, FABP7, SLC45A2, KIT, EDNRB, TRPM1, APOE, MLANA, MLPH, TYRP1, GPR143, TYR, RAB27A, SNAI2, MITF, DCT besteht; und/oder
- einer Population mit erhöhter Expression von 4 bis 8 Genen, die aus der Gensignatur D1 ausgewählt sind, die aus den Genen VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4 besteht;
wobei die erhöhte Expression eines ausgewählten Gens im Vergleich zu einem Referenzexpressionsniveau des ausgewählten Gens bestimmt wird;
- Inkontaktbringen der einen oder der mehreren Populationen von Tumorzellen, die gegen die Therapie reversibel resistent sind, mit einer Verbindung, die eine Kandidatenverbindung zum Modifizieren einer Expression oder einer Funktion eines Gens ist, das aus einer beliebigen der Gensignaturen A1, B1, C1 oder D1 ausgewählt ist;
- Identifizieren einer Verbindung, die eine Expression oder eine Funktion eines Gens modifiziert, das aus einer beliebigen der Gensignaturen A1, B1, C1 oder D1 ausgewählt ist, als eine zytotoxische oder zytostatische Verbindung.

## Revendications

1. Procédé d'analyse d'une tumeur humaine, le procédé comprenant détecter dans un échantillon biologique provenant de la tumeur humaine ou dans un échantillon biologique comprenant des acides nucléiques de tumeur humaine un niveau d'expression accru :
- de 4 à 8 gènes sélectionnés parmi la signature génomique A1 constituée des gènes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B ; et dans lequel la détection d'un niveau d'expression accru des gènes sélectionnés indique l'émergence ou la présence de cellules tumorales neurales tolérantes au médicament (NDTC) ; et
- de 4 à 8 gènes sélectionnés parmi la signature génomique B1 constituée des gènes SLC7A8, DLX5, TRIM67, CD36, PAX3, IP6K3, UBXN10, KIAA1161, LSMEM1 ; et dans lequel la détection d'un niveau d'expression accru des gènes sélectionnés indique l'émergence ou la présence de cellules tumorales hypométaboliques (HMTC) ; et
- de 4 à 8 gènes sélectionnés parmi la signature génomique C1 constituée des gènes SLC24A5, PMEL, FABP7, SLC45A2, KIT, EDNRB, TRPM1, APOE, MLANA, MLPH, TYRP1, GPR143, TYR, RAB27A, SNAI2, MITF, DCT ; et dans lequel la détection d'un niveau d'expression accru des gènes sélectionnés indique l'émergence ou la présence de cellules tumorales pigmentées ; et
- de 4 à 8 gènes sélectionnés parmi la signature génomique D1 constituée de VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4 ; et dans lequel la détection d'un niveau d'expression accru des gènes sélectionnés indique l'émergence ou la présence de cellules tumorales invasives ; et
dans lequel le niveau d'expression accru d'un gène sélectionné est déterminé par rapport à un niveau d'expression de référence de ce gène sélectionné.

2. Procédé selon la revendication 1 destiné à être utilisé dans la sélection de la thérapie ou dans l'optimisation de la thérapie pour un patient ayant une tumeur, ou destiné à être utilisé dans la prédiction de la réponse d'une tumeur à la thérapie.

3. Procédé selon la revendication 2, comprenant en outre l'étape de sélection de la thérapie pour un patient ayant une tumeur, dans lequel la thérapie sélectionnée normalise les niveaux d'expression d'un gène sélectionné détectés pour être accrus dans l'échantillon biologique par rapport au niveau d'expression de référence du gène sélectionné.

4. Procédé selon la revendication 3, dans lequel la thérapie est choisie parmi :
- un composé qui est cytotoxique ou cytostatique pour la population de cellules dans la tumeur avec, par rapport à des niveaux d'expression de référence, des niveaux d'expression accrus des 4 à 8 gènes sélectionnés parmi la signature génomique A1 ; et/ou
- un composé qui est cytotoxique ou cytostatique pour la population de cellules dans la tumeur avec, par rapport à des niveaux d'expression de référence, des niveaux d'expression accrus des 4 à 8 gènes sélectionnés parmi la signature génomique B1 ; et/ou
- un composé qui est cytotoxique ou cytostatique pour la population de cellules dans la tumeur avec, par rapport à des niveaux d'expression de référence, des niveaux d'expression accrus des 4 à 8 gènes sélectionnés parmi la signature génomique C1 ; et/ou
- un composé qui est cytotoxique ou cytostatique pour les cellules dans la tumeur avec, par rapport à des niveaux d'expression de référence, des niveaux d'expression accrus des 4 à 8 gènes sélectionnés parmi la signature génomique D1.

5. Procédé selon la revendication 4, dans lequel :
- le composé qui est cytotoxique ou cytostatique pour la population de cellules dans la tumeur avec, par rapport à des niveaux d'expression de référence, des niveaux d'expression accrus des 4 à 8 gènes sélectionnés parmi la signature génomique A1, est un antagoniste du récepteur X de rétinoïdes gamma (RXRG), un antagoniste de RXRG combiné à un inhibiteur FAK, un antagoniste CD36 ou un antagoniste CD36 combiné à un médicament antifolate, un promédicament enzymatique dirigé contre les mélanocytes, un conjugué anticorps médicament dans lequel l'anticorps cible GPNMB ou du nelfinavir ;
- le composé qui est cytotoxique ou cytostatique pour la population de cellules dans la tumeur avec, par rapport à des niveaux d'expression de référence, des niveaux d'expression accrus des 4 à 8 gènes sélectionnés parmi la signature génomique B1, est un antagoniste CD36, un inhibiteur de PAX3 ou un antagoniste CD36 combiné à un médicament antifolate, un promédicament enzymatique dirigé contre les mélanocytes, un conjugué anticorps médicament dans lequel l'anticorps cible GPNMB ou du nelfinavir ;
- le composé qui est cytotoxique ou cytostatique pour la population de cellules dans la tumeur avec, par rapport à des niveaux d'expression de référence, des niveaux d'expression accrus des 4 à 8 gènes sélectionnés parmi la signature génomique C1, est un médicament antifolate, un promédicament enzymatique dirigé contre les mélanocytes, un conjugué anticorps médicament dans lequel l'anticorps cible GPNMB ou du nelfinavir ;
- le composé qui est cytotoxique ou cytostatique pour les cellules dans la tumeur avec, par rapport à des niveaux d'expression de référence, des niveaux d'expression accrus des 4 à 8 gènes sélectionnés parmi la signature génomique D1, est un inhibiteur de AXL.

6. Procédé selon l'une quelconque des revendications 1 à 5, dans lequel la tumeur est un mélanome.

7. Procédé selon l'une quelconque des revendications 1 à 6, dans lequel le niveau d'expression est un niveau d'expression d'ARNm.

8. Procédé selon la revendication 7, dans lequel le niveau d'expression d'ARNm est déterminé par séquençage d'ARN, PCR, RT-PCR, profilage d'expression génique, analyse en série d'expression génique, analyse par puce à ADN, séquençage génomique entier, ou est déterminé en fonction d'au moins une d'une réaction d'amplification, d'une réaction de séquençage, d'une réaction de fusion, d'une réaction d'hybridation ou d'une réaction d'hybridation inverse.

9. Procédé selon l'une quelconque des revendications 1 à 8, dans lequel le niveau d'expression d'au plus 250 gènes est déterminé.

10. Procédé selon l'une quelconque des revendications 1 à 6, dans lequel le niveau d'expression est un niveau d'expression protéique.

11. Procédé de criblage pour des composés cytotoxiques ou cytostatiques, le procédé comprenant :
- mettre en culture des cellules tumorales ;
- appliquer une thérapie aux cellules tumorales mises en culture, dans lequel la thérapie induit l'occurrence d'une ou plusieurs populations de cellules tumorales qui sont résistantes de manière réversible à la thérapie, et dans lequel les populations sont une ou plusieurs parmi :
- une population avec une expression accrue de 4 à 8 gènes sélectionnés parmi la signature génomique A1 constituée des gènes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B ; et/ou
- une population avec une expression accrue de 4 à 8 gènes sélectionnés parmi la signature génomique B1 constituée des gènes SLC7A8, DLX5, TRIM67, CD36, PAX3, IP6K3, UBXN10, KIAA1161, LSMEM1 ; et/ou
- une population avec une expression accrue de 4 à 8 gènes sélectionnés parmi la signature génomique C1 constituée des gènes SLC24A5, PMEL, FABP7, SLC45A2, KIT, EDNRB, TRPM1, APOE, MLANA, MLPH, TYRP1, GPR143, TYR, RAB27A, SNAI2, MITF, DCT ; et/ou
- une population avec une expression accrue de 4 à 8 gènes sélectionnés parmi la signature génomique D1 constituée de VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4 ;
dans lequel l'expression accrue d'un gène sélectionné est déterminée par rapport à un niveau d'expression de référence du gène sélectionné ;
- mettre en contact la ou les populations de cellules tumorales qui sont résistantes de manière réversible à la thérapie avec un composé qui est un composé candidat cytotoxique ou cytostatique à une ou plusieurs des populations de cellules tumorales qui sont résistantes de manière réversible à la thérapie ;
- identifier un composé cytotoxique ou cytostatique à une ou plusieurs des populations de cellules tumorales qui sont résistantes de manière réversible à la thérapie.

12. Procédé de criblage pour des composés cytotoxiques ou cytostatiques, le procédé comprenant :
- mettre en culture des cellules tumorales ;
- appliquer une thérapie aux cellules tumorales mises en culture, dans lequel la thérapie induit l'occurrence d'une ou plusieurs populations de cellules tumorales qui sont résistantes de manière réversible à la thérapie, et dans lequel les populations sont une ou plusieurs parmi :
- une population avec une expression accrue de 4 à 8 gènes sélectionnés parmi la signature génomique A1 constituée des gènes AQP1, ITGA1, L1CAM, NLGN3, S100A4, IL1RAP, COL4A1, THBS2, SLITRK6, CADM1, NRXN1, A2M, PRIMA1, GFRA2, MPZ, ADAMTS4, GFRA1, RSPO3, GFRA3, LAMC1, ANXA1, SYT11, MATN2, ATP1B2, ADGB, CNN3, COL1A1, TMEM176B, PLAT, PDGFB, SLC22A17, ITGA6, NGFR, VCAN, ATP1A2, IGF1, SEMA3B ; et/ou
- une population avec une expression accrue de 4 à 8 gènes sélectionnés parmi la signature génomique B1 constituée des gènes SLC7A8, DLX5, TRIM67, CD36, PAX3, IP6K3, UBXN10, KIAA1161, LSMEM1 ; et/ou
- une population avec une expression accrue de 4 à 8 gènes sélectionnés parmi la signature génomique C1 constituée des gènes SLC24A5, PMEL, FABP7, SLC45A2, KIT, EDNRB, TRPM1, APOE, MLANA, MLPH, TYRP1, GPR143, TYR, RAB27A, SNAI2, MITF, DCT ; et/ou
- une population avec une expression accrue de 4 à 8 gènes sélectionnés parmi la signature génomique D1 constituée de VCAN, TNC, BCAT1, FOSL2, UNC5B, CCL2, COL1A1, SH2B3, MGP, VEGFA, LOX, FGF1, PDGFRB, IGFBP5, ERRFI1, PRDX1, TGFBI, IL13RA2, SOX4, NES, LOXL2, SPRY2, CDH13, LMO4, RGS5, RGS16, DLX1, SLIT2, GPC3, ADM, EDNRA, CYSLTR2, DDAH1, PLXDC1, VSNL1, COL1A2, DLC1, AXL, ANGPTL4, IGFBP6, COL3A1, FABP4, CDH2, PTGER4, NDNF, NR2F1, BGN, TGM2, TMSB4X, CYR61, WNT5A, TCF4 ;
dans lequel l'expression accrue d'un gène sélectionné est déterminée par rapport à un niveau d'expression de référence du gène sélectionné ;
- mettre en contact la ou les populations de cellules tumorales qui sont résistantes de manière réversible à la thérapie avec un composé qui est un composé candidat pour modifier l'expression ou la fonction d'un gène sélectionné parmi l'une quelconque des signatures génomiques A1, B1, C1 ou D1 ;
- identifier comme composé cytotoxique ou cytostatique un composé qui modifie l'expression ou la fonction d'un gène sélectionné parmi l'une quelconque des signatures génomiques A1, B1, C1 ou D1.
